(19) Europäisches Patentamt
European Patent Office
Office européen des brevets

(11) EP 4 019 021 A1

(12) **EUROPEAN PATENT APPLICATION**
published in accordance with Art. 153(4) EPC

(43) Date of publication:
**29.06.2022 Bulletin 2022/26**

(21) Application number: **20857158.8**

(22) Date of filing: **21.08.2020**

(51) International Patent Classification (IPC):
***A61K 31/506*** *(2006.01)* ***C07D 401/12*** *(2006.01)*
***A61P 35/00*** *(2006.01)*

(52) Cooperative Patent Classification (CPC):
**A61K 31/506; A61P 35/00; C07D 401/12**

(86) International application number:
**PCT/CN2020/110442**

(87) International publication number:
**WO 2021/036922 (04.03.2021 Gazette 2021/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.08.2019 CN 201910785651**
**21.01.2020 CN 202010072446**
**20.08.2020 CN 202010840485**

(71) Applicant: **Beijing Tide Pharmaceutical Co., Ltd.**
**Beijing 100176 (CN)**

(72) Inventors:
- **ZHAO, Yanping**
  **Beijing 100176 (CN)**
- **WANG, Hongjun**
  **Beijing 100176 (CN)**
- **WANG, Yeming**
  **Beijing 100176 (CN)**
- **FAN, Futian**
  **Beijing 100176 (CN)**
- **JIANG, Yuanyuan**
  **Beijing 100176 (CN)**
- **WANG, Xiaoqian**
  **Beijing 100176 (CN)**
- **LIANG, Huining**
  **Beijing 100176 (CN)**
- **XIE, Yong**
  **Beijing 100176 (CN)**
- **ZHANG, Yanhao**
  **Beijing 100176 (CN)**
- **LIU, Kai**
  **Beijing 100176 (CN)**
- **FENG, Zewang**
  **Beijing 100176 (CN)**
- **LIU, Xuelian**
  **Beijing 100176 (CN)**

(74) Representative: **Novitas Patent AB**
**P.O. Box 55557**
**102 04 Stockholm (SE)**

(54) **COMPOUND INHIBITING AND INDUCING DEGRADATION OF EGFR AND ALK**

(57) A new compound of general formula (X) inhibiting and inducing degradation of an EGFR and ALK, and a pharmaceutical composition containing said compound. The compound and the pharmaceutical composition can be used for treating diseases related to the EGFR and the ALK kinase, such as cancer. The present invention further provides the preparation and use of the compound.

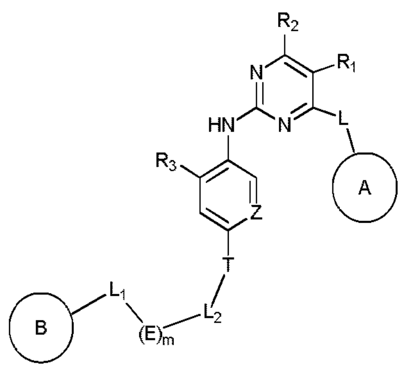

(X)

EP 4 019 021 A1

## Description

### FIELD OF THE INVENTION

The present disclosure relates to the field of medicine, specifically, the present disclosure provides compounds that can inhibit or induce the degradation of EGFR and ALK, and preparation and application thereof.

### BACKGROUND OF THE INVENTION

Lung cancer is one of the most common malignant tumors. In 2018, there were 2.1 million new lung cancer cases worldwide, accounting for 11.6% of all new tumor cases; there were 1.8 million deaths, accounting for 18.4% of all tumor deaths, wherein non-small cell lung cancer (NSCLC) accounts for 80%-85% of the total number of lung cancer. Epithelial growth factor receptor (EGFR) and anaplastic lymphoma kinase (ALK) are driving genes of common non-small cell lung cancer.

In non-small cell lung cancer, about 50% of Chinese patients and 11-16% of patients in western countries have EGFR gene mutations, wherein the most common mutation types are exon 19 deletion mutation (del E746-A750) and exon 21 L858R point mutation, accounting for about 90% of all EGFR mutation populations. EGFR small molecule inhibitors are the standard first-line treatment for non-small cell lung cancer with EGFR gene mutation and have been widely used in the field of lung cancer treatment. They competitively bind to EGFR with endogenous ligands, and inhibit the activation of tyrosine kinases, thereby blocking the EGFR signaling pathway, inhibiting tumor cell proliferation and metastasis, and promoting a series of biological effects such as tumor cell apoptosis.

The first-generation EGFR small-molecule inhibitors Gefitinib and Erlotinib have been used to treat advanced non-small cell lung cancer with activating EGFR mutations (L858R, del E746-A750). However, patients develop resistance after 10-12 months of administration of Gefitinib and Erlotinib, wherein more than 50 % of drug-resistant patients are due to the secondary mutation of T790M in EGFR. Afatinib, the second-generation irreversible EGFR inhibitor, is effective for advanced non-small cell lung cancer patients with activating EGFR mutations (L858R, del E746-A750), but cannot resolve the clinical drug resistance caused by EGFR T790M mutation. Moreover, Afatinib lacks selectivity to wildtype EGFR and has great toxicity. Osimertinib, the third-generation irreversible inhibitor, overcomes the drug resistance caused by EGFR T790M and can effectively treat advanced non-small cell lung cancer patients with drug resistance caused by EGFR T790M mutation. Although Osimertinib has achieved great success in the clinical treatment of non-small cell lung cancer with EGFR T790M mutant, some patients who benefits from the treatment develops drug resistance after 9-14 months of treatment (Nature Medicine, 2015, 21(6), 560-562). Studies have shown that up to 22% of patients with drug resistance of Osimertinib are due to EGFR C797S mutation (JAMA Oncol. 2018;4(11):1527-1534). EGFR C797S mutation causes cysteine at position 797 to be mutated into serine, and Osimertinib cannot covalently bind to EGFR, resulting in drug resistance. At present, there is no effective single EGFR inhibitor for EGFR C797S in clinical practice. Therefore, the development of a new generation of EGFR inhibitors to meet the needs of clinical treatment is an urgent problem to be solved.

Anaplastic lymphoma kinase (ALK) is a receptor tyrosine protein kinase. ALK gene rearrangement, point mutation and gene amplification can lead to carcinogenesis in the body. ALK rearrangement gene is a strong oncogenic driver gene, wherein echinoderm microtubule associated protein like 4 (EML4-ALK) and nucleophosmin (NPM-ALK) are common types. ALK gene rearrangement leads to the phosphorylation of ALK before dimer formation. Therefore, ALK fusion protein will continue to be activated and activate its downstream pathway, resulting in excessive cell proliferation and tumorigenesis. In non-small cell lung cancer, 3-7% of patients have ALK gene rearrangement. Small molecule inhibitors targeting ALK have been widely used in clinical practice. They competitively bind to ALK with endogenous ligands, and inhibit the activation of tyrosine kinases, thereby blocking the ALK signaling pathway, and inhibiting a series of biological effects such as tumor cell proliferation and metastasis. At present, the ALK inhibitors on the market include crizotinib, ceritinib, alectinib, brigatinib and loratinib, but it is inevitable that these inhibitors have drug resistance. Common ALK mutations leading to resistance are L1196M, G1269A, S1206Y, G1202R, C1156Y, L1198F and so on. Therefore, it is of great significance to develop new ALK inhibitors to meet the needs of clinical treatment.

Ubiquitin-proteasome system (UPS) is a multi-component system of intracellular protein degradation, which is involved in important physiological and biochemical processes such as cell growth and differentiation, DNA replication and repair, cell metabolism, immune response and so on. Protein degradation mediated by the ubiquitin-proteasome pathway is an important mechanism of the body for regulating intracellular protein level and function, and plays an important role in maintaining protein homeostasis in vivo. Inducing the degradation of EGFR or ALK through the intracellular ubiquitin-proteasome pathway provides a new idea for the treatment of cancer.

The present disclosure provides compounds that can inhibit and/or induce the degradation of EGFR and ALK, and preparation and application thereof.

## SUMMARY OF THE INVENTION

In one aspect, the present disclosure provides a compound of general formula (X), or a pharmaceutically acceptable salt, enantiomer, diastereomer, racemate, solvate, hydrate, polymorph, prodrug, or isotopic variant thereof, or mixture thereof, which can be used for treating diseases mediated by EGFR and/or ALK kinase, such as cancer.

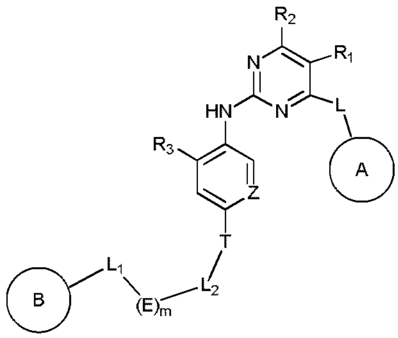

(X)

wherein

ring A is selected from the following optionally substituted groups: $C_{3-7}$ cycloalkyl, 4- to 8-membered heterocyclyl, $C_{6-10}$ aryl or 5- to 10-membered heteroaryl, wherein the substituent is selected from halogen, -CN, $C_{1-6}$ alkyl, $C_{1-6}$ haloalkyl, $C_{2-6}$ alkenyl, $C_{3-7}$ cycloalkyl, 4- to 8-membered heterocyclyl, -P(O)($C_{1-6}$ alkyl)$_2$, -P(O)($C_{2-6}$ alkenyl)$_2$, -O-$C_{1-6}$ alkyl, -O-$C_{1-6}$ haloalkyl, -O-$C_{2-6}$ alkenyl, -O-$C_{3-7}$ cycloalkyl, -O-4- to 8-membered heterocyclyl, -NH-$C_{1-6}$ alkyl, -NH-$C_{2-6}$ alkenyl, -NH-$C_{3-7}$ cycloalkyl, -NH-4- to 8-membered heterocyclyl, -C(O)-$C_{1-6}$ alkyl, -C(O)-$C_{2-6}$ alkenyl, -C(O)-$C_{3-7}$ cycloalkyl, -C(O)-4- to 8-membered heterocyclyl, -S(O)$_2$-$C_{1-6}$ alkyl, -S(O)$_2$-$C_{2-6}$ alkenyl, -S(O)$_2$-$C_{3-7}$ cycloalkyl, - S(O)$_2$-4- to 8-membered heterocyclyl, -NHC(O)-$C_{1-6}$ alkyl, -NHC(O)-$C_{2-6}$ alkenyl, -NHC(O)-$C_{3-7}$ cycloalkyl, -NHC(O)-4- to 8-membered heterocyclyl, -NHS(O)$_2$-$C_{1-6}$ alkyl, -NHS(O)$_2$-$C_{2-6}$ alkenyl, -NHS(O)$_2$-$C_{3-7}$ cycloalkyl or -NHS(O)$_2$-4- to 8-membered heterocyclyl;

ring B is selected from the following groups:

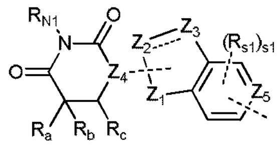

;

represents single bond or double bond;

represents that the point of attachment to the rest of the molecule can be located at the available point of the ring;

$Z_1$ is O, S, N or C atom, which is optionally substituted with one or two Rzi; or $Z_1$ is absent, and thus $Z_4$ is connected to $Z_2$, $Z_3$ or the C atom on the aromatic ring connected to $Z_1$, and the $Z_2$ and the C atom on the aromatic ring that are connected to $Z_1$ are connected to Rw respectively; or $Z_1$,$Z_2$ and $Z_3$ are all absent, and thus $Z_4$ is connected to one of the C atoms on the aromatic ring connected to $Z_1$ or $Z_3$, and the other C atom on the aromatic ring is connected to Rw;

$Z_2$ is O, S, N or C atom, which is optionally substituted with one or two $R_{Z2}$;

$Z_3$ is O, S, N or C atom, which is optionally substituted with one or two Rz3; with the proviso that when

represents double bond, $Z_2$ is N or C atom, and $Z_3$ is N or C atom;

$Z_4$ is N or CRz4;

$Z_5$ is N or $CR_{Z5}$;

$R_a$, $R_b$ and $R_c$ are independently H, halogen, -$(CH_2)_{0-5}$-OR", -$(CH_2)_{0-5}$-NR″R‴ $C_{1-6}$ alkyl or $C_{1-6}$ haloalkyl; or $R_a$ and $R_b$ are taken together with the carbon atom to which they are attached to form C=O, $C_{3-7}$ cycloalkyl or 4- to 8-membered heterocyclyl; or $R_a$ and $R_c$ are taken together with the cabin atoms to which they are attached to form

$C_{3-7}$ cycloalkyl or 4-to 8-membered heterocyclyl; or $R_a$ and $R_c$ are taken together to form bond;
$R_{N1}$ is H, $C_{1-6}$ alkyl or $C_{1-6}$ haloalkyl, alternatively H;
Rzi is absent, H, CN, halogen, $-(CH_2)_{0-5}$-OR", $-(CH_2)_{0-5}$-NR″R‴, $C_{1-6}$ alkyl, $C_{1-6}$ haloalkyl, $-(CH_2)_{0-5}-C_{3-7}$ cycloalkyl or $-(CH_2)_{0-5}$-4- to 8-membered heterocyclyl; or two $R_{Z1}$ are taken together with $Z_1$ to form C=O, $C_{3-7}$ cycloalkyl or 4- to 8-membered heterocyclyl;
Rz2 is absent, H, CN, halogen, $-(CH_2)_{0-5}$-OR", $-(CH_2)_{0-5}$-NR″R‴, $C_{1-6}$ alkyl, $C_{1-6}$ haloalkyl, $-(CH_2)_{0-5}-C_{3-7}$ cycloalkyl or $-(CH_2)_{0-5}$-4- to 8-membered heterocyclyl; or two Rz2 are taken together with $Z_2$ to form C=O, $C_{3-7}$ cycloalkyl or 4- to 8-membered heterocyclyl;
$R_{Z3}$ is absent, H, CN, halogen, $-(CH_2)_{0-5}$-OR", $-(CH_2)_{0-5}$-NR″R‴, $C_{1-6}$ alkyl, $C_{1-6}$ haloalkyl, $-(CH_2)_{0-5}-C_{3-7}$ cycloalkyl or $-(CH_2)_{0-5}$-4- to 8-membered heterocyclyl; or two $R_{Z3}$ are taken together with $Z_3$ to form C=O, $C_{3-7}$ cycloalkyl or 4- to 8-membered heterocyclyl;
$R_{Z4}$ is H, CN, halogen, $-(CH_2)_{0-5}$-OR", $-(CH_2)_{0-5}$-NR″R‴, $C_{1-6}$ alkyl or $C_{1-6}$ haloalkyl;
$R_{Z5}$ is H, CN, halogen, $-(CH_2)_{0-5}$-OR", $-(CH_2)_{0-5}$-NR″R‴, $C_{1-6}$ alkyl, $C_{1-6}$ haloalkyl, $-(CH_2)_{0-5}-C_{3-7}$ cycloalkyl or $-(CH_2)_{0-5}$-4- to 8-membered heterocyclyl;
or the ring where $Z_4$ is located is absent;
wherein $R_w$ is H, CN, halogen, $-(CH_2)_{0-5}$-OR", $-(CH_2)_{0-5}$-NR″R‴, $-C_{1-6}$ alkyl, $C_{1-6}$ haloalkyl, $-(CH_2)_{0-5}-C_{3-7}$ cycloalkyl, $-(CH_2)_{0-5}$-4- to 8-membered heterocyclyl, $C_{2-6}$ alkenyl, $C_{2-6}$ alkynyl, $-(CH_2)_{0-5}-C_{3-10}$ halocycloalkyl, $-(CH_2)_{0-5}-C_{6-10}$ aryl or $-(CH_2)_{0-5}$-5- to 14-membered heteroaryl;
R" is H, $C_{1-6}$ alkyl, $C_{1-6}$ haloalkyl or $-(CH_2)_{0-5}-C_{3-7}$ cycloalkyl;
R'" is H, $C_{1-6}$ alkyl or $C_{1-6}$ haloalkyl;
$L_1$ is selected from bond, -O-, $-S(O)_p$-, -S(O)(=NR*)-, $-NR^{\#}$-, $-CR^{\#}R^{\#'}$-, $-C_aR^{\#}R^{\#'}-C_bR^{\#}R^{\#'}$-, -N=S(O)(R*)- or -S(O)(R*)=N-;
$L_2$ is selected from bond, -O-, $-S(O)_p$-, -S(O)(=NR*)-, $-NR^{\#}$-, $-CR^{\#}R^{\#'}$-, $-C_aR^{\#}R^{\#'}-C_bR^{\#}R^{\#'}$-, -N=S(O)(R*)- or -S(O)(R*)=N-;
wherein one of $C_aR^{\#}R^{\#'}$ and $C_bR^{\#}R^{\#'}$ can be replaced by O, $S(O)_p$, S(O)(=NR*) or $NR^{\#}$, and when one of $C_aR^{\#}R^{\#'}$ and $C_bR^{\#}R^{\#''}$ is replaced by O, S or $NR^{\#}$, the other of $C_aR^{\#}R^{\#'}$ and $C_bR^{\#}R^{\#'}$ can also be replaced by $S(O)_q$;
E is independently selected from: bond, $-C_cR^{\#}R^{\#'}-C_dR^{\#}R^{\#'}-C_eR^{\#}R^{\#'}$,

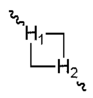 ,

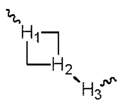 , 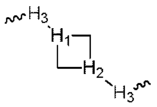 , 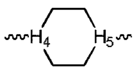 or 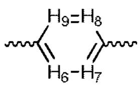 ;

wherein one of $C_cR^{\#}R^{\#'}$, $C_dR^{\#}R^{\#'}$ or $C_eR^{\#}R^{\#'}$, or both of $C_cR^{\#}R^{\#'}$ and $C_eR^{\#}R^{\#'}$ can be replaced by O, $S(O)_p$, S(O)(=NR*) or NR#, and when one of $C_cR^{\#}R^{\#'}$, $C_dR^{\#}R^{\#'}$ or $C_eR^{\#}R^{\#'}$ is replaced by O, S or $NR^{\#}$, the other adjacent one or two of $C_cR^{\#}R^{\#'}$, $C_dR^{\#}R^{\#'}$ or $C_eR^{\#}R^{\#'}$ can also be replaced by $S(O)_q$;
or two E moieties can be taken together to form $-CH_2CH_2OCH_2CH_2$-, - $OCH_2CH_2CH_2CH_2$-, $-CH_2CH_2CH_2CH_2O$-,

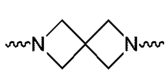 , 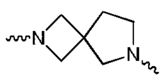 , 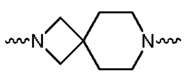 ,

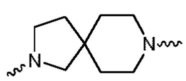 , 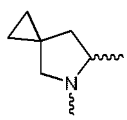 or 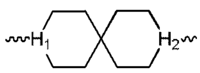 ;

wherein represents the point of attachment to $L_1$ or $L_2$;
$H_1$ and $H_2$ are N or C atom, $H_3$ is O, S, N or C atom, and $H_1$ and $H_3$, and $H_2$ and $H_3$ are not heteroatoms at the same time;
$H_4$ and $H_5$ are N or C atom;
$H_6$, $H_7$, $H_8$ and $H_9$ are C or N atom;

p is 0, 1 or 2;

q is 1 or 2;

$R^*$ is H, $C_{1-6}$ alkyl, $C_{1-6}$haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl or 5- to 14-membered heteroaryl;

$R^\#$ is H, halogen, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl or 5- to 14-membered heteroaryl;

$R^{\#'}$ is H, halogen, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl or 5- to 14-membered heteroaryl;

or, $R^\#$ and $R^\#$ on adjacent atoms can be taken together to form bond, and $R^{\#'}$ and $R^{\#'}$ on adjacent atoms can be taken together to form bond;

or, $R^\#$ and $R^{\#'}$ on the same or different atoms can be taken together to form =O, or $C_{3-7}$ cycloalkyl, 4- to 8-membered heterocyclyl, $C_{6-10}$ aryl or 5- to 6-membered heteroaryl, wherein the $C_{3-7}$ cycloalkyl, 4- to 8-membered heterocyclyl, $C_{6-10}$ aryl or 5- to 6-membered heteroaryl is optionally substituted with $R_x$, and the $R_x$ is H, CN, halogen, $C_{1-6}$ alkyl or $C_{1-6}$ haloalkyl;

m is 0, 1, 2, 3, 4, 5, 6, 7, 8, 9 or 10;

$R_{s1}$ is selected from H, CN, halogen, $-(CH_2)_{0-5}$-OR", $-(CH_2)_{0-5}$-NR″R‴, $C_{1-6}$ alkyl, $C_{1-6}$ haloalkyl, $-(CH_2)_{0-5}$-4- to 8-membered heterocyclyl, $C_{2-6}$ alkenyl, $C_{2-6}$ alkynyl, $-(CH_2)_{0-5}-C_{3-7}$ cycloalkyl, $-(CH_2)_{0-5}-C_{3-10}$ halocycloalkyl, $-(CH_2)_{0-5}-C_{6-10}$ aryl, $-(CH_2)_{0-5}$-5- to 14-membered heteroaryl, $-C(O)R_W$, $-S(O)R_W$ or $-S(O)_2R_W$;

s1 is 0, 1, 2 or 3;

R' is selected from H, $-C(O)-C_{1-6}$ alkyl, $-C(O)-C_{1-6}$ haloalkyl, $-C(O)-C_{2-6}$ alkenyl or - $C(O)-C_{6-10}$ aryl;

L is bond, -O- or -NR-;

wherein R is H or $C_{1-6}$ alkyl;

Z is -N= or $-C(R_4)=$;

T is selected from bond, $C_{2-6}$ heteroalkylene, 4- to 12-membered heterocyclylene, or 5- to 6-membered heterocyclylene, wherein the 5- to 6-membered heterocyclylene is substituted with 5- to 6-membered heterocyclylene;

$R_1$ is selected from H, halogen, cyano, $C_{1-6}$ alkyl, $C_{3-7}$ cycloalkyl, 4- to 8-membered heterocyclyl, $C_{1-6}$ haloalkyl, $C_{2-6}$ alkenyl or $C_{2-6}$ alkynyl;

or, when L is -NR-, $R_1$ and R are taken together with the atoms to which they are attached to form optionally substituted 5- to 6-membered heterocyclyl, wherein the substituent is selected from halogen, oxo, $C_{1-6}$ alkyl, $C_{1-6}$ haloalkyl, or $C_{6-10}$ aryl, wherein the $C_{6-10}$ aryl is mono- or poly-substituted with halogen;

$R_2$ is H, halogen, hydroxyl, amino, $C_{1-6}$ alkyl or $C_{1-6}$ haloalkyl;

or $R_1$ and $R_2$ are taken together with the atoms to which they are attached to form 5-to 6-membered heterocyclyl or 5- to 6-membered heteroaryl;

$R_3$ is selected from H, $-O-C_{1-6}$ alkyl or $-O-C_{1-6}$ haloalkyl;

$R_4$ is selected from H, halogen, $C_{1-6}$ alkyl, $C_{1-6}$ haloalkyl, $-NHC(O)-C_{1-6}$ alkyl or - $NHC(O)-C_{2-6}$ alkenyl;

if the above groups are H or H-containing groups, the one or more H atom(s) may be substituted with D atom(s);

the groups containing OH, NH, $NH_2$, CH, $CH_2$, or $CH_3$ in $L_1$, E, $L_2$, and T, or the above alkyl, alkylene, haloalkyl, alkenyl, alkynyl, cycloalkyl, halocycloalkyl, heterocyclyl, aryl, and heteroaryl are each optionally substituted with 1, 2, 3 or more $R^s$ at each occurrence, and the $R^s$ is independently selected from the following groups at each occurrence: halogen, hydroxyl, amino, cyano, nitro, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl, 5- to 14-membered heteroaryl, $C_{6-12}$ aralkyl, $-OR^{a'}$, $-OC(O)R^{a'}$, $-C(O)R^{a'}$, $-C(O)OR^{a'}$, $-C(O)NR^{a'}R^{b'}$, - $S(O)_nR^{a'}$, $-S(O)_nOR^{a'}$, $-S(O)_nNR^{a'}R^{b'}$, $-NR^{a'}R^{b'}$, $-NR^{a'}C(O)R^{b'}$, $-NR^{a'}-C(O)OR^{b'}$, $-NR^{a'}-S(O)_n-R^{b'}$, $-NR^{a'}C(O)NR^{a'}R^{b'}$, $-C_{1-6}$ alkylene-$R^{a'}$, $-C_{1-6}$ alkylene-$OR^{a'}$, $-C_{1-6}$ alkylene-$OC(O)R^{a'}$, $-C_{1-6}$ alkylene-$C(O)OR^{a'}$, $-C_{1-6}$ alkylene-$S(O)_nR^{a'}$, $-C_{1-6}$ alkylene-$S(O)_nOR^{a'}$, $-C_{1-6}$ alkylene-$OC(O)NR^{a'}R^{b'}$, $-C_{1-6}$ alkylene-$C(O)NR^{a'}R^{b'}$, $-C_{1-6}$ alkylene-$NR^{a'}-C(O)NR^{a'}R^{b'}$, $-C_{1-6}$ alkylene-$OS(O)_nR^{a'}$, $-C_{1-6}$ alkylene-$S(O)_nNR^{a'}R^{b'}$, $-C_{1-6}$ alkylene-$NR^{a'}-S(O)_nNR^{a'}R^{b'}$, $-C_{1-6}$ alkylene-$NR^{a'}R^{b'}$ and $-O-C_{1-6}$ alkylene-$NR^{a'}R^{b'}$, and wherein the hydroxyl, amino, alkyl, alkylene, cycloalkyl, heterocyclyl, aryl, heteroaryl and aralkyl described with respect to the substituent $R^s$ are further optionally substituted with 1, 2, 3 or more substituents independently selected from: halogen, OH, amino, cyano, nitro, $C_{1-6}$ alkyl, $C_{1-6}$ haloalkyl, $C_{1-6}$ alkyl hydroxyl, $C_{3-6}$ cycloalkyl, 3- to 10-membered heterocyclyl, $C_{6-10}$ aryl, 5- to 14-membered heteroaryl and $C_{6-12}$ aralkyl;

n is independently 1 or 2 at each occurrence;

$R^{a'}$ and $R^{b'}$ are each independently selected from H, $C_{1-6}$ alkyl, $C_{2-6}$ alkenyl, $C_{2-6}$ alkynyl, $C_{1-6}$ alkyl-O-, $C_{1-6}$ alkyl-S-, $C_{3-10}$ cycloalkyl, 3- to 10-membered heterocyclyl, $C_{6-10}$ aryl, 5- to 14-membered heteroaryl and $C_{6-12}$ aralkyl at each occurrence.

In another aspect, the present disclosure provides a pharmaceutical composition comprising a compound disclosed herein, and optionally pharmaceutically acceptable excipients.

In another aspect, the present disclosure provides a pharmaceutical composition comprising a compound

disclosed herein and pharmaceutically acceptable excipients, which further comprises other therapeutic agent(s).

In another aspect, the present disclosure provides a kit comprising a compound disclosed herein, other therapeutic agent(s), and pharmaceutically acceptable carriers, adjuvants or vehicles.

In another aspect, the present disclosure provides a use of a compound disclosed herein in the manufacture of a medicament for treating and/or preventing a disease mediated by EGFR and/or ALK kinase.

In another aspect, the present disclosure provides a method of treating and/or preventing a disease mediated by EGFR and/or ALK kinase in a subject, comprising administering to the subject a compound disclosed herein or a composition disclosed herein.

In another aspect, the present disclosure provides a compound disclosed herein or a composition disclosed herein, for use in treating and/or preventing a disease mediated by EGFR and/or ALK kinase.

In a specific embodiment, the diseases treated by the present disclosure include cancer, such as ovarian cancer, cervical cancer, colorectal cancer, breast cancer, pancreatic cancer, glioma, glioblastoma, melanoma, prostate cancer, leukemia, lymphoma, non-Hodgkin's lymphoma, gastric cancer, lung cancer, hepatocellular cancer, stomach cancer, gastrointestinal stromal tumor (GIST), thyroid cancer, cancer of bile duct, endometrial cancer, kidney cancer, anaplastic large cell lymphoma, acute myeloid leukemia (AML), multiple myeloma, melanoma, and mesothelioma.

Other objects and advantages of the present disclosure will be apparent to those skilled in the art from the following specific embodiments, examples and claims disclosed herein.

Definition

Chemical definitions

Definitions of specific functional groups and chemical terms are described in more detail below.

When a range of values is listed, it is intended to encompass each value and subrange within the range. For example, "$C_{1-6}$ alkyl" is intended to include $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$ and $C_{5-6}$ alkyl.

"$C_{1-6}$ alkyl" refers to a radical of a straight or branched, saturated hydrocarbon group having 1 to 6 carbon atoms. In some embodiments, $C_{1-4}$ alkyl is alternative. Examples of $C_{1-6}$ alkyl include methyl ($C_1$), ethyl ($C_2$), n-propyl ($C_3$), iso-propyl ($C_3$), n-butyl ($C_4$), tert-butyl (C4), sec-butyl ($C_4$), iso-butyl ($C_4$), n-pentyl ($C_5$), 3-pentyl ($C_5$), pentyl ($C_5$), neopentyl ($C_5$), 3-methyl-2-butyl ($C_5$), tert-pentyl ($C_5$) and n-hexyl ($C_6$). The term "$C_{1-6}$ alkyl" also includes heteroalkyl, wherein one or more (e.g., 1, 2, 3 or 4) carbon atoms are substituted with heteroatoms (e.g., oxygen, sulfur, nitrogen, boron, silicon, phosphorus). Alkyl groups can be optionally substituted with one or more substituents, for example, with 1 to 5 substituents, 1 to 3 substituents or 1 substituent. Conventional abbreviations of alkyl include Me ($-CH_3$), Et ($-CH_2CH_3$), iPr ($-CH(CH_3)_2$), nPr ($-CH_2CH_2CH_3$), n-Bu ($-CH_2CH_2CH_2CH_3$) or i-Bu ($-CH_2CH(CH_3)_2$).

"$C_{2-6}$ alkenyl" refers to a radical of a straight or branched hydrocarbon group having 2 to 6 carbon atoms and at least one carbon-carbon double bond. In some embodiments, $C_{2-4}$ alkenyl is alternative. Examples of $C_{2-6}$ alkenyl include vinyl ($C_2$), 1-propenyl ($C_3$), 2-propenyl ($C_3$), 1-butenyl ($C_4$), 2-butenyl ($C_4$), butadienyl ($C_4$), pentenyl ($C_5$), pentadienyl ($C_5$), hexenyl ($C_6$), etc. The term "$C_{2-6}$ alkenyl" also includes heteroalkenyl, wherein one or more (e.g., 1, 2, 3 or 4) carbon atoms are replaced by heteroatoms (e.g., oxygen, sulfur, nitrogen, boron, silicon, phosphorus). The alkenyl groups can be optionally substituted with one or more substituents, for example, with 1 to 5 substituents, 1 to 3 substituents or 1 substituent.

"$C_{2-6}$ alkynyl" refers to a radical of a straight or branched hydrocarbon group having 2 to 6 carbon atoms, at least one carbon-carbon triple bond and optionally one or more carbon-carbon double bonds. In some embodiments, $C_{2-4}$ alkynyl is alternative. Examples of $C_{2-6}$ alkynyl include, but are not limited to, ethynyl ($C_2$), 1-propynyl ($C_3$), 2-propynyl ($C_3$), 1-butynyl ($C_4$), 2-butynyl ($C_4$), pentynyl ($C_5$), hexynyl ($C_6$), etc. The term "$C_{2-6}$ alkynyl" also includes heteroalkynyl, wherein one or more (e.g., 1, 2, 3 or 4) carbon atoms are replaced by heteroatoms (e.g., oxygen, sulfur, nitrogen, boron, silicon, phosphorus). The alkynyl groups can be substituted with one or more substituents, for example, with 1 to 5 substituents, 1 to 3 substituents or 1 substituent.

"$C_{1-6}$ alkylene, $C_{2-6}$ alkenylene or $C_{2-6}$ alkynylene" refers to a divalent group of the "$C_{1-6}$ alkyl, $C_{2-6}$ alkenyl or $C_{2-6}$ alkynyl" as defined above.

"$C_{1-6}$ alkylene" refers to a divalent group formed by removing another hydrogen of the $C_{1-6}$ alkyl, and can be substituted or unsubstituted. In some embodiments, $C_{1-4}$ alkylene is yet alternative. The unsubstituted alkylene groups include, but are not limited to, methylene ($-CH_2-$), ethylene ($-CH_2CH_2-$), propylene ($-CH_2CH_2CH_2-$), butylene ($-CH_2CH_2CH_2CH_2-$), pentylene ($-CH_2CH_2CH_2CH_2CH_2-$), hexylene ($-CH_2CH_2CH_2CH_2CH_2CH_2-$), etc. Examples of substituted alkylene groups, such as those substituted with one or more alkyl (methyl) groups, include, but are not limited to, substituted methylene ($-CH(CH_3)-$, $-C(CH_3)_2-$), substituted ethylene ($-CH(CH_3)CH_2-$, $-CH_2CH(CH_3)-$, $-C(CH_3)_2CH_2-$, $-CH_2C(CH_3)_2-$), substituted propylene ($-CH(CH_3)CH_2CH_2-$, $-CH_2CH(CH_3)CH_2-$, $-CH_2CH_2CH(CH_3)-$, $-C(CH_3)_2CH_2CH_2-$, $-CH_2C(CH_3)_2CH_2-$, $-CH_2CH_2C(CH_3)_2-$), etc.

"$C_{2-6}$ alkenylene" refers to a $C_{2-6}$ alkenyl group wherein another hydrogen is removed to provide a divalent radical of alkenylene, and which may be substituted or unsubstituted. In some embodiments, $C_{2-4}$ alkenylene is yet alternative. Exemplary unsubstituted alkenylene groups include, but are not limited to, ethenylene (-CH=CH-) and propenylene (e.g., $-CH=CHCH_2-$, $-CH_2-CH=CH-$). Exemplary substituted alkenylene groups, e.g., substituted with one or more alkyl (methyl) groups, include but are not limited to, substituted ethylene ($-C(CH_3)=CH-$, $-CH=C(CH_3)-$), substituted propylene (e.g., - $C(CH_3)=CHCH_2-$, $-CH=C(CH_3)CH_2-$, $-CH=CHCH(CH_3)-$, $-CH=CHC(CH_3)_2-$, $-CH(CH_3)-CH=CH-$, $-C(CH_3)_2-CH=CH-$, $-CH_2-C(CH_3)=CH-$, $-CH_2-CH=C(CH_3)-$), and the like.

"$C_{2-6}$ alkynylene" refers to a $C_{2-6}$ alkynyl group wherein another hydrogen is removed to provide a divalent radical of alkynylene, and which may be substituted or unsubstituted. In some embodiments, $C_{2-4}$ alkynylene is yet alternative. Exemplary alkynylene groups include, but are not limited to, ethynylene (-C=C-), substituted or unsubstituted propynylene ($-C=CCH_2-$), and the like.

"$C_{1-6}$ heteroalkyl" refers to $C_{1-6}$ alkyl, as defined herein, which further contains one or more (e.g., 1, 2, 3 or 4) heteroatoms (e.g., oxygen, sulfur, nitrogen, boron, silicon, phosphorus) in the parent carbon chain, wherein one or more heteroatoms are between adjacent carbon atoms in the parent carbon chain, and/or one or more heteroatoms are between the carbon atom and the parent molecule, that is, between the connection points. The point of attachment between $C_{1-6}$ heteroalkyl and the parent molecule can be a carbon atom or a heteroatom.

"$C_{2-6}$ heteroalkylene" refers to a divalent group formed by removing another hydrogen of $C_{1-6}$ heteroalkyl, and can be substituted or unsubstituted. The point of attachment of $C_{1-6}$ heteroalkylene to other parts of the parent molecule can be two carbon atoms, or two heteroatoms, or one carbon atom and one heteroatom.

"Halo" or "halogen" refers to fluorine (F), chlorine (Cl), bromine (Br) and iodine (I).

Thus, "$C_{1-6}$ haloalkyl" refers to the above "$C_{1-6}$ alkyl", which is substituted by one or more halogen. In some embodiments, $C_{1-4}$ haloalkyl is yet alternative, and still alternatively $C_{1-2}$ haloalkyl. Exemplary haloalkyl groups include, but are not limited to, $-CF_3$, $-CH_2F$, - $CHF_2$, $-CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, and the like. The haloalkyl can be substituted at any available point of attachment, for example, with 1 to 5 substituents, 1 to 3 substituents or 1 substituent.

"$C_{3-10}$ cycloalkyl" refers to a radical of a non-aromatic cyclic hydrocarbon group having from 3 to 10 ring carbon atoms and zero heteroatoms. In some embodiments, $C_{3-7}$ cycloalkyl and $C_{3-6}$ cycloalkyl are yet alternative, and still alternatively $C_{5-6}$ cycloalkyl. The cycloalkyl also includes a ring system in which the cycloalkyl described herein is fused with one or more aryl or heteroaryl groups, wherein the point of attachment is on the cycloalkyl ring, and in such case, the number of carbon atoms continues to represent the number of carbon atoms in the cycloalkyl system. Exemplary cycloalkyl groups include, but are not limited to, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), etc. The cycloalkyl can be substituted with one or more substituents, for example, with 1 to 5 substituents, 1 to 3 substituents or 1 substituent.

"$C_{3-10}$ halocycloalkyl" refers to the above "$C_{3-10}$ cycloalkyl", which is substituted by one or more halogen.

"3- to 12-membered heterocyclyl" refers to a radical of 3- to 12-membered non-aromatic ring system having ring carbon atoms and 1 to 5 ring heteroatoms, wherein each of the heteroatoms is independently selected from nitrogen, oxygen, sulfur, boron, phosphorus and silicon. In the heterocyclyl containing one or more nitrogen atoms, the point of attachment can be a carbon or nitrogen atom as long as the valence permits. In some embodiments, 4- to 12-membered heterocyclyl is alternative, which is a radical of 4- to 12-membered non-aromatic ring system having ring carbon atoms and 1 to 5 ring heteroatoms. In some embodiments, 3- to 10-membered heterocyclyl is alternative, which is a radical of 3-to 10-membered non-aromatic ring system having ring carbon atoms and 1 to 5 ring heteroatoms. In some embodiments, 3- to 8-membered heterocyclyl is alternative, which is a radical of 3- to 8-membered non-aromatic ring system having ring carbon atoms and 1 to 4 ring heteroatoms. 3- to 6-membered heterocyclyl is alternative, which is a radical of 3- to 6-membered non-aromatic ring system having ring carbon atoms and 1 to 3 ring heteroatoms. 4- to 8-membered heterocyclyl is alternative, which is a radical of 4- to 8-membered non-aromatic ring system having ring carbon atoms and 1 to 3 ring heteroatoms. 5- to 6-membered heterocyclyl is more alternative, which is a radical of 5- to 6-membered non-aromatic ring system having ring carbon atoms and 1 to 3 ring heteroatoms. The heterocyclyl also includes a ring system wherein the heterocyclyl described above is fused with one or more cycloalkyl groups, wherein the point of attachment is on the cycloalkyl ring, or the heterocyclyl described above is fused with one or more aryl or heteroaryl groups, wherein the point of attachment is on the heterocyclyl ring; and in such cases, the number of ring members continues to represent the number of ring members in the heterocyclyl ring system. Exemplary 3-membered heterocyclyl groups containing one heteroatom include, but are not limited to, aziridinyl, oxiranyl and thiorenyl. Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, dioxolanyl, oxasulfuranyl, disulfuranyl, and oxazolidin-2-one. Exemplary 5-membered heterocyclyl groups containing three heteroatoms include, but are not

limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocyclyl groups containing one heteroatom include, but are not limited to, azepanyl, oxepanyl and thiepanyl. Exemplary 5-membered heterocyclyl groups fused with a $C_6$ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a $C_6$ aryl (also referred as 6,6-bicyclic heterocyclyl herein) include, but are not limited to, tetrahydroquinolinyl, tetrahydroisoquinolinyl, etc. The heterocyclyl can be substituted with one or more substituents, for example, with 1 to 5 substituents, 1 to 3 substituents or 1 substituent.

"4- to 12-membered heterocyclylene" and "5- to 6-membered heterocyclylene" refer to the above "4- to 12-membered heterocyclyl" and "5- to 6-membered heterocyclyl", respectively, wherein another hydrogen is removed and formed divalent groups, and can be substituted or unsubstituted.

"$C_{6-10}$ aryl" refers to a radical of monocyclic or polycyclic (e.g., bicyclic) 4n+2 aromatic ring system having 6-10 ring carbon atoms and zero heteroatoms (e.g., having 6 or 10 shared $\pi$ electrons in a cyclic array). In some embodiments, the aryl group has six ring carbon atoms ("$C_6$ aryl"; for example, phenyl). In some embodiments, the aryl group has ten ring carbon atoms ("$C_{10}$ aryl"; for example, naphthyl, e.g., 1-naphthyl and 2-naphthyl). The aryl group also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system. The aryl can be substituted with one or more substituents, for example, with 1 to 5 substituents, 1 to 3 substituents or 1 substituent.

"$C_{6-12}$ aralkyl " refers to the group -R-R', wherein R is alkyl, R' is aryl, and alkyl and aryl have a total of 6-12 carbon atoms.

"5- to 14-membered heteroaryl" refers to a radical of 5- to 14-membered monocyclic or bicyclic 4n+2 aromatic ring system (e.g., having 6, 10 or 14 shared $\pi$ electrons in a cyclic array) having ring carbon atoms and 1-4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. Heteroaryl bicyclic systems may include one or more heteroatoms in one or two rings. Heteroaryl also includes ring systems wherein the heteroaryl ring described above is fused with one or more cycloalkyl or heterocyclyl groups, and the point of attachment is on the heteroaryl ring. In such case, the number the carbon atoms continues to represent the number of carbon atoms in the heteroaryl ring system. In some embodiments, 5-to 10-membered heteroaryl groups are alternative, which are radicals of 5- to 10-membered monocyclic or bicyclic 4n+2 aromatic ring systems having ring carbon atoms and 1-4 ring heteroatoms. In other embodiments, 5- to 6-membered heteroaryl groups are yet alternative, which are radicals of 5- to 6-membered monocyclic or bicyclic 4n+2 aromatic ring systems having ring carbon atoms and 1-4 ring heteroatoms. Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (such as, 1,2,4- oxadiazolyl), and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl. The heteroaryl can be substituted with one or more substituents, for example, with 1 to 5 substituents, 1 to 3 substituents or 1 substituent.

"Oxo" represents =O.

Alkyl, alkenyl, alkynyl, cycloalkyl, heterocyclyl, aryl and heteroaryl as defined herein are optionally substituted groups.

Exemplary substituents on carbon atoms include, but are not limited to, halogen, - CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3{}^+X^-$, $-N(OR^{cc})R^{bb}$, - SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, - $C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, - $C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})_2$, - $OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, $-SO_2R^{aa}$, $-SO_2OR^{aa}$,

-OSO$_2$R$^{aa}$, -S(=O)R$^{aa}$, -OS(=O)R$^{aa}$, -Si(R$^{aa}$)$_3$, -OSi(R$^{aa}$)$_3$, -C(=S)N(R$^{bb}$)$_2$, -C(=O)SR$^{aa}$, -C(=S)SR$^{aa}$, -SC(=S)SR$^{aa}$, -SC(=O)SR$^{aa}$, -OC(=O)SR$^{aa}$, -SC(=O)OR$^{aa}$, - SC(=O)R$^{aa}$, -P(=O)$_2$R$^{aa}$, -OP(=O)$_2$R$^{aa}$, -P(=O)(R$^{aa}$)$_2$, -OP(=O)(R$^{aa}$)$_2$, -OP(=O)(OR$^{cc}$)$_2$, - P(=O)$_2$N(R$^{bb}$)$_2$, -OP(=O)$_2$N(R$^{bb}$)$_2$, -P(=O)(NR$^{bb}$)$_2$, -OP(=O)(NR$^{bb}$)$_2$, -NR$^{bb}$P(=O)(OR$^{cc}$)$_2$, -NR$^{bb}$P(=O)(NR$^{bb}$)$_2$, -P(R$^{cc}$)$_2$, -P(R$^{cc}$)$_3$, -OP(R$^{cc}$)$_2$, -OP(R$^{cc}$)$_3$, -B(R$^{aa}$)$_2$, -B(OR$^{cc}$)$_2$, -BR$^{aa}$(OR$^{cc}$), alkyl, haloalkyl, alkenyl, alkynyl, cycloalkyl, heterocyclyl, aryl and heteroaryl, wherein each of the alkyl, alkenyl, alkynyl, cycloalkyl, heterocyclyl, aryl and heteroaryl is independently substituted with 0, 1, 2, 3, 4 or 5 R$^{dd}$ groups;

or two geminal hydrogen on a carbon atom are replaced with =O, =S, =NN(R$^{bb}$)$_2$, =NNR$^{bb}$C(=O)R$^{aa}$, =NNR$^{bb}$C(=O)OR$^{aa}$, =NNR$^{bb}$S(=O)$_2$R$^{aa}$, =NR$^{bb}$ or =NOR$^{cc}$ groups;
each of the R$^{aa}$ is independently selected from alkyl, haloalkyl, alkenyl, alkynyl, cycloalkyl, heterocyclyl, aryl and heteroaryl, or two of the R$^{aa}$ groups are combined to form a heterocyclyl or heteroaryl ring, wherein each of the alkyl, alkenyl, alkynyl, cycloalkyl, heterocyclyl, aryl and heteroaryl is independently substituted with 0, 1, 2, 3, 4 or 5 R$^{dd}$ groups;
each of the R$^{bb}$ is independently selected from hydrogen, -OH, -OR$^{aa}$, -N(R$^{cc}$)$_2$, -CN, -C(=O)R$^{aa}$, -C(=O)N(R$^{cc}$)$_2$, -CO$_2$R$^{aa}$, -SO$_2$R$^{aa}$, -C(=NR$^{cc}$)OR$^{aa}$, -C(=NR$^{cc}$)N(R$^{cc}$)$_2$, - SO$_2$N(R$^{cc}$)$_2$, -SO$_2$R$^{cc}$, -SO$_2$OR$^{cc}$, -SOR$^{aa}$, -C(=S)N(R$^{cc}$)$_2$, -C(=O)SR$^{cc}$, -C(=S)SR$^{cc}$, - P(=O)$_2$R$^{aa}$, -P(=O)(R$^{aa}$)$_2$, -P(=O)$_2$N(R$^{cc}$)$_2$, -P(=O)(NR$^{cc}$)$_2$, alkyl, haloalkyl, alkenyl, alkynyl, cycloalkyl, heterocyclyl, aryl and heteroaryl, or two R$^{bb}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R$^{dd}$ groups;
each of the R$^{cc}$ is independently selected from hydrogen, alkyl, haloalkyl, alkenyl, alkynyl, cycloalkyl, heterocyclyl, aryl and heteroaryl, or two R$^{cc}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R$^{dd}$ groups;
each of the R$^{dd}$ is independently selected from halogen, -CN, -NO$_2$, -N$_3$, -SO$_2$H, - SO$_3$H, -OH, -OR$^{ee}$, -ON(R$^{ff}$)$_2$, -N(R$^{ff}$)$_2$, -N(R$^{ff}$)$_3$$^+$X$^-$, -N(OR$^{ee}$)R$^{ff}$, -SH, -SR$^{ee}$, -SSR$^{ee}$, - C(=O)R$^{ee}$, -CO$_2$H, -CO$_2$R$^{ee}$, -OC(=O)R$^{ee}$, -OCO$_2$R$^{ee}$, -C(=O)N(R$^{ff}$)$_2$, -OC(=O)N(R$^{ff}$)$_2$, - NR$^{ff}$C(=O)R$^{ee}$, -NR$^{ff}$CO$_2$R$^{ee}$, -NR$^{ff}$C(=O)N(R$^{ff}$)$_2$, -C(=NR$^{ff}$)OR$^{ee}$, -OC(=NR$^{ff}$)R$^{ee}$, - OC(=NR$^{ff}$)OR$^{ee}$, -C(=NR$^{ff}$)N(R$^{ff}$)$_2$, -OC(=NR$^{ff}$)N(R$^{ff}$)$_2$, -NR$^{ff}$C(=NR$^{ff}$)N(R$^{ff}$)$_2$, -NR$^{ff}$SO$_2$R$^{ee}$, - SO$_2$N(R$^{ff}$)$_2$, -SO$_2$R$^{ee}$, -SO$_2$OR$^{ee}$, -OSO$_2$R$^{ee}$, -S(=O)R$^{ee}$, -Si(R$^{ee}$)$_3$, -OSi(R$^{ee}$)$_3$, -C(=S)N(R$^{ff}$)$_2$, - C(=O)SR$^{ee}$, -C(=S)SR$^{ee}$, -SC(=S)SR$^{ee}$, -P(=O)$_2$R$^{ee}$, -P(=O)(R$^{ee}$)$_2$, -OP(=O)(R$^{ee}$)$_2$, - OP(=O)(OR$^{ee}$)$_2$, alkyl, haloalkyl, alkenyl, alkynyl, cycloalkyl, heterocyclyl, aryl, heteroaryl, wherein each of the alkyl, alkenyl, alkynyl, cycloalkyl, heterocyclyl, aryl and heteroaryl is independently substituted with 0, 1, 2, 3, 4 or 5 R$^{gg}$ groups, or two geminal R$^{dd}$ substituents can be combined to form=O or =S;
each of the R$^{ee}$ is independently selected from alkyl, haloalkyl, alkenyl, alkynyl, cycloalkyl, aryl, heterocyclyl, and heteroaryl, wherein each of the alkyl, alkenyl, alkynyl, cycloalkyl, heterocyclyl, aryl and heteroaryl is independently substituted with 0, 1, 2, 3, 4 or 5 R$^{gg}$ groups;
each of the R$^{ff}$ is independently selected from hydrogen, alkyl, haloalkyl, alkenyl, alkynyl, cycloalkyl, heterocyclyl, aryl and heteroaryl, or two R$^{ff}$ groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R$^{gg}$ groups;
each of the R$^{gg}$ is independently selected from halogen, -CN, -NO$_2$, -N$_3$, -SO$_2$H, - SO$_3$H, -OH, -OC$_{1-6}$ alkyl, -ON(C$_{1-6}$ alkyl)$_2$, -N(C$_{1-6}$ alkyl)$_2$, -N(C$_{1-6}$ alkyl)$_3$$^+$X$^-$, -NH(C$_{1-6}$ alkyl)$_2$$^+$X$^-$, -NH$_2$(C$_{1-6}$ alkyl)$^+$X$^-$, -NH$_3$$^+$X$^-$, -N(OC$_{1-6}$ alkyl)(C$_{1-6}$ alkyl), -N(OH)(C$_{1-6}$ alkyl), - NH(OH), -SH, -SC$_{1-6}$ alkyl, -SS(C$_{1-6}$ alkyl), -C(=O)(C$_{1-6}$ alkyl), -CO$_2$H, -CO$_2$(C$_{1-6}$ alkyl), - OC(=O)(C$_{1-6}$ alkyl), -OCO$_2$(C$_{1-6}$ alkyl), -C(=O)NH$_2$, -C(=O)N(C$_{1-6}$ alkyl)$_2$, -OC(=O)NH(C$_{1-6}$ alkyl), -NHC(=O)(C$_{1-6}$ alkyl), -N(C$_{1-6}$ alkyl)C(=O)(C$_{1-6}$ alkyl), -NHCO$_2$(C$_{1-6}$ alkyl), - NHC(=O)N(C$_{1-6}$ alkyl)$_2$, -NHC(=O)NH(C$_{1-6}$ alkyl), -NHC(=O)NH$_2$, -C(=NH)O(C$_{1-6}$ alkyl), - OC(=NH)(C$_{1-6}$ alkyl), -OC(=NH)OC$_{1-6}$ alkyl, -C(=NH)N(C$_{1-6}$ alkyl)$_2$, -C(=NH)NH(C$_{1-6}$ alkyl), -C(=NH)NH$_2$, -OC(=NH)N(C$_{1-6}$ alkyl)$_2$, -OC(NH)NH(C$_{1-6}$ alkyl), -OC(NH)NH$_2$, -NHC(NH)N(C$_{1-6}$ alkyl)$_2$, -NHC(=NH)NH$_2$, -NHSO$_2$(C$_{1-6}$ alkyl), -SO$_2$N(C$_{1-6}$ alkyl)$_2$, - SO$_2$NH(C$_{1-6}$ alkyl), -SO$_2$NH$_2$, -SO$_2$C$_{1-6}$ alkyl, -SO$_2$OC$_{1-6}$ alkyl, -OSO$_2$C$_{1-6}$ alkyl, -SOC$_{1-6}$ alkyl, -Si(C$_{1-6}$ alkyl)$_3$, -OSi(C$_{1-6}$ alkyl)$_3$, -C(=S)N(C$_{1-6}$ alkyl)$_2$, C(=S)NH(C$_{1-6}$ alkyl), C(=S)NH$_2$, - C(=O)S(C$_{1-6}$ alkyl), -C(=S)SC$_{1-6}$ alkyl, -SC(=S)SC$_{1-6}$ alkyl, -P(=O)$_2$(C$_{1-6}$ alkyl), -P(=O)(C$_{1-6}$ alkyl)$_2$, -OP(=O)(C$_{1-6}$ alkyl)$_2$, -OP(=O)(OC$_{1-6}$ alkyl)$_2$, C$_{1-6}$ alkyl, C$_{1-6}$ haloalkyl, C$_2$-C$_6$ alkenyl, C$_2$-C$_6$ alkynyl, C$_3$-C$_7$ cycloalkyl, C$_6$-C$_{10}$ aryl, C$_3$-C$_7$ heterocyclyl, C$_5$-C$_{10}$ heteroaryl; or two geminal R$^{gg}$ substituents may combine to form =O or =S; wherein X$^-$ is a counter-ion.

Exemplary substituents on nitrogen atoms include, but are not limited to, hydrogen, - OH, -OR$^{aa}$, -N(R$^{cc}$)$_2$, -CN, -C(=O)R$^{aa}$, -C(=O)N(R$^{cc}$)$_2$, -CO$_2$R$^{aa}$, -SO$_2$R$^{aa}$, -C(=NR$^{bb}$)R$^{aa}$, - C(=NR$^{cc}$)OR$^{aa}$, -C(=NR$^{cc}$)N(R$^{cc}$)$_2$, -SO$_2$N(R$^{cc}$)$_2$, -SO$_2$R$^{cc}$, -SO$_2$OR$^{cc}$, -SOR$^{aa}$, -C(=S)N(R$^{cc}$)$_2$, - C(=O)SR$^{cc}$, -C(=S)SR$^{cc}$, -P(=O)$_2$R$^{aa}$, -P(=O)(R$^{aa}$)$_2$, -P(=O)$_2$N(R$^{cc}$)$_2$, -P(=O)(NR$^{cc}$)$_2$, alkyl, haloalkyl, alkenyl, alkynyl, cycloalkyl, heterocyclyl, aryl and heteroaryl, or two R$^{cc}$ groups attached to a nitrogen atom combine to form a heterocyclyl or a heteroaryl ring, wherein each of the alkyl, alkenyl, alkynyl,

cycloalkyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as described herein.

Other Definitions

The term "cancer" includes, but is not limited to, the following cancers: breast, ovary, cervix, prostate, testis, esophagus, stomach, skin, lung, bone, colon, pancreas, thyroid, biliary tract, buccal cavity and pharynx (mouth), lips, tongue, oral cavity, pharynx, small intestine, colorectal, large intestine, rectum, cancer of brain and central nervous system, glioblastoma, neuroblastoma, keratoacanthoma, epidermoid carcinoma, large cell carcinoma, adenocarcinoma, adenoma, follicular carcinoma, undifferentiated carcinoma, papillary carcinoma, seminoma, melanoma, sarcoma, bladder cancer, liver cancer, kidney cancer, bone marrow disorder, lymphatic disorder, Hodgkin’s disease, hairy cell carcinoma and leukemia.

The term "treating" as used herein relates to reversing, alleviating or inhibiting the progression or prevention of the disorders or conditions to which the term applies, or of one or more symptoms of such disorders or conditions. The noun "treatment" as used herein relates to the action of treating, which is a verb, and the latter is as just defined.

The term "pharmaceutically acceptable salt" as used herein refers to those carboxylate and amino acid addition salts of the compounds of the present disclosure, which are suitable for the contact with patients’ tissues within a reliable medical judgment, and do not produce inappropriate toxicity, irritation, allergy, etc. They are commensurate with a reasonable benefit/risk ratio, and are effective for their intended use. The term includes, if possible, the zwitterionic form of the compounds of the disclosure.

The pharmaceutically acceptable base addition salts are formed with metals or amines, such as alkali metal and alkaline earth metal hydroxides or organic amines. Examples of the metals used as cations include sodium, potassium, magnesium, calcium, etc. Examples of suitable amines are N,N’-dibenzylethylenediamine, chloroprocaine, choline, diethanolamine, ethylenediamine, N-methylglucamine and procaine.

The base addition salt of the acidic compound can be prepared by contacting the free acid form with a sufficient amount of the required base to form a salt in a conventional manner. The free acid can be regenerated by contacting the salt form with an acid in a conventional manner and then isolating the free acid. The free acid forms are somewhat different from their respective salt forms in their physical properties, such as solubility in polar solvents. But for the purposes of the present disclosure, the salts are still equivalent to their respective free acids.

The salts can be prepared from the inorganic acids, which include sulfates, pyrosulfates, bisulfates, sulfites, bisulfites, nitrates, phosphates, monohydrogen phosphates, dihydrogen phosphates, metaphosphates, pyrophosphates, chlorides, bromides and iodides. Examples of the acids include hydrochloric acid, nitric acid, sulfuric acid, hydrobromic acid, hydroiodic acid, phosphoric acid, etc. The representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthalate, methanesulfonate, glucoheptanate, lactobionate, lauryl sulfonate, isethionate, etc. The salts can also be prepared from the organic acids, which include aliphatic monocarboxylic and dicarboxylic acids, phenyl-substituted alkanoic acids, hydroxyalkanoic acids, alkanedioic acid, aromatic acids, aliphatic and aromatic sulfonic acids, etc. The representative salts include acetate, propionate, octanoate, isobutyrate, oxalate, malonate, succinate, suberate, sebacate, fumarate, maleate, mandelate, benzoate, chlorobenzoate, methyl benzoate, dinitrobenzoate, naphthoate, besylate, tosylate, phenylacetate, citrate, lactate, maleate, tartrate, methanesulfonate, etc. The pharmaceutically acceptable salts can include cations based on alkali metals and alkaline earth metals, such as sodium, lithium, potassium, calcium, magnesium, etc., as well as non-toxic ammonium, quaternary ammonium, and amine cations including, but not limited to, ammonium, tetramethylammonium, tetraethylammonium, methylamine, dimethylamine, trimethylamine, triethylamine, ethylamine, etc. Salts of amino acids are also included, such as arginine salts, gluconates, galacturonates, etc. (for example, see Berge S. M. et al., "Pharmaceutical Salts," J. Pharm. Sci., 1977; 66: 1- 19 for reference).

"Subjects" to which administration is contemplated include, but are not limited to, humans (e.g., males or females of any age group, e.g., paediatric subjects (e.g., infants, children, adolescents) or adult subjects (e.g., young adults, middle-aged adults or older adults) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "human", "patient" and "subject" can be used interchangeably herein.

"Disease," "disorder," and "condition" can be used interchangeably herein.

Unless indicated, otherwise the term "treatment" as used herein includes the effect on a subject who is suffering from a particular disease, disorder, or condition, which reduces the severity of the disease, disorder, or condition, or delays or slows the progression of the disease, disorder or condition ("therapeutic treatment"). The term also includes the effect that occurs before the subject begins to suffer from a specific disease, disorder or condition ("prophylactic treatment ").

Generally, the "effective amount" of a compound refers to an amount sufficient to elicit a target biological response. As understood by those skilled in the art, the effective amount of the compound of the disclosure can vary depending on the following factors, such as the desired biological endpoint, the pharmacokinetics of the compound, the diseases being treated, the mode of administration, and the age, health status and symptoms of the subjects. The effective amount includes therapeutically effective amount and prophylactically effective amount.

Unless indicated, otherwise the "therapeutically effective amount" of the compound as used herein is an amount sufficient to provide therapeutic benefits in the course of treating a disease, disorder or condition, or to delay or minimize one or more symptoms associated with the disease, disorder or condition. The therapeutically effective amount of a compound refers to the amount of the therapeutic agent that, when used alone or in combination with other therapies, provides a therapeutic benefit in the treatment of a disease, disorder or condition. The term "therapeutically effective amount" can include an amount that improves the overall treatment, reduces or avoids the symptoms or causes of the disease or condition, or enhances the therapeutic effect of other therapeutic agents.

Unless indicated, otherwise the "prophylactically effective amount" of the compound as used herein is an amount sufficient to prevent a disease, disorder or condition, or an amount sufficient to prevent one or more symptoms associated with a disease, disorder or condition, or an amount sufficient to prevent the recurrence of a disease, disorder or condition. The prophylactically effective amount of a compound refers to the amount of a therapeutic agent that, when used alone or in combination with other agents, provides a prophylactic benefit in the prevention of a disease, disorder or condition. The term "prophylactically effective amount" can include an amount that improves the overall prevention, or an amount that enhances the prophylactic effect of other preventive agents.

"Combination" and related terms refer to the simultaneous or sequential administration of the compounds of the present disclosure and other therapeutic agents. For example, the compounds of the present disclosure can be administered simultaneously or sequentially in separate unit dosage with other therapeutic agents, or simultaneously in a single unit dosage with other therapeutic agents.

## BRIEF DESCRIPTION OF THE DRAWINGS

FIG. 1 shows the effect of compound C176 on $EGFR^{L858R/T790M/C797S}$ protein level.

FIG. 2 shows the effect of compound C213 on $EGFR^{Del19/T790M/C797S}$ protein level.

## DETAILED DESCRIPTION OF THE INVENTION

As used herein, the term "compound disclosed herein" refers to the following compounds of formula (X) (including sub general formulas, such as formula (I), (I-1), (I-5-1), etc.), or a pharmaceutically acceptable salt, enantiomer, diastereomer, racemate, solvate, hydrate, polymorph, prodrug, or isotopic variant thereof, or mixture thereof.

In the present disclosure, compounds are named using standard nomenclature. For compounds having an asymmetric center, it should be understood, unless otherwise stated, that all optical isomers and mixtures thereof are included. Furthermore, unless otherwise specified, all isomer compounds and carbon-carbon double bonds included in the present disclosure may occur in the form of Z and E. Compounds which exist in different tautomeric forms, one of which is not limited to any particular tautomer, but is intended to cover all tautomeric forms.

In one embodiment, the present disclosure relates to a compound of general formula (X), or a pharmaceutically acceptable salt, enantiomer, diastereomer, racemate, solvate, hydrate, polymorph, prodrug, or isotopic variant thereof, or mixture thereof:

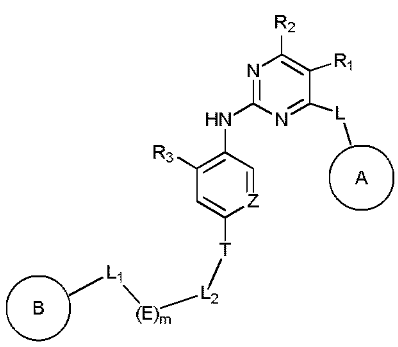

(X)

wherein

ring A is selected from the following optionally substituted groups: $C_{3-7}$ cycloalkyl, 4- to 8-membered heterocyclyl, $C_{6-10}$ aryl or 5- to 10-membered heteroaryl, wherein the substituent is selected from halogen, -CN, $C_{1-6}$ alkyl, $C_{1-6}$ haloalkyl, $C_{2-6}$ alkenyl, $C_{3-7}$ cycloalkyl, 4- to 8-membered heterocyclyl, -P(O)($C_{1-6}$ alkyl)$_2$, -P(O)($C_{2-6}$ alkenyl)$_2$, -O-$C_{1-6}$ alkyl, -O-$C_{1-6}$ haloalkyl, -O-$C_{2-6}$ alkenyl, -O-$C_{3-7}$ cycloalkyl, -O-4- to 8-membered heterocyclyl, -NH-$C_{1-6}$ alkyl, -NH-$C_{2-6}$ alkenyl, -NH-$C_{3-7}$ cycloalkyl, -NH-4- to 8-membered heterocyclyl, -C(O)-$C_{1-6}$ alkyl, -C(O)-$C_{2-6}$ alkenyl, -C(O)-$C_{3-7}$ cycloalkyl, -C(O)-4- to 8-membered heterocyclyl, -S(O)$_2$-$C_{1-6}$ alkyl, -S(O)$_2$-$C_{2-6}$ alkenyl, -S(O)$_2$-$C_{3-7}$ cycloalkyl, - S(O)$_2$-4- to 8-membered heterocyclyl, -NHC(O)-$C_{1-6}$ alkyl, -NHC(O)-$C_{2-6}$ alkenyl, -NHC(O)-$C_{3-7}$ cycloalkyl, -NHC(O)-4- to 8-membered heterocyclyl, -NHS(O)$_2$-$C_{1-6}$ alkyl, -NHS(O)$_2$-$C_{2-6}$ alkenyl, -NHS(O)$_2$-$C_{3-7}$ cycloalkyl or -NHS(O)$_2$-4- to 8-membered heterocyclyl;

ring B is selected from the following groups:

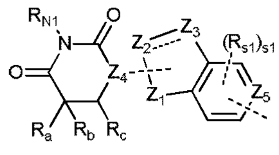;



represents single bond or double bond;



represents that the point of attachment to the rest of the molecule can be located at the available point of the ring;

$Z_1$ is O, S, N or C atom, which is optionally substituted with one or two Rzi; or $Z_1$ is absent, and thus $Z_4$ is connected to $Z_2$, $Z_3$ or the C atom connected to $Z_1$ on the aromatic ring, and the $Z_2$ and the C atom on the aromatic ring that are connected to $Z_1$ are connected to Rw respectively; or $Z_1$, $Z_2$ and $Z_3$ are all absent, and thus $Z_4$ is connected to one of the C atoms connected to $Z_1$ or $Z_3$ on the aromatic ring, and the other C atom on the aromatic ring is connected to $R_W$;

$Z_2$ is O, S, N or C atom, which is optionally substituted with one or two $R_{Z2}$;

$Z_3$ is O, S, N or C atom, which is optionally substituted with one or two $R_{Z3}$; with the proviso that when



represents double bond, $Z_2$ is N or C atom, and $Z_3$ is N or C atom;

$Z_4$ is N or $CR_{Z4}$;

$Z_5$ is N or $CR_{Z5}$;

$R_a$, $R_b$ and $R_c$ are independently H, halogen, -$(CH_2)_{0-5}$-OR", -$(CH_2)_{0-5}$-NR″R‴, $C_{1-6}$ alkyl or $C_{1-6}$ haloalkyl; or $R_a$ and $R_b$ are taken together with the carbon atom to which they are attached to form C=O, $C_{3-7}$ cycloalkyl or 4- to 8-membered heterocyclyl; or, $R_a$ and $R_c$ are taken together with the cabin atoms to which they are attached to form $C_{3-7}$ cycloalkyl or 4-to 8-membered heterocyclyl; or $R_a$ and $R_c$ are taken together to form bond;

$R_{N1}$ is H, $C_{1-6}$ alkyl or $C_{1-6}$ haloalkyl, alternatively H;

Rzi is absent, H, CN, halogen, -$(CH_2)_{0-5}$-OR", -$(CH_2)_{0-5}$-NR″R‴, $C_{1-6}$ alkyl, $C_{1-6}$ haloalkyl, -$(CH_2)_{0-5}$-$C_{3-7}$ cycloalkyl or -$(CH_2)_{0-5}$-4- to 8-membered heterocyclyl; or two $R_{Z1}$ are taken together with $Z_1$ to form C=O, $C_{3-7}$ cycloalkyl or 4- to 8-membered heterocyclyl;

$R_{Z2}$ is absent, H, CN, halogen, -$(CH_2)_{0-5}$-OR", -$(CH_2)_{0-5}$-NR″R‴, $C_{1-6}$ alkyl, $C_{1-6}$ haloalkyl, -$(CH_2)_{0-5}$-$C_{3-7}$ cycloalkyl or -$(CH_2)_{0-5}$-4- to 8-membered heterocyclyl; or two $R_{Z2}$ are taken together with $Z_2$ to form C=O, $C_{3-7}$ cycloalkyl or 4- to 8-membered heterocyclyl;

$R_{Z3}$ is absent, H, CN, halogen, -$(CH_2)_{0-5}$-OR", -$(CH_2)_{0-5}$-NR″R‴, $C_{1-6}$ alkyl, $C_{1-6}$ haloalkyl, -$(CH_2)_{0-5}$-$C_{3-7}$ cycloalkyl or -$(CH_2)_{0-5}$-4- to 8-membered heterocyclyl; or two $R_{Z3}$ are taken together with $Z_3$ to form C=O, $C_{3-7}$ cycloalkyl or 4- to 8-membered heterocyclyl;

$R_{Z4}$ is H, CN, halogen, -$(CH_2)_{0-5}$-OR", -$(CH_2)_{0-5}$-NR″R‴, $C_{1-6}$ alkyl or $C_{1-6}$ haloalkyl;

$R_{Z5}$ is H, CN, halogen, -$(CH_2)_{0-5}$-OR", -$(CH_2)_{0-5}$-NR″R‴, $C_{1-6}$ alkyl, $C_{1-6}$ haloalkyl, -$(CH_2)_{0-5}$-$C_{3-7}$ cycloalkyl or

$-(CH_2)_{0-5}$-4- to 8-membered heterocyclyl;

or the ring where $Z_4$ is located is absent;

wherein $R_w$ is H, CN, halogen, $-(CH_2)_{0-5}$-OR", $-(CH_2)_{0-5}$-NR″R‴, $-C_{1-6}$ alkyl, $C_{1-6}$ haloalkyl, $-(CH_2)_{0-5}-C_{3-7}$ cycloalkyl, $-(CH_2)_{0-5}$-4- to 8-membered heterocyclyl, $C_{2-6}$ alkenyl, $C_{2-6}$ alkynyl, $-(CH_2)_{0-5}-C_{3-10}$ halocycloalkyl, $-(CH_2)_{0-5}-C_{6-10}$ aryl or $-(CH_2)_{0-5}$-5- to 14-membered heteroaryl;

R" is H, $C_{1-6}$ alkyl, $C_{1-6}$ haloalkyl or $-(CH_2)_{0-5}-C_{3-7}$ cycloalkyl;

R‴ is H, $C_{1-6}$ alkyl or $C_{1-6}$ haloalkyl;

$L_1$ is selected from bond, -O-, $-S(O)_p-$, -S(O)(=NR*)-, $-NR^{\#}-$, $-CR^{\#}R^{\#'}-$, $-C_aR^{\#}R^{\#'}-C_bR^{\#}R^{\#'}-$, -N=S(O)(R*)- or -S(O)(R*)=N-;

$L_2$ is selected from bond, -O-, $-S(O)_p-$, -S(O)(=NR*)-, $-NR^{\#}-$, $-CR^{\#}R^{\#'}-$, $-C_aR^{\#}R^{\#'}-C_bR^{\#}R^{\#'}-$, -N=S(O)(R*)- or -S(O)(R*)=N-;

wherein one of $C_aR^{\#}R^{\#'}$ and $C_bR^{\#}R^{\#'}$ can be replaced by O, $S(O)_p$, S(O)(=NR*) or $NR^{\#}$, and when one of $C_aR^{\#}R^{\#'}$ and $C_bR^{\#}R^{\#''}$ is replaced by O, S or $NR^{\#}$, the other of $C_aR^{\#}R^{\#'}$ and $C_bR^{\#}R^{\#'}$ can also be replaced by $S(O)_q$;

E is independently selected from: bond, $-C_cR^{\#}R^{\#'}-C_dR^{\#}R^{\#'}-C_eR^{\#}R^{\#'}$,

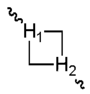,

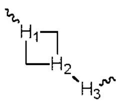, 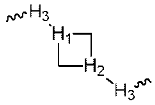, 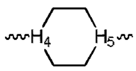 or 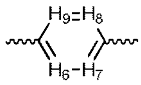;

wherein one of $C_cR^{\#}R^{\#'}$, $C_dR^{\#}R^{\#'}$ or $C_eR^{\#}R^{\#'}$, or both of $C_cR^{\#}R^{\#'}$ and $C_eR^{\#}R^{\#'}$ can be replaced by O, $S(O)_p$, S(O)(=NR*) or $NR^{\#}$, and when one of $C_cR^{\#}R^{\#'}$, $C_dR^{\#}R^{\#'}$ or $C_eR^{\#}R^{\#'}$ is replaced by O, S or $NR^{\#}$, the other adjacent one or two of $C_cR^{\#}R^{\#'}$, $C_dR^{\#}R^{\#'}$ or $C_eR^{\#}R^{\#'}$ can also be replaced by $S(O)_q$;

or two E moieties can be taken together to form $-CH_2CH_2OCH_2CH_2-$, - $OCH_2CH_2CH_2CH_2-$, $-CH_2CH_2CH_2CH_2O-$,

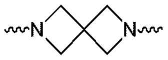, 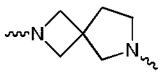, 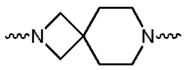,

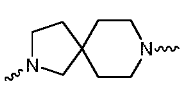, 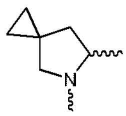 or 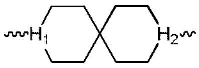;

wherein



represents the point of attachment to $L_1$ or $L_2$;

$H_1$ and $H_2$ are N or C atom, $H_3$ is O, S, N or C atom, and $H_1$ and $H_3$, $H_2$ and $H_3$ are not heteroatoms at the same time;

$H_4$ and $H_5$ are N or C atom;

$H_6$, $H_7$, $H_8$ and $H_9$ are C or N atom;

p is 0, 1 or 2;

q is 1 or 2;

R* is H, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl or 5- to 14-membered heteroaryl;

$R^{\#}$ is H, halogen, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl or 5- to 14-membered heteroaryl;

$R^{\#'}$ is H, halogen, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl or 5- to 14-membered heteroaryl;

or, $R^{\#}$ and $R^{\#}$ on adjacent atoms can be taken together to form bond, and $R^{\#'}$ and $R^{\#'}$ on adjacent atoms can be

taken together to form bond;

or, $R^{\#}$ and $R^{\#'}$ on the same or different atoms can be taken together to form =O, or $C_{3-7}$ cycloalkyl, 4- to 8-membered heterocyclyl, $C_{6-10}$ aryl or 5- to 6-membered heteroaryl, wherein the $C_{3-7}$ cycloalkyl, 4- to 8-membered heterocyclyl, $C_{6-10}$ aryl or 5- to 6-membered heteroaryl is optionally substituted with $R_x$, and the $R_x$ is H, CN, halogen, $C_{1-6}$ alkyl or $C_{1-6}$ haloalkyl;

m is 0, 1, 2, 3, 4, 5, 6, 7, 8, 9 or 10;

$R_{s1}$ is selected from H, CN, halogen, $-(CH_2)_{0-5}$-OR", $-(CH_2)_{0-5}$-NR"R‴, $C_{1-6}$ alkyl, $C_{1-6}$ haloalkyl, $-(CH_2)_{0-5}$-4- to 8-membered heterocyclyl, $C_{2-6}$ alkenyl, $C_{2-6}$ alkynyl, $-(CH_2)_{0-5}-C_{3-7}$ cycloalkyl, $-(CH_2)_{0-5}-C_{3-10}$ halocycloalkyl, $-(CH_2)_{0-5}-C_{6-10}$ aryl, $-(CH_2)_{0-5}$-5- to 14-membered heteroaryl, $-C(O)R_W$, $-S(O)R_W$ or $-S(O)_2R_W$;

s1 is 0, 1, 2 or 3;

R' is selected from H, -C(O)-$C_{1-6}$ alkyl, -C(O)-$C_{1-6}$ haloalkyl, -C(O)-$C_{2-6}$ alkenyl or - C(O)-$C_{6-10}$ aryl;

L is bond, -O- or -NR-;

wherein R is H or $C_{1-6}$ alkyl;

Z is -N= or -C($R_4$)=;

T is selected from bond, $C_{2-6}$ heteroalkylene, 4- to 12-membered heterocyclylene, or 5- to 6-membered heterocyclylene, wherein the 5- to 6-membered heterocyclylene is substituted with 5- to 6-membered heterocyclylene;

$R_1$ is selected from H, halogen, cyano, $C_{1-6}$ alkyl, $C_{3-7}$ cycloalkyl, 4- to 8-membered heterocyclyl, $C_{1-6}$ haloalkyl, $C_{2-6}$ alkenyl or $C_{2-6}$ alkynyl;

or, when L is -NR-, $R_1$ and R are taken together with the atoms to which they are attached to form optionally substituted 5- to 6-membered heterocyclyl, wherein the substituent is selected from halogen, oxo, $C_{1-6}$ alkyl, $C_{1-6}$ haloalkyl, $C_{6-10}$ aryl, wherein the $C_{6-10}$ aryl is mono- or poly-substituted with halogen;

$R_2$ is H, halogen, hydroxyl, amino, $C_{1-6}$ alkyl or $C_{1-6}$ haloalkyl;

or $R_1$ and $R_2$ are taken together with the atoms to which they are attached to form 5-to 6-membered heterocyclyl or 5- to 6-membered heteroaryl;

$R_3$ is selected from H, -O-$C_{1-6}$ alkyl or -O-$C_{1-6}$ haloalkyl;

$R_4$ is selected from H, halogen, $C_{1-6}$ alkyl, $C_{1-6}$ haloalkyl, -NHC(O)-$C_{1-6}$ alkyl or - NHC(O)-$C_{2-6}$ alkenyl;

if the above groups are H or H-containing groups, the one or more H atom(s) may be substituted with D atom(s);

the groups containing OH, NH, $NH_2$, CH, $CH_2$, or $CH_3$ in $L_1$, E, $L_2$, and T, or the above alkyl, alkylene, haloalkyl, alkenyl, alkynyl, cycloalkyl, halocycloalkyl, heterocyclyl, aryl, and heteroaryl are each optionally substituted with 1, 2, 3 or more $R^s$ at each occurrence, and the $R^s$ is independently selected from the following groups at each occurrence: halogen, hydroxyl, amino, cyano, nitro, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl, 5- to 14-membered heteroaryl, $C_{6-12}$ aralkyl, $-OR^{a'}$, $-OC(O)R^{a'}$, $-C(O)R^{a'}$, $-C(O)OR^{a'}$, $-C(O)NR^{a'}R^{b'}$, $- S(O)_nR^{a'}$, $-S(O)_nOR^{a'}$, $-S(O)_nNR^{a'}R^{b'}$, $-NR^{a'}R^{b'}$, $-NR^{a'}C(O)R^{b'}$, $-NR^{a'}-C(O)OR^{b'}$, $-NR^{a'}-S(O)_n-R^{b'}$, $-NR^{a'}C(O)NR^{a'}R^{b'}$, $-C_{1-6}$ alkylene-$R^{a'}$, $-C_{1-6}$ alkylene-$OR^{a'}$, $-C_{1-6}$ alkylene-$OC(O)R^{a'}$, $-C_{1-6}$ alkylene-$C(O)OR^{a'}$, $-C_{1-6}$ alkylene-$S(O)_nR^{a'}$, $-C_{1-6}$ alkylene-$S(O)_nOR^{a'}$, $-C_{1-6}$ alkylene-$OC(O)NR^{a'}R^{b'}$, $-C_{1-6}$ alkylene-$C(O)NR^{a'}R^{b'}$, $-C_{1-6}$ alkylene-$NR^{a'}-C(O)NR^{a'}R^{b'}$, $-C_{1-6}$ alkylene-$OS(O)_nR^{a'}$, $-C_{1-6}$ alkylene-$S(O)_nNR^{a'}R^{b'}$, $-C_{1-6}$ alkylene-$NR^{a'}-S(O)_nNR^{a'}R^{b'}$, $-C_{1-6}$ alkylene-$NR^{a'}R^{b'}$ and $-O-C_{1-6}$ alkylene-$NR^{a'}R^{b'}$, and wherein the hydroxyl, amino, alkyl, alkylene, cycloalkyl, heterocyclyl, aryl, heteroaryl and aralkyl described with respect to the substituent $R^s$ are further optionally substituted with 1, 2, 3 or more substituents independently selected from: halogen, OH, amino, cyano, nitro, $C_{1-6}$ alkyl, $C_{1-6}$ haloalkyl, $C_{1-6}$ alkyl hydroxyl, $C_{3-6}$ cycloalkyl, 3- to 10-membered heterocyclyl, $C_{6-10}$ aryl, 5- to 14-membered heteroaryl and $C_{6-12}$ aralkyl;

n is independently 1 or 2 at each occurrence;

each of $R^{a'}$ and $R^{b'}$ is independently selected from H, $C_{1-6}$ alkyl, $C_{2-6}$ alkenyl, $C_{2-6}$ alkynyl, $C_{1-6}$ alkyl-O-, $C_{1-6}$ alkyl-S-, $C_{3-10}$ cycloalkyl, 3- to 10-membered heterocyclyl, $C_{6-10}$ aryl, 5- to 14-membered heteroaryl and $C_{6-12}$ aralkyl at each occurrence.

In another specific embodiment, the present disclosure relates to a compound of general formula (X), or a pharmaceutically acceptable salt, enantiomer, diastereomer, racemate, solvate, hydrate, polymorph, prodrug, or isotopic variant thereof, or mixture thereof:

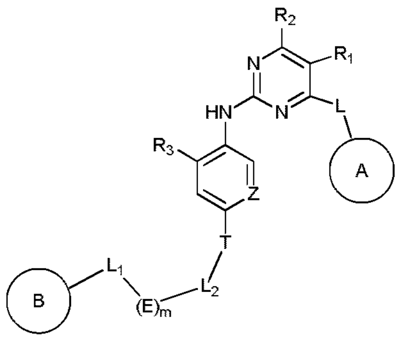

(X)

wherein

ring A is selected from the following optionally substituted groups: $C_{3-7}$ cycloalkyl, 4- to 8-membered heterocyclyl, $C_{6-10}$ aryl or 5- to 10-membered heteroaryl, wherein the substituent is selected from halogen, -CN, $C_{1-6}$ alkyl, $C_{1-6}$ haloalkyl, $C_{2-6}$ alkenyl, $C_{3-7}$ cycloalkyl, 4- to 8-membered heterocyclyl, $-P(O)(C_{1-6}$ alkyl$)_2$, $-P(O)(C_{2-6}$ alkenyl$)_2$, -O-$C_{1-6}$ alkyl, -O-$C_{1-6}$ haloalkyl, -O-$C_{2-6}$ alkenyl, -O-$C_{3-7}$ cycloalkyl, -O-4- to 8-membered heterocyclyl, -NH-$C_{1-6}$ alkyl, -NH-$C_{2-6}$ alkenyl, -NH-$C_{3-7}$ cycloalkyl, -NH-4- to 8-membered heterocyclyl, -C(O)-$C_{1-6}$ alkyl, -C(O)-$C_{2-6}$ alkenyl, -C(O)-$C_{3-7}$ cycloalkyl, -C(O)-4- to 8-membered heterocyclyl, $-S(O)_2$-$C_{1-6}$ alkyl, $-S(O)_2$-$C_{2-6}$ alkenyl, $-S(O)_2$-$C_{3-7}$ cycloalkyl, - $S(O)_2$-4- to 8-membered heterocyclyl, -NHC(O)-$C_{1-6}$ alkyl, -NHC(O)-$C_{2-6}$ alkenyl, -NHC(O)-$C_{3-7}$ cycloalkyl, -NHC(O)-4- to 8-membered heterocyclyl, $-NHS(O)_2$-$C_{1-6}$ alkyl, $-NHS(O)_2$-$C_{2-6}$ alkenyl, $-NHS(O)_2$-$C_{3-7}$ cycloalkyl or $-NHS(O)_2$-4- to 8-membered heterocyclyl;

ring B is selected from the following groups:

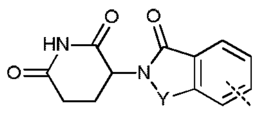
;

wherein Y is $-CH_2$- or -C(O)-;

R' is selected from H, -C(O)-$C_{1-6}$ alkyl, -C(O)-$C_{1-6}$ haloalkyl, -C(O)-$C_{2-6}$ alkenyl or - C(O)-$C_{6-10}$ aryl;

----- represents the point of attachment to $L_1$;

L is bond, -O- or -NR-;

wherein R is H or $C_{1-6}$ alkyl;

Z is -N= or $-C(R_4)$=;

T is selected from bond, $C_{2-6}$ heteroalkylene, 4- to 12-membered heterocyclylene, or 5- to 6-membered heterocyclylene, wherein the 5- to 6-membered heterocyclylene is substituted with 5- to 6-membered heterocyclylene;

$L_1$ is selected from bond, -O-, -NH-, $-CH_2$-, -C(O)-, $-CH_2CH_2$-, -CH=CH-, -C=C-, - $OCH_2$-, $-CH_2O$-, $-NHCH_2$-, $-CH_2NH$-, $-C(O)CH_2$-, $-CH_2C(O)$-, -OC(O)-, -C(O)O-, -NHC(O)- or -C(O)NH-;

E is independently selected from $-CH_2CH_2CH_2$-, $-CH_2CH_2C(O)$-, $-CH_2C(O)CH_2$-, - $C(O)CH_2CH_2$-, -C(O)CH=CH-, -C(O)C=C-, $-CH_2CH_2O$-, $-CH_2OCH_2$-, $-OCH_2CH_2$-, - $C(O)CH_2O$-, $-OCH_2C(O)$-, $-CH_2C(O)O$-, $-OC(O)CH_2$-, $-C(O)OCH_2$-, $-CH_2OC(O)$-, - $CH_2CH_2NH$-, $-CH_2NHCH_2$-, $-NHCH_2CH_2$-, $-C(O)CH_2NH$-, $-NHCH_2C(O)$-, $-CH_2C(O)NH$-, - $NHC(O)CH_2$-, $-C(O)NHCH_2$-, $-CH_2NHC(O)$-,

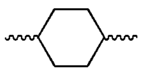 , 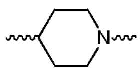 , 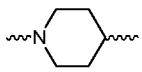 ,

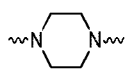 , 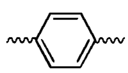 , 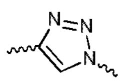 , 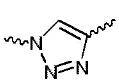 , 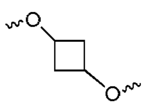 , 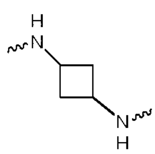 , 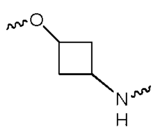 ,

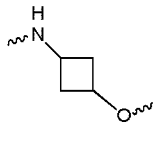, 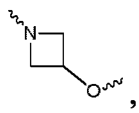, 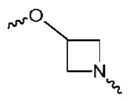, 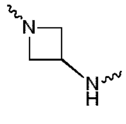 or 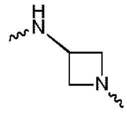;

wherein



represents the point of attachment to $L_1$ or $L_2$;

m is 0, 1, 2, 3, 4, 5, 6, 7, 8, 9 or 10;

$L_2$ is selected from bond, $-CH_2-$, $-CH_2CH_2-$, $-OCH_2-$, $-NHCH_2-$, -OC(O)-, -NHC(O)-, $-CH_2C(O)-$ or $-C(O)CH_2-$;

$R_1$ is selected from H, halogen, cyano, $C_{1-6}$ alkyl, $C_{3-7}$ cycloalkyl, 4- to 8-membered heterocyclyl, $C_{1-6}$ haloalkyl, $C_{2-6}$ alkenyl or $C_{2-6}$ alkynyl;

or, when L is -NR-, $R_1$ and R are taken together with the atoms to which they are attached to form optionally substituted 5- to 6-membered heterocyclyl, wherein the substituent is selected from halogen, oxo, $C_{1-6}$ alkyl, $C_{1-6}$ haloalkyl, or $C_{6-10}$ aryl, wherein the $C_{6-10}$ aryl is mono- or poly-substituted with halogen;

$R_2$ is H, halogen, hydroxyl, amino, $C_{1-6}$ alkyl or $C_{1-6}$ haloalkyl;

or $R_1$ and $R_2$ are taken together with the atoms to which they are attached to form 5-to 6-membered heterocyclyl or 5- to 6-membered heteroaryl;

$R_3$ is selected from H, $-O-C_{1-6}$ alkyl or $-O-C_{1-6}$ haloalkyl;

$R_4$ is selected from H, halogen, $C_{1-6}$ alkyl, $C_{1-6}$ haloalkyl, $-NHC(O)-C_{1-6}$ alkyl or - $NHC(O)-C_{2-6}$ alkenyl;

the groups containing OH, NH, $NH_2$, CH, $CH_2$, or $CH_3$ in $L_1$, E, $L_2$, and T, or the above alkyl, alkylene, alkenyl, alkynyl, cycloalkyl, heterocyclyl, aryl, and heteroaryl are each optionally substituted with 1, 2, 3 or more $R^s$ at each occurrence, and the $R^s$ is independently selected from the following groups at each occurrence: halogen, hydroxyl, amino, cyano, nitro, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl, 5- to 14-membered heteroaryl, $C_{6-12}$ aralkyl, $-OR^a$, $-OC(O)R^a$, $-C(O)R^a$, $-C(O)OR^a$, $-C(O)NR^aR^b$, $-S(O)_nR^a$, $-S(O)_nOR^a$, - $S(O)_nNR^aR^b$, $-NR^aR^b$, $-NR^aC(O)R^b$, $-NR^a-C(O)OR^b$, $-NR^a-S(O)_n-R^b$, $-NR^aC(O)NR^aR^b$, $-C_{1-6}$ alkylene-$R^a$, $-C_{1-6}$ alkylene-$OR^a$, $-C_{1-6}$ alkylene-$OC(O)R^a$, $-C_{1-6}$ alkylene-$C(O)OR^a$, $-C_{1-6}$ alkylene-$S(O)_nR^a$, $-C_{1-6}$ alkylene-$S(O)_nOR^a$, $-C_{1-6}$ alkylene-$OC(O)NR^aR^b$, $-C_{1-6}$ alkylene-$C(O)NR^aR^b$, $-C_{1-6}$ alkylene-$NR^a-C(O)NR^aR^b$, $-C_{1-6}$ alkylene-$OS(O)_nR^a$, $-C_{1-6}$ alkylene$S(O)_nNR^aR^b$, $-C_{1-6}$ alkylene-$NR^a-S(O)_nNR^aR^b$, $-C_{1-6}$ alkylene-$NR^aR^b$ and $-O-C_{1-6}$ alkylene-$NR^aR^b$, and wherein the halogen, hydroxyl, amino, alkyl, alkylene, cycloalkyl, heterocyclyl, aryl, heteroaryl and aralkyl described with respect to the substituent $R^s$ are further optionally substituted with 1, 2, 3 or more substituents independently selected from: halogen, OH, amino, cyano, nitro, $C_{1-6}$ alkyl, $C_{1-6}$ haloalkyl, $C_{1-6}$ alkyl hydroxyl, $C_{3-6}$ cycloalkyl, 3- to 10-membered heterocyclyl, $C_{6-10}$ aryl, 5- to 14-membered heteroaryl and $C_{6-12}$ aralkyl;

n is independently 1 or 2 at each occurrence;

$R^a$ and $R^b$ are independently selected from H, $C_{1-6}$ alkyl, $C_{2-6}$ alkenyl, $C_{2-6}$ alkynyl, $C_{1-6}$ alkyl-O-, $C_{1-6}$ alkyl-S-, $C_{3-10}$ cycloalkyl, 3- to 10-membered heterocyclyl, $C_{6-10}$ aryl, 5- to 14-membered heteroaryl and $C_{6-12}$ aralkyl at each occurrence.

In a more specific embodiment, the present disclosure provides a compound of formula (I) or (I-G), or a pharmaceutically acceptable salt, enantiomer, diastereomer, racemate, solvate, hydrate, polymorph, prodrug, or isotopic variant thereof, or mixture thereof:

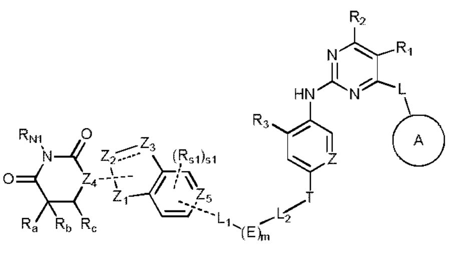 (I), 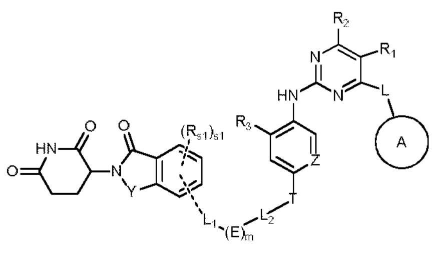 (I-G),

wherein each group is as defined above.

In a more specific embodiment, the present disclosure provides a compound of formula (I) or (I-G), or a pharmaceutically acceptable salt, enantiomer, diastereomer, racemate, solvate, hydrate, polymorph, prodrug, or isotopic variant thereof, or mixture thereof:

wherein

ring A is the following groups:

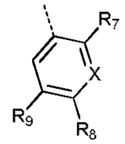 , 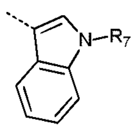 or 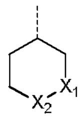 ;

wherein

$R_7$ is selected from $C_{1-6}$ alkyl, $C_{1-6}$ haloalkyl, $C_{2-6}$ alkenyl, $C_{3-7}$ cycloalkyl, -P(O)($C_{1-6}$ alkyl)$_2$ or -P(O)($C_{2-6}$ alkenyl)$_2$;

$R_8$ is selected from H, -NH-$C_{1-6}$ alkyl, -NH-$C_{2-6}$ alkenyl, -NH-$C_{3-7}$ cycloalkyl, -NH-4- to 8-membered heterocyclyl, -NHC(O)-$C_{1-6}$ alkyl, -NHC(O)-$C_{2-6}$ alkenyl, -NHC(O)-$C_{3-7}$ cycloalkyl, -NHC(O)-4- to 8-membered heterocyclyl, -NHS(O)$_2$-$C_{1-6}$ alkyl or -NHS(O)$_2$-$C_{3-7}$ cycloalkyl;

$R_9$ is selected from H, halogen, -CN, $C_{1-6}$ haloalkyl, -O-$C_{1-6}$ alkyl, -O-$C_{1-6}$ haloalkyl, $C_{3-7}$ cycloalkyl, -O-$C_{3-7}$ cycloalkyl, $C_{1-6}$ alkyl, -NH-$C_{1-6}$ alkyl, -NH-$C_{2-6}$ alkenyl, -NH-$C_{3-7}$ cycloalkyl, -NH-4- to 8-membered heterocyclyl, -NHC(O)-$C_{1-6}$ alkyl, -NHC(O)-$C_{2-6}$ alkenyl, -NHC(O)-$C_{3-7}$ cycloalkyl, -NHC(O)-4- to 8-membered heterocyclyl, -NHS(O)$_2$-$C_{1-6}$ alkyl or - NHS(O)$_2$-$C_{3-7}$ cycloalkyl;

X is -C($R_x$)= or -N=;

wherein $R_x$ is H, or $R_x$ and $R_8$ are taken together with the C atoms to which they are attached to form 5- to 6-membered heterocyclyl or 5- to 6-membered heteroaryl; alternatively, $R_x$ and $R_8$ are taken together with the C atoms to which they are attached to form 5- to 6-membered heteroaryl, alternatively pyrazinyl;

$X_1$ is -CH(Rxi)- or -N($R_{X1}$)-;

$X_2$ is -CH($R_{X2}$)- or -N($R_{X2}$)-;

wherein Rxi is selected from H, $C_{1-6}$ alkyl, $C_{3-7}$ cycloalkyl, -O-$C_{1-6}$ alkyl, -O-$C_{3-7}$ cycloalkyl, -NH-$C_{1-6}$ alkyl, -NH-$C_{3-7}$ cycloalkyl, -S(O)$_2$-$C_{1-6}$ alkyl, -S(O)$_2$-$C_{3-7}$ cycloalkyl, - NHS(O)$_2$-$C_{1-6}$ alkyl, -NHS(O)$_2$-$C_{3-7}$ cycloalkyl, -C(O)-$C_{1-6}$ alkyl, -C(O)-$C_{2-6}$ alkenyl, -C(O)-$C_{3-7}$ cycloalkyl, -NHC(O)-$C_{1-6}$ alkyl, -NHC(O)-$C_{3-7}$ cycloalkyl or -C(O)-4- to 8-membered heterocyclyl; $R_{X2}$ is selected from H, $C_{1-6}$ alkyl, $C_{3-7}$ cycloalkyl, -O-$C_{1-6}$ alkyl, -O-$C_{3-7}$ cycloalkyl, -NH-$C_{1-6}$ alkyl, -NH-$C_{3-7}$ cycloalkyl, -C(O)-$C_{1-6}$ alkyl, -C(O)-$C_{3-7}$ cycloalkyl, - C(O)-4- to 8-membered heterocyclyl, -S(O)$_2$-$C_{1-6}$ alkyl, -S(O)$_2$-$C_{3-7}$ cycloalkyl, -NHS(O)$_2$-$C_{1-6}$ alkyl, -NHS(O)$_2$-$C_{3-7}$ cycloalkyl, -NHC(O)-$C_{1-6}$ alkyl, -NHC(O)-$C_{2-6}$ alkenyl, -NHC(O)-$C_{3-7}$ cycloalkyl or -NHC(O)-4- to 8-membered heterocyclyl;

----- represents the point of attachment to L;

other groups are as defined above.

In a more specific embodiment, the present disclosure provides a compound of formula (I) or (I-G), or a pharmaceutically acceptable salt, enantiomer, diastereomer, racemate, solvate, hydrate, polymorph, prodrug, or isotopic variant thereof, or mixture thereof:

wherein

T is bond,

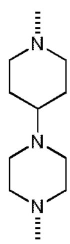 , 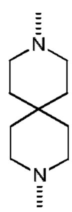 , 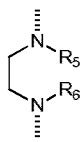 , 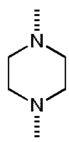 , 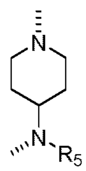 or 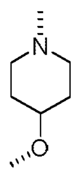 ;

wherein

$R_5$ is H or $C_{1-6}$ alkyl;

$R_6$ is H or $C_{1-6}$ alkyl;

or $R_5$ and R6 are connected to form $C_{1-6}$ alkylene;

 represents the point of attachment to parent core or $L_2$;
other groups are as defined above.

In a more specific embodiment, the present disclosure provides a compound of formula (I) or (I-G), or a pharmaceutically acceptable salt, enantiomer, diastereomer, racemate, solvate, hydrate, polymorph, prodrug, or isotopic variant thereof, or mixture thereof:

wherein
when L is -NR-, $R_1$ and R are taken together to form the following groups: - C(O)N($R_N$)C(O)- or -C($C_{1-6}$ alkyl)=C($R_N$)C(O)-;
wherein
$R_N$ is selected from $C_{1-6}$ alkyl or

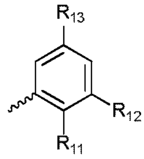 ;

$R_{11}$ is H or halogen;
$R_{12}$ is H or halogen;
$R_{13}$ is H or halogen;



represents the point of attachment;
other groups are as defined above.

In a more specific embodiment, the present disclosure provides a compound of formula (I) or (I-G), or a pharmaceutically acceptable salt, enantiomer, diastereomer, racemate, solvate, hydrate, polymorph, prodrug, or isotopic variant thereof, or mixture thereof:
wherein $R_1$ and $R_2$ are taken together with the atoms to which they are attached to form 5- to 6-membered heteroaryl; alternatively form pyrrolyl; other groups are as defined above.

Ring A

In a specific embodiment, ring A is optionally substituted $C_{3-7}$ cycloalkyl; in another specific embodiment, ring A is optionally substituted 4- to 8-membered heterocyclyl; in another specific embodiment, ring A is optionally substituted $C_{6-10}$ aryl; in another specific embodiment, ring A is optionally substituted 5- to 10-membered heteroaryl; in another specific embodiment, ring A is

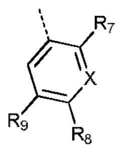

; in another specific embodiment, ring A is

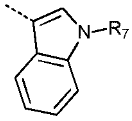 ;

in another specific embodiment, ring A is

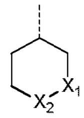

.

In a specific embodiment described above, the substituent of ring A is halogen; in another specific embodiment described above, the substituent of ring A is -CN; in another specific embodiment described above, the substituent of ring A is $C_{1-6}$ alkyl; in another specific embodiment described above, the substituent of ring A is $C_{1-6}$ haloalkyl; in another specific embodiment described above, the substituent of ring A is $C_{2-6}$ alkenyl; in another specific embodiment described above, the substituent of ring A is $C_{3-7}$ cycloalkyl; in another specific embodiment described above, the substituent of ring A is 4- to 8-membered heterocyclyl; in another specific embodiment described above, the substituent of ring A is -$P(O)(C_{1-6}$ alkyl$)_2$; in another specific embodiment described above, the substituent of ring A is -$P(O)(C_{2-6}$ alkenyl$)_2$; in another specific embodiment described above, the substituent of ring A is -O-$C_{1-6}$ alkyl; in another specific embodiment described above, the substituent of ring A is -O-$C_{1-6}$ haloalkyl; in another specific embodiment described above, the substituent of ring A is -O-$C_{2-6}$ alkenyl; in another specific embodiment described above, the substituent of ring A is -O-$C_{3-7}$ cycloalkyl; in another specific embodiment described above, the substituent of ring A is -O-4- to 8-membered heterocyclyl; in another specific embodiment described above, the substituent of ring A is -NH-$C_{1-6}$ alkyl; in another specific embodiment described above, the substituent of ring A is -NH-$C_{2-6}$ alkenyl; in another specific embodiment described above, the substituent of ring A is -NH-$C_{3-7}$ cycloalkyl; in another specific embodiment described above, the substituent of ring A is -NH-4- to 8-membered heterocyclyl; in another specific embodiment described above, the substituent of ring A is - C(O)-$C_{1-6}$ alkyl; in another specific embodiment described above, the substituent of ring A is -C(O)-$C_{2-6}$ alkenyl; in another specific embodiment described above, the substituent of ring A is -C(O)-$C_{3-7}$ cycloalkyl; in another specific embodiment described above, the substituent of ring A is -C(O)-4- to 8-membered heterocyclyl; in another specific embodiment described above, the substituent of ring A is -$S(O)_2$-$C_{1-6}$ alkyl; in another specific embodiment described above, the substituent of ring A is -$S(O)_2$-$C_{2-6}$ alkenyl; in another specific embodiment described above, the substituent of ring A is -$S(O)_2$-$C_{3-7}$ cycloalkyl; in another specific embodiment described above, the substituent of ring A is -$S(O)_2$-4- to 8-membered heterocyclyl; in another specific embodiment described above, the substituent of ring A is - NHC(O)-$C_{1-6}$ alkyl; in another specific embodiment described above, the substituent of ring A is -NHC(O)-$C_{2-6}$ alkenyl; in another specific embodiment described above, the substituent of ring A is -NHC(O)-$C_{3-7}$ cycloalkyl; in another specific embodiment described above, the substituent of ring A is -NHC(O)-4- to 8-membered heterocyclyl; in another specific embodiment described above, the substituent of ring A is -$NHS(O)_2$-$C_{1-6}$ alkyl; in another specific embodiment described above, the substituent of ring A is -$NHS(O)_2$-$C_{2-6}$ alkenyl; in another specific embodiment described above, the substituent of ring A is -$NHS(O)_2$-$C_{3-7}$ cycloalkyl; in another specific embodiment described above, the substituent of ring A is - $NHS(O)_2$-4- to 8-membered heterocyclyl.

L

In a specific embodiment, L is bond; in another specific embodiment, L is -O-; in another specific embodiment, L is -NR-.

Y

In a specific embodiment, Y is -$CH_2$-; in another specific embodiment, Y is -C(O)-.

Z

In a specific embodiment, Z is -N=; in another specific embodiment, Z is -$C(R_4)$=.

T

In a specific embodiment, T is $C_{2-6}$ heteroalkylene; in another specific embodiment, T is 4- to 12-membered heterocyclylene; in another specific embodiment, T is 5- to 6-membered heterocyclylene substituted with 5- to 6-membered heterocyclylene; in another specific embodiment, T is

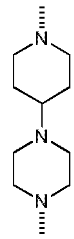
;

in another specific embodiment, T is

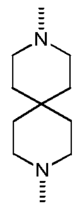
;

in another specific embodiment, T is

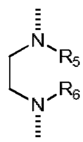
;

in another specific embodiment, T is

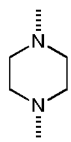

; in another specific embodiment, T is bond.

In a specific embodiment,



represents single bond; in another specific embodiment,



represents double bond.

$Z_1$

In a specific embodiment, $Z_1$ is O atom; in another specific embodiment, $Z_1$ is S atom; in another specific embodiment, $Z_1$ is N atom; in another specific embodiment, $Z_1$ is C atom; in another specific embodiment, $Z_1$ is substituted with one Rzi; in another specific embodiment, $Z_1$ is substituted with two Rzi; in another specific embodiment, $Z_1$ is absent.

$Z_2$

In a specific embodiment, $Z_2$ is O atom; in another specific embodiment, $Z_2$ is S atom; in another specific embodiment, $Z_2$ is N atom; in another specific embodiment, $Z_2$ is C atom; in another specific embodiment, $Z_2$ is substituted with one $R_{Z2}$; in another specific embodiment, $Z_2$ is substituted with two $R_{Z2}$.

$Z_3$

In a specific embodiment, $Z_3$ is O atom; in another specific embodiment, $Z_3$ is S atom; in another specific embodiment, $Z_3$ is N atom; in another specific embodiment, $Z_3$ is C atom; in another specific embodiment, $Z_3$ is substituted with one $R_{Z3}$; in another specific embodiment, $Z_3$ is substituted with two $R_{Z3}$.
In a specific embodiment, $Z_1$, $Z_2$ and $Z_3$ are all absent.

$Z_4$

In a specific embodiment, $Z_4$ is N; in another specific embodiment, $Z_4$ is $CR_{Z4}$.

$Z_5$

In a specific embodiment, $Z_5$ is N; in another specific embodiment, $Z_5$ is CRzs.

$R_a$, $R_b$ and $R_c$

In a specific embodiment, $R_a$ is H; in another specific embodiment, $R_a$ is halogen; in another specific embodiment, $R_a$ is OR'; in another specific embodiment, $R_a$ is NR'R"; in another specific embodiment, $R_a$ is $C_{1-6}$ alkyl; in another specific embodiment, $R_a$ is $C_{1-6}$ haloalkyl.
In a specific embodiment, $R_b$ is H; in another specific embodiment, $R_b$ is halogen; in another specific embodiment, $R_b$ is OR'; in another specific embodiment, $R_b$ is NR'R"; in another specific embodiment, $R_b$ is $C_{1-6}$ alkyl; in another specific embodiment, $R_b$ is $C_{1-6}$ haloalkyl.
In a specific embodiment, $R_c$ is H; in another specific embodiment, $R_c$ is halogen; in another specific embodiment, $R_c$ is OR'; in another specific embodiment, $R_c$ is NR'R"; in another specific embodiment, $R_c$ is $C_{1-6}$ alkyl; in another specific embodiment, $R_c$ is $C_{1-6}$ haloalkyl.
In a specific embodiment, $R_a$ and $R_b$ are taken together with the carbon atom to which they are attached to form C=O; in another specific embodiment, $R_a$ and $R_b$ are taken together with the cabin atom to which they are attached to form $C_{3-7}$ cycloalkyl; in another specific embodiment, $R_a$ and $R_b$ are taken together with the carbon atom to which they are attached to form 4- to 8-membered heterocyclyl; in another specific embodiment, $R_a$ and $R_c$ are taken together to form bond; in another specific embodiment, $R_a$ and $R_c$ are taken together with the cabin atoms to which they are attached to form $C_{3-7}$ cycloalkyl or 4- to 8-membered heterocyclyl.

$R_{Z1}$

In a specific embodiment, Rzi is absent; in another specific embodiment, Rzi is H; in another specific embodiment, Rzi is CN; in another specific embodiment, Rzi is halogen; in another specific embodiment, Rzi is $-(CH_2)_{0-5}$-OR'; in another specific embodiment, Rzi is -
**[0083]** $(CH_2)_{0-5}$-NR'R"; in another specific embodiment, Rzi is $C_{1-6}$ alkyl; in another specific embodiment, Rzi is $C_{1-6}$ haloalkyl; in another specific embodiment, Rzi is $-(CH_2)_{0-5}-C_{3-7}$ cycloalkyl; in another specific embodiment, Rzi is $-(CH_2)_{0-5}$-4- to 8-membered heterocyclyl; in another specific embodiment, two Rzi are taken together with $Z_1$ to form C=O; in another specific embodiment, two Rzi are taken together with $Z_1$ to form $C_{3-7}$ cycloalkyl; in another specific embodiment, two Rzi are taken together with $Z_1$ to form 4- to 8-membered heterocyclyl.

$R_{Z2}$

In a specific embodiment, $R_{Z2}$ is absent; in another specific embodiment, $R_{Z2}$ is H; in another specific embodiment, $R_{Z2}$ is CN; in another specific embodiment, $R_{Z2}$ is halogen; in another specific embodiment, $R_{Z2}$ is $-(CH_2)_{0-5}$-OR'; in another specific embodiment, $R_{Z2}$ is - $(CH2)_{0-5}$-NR'R"; in another specific embodiment, $R_{Z2}$ is $C_{1-6}$ alkyl; in another specific embodiment, $R_{Z2}$ is $C_{1-6}$ haloalkyl; in another specific embodiment, $R_{Z2}$ is $-(CH2)_{0-5}-C_{3-7}$ cycloalkyl; in another specific embodiment, $R_{Z2}$ is $-(CH_2)_{0-5}$-4- to 8-membered heterocyclyl; in another specific embodiment, two $R_{Z2}$ are taken together with $Z_2$ to form C=O; in another specific embodiment, two $R_{Z2}$ are taken together with $Z_2$ to form $C_{3-7}$ cycloalkyl;

in another specific embodiment, two $R_{Z2}$ are taken together with $Z_2$ to form 4- to 8-membered heterocyclyl.

Rz3

In a specific embodiment, $R_{Z3}$ is absent; in another specific embodiment, $R_{Z3}$ is H; in another specific embodiment, $R_{Z3}$ is CN; in another specific embodiment, $R_{Z3}$ is halogen; in another specific embodiment, $R_{Z3}$ is $-(CH_2)_{0-5}$-OR'; in another specific embodiment, $R_{Z3}$ is - $(CH_2)_{0-5}$-NR'R"; in another specific embodiment, $R_{Z3}$ is $C_{1-6}$ alkyl; in another specific embodiment, Rz3 is $C_{1-6}$ haloalkyl; in another specific embodiment, Rz3 is $-(CH_2)_{0-5}-C_{3-7}$ cycloalkyl; in another specific embodiment, Rz3 is $-(CH_2)_{0-5}$-4- to 8-membered heterocyclyl; in another specific embodiment, two $R_{Z3}$ are taken together with $Z_3$ to form C=O; in another specific embodiment, two $R_{Z3}$ are taken together with $Z_3$ to form $C_{3-7}$ cycloalkyl; in another specific embodiment, two $R_{Z3}$ are taken together with $Z_3$ to form 4- to 8-membered heterocyclyl.

$R_{Z4}$

In a specific embodiment, $R_{Z4}$ is H; in another specific embodiment, $R_{Z4}$ is CN; in another specific embodiment, $R_{Z4}$ is halogen; in another specific embodiment, $R_{Z4}$ is $-(CH_2)_{0-5}$-OR'; in another specific embodiment, $R_{Z4}$ is $-(CH_2)_{0-5}$-NR'R"; in another specific embodiment, $R_{Z4}$ is $C_{1-6}$ alkyl; in another specific embodiment, $R_{Z4}$ is $C_{1-6}$ haloalkyl.

$R_{Z5}$

In a specific embodiment, Rzs is H; in another specific embodiment, $R_{Z5}$ is CN; in another specific embodiment, Rzs is halogen; in another specific embodiment, $R_{Z5}$ is $-(CH_2)_{0-5}$-OR'; in another specific embodiment, Rzs is $-(CH_2)_{0-5}$-NR'R"; in another specific embodiment, Rzs is $C_{1-6}$ alkyl; in another specific embodiment, Rzs is $C_{1-6}$ haloalkyl; in another specific embodiment, Rzs is $-(CH_2)_{0-5}-C_{3-7}$ cycloalkyl; in another specific embodiment, Rzs is $-(CH_2)_{0-5}$-4- to 8-membered heterocyclyl.

In a specific embodiment, the ring in which $Z_4$ is located is absent.

$L_1$

In a specific embodiment, $L_1$ is bond; in another specific embodiment, $L_1$ is -O-; in another specific embodiment, $L_1$ is $-S(O)_p-$; in another specific embodiment, $L_1$ is - S(O)(=NR*)-; in another specific embodiment, $L_1$ is $-NR^{\#}-$; in another specific embodiment, $L_1$ is $-CR^{\#}R^{\#'}-$; in another specific embodiment, $L_1$ is $-C_aR^{\#}R^{\#'}-C_bR^{\#}R^{\#'}-$; in another specific embodiment, $L_1$ is -N=S(O)(R*)-; in another specific embodiment, $L_1$ is -S(O)(R*)=N-.

$L_2$

In a specific embodiment, $L_2$ is bond; in another specific embodiment, $L_2$ is -O-; in another specific embodiment, $L_2$ is $-S(O)_p-$; in another specific embodiment, $L_2$ is - S(O)(=NR*)-; in another specific embodiment, $L_2$ is $-NR^{\#}-$; in another specific embodiment, $L_2$ is $-CR^{\#}R^{\#'}-$; in another specific embodiment, $L_2$ is $-C_aR^{\#}R^{\#'}-C_bR^{\#}R^{\#'}-$; in another specific embodiment, $L_2$ is -N=S(O)(R*)-; in another specific embodiment, $L_2$ is -S(O)(R*)=N-.

In another specific embodiment, one of $C_aR^{\#}R^{\#'}$ and $C_bR^{\#}R^{\#'}$ in $L_1$ or $L_2$ can be replaced by O, $S(O)_p$, S(O)(=NR*) or $NR^{\#}$, and when one of $C_aR^{\#}R^{\#'}$ and $C_bR^{\#}R^{\#''}$ is replaced by O, S or $NR^{\#}$, the other of $C_aR^{\#}R^{\#'}$ and $C_bR^{\#}R^{\#'}$ can also be replaced by $S(O)_q$;

E

In a specific embodiment, E is bond; in another specific embodiment, E is $-C_cR^{\#}R^{\#'}-C_dR^{\#}R^{\#'}-C_eR^{\#}R^{\#'}$; in another specific embodiment, E is

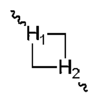;

in another specific embodiment, E is

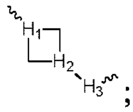

in another specific embodiment, E is

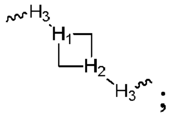

in another specific embodiment, E is

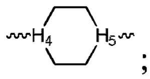

in another specific embodiment, E is

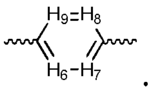

In another specific embodiment, one of $C_cR^{\#}R^{\#'}$, $C_dR^{\#}R^{\#'}$ or $C_eR^{\#}R^{\#'}$, or both of $C_cR^{\#}R^{\#'}$ and $C_eR^{\#}R^{\#'}$ can be replaced by O, $S(O)_p$, S(O)(=NR*) or $NR^{\#}$, and when one of $C_cR^{\#}R^{\#'}$, $C_dR^{\#}R^{\#'}$ or $C_eR^{\#}R^{\#'}$ is replaced by O, S or NR#, the other adjacent one or two of $C_cR^{\#}R^{\#'}$, $C_dR^{\#}R^{\#'}$ or $C_eR^{\#}R^{\#'}$ can also be replaced by $S(O)_q$;

In another specific embodiment, two E moieties can be taken together to form - $CH_2CH_2OCH_2CH_2$-; in another specific embodiment, two E moieties can be taken together to form -$OCH_2CH_2CH_2CH_2$-; in another specific embodiment, two E moieties can be taken together to form -$CH_2CH_2CH_2CH_2O$-; in another specific embodiment, two E moieties can be taken together to form

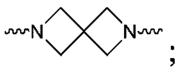

in another specific embodiment, two E moieties can be taken together to form

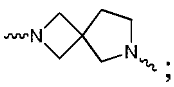

in another specific embodiment, two E moieties can be taken together to form

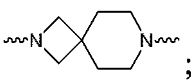

in another specific embodiment, two E moieties can be taken together to form

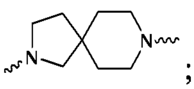

in another specific embodiment, two E moieties can be taken together to form

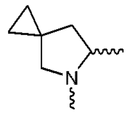;

in another specific embodiment, two E moieties can be taken together to form

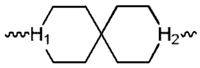.

In another specific embodiment, in the embodiment of $L_1$, $L_2$ or E, $R^\#$ and $R^\#$ on adjacent atoms can be taken together to form bond, and $R^{\#'}$ and $R^{\#'}$ on adjacent atoms can be taken together to form bond;

In another specific embodiment, in the embodiment of $L_1$, $L_2$ or E, $R^\#$ and $R^{\#'}$ on the same atom can be taken together to form =O, or $C_{3-7}$ cycloalkyl, 4- to 8-membered heterocyclyl, $C_{6-10}$ aryl or 5- to 6-membered heteroaryl, each of which is optionally substituted with $R_x$ group; in another specific embodiment, in the embodiment of $L_1$, $L_2$ or E, $R^\#$ and $R^{\#'}$ on different atoms can be taken together to form $C_{3-7}$ cycloalkyl, 4- to 8-membered heterocyclyl, $C_{6-10}$ aryl or 5- to 6-membered heteroaryl, each of which is optionally substituted with $R_x$ group.

m

In a specific embodiment, m is 0; in another specific embodiment, m is 1; in another specific embodiment, m is 2; in another specific embodiment, m is 3; in another specific embodiment, m is 4; in another specific embodiment, m is 5; in another specific embodiment, m is 6; in another specific embodiment, m is 7; in another specific embodiment, m is 8; in another specific embodiment, m is 9; in another specific embodiment, m is 10.

$R_1$

In a specific embodiment, $R_1$ is H; in another specific embodiment, $R_1$ is halogen; in another specific embodiment, $R_1$ is cyano; in another specific embodiment, $R_1$ is $C_{1-6}$ alkyl; in another specific embodiment, $R_1$ is $C_{3-7}$ cycloalkyl; in another specific embodiment, $R_1$ is 4-to 8-membered heterocyclyl; in another specific embodiment, $R_1$ is $C_{1-6}$ haloalkyl; in another specific embodiment, $R_1$ is $C_{2-6}$ alkenyl; in another specific embodiment, $R_1$ is $C_{2-6}$ alkynyl. In another specific embodiment, when L is -NR-, $R_1$ and R are taken together with the atoms to which they are attached to form optionally substituted 5- to 6-membered heterocyclyl, wherein the substituent is selected from halogen, oxo, $C_{1-6}$ alkyl, $C_{1-6}$ haloalkyl, $C_{6-10}$ aryl, wherein the $C_{6-10}$ aryl is mono- or poly-substituted with halogen; in another specific embodiment, $R_1$ and R are taken together to form -C(O)N($R_N$)C(O)-; in another specific embodiment, $R_1$ and R are taken together to form -C($C_{1-6}$ alkyl)=C($R_N$)C(O)-.

$R_2$

In a specific embodiment, $R_2$ is H; in another specific embodiment, $R_2$ is halogen; in another specific embodiment, $R_2$ is hydroxyl; in another specific embodiment, $R_2$ is amino; in another specific embodiment, $R_2$ is $C_{1-6}$ alkyl; in another specific embodiment, $R_2$ is $C_{1-6}$ haloalkyl; in another specific embodiment, $R_1$ and $R_2$ are taken together with the atoms to which they are attached to form 5- to 6-membered heterocyclyl; in another specific embodiment, $R_1$ and $R_2$ are taken together with the atoms to which they are attached to form 5- to 6-membered heteroaryl; in another specific embodiment, $R_1$ and $R_2$ are taken together with the atoms to which they are attached to form pyrrolyl.

$R_3$

In a specific embodiment, $R_3$ is H; in another specific embodiment, $R_3$ is H-O-$C_{1-6}$ alkyl; in another specific embodiment, $R_3$ is -O-$C_{1-6}$ haloalkyl.

$R_4$

In a specific embodiment, $R_4$ is H; in another specific embodiment, $R_4$ is halogen; in another specific embodiment, $R_4$ is $C_{1-6}$ alkyl; in another specific embodiment, $R_4$ is $C_{1-6}$ haloalkyl; in another specific embodiment, $R_4$ is -NHC(O)-$C_{1-6}$ alkyl; in another specific embodiment, $R_4$ is -NHC(O)-$C_{2-6}$ alkenyl.

Any technical solution in any one of the above specific embodiments, or any combination thereof, may be combined with any technical solution in other specific embodiments or any combination thereof. For example, any technical solution of ring A or any combination thereof may be combined with any technical solution of L, Y, $Z_1$-$Z_5$, R', T, $L_1$, E, m, $L_2$, $R_1$-$R_4$ or any combination thereof. The present disclosure is intended to include all combination of such technical solutions, which are not exhaustively listed here to save space.

In a more specific embodiment, the present disclosure provides a compound of formula (I) or (I-G), or a pharmaceutically acceptable salt, enantiomer, diastereomer, racemate, solvate, hydrate, polymorph, prodrug, or isotopic variant thereof, or mixture thereof, wherein

ring A is selected from the following optionally substituted groups: $C_{3-7}$ cycloalkyl, 4- to 8-membered heterocyclyl, $C_{6-10}$ aryl or 5- to 10-membered heteroaryl, wherein the substituent is selected from -F, -Cl, -Br, -Me, -OMe, $-CF_3$, $-OCF_3$, -CN, -NHMe, cyclopropyl, $-P(O)Me_2$, $-NHC(O)CH_2CH_3$, $-C(O)CH=CH_2$, $-NHS(O)_2CH_2CH_3$, -NH-cyclopropyl, $-NHC(O)CH=CH_2$ or $-C(O)CH_2CH_3$; ring A is alternatively the following groups:

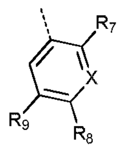
,

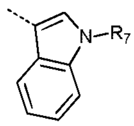
or
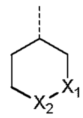
;

wherein

$R_7$ is selected from -Me, cyclopropyl or $-P(O)Me_2$;
$R_8$ is selected from H, -NHMe, $-NHC(O)CH_2CH_3$ or -NH-cyclopropyl;
$R_9$ is selected from H, -F, -Cl, -Br, -Me, $-CF_3$, -OMe, $-OCF_3$, -CN, $-NHC(O)CH_2CH_3$, $-NHS(O)_2CH_2CH_3$, $-NHC(O)CH=CH_2$ or -NH-cyclopropyl;
X is $-C(R_x)=$ or -N=;
wherein $R_x$ is H, or $R_x$ and $R_8$ are taken together with the C atoms to which they are attached to form 5- to 6-membered heterocyclyl or 5- to 6-membered heteroaryl; alternatively 5- to 6-membered heteroaryl (alternatively pyrazinyl);
$X_1$ is $-CH_2-$ or $-N(R_{X1})-$;
$X_2$ is $-CH(R_{X2})-$ or $-N(R_{X2})-$;
wherein Rxi is $-C(O)CH_2CH_3$ or $-C(O)CH=CH_2$; $R_{X2}$ is H, -Me, -OMe, -NHMe, $-C(O)CH_2CH_3$, $-NHS(O)_2CH_2CH_3$ or $-NHC(O)CH_2CH_3$;
----- represents the point of attachment to L;
T is selected from bond, $C_{2-6}$ heteroalkylene, 4- to 12-membered heterocyclylene, or 5- to 6-membered heterocyclylene, wherein the 5- to 6-membered heterocyclylene is substituted with 5- to 6-membered heterocyclylene; or is alternatively the following groups:

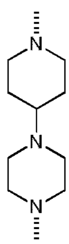
,
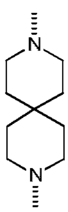
,
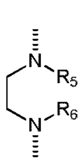
,
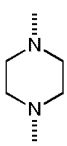
,
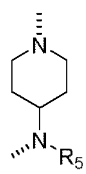
or
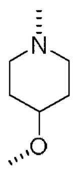
;

wherein

$R_5$ is -Me;
$R_6$ is -Me;
or $R_5$ and $R_6$ are connected to form $-CH_2CH_2-$;

represents the point of attachment to parent core or $L_2$;

$R_1$ is selected from H, -Cl, -Br, $-CH_3$, $-CF_3$, cyclopropyl or $-CH=CH_2$;

or, when L is -NR-, $R_1$ and R are taken together with the atoms to which they are attached to form optionally substituted 5- to 6-membered heterocyclyl, wherein the substituent is selected from halogen, oxo, -iPr, -Et, or phenyl, wherein the phenyl is mono- or poly-substituted with halogen; $R_1$ and R are alternatively taken together to form the following groups: $-C(O)N(R_N)C(O)-$ or $-C(CH_3)=C(R_N)C(O)-$;

wherein

$R_N$ is selected from -iPr, -Et or

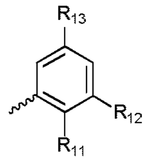 ;

$R_{11}$ is -Cl, -Br;

$R_{12}$ is H or -F;

$R_{13}$ is H or -F;



represents the point of attachment;

$R_2$ is H;

or $R_1$ and $R_2$ are taken together with the atoms to which they are attached to form 5-to 6-membered heterocyclyl or 5- to 6-membered heteroaryl; alternatively form pyrrolyl;

$R_3$ is selected from H or -OMe;

$R_4$ is selected from H, -F, -Me, $-CF_3$ or $-NHC(O)CH=CH_2$;

other groups are as defined above;

In an embodiment of the compound of formula (I) or (I-G), $L_1$ is selected from bond, -O-, -NH-, $-CH_2-$, -C(O)-, $-CH_2CH_2-$, -CH=CH-, -C=C-, $-OCH_2-$, $-CH_2O-$, $-NHCH_2-$, - $CH_2NH-$, $-C(O)CH_2-$, $-CH_2C(O)-$, -OC(O)-, -C(O)O-, -NHC(O)- or -C(O)NH-;

E is independently selected from $-CH_2CH_2CH_2-$, $-CH_2CH_2C(O)-$, $-CH_2C(O)CH_2-$, - $C(O)CH_2CH_2-$, -C(O)CH=CH-, -C(O)C≡C-, $-CH_2CH_2O-$, $-CH_2OCH_2-$, $-OCH_2CH_2-$, - $C(O)CH_2O-$, $-OCH_2C(O)-$, $-CH_2C(O)O-$, $-OC(O)CH_2-$, $-C(O)OCH_2-$, $-CH_2OC(O)-$, - $CH_2CH_2NH-$, $-CH_2NHCH_2-$, $-NHCH_2CH_2-$, $-C(O)CH_2NH-$, $-NHCH_2C(O)-$, $-CH_2C(O)NH-$, - $NHC(O)CH_2-$, $-C(O)NHCH_2-$, $-CH_2NHC(O)-$,

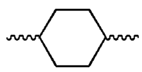 , 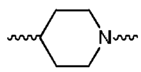 , 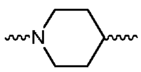 ,

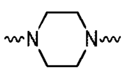 , 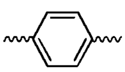 , 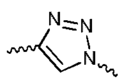 , 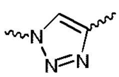 , 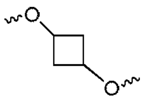 , 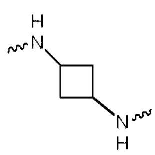 , 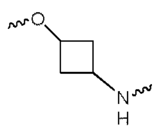 ,

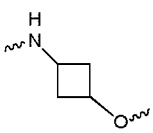 , 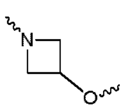 , 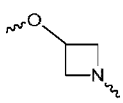 , 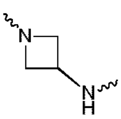 or 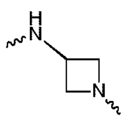 ;

m is 0, 1, 2, 3, 4, 5, 6, 7, 8, 9 or 10;
$L_2$ is selected from bond, $-CH_2-$, $-CH_2CH_2-$, $-OCH_2-$, $-NHCH_2-$, -OC(O)-, -NHC(O)-, $-CH_2C(O)-$ or $-C(O)CH_2-$; wherein



represents the point of attachment to $L_1$ or $L_2$.

In a more specific embodiment, the present disclosure provides a compound of formula (I), or a pharmaceutically acceptable salt, enantiomer, diastereomer, racemate, solvate, hydrate, polymorph, prodrug, or isotopic variant thereof, or mixture thereof, wherein the compound of formula (I) has the structure of the following formulas:

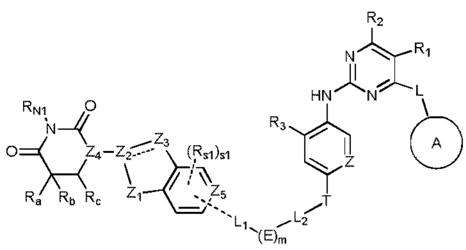

(I-A),

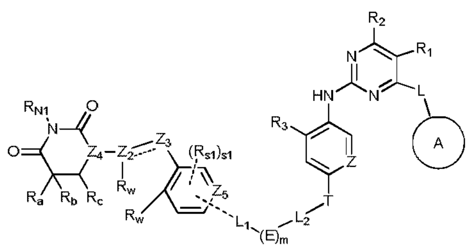

(I-B),

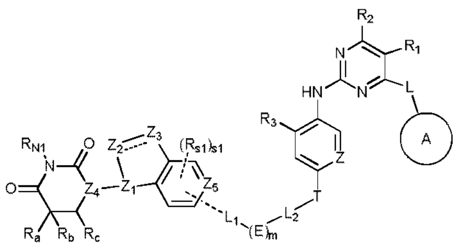

(I-C),

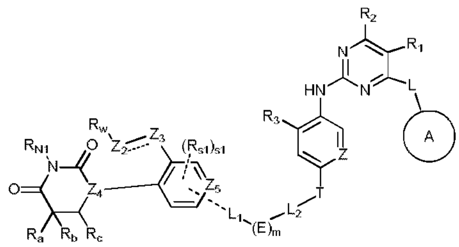

(I-D),

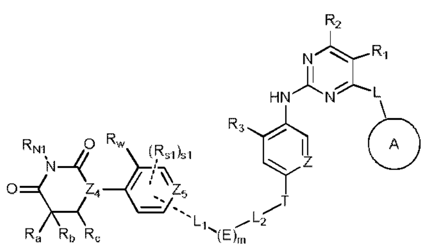

(I-E),

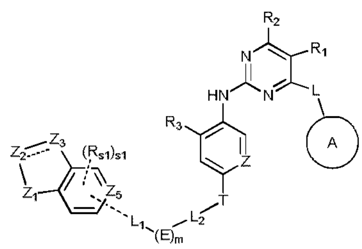

(I-F),

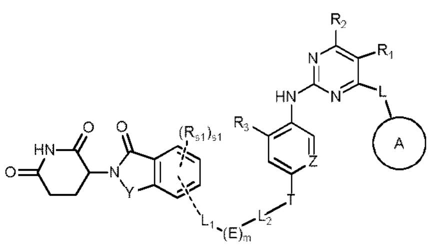

(I-G),

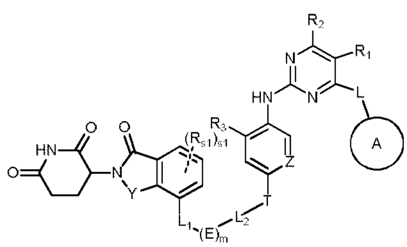

(I-H),

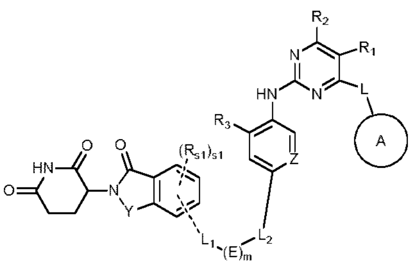

(I-I),

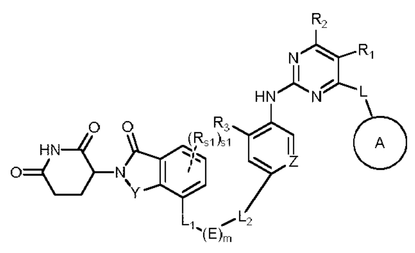

(I-J),

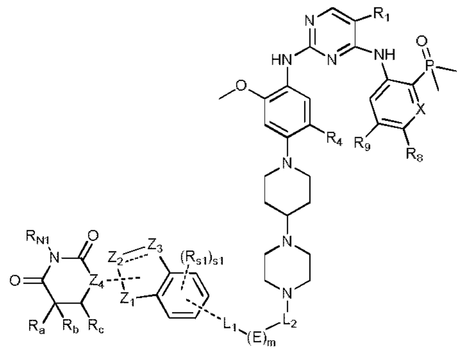

(I-1),

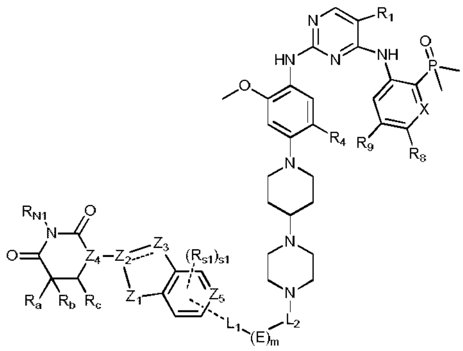

(I-1-A),

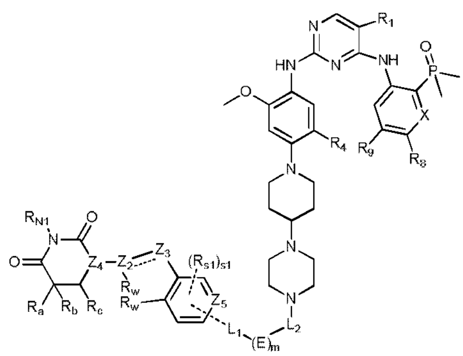

(I-1-B),

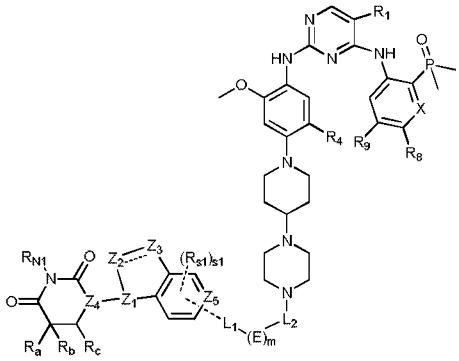

(I-1-C),

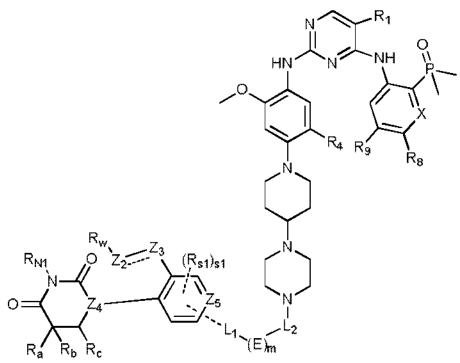

(I-1-D),

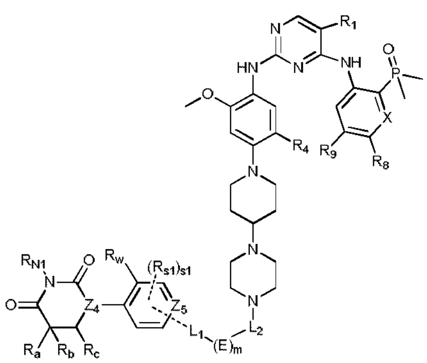

(I-1-E),

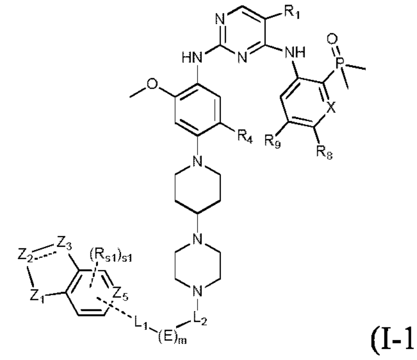 (I-1-F),

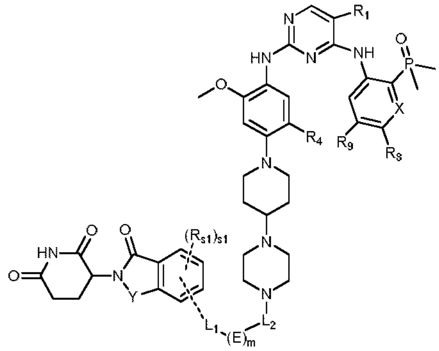 (I-1-G),

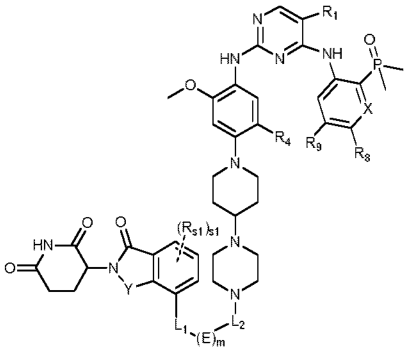 (I-1-H),

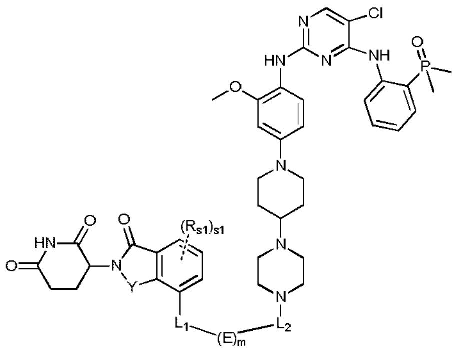 (I-1-H'),

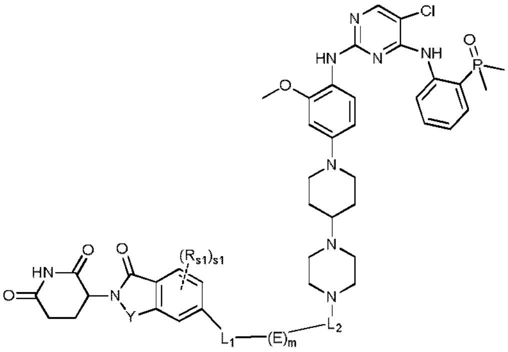 (I-1-H''),

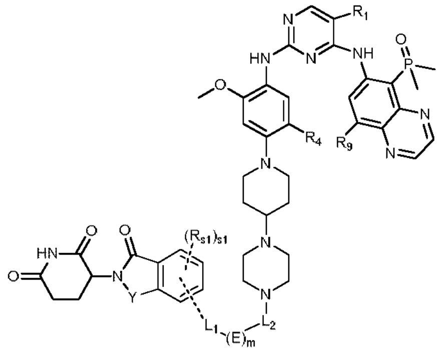 (I-1-I),

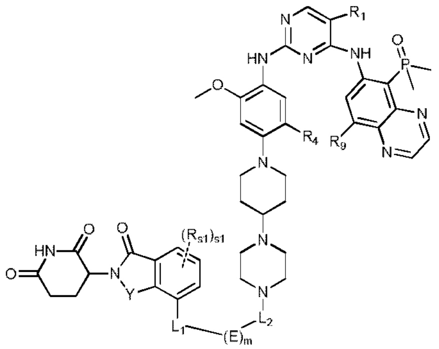 (I-1-I'),

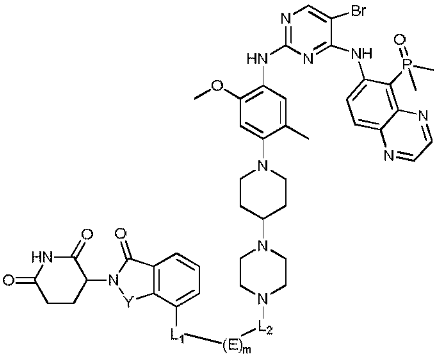 (I-1-I''),

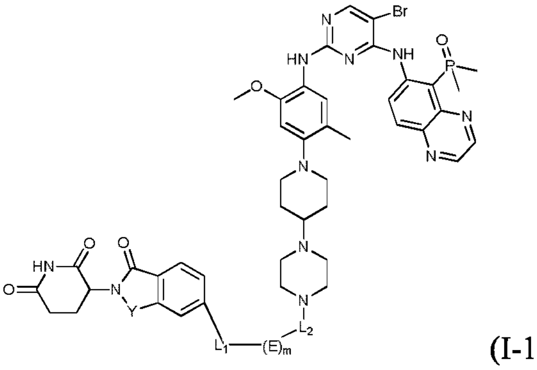

(I-1-I'''),

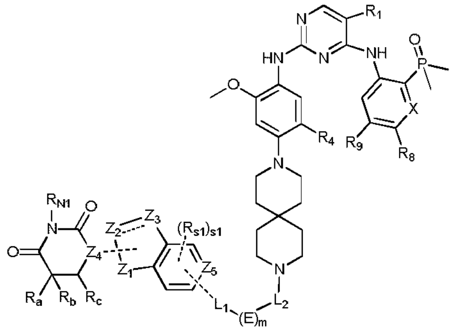

(I-2),

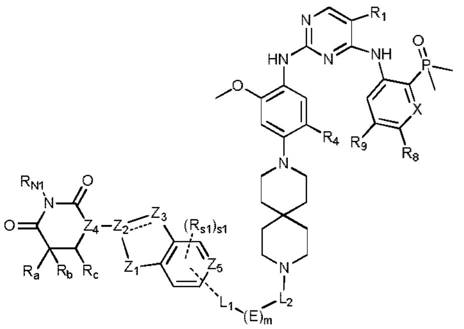

(I-2-A),

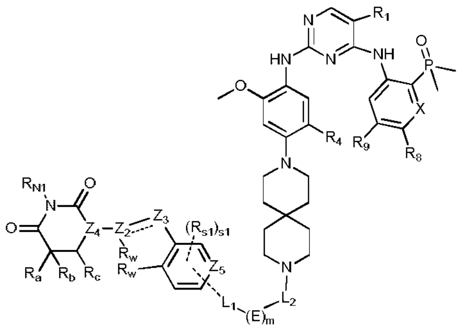

(I-2-B),

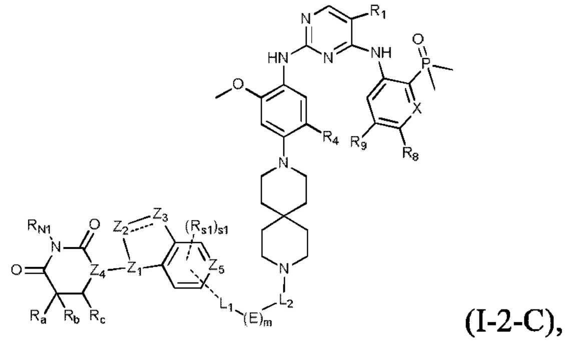

(I-2-C),

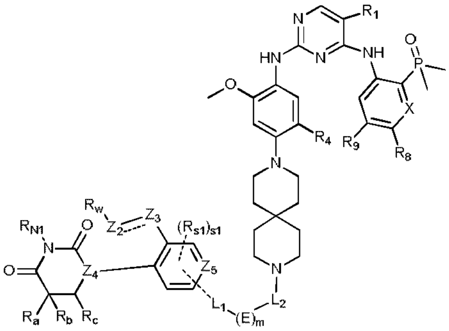

(I-2-D),

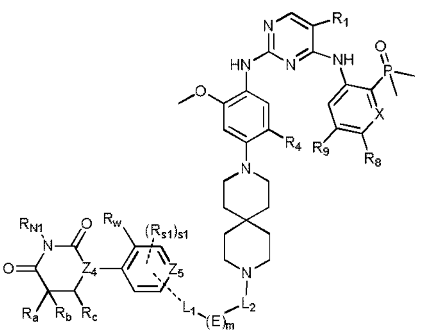

(I-2-E),

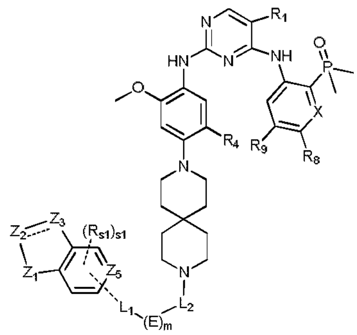

(I-2-F),

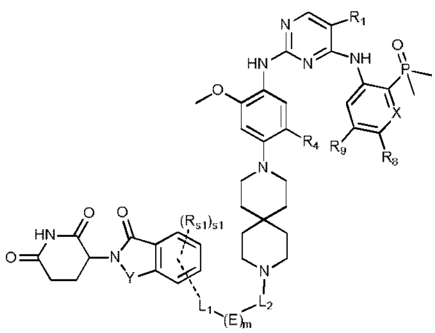
(I-2-G),

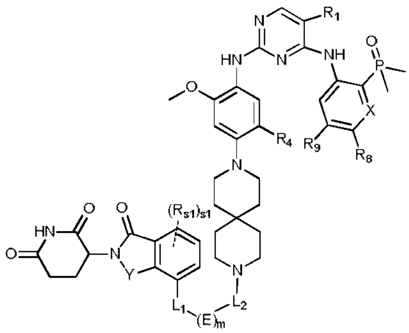
(I-2-H),

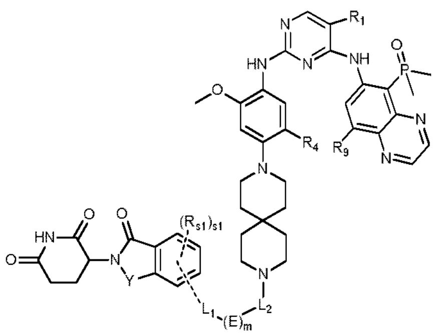
(I-2-I),

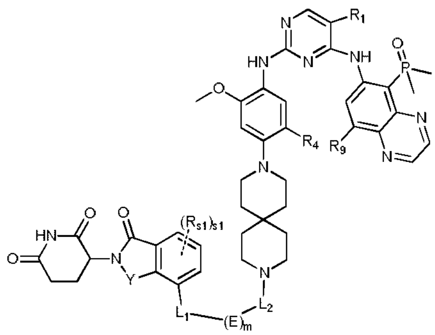
(I-2-I'),

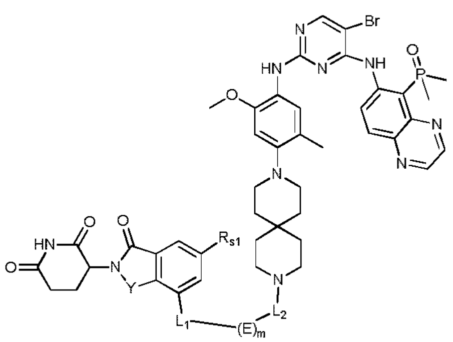
(I-2-I''),

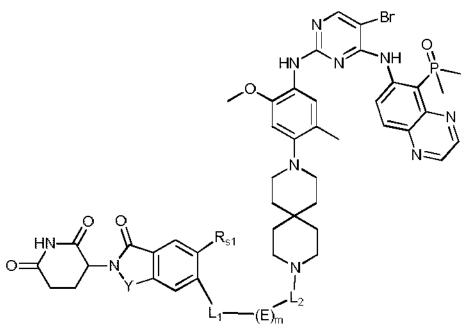
(I-2-I'''),

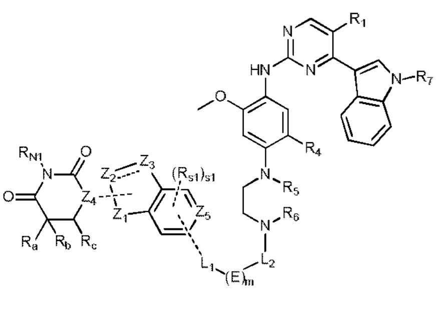
(I-3),

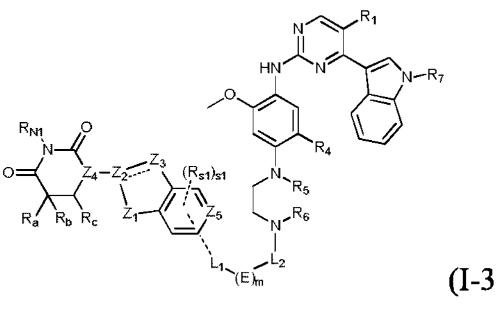
(I-3-A),

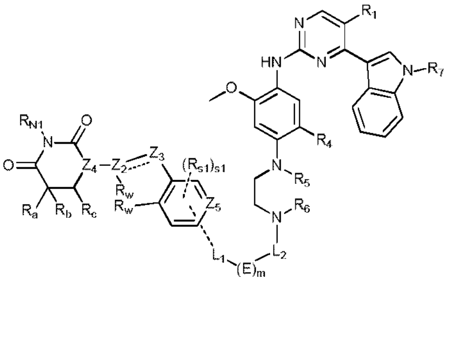
(I-3-B),

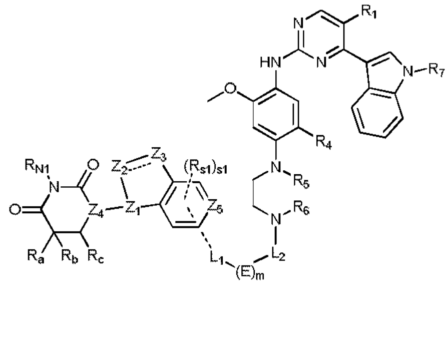
(I-3-C),

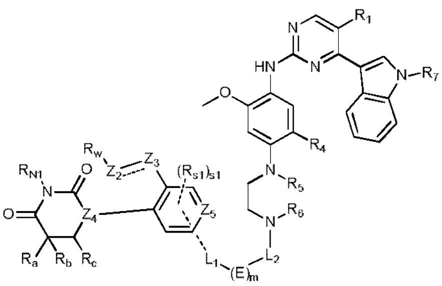

(I-3-D),

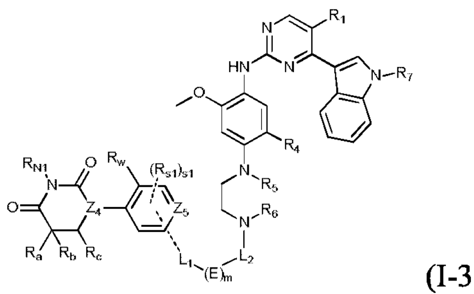

(I-3-E),

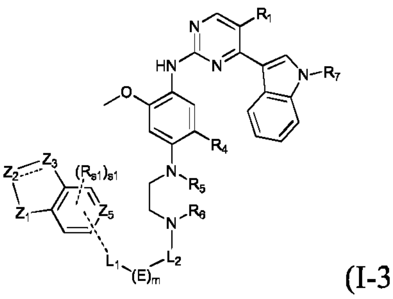

(I-3-F),

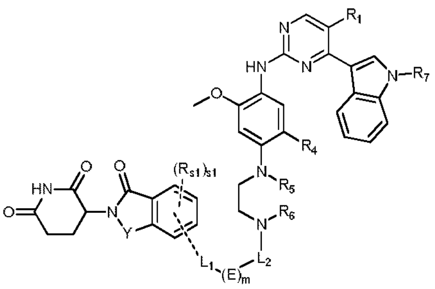

(I-3-G),

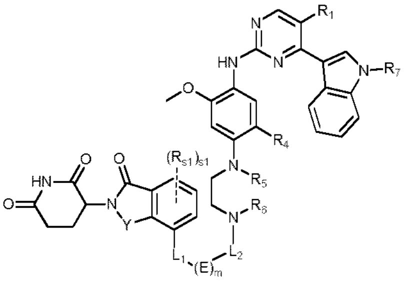

(I-3-H),

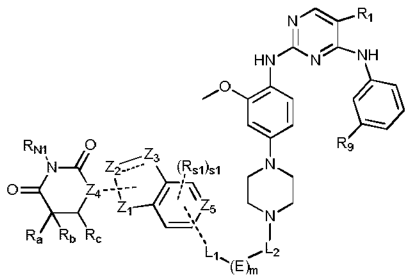

(I-4),

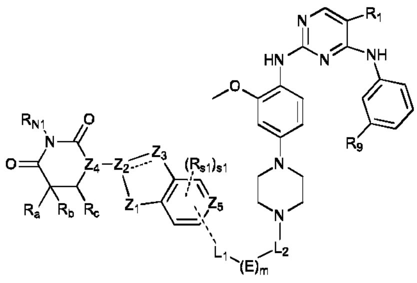

(I-4-A),

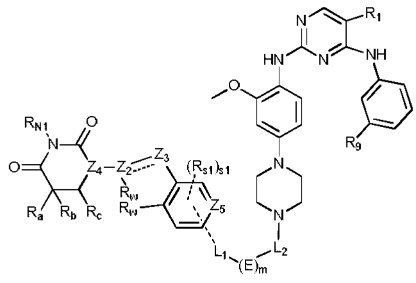

(I-4-B),

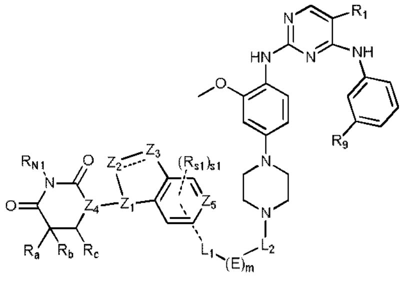

(I-4-C),

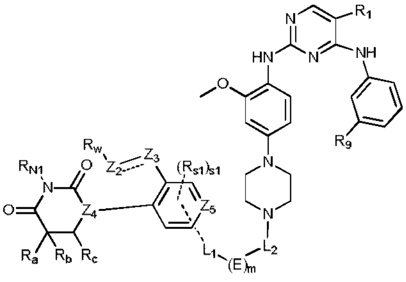

(I-4-D),

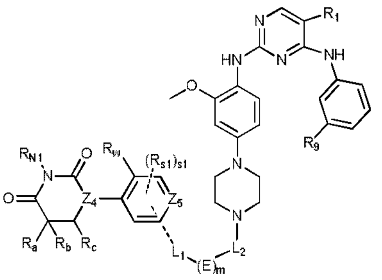
(I-4-E),

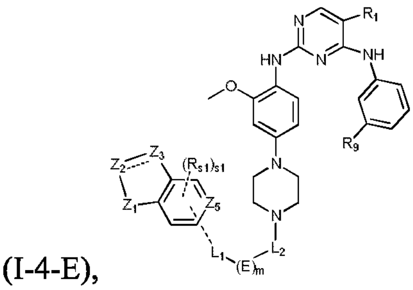
(I-4-F),

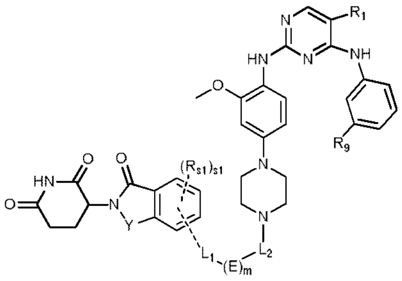
(I-4-G),

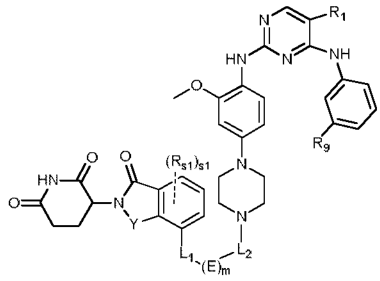
(I-4-H),

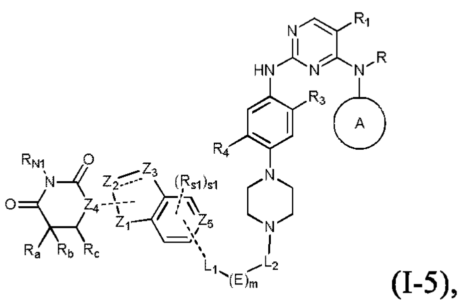
(I-5),

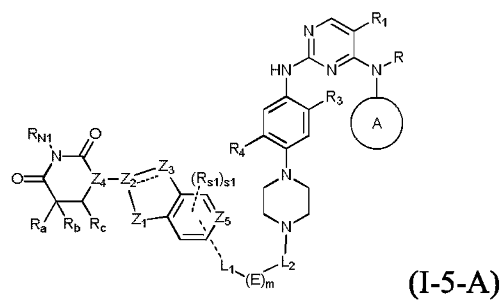
(I-5-A),

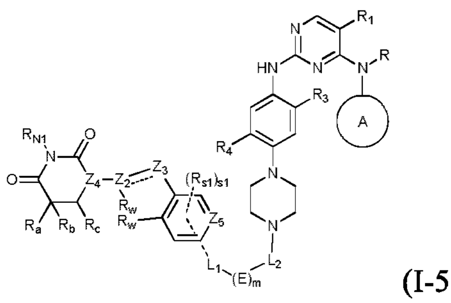
(I-5-B),

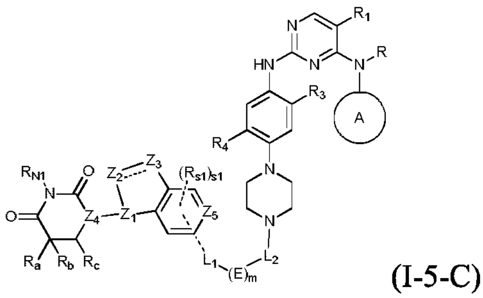
(I-5-C),

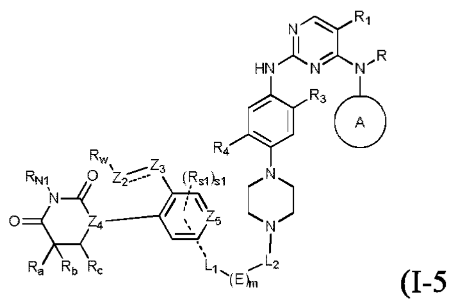
(I-5-D),

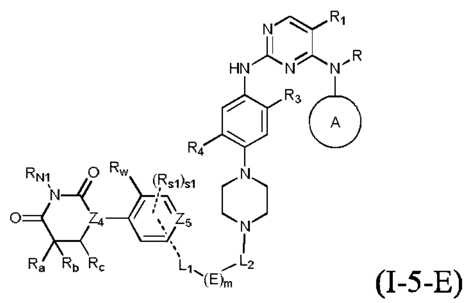
(I-5-E),

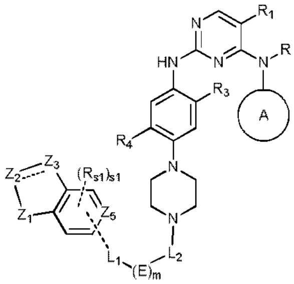 (I-5-F),

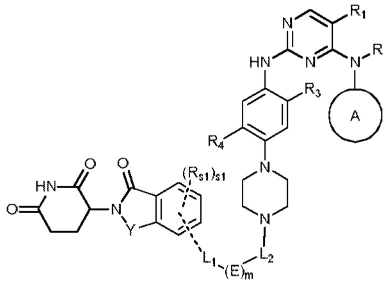 (I-5-G),

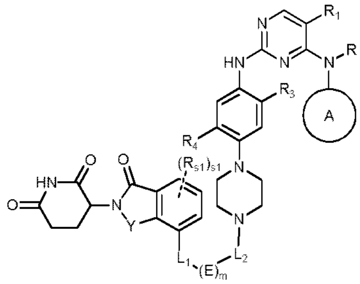 (I-5-H),

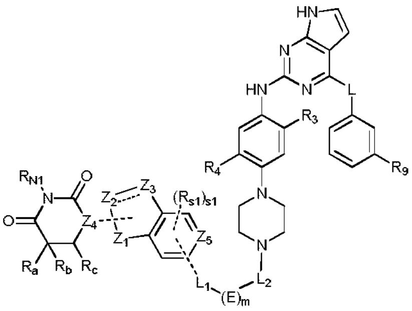 (I-6),

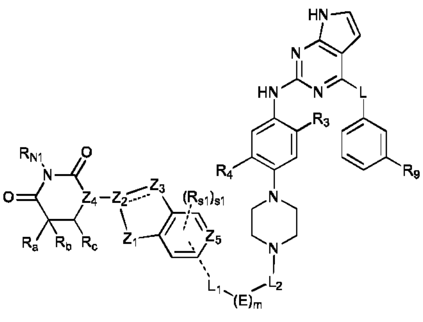 (I-6-A),

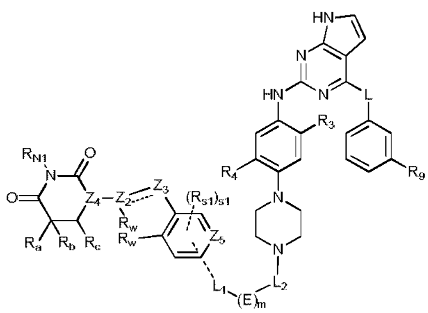 (I-6-B),

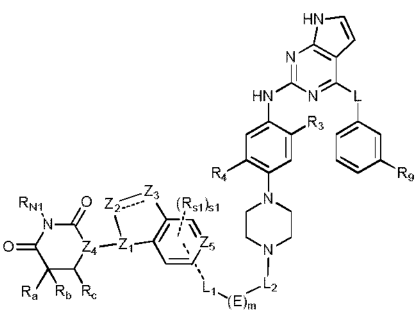 (I-6-C),

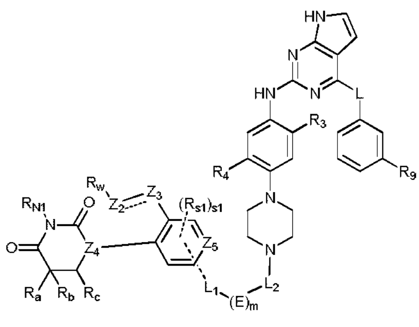 (I-6-D),

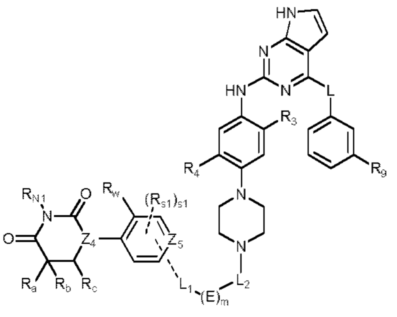 (I-6-E),

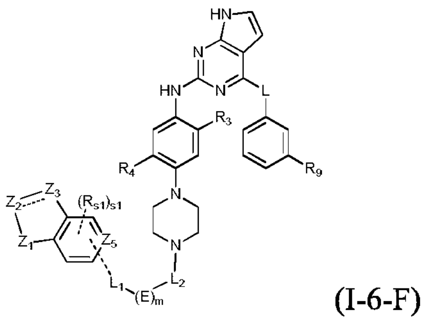 (I-6-F),

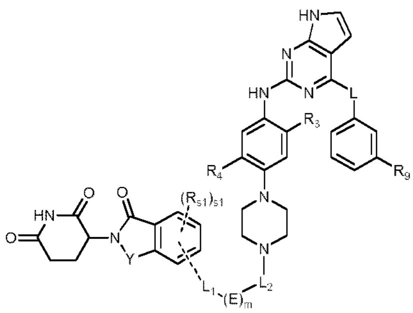

(I-6-G),

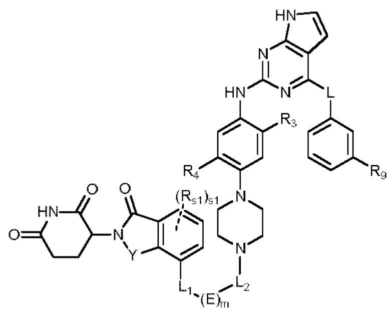

(I-6-H),

wherein each group is as defined in above.

In a more specific embodiment, the present disclosure provides a compound of formula (I-1), (I-1-A), (I-1-B), (I-1-C), (I-1-D), (I-1-E), (I-1-F), (I-1-G), (I-1-H) or (I-1-I), or a pharmaceutically acceptable salt, enantiomer, diastereomer, racemate, solvate, hydrate, polymorph, prodrug, or isotopic variant thereof, or mixture thereof, wherein

$R_1$ is selected from -Cl, -Br, $-CF_3$ or $-CH=CH_2$; alternatively, $R_1$ is selected from -Cl or -Br;
$R_4$ is H or -Me;
$R_8$ is H, -NHMe, $-NHC(O)CH_2CH_3$ or -NH-cyclopropyl;
$R_9$ is H, -F, -Cl, -Br, -Me, $-CF_3$, -OMe, $-OCF_3$, -CN, $-NHC(O)CH_2CH_3$, - $NHS(O)_2CH_2CH_3$ or $-NHC(O)CH=CH_2$;
X is $-C(R_x)=$ or -N=;
$R_x$ is H, or $R_x$ and $R_8$ are taken together with the C atoms to which they are attached to form pyrazinyl;
and other groups are as defined in above.

In a more specific embodiment, the present disclosure provides a compound of formula (I-G), or a pharmaceutically acceptable salt, enantiomer, diastereomer, racemate, solvate, hydrate, polymorph, prodrug, or isotopic variant thereof, or mixture thereof:

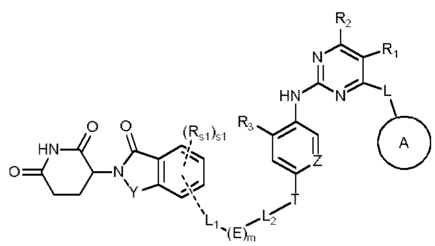

(I-G),

wherein
ring A is the following group:

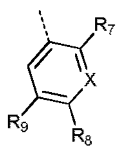

;

wherein

$R_7$ is $-P(O)(C_{1-6}\ alkyl)_2$;
$R_8$ is H;
$R_9$ is selected from H, halogen, $C_{1-6}$ alkyl, -CN or $C_{1-6}$ haloalkyl;
X is $-C(R_x)=$;
wherein $R_x$ is H, or $R_x$ and $R_8$ are taken together with the C atoms to which they are attached to form 5- to 6-membered heteroaryl; alternatively form pyrazinyl;
Y is C atom, which is optionally substituted with one or two Rzi, wherein Rzi is H, CN or halogen; or two Rzi are taken together with Y to form C=O; alternatively, Y is $CH_2$ or C=O; alternatively, Y is $CH_2$;
$L_1$ is $-C_aR^{\#}R^{\#'}-C_bR^{\#}R^{\#'}-$;

$L_2$ is selected from bond, $-CR^{\#}R^{\#'}-$ or $-C_aR^{\#}R^{\#'}-C_bR^{\#}R^{\#'}-$;
wherein one of $C_aR^{\#}R^{\#'}$ and $C_bR^{\#}R^{\#'}$ can be replaced by O, $S(O)_p$ or $NR^{\#}$;
E is independently selected from: bond or $-C_cR^{\#}R^{\#'}-C_dR^{\#}R^{\#'}-C_eR^{\#}R^{\#'}$;
wherein one of $C_cR^{\#}R^{\#'}$, $C_dR^{\#}R^{\#'}$ or $C_eR^{\#}R^{\#'}$, or both of $C_cR^{\#}R^{\#'}$ and $C_eR^{\#}R^{\#'}$ can be replaced by O, $S(O)_p$ or $NR^{\#}$;
p is 0, 1 or 2;
$R^{\#}$ is H, halogen, $C_{1-6}$ alkyl, $C_{1-6}$ haloalkyl, $C_{2-6}$ alkenyl or $C_{2-6}$ alkynyl;
$R^{\#'}$ is H, halogen, $C_{1-6}$ alkyl, $C_{1-6}$ haloalkyl, $C_{2-6}$ alkenyl or $C_{2-6}$ alkynyl;
or, $R^{\#}$ and $R^{\#}$ on adjacent atoms can be taken together to form bond, $R^{\#'}$ and $R^{\#'}$ on adjacent atoms can be taken together to form bond;
or, $R^{\#}$ and $R^{\#'}$ on the same or different atoms can be taken together to form =O;
m is 0, 1, 2, 3, 4 or 5;
$R_{s1}$ is selected from H, CN, halogen, OH, $NH_2$, $C_{2-6}$ alkenyl, $C_{2-6}$ alkynyl, $-O-C_{1-6}$ alkyl, $-O-C_{1-6}$ haloalkyl, $-NH-C_{1-6}$ alkyl, $C_{1-6}$ alkyl or $C_{1-6}$ haloalkyl;
sl is 0, 1, 2 or 3;
L is -NR-; wherein R is H or $C_{1-6}$ alkyl;
Z is $-C(R_4)=$;
T is

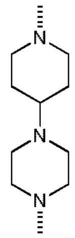 or 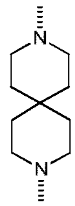 ;

$R_1$ is selected from H, halogen, cyano, $C_{1-6}$ alkyl, $C_{1-6}$ haloalkyl, $C_{2-6}$ alkenyl or $C_{2-6}$ alkynyl;
$R_2$ is H, halogen, hydroxyl, amino, $C_{1-6}$ alkyl or $C_{1-6}$ haloalkyl;
$R_3$ is selected from H, $-O-C_{1-6}$ alkyl or $-O-C_{1-6}$ haloalkyl;
$R_4$ is selected from H, halogen, $C_{1-6}$ alkyl or $C_{1-6}$ haloalkyl;
if the above groups are H or H-containing groups, the one or more H atom(s) may be substituted with D atom(s).

In a more specific embodiment, the present disclosure provides a compound of formula (I-1-G), (I-1-H), (I-1-H'), (I-1-H"), or a pharmaceutically acceptable salt, enantiomer, diastereomer, racemate, solvate, hydrate, polymorph, prodrug, or isotopic variant thereof, or mixture thereof:

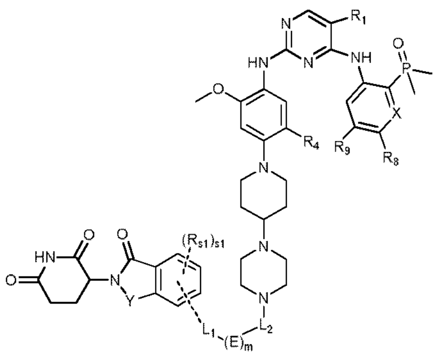 (I-1-G),

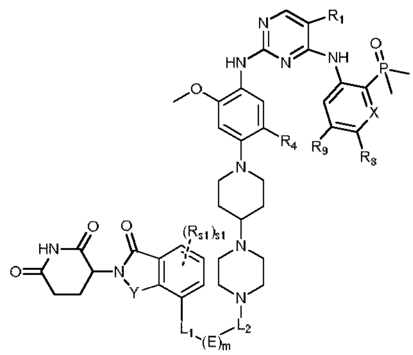 (I-1-H),

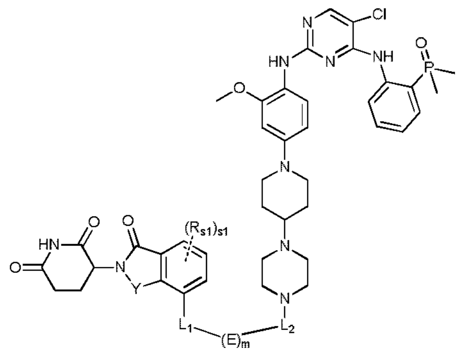

(I-1-H'),

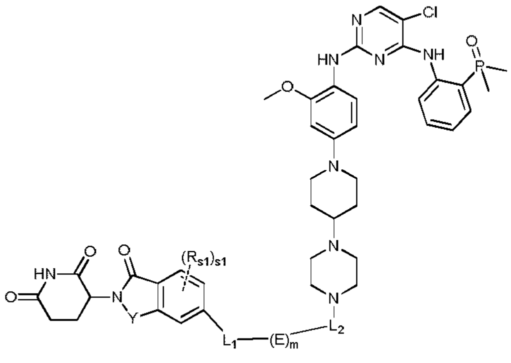

(I-1-H"),

wherein

X is -CH=;

Y is C atom, which is optionally substituted with one or two Rzi, wherein Rzi is H, CN or halogen; or two Rzi are taken together with Y to form C=O; alternatively, Y is $CH_2$ or C=O;

$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is H or halogen;

$R_4$ is selected from H, halogen, $C_{1-6}$ alkyl or $C_{1-6}$ haloalkyl; alternatively, $R_4$ is H;

$R_8$ is H;

$R_9$ is selected from H, halogen, $C_{1-6}$ alkyl, -CN or $C_{1-6}$ haloalkyl; alternatively, $R_9$ is H;

$L_1$ is selected from $-CH_2CH_2-$, -CH=CH-, -C=C-, $-OCH_2-$, $-SCH_2-$, $-S(O)CH_2-$,$-S(O)_2CH_2-$, $-NHCH_2-$, $-N(Me)CH_2-$, $-C(O)CH_2-$, $-CH_2C(O)-$, -OC(O)-, -SC(O)-, -NHC(O)-or -N(Me)C(O)-, and one or more H atom(s) in the above groups can be substituted with D atom(s); alternatively, $L_1$ is selected from $-CH_2CH_2-$, -CH=CH-, -C=C-, $-OCH_2-$ or $-NHCH_2-$; alternatively, $L_1$ is selected from $-CH_2CH_2-$, -C=C- or $-OCH_2-$;

$L_2$ is selected from bond, $-CH_2-$ or $-CH_2CH_2-$, and one or more H atom(s) in the above groups can be substituted with D atom(s);

E is $-CH_2CH_2CH_2-$, $-CH_2CH_2O-$, $-CH_2OCH_2-$, $-OCH_2CH_2-$, $-CH_2CH_2S-$, $-CH_2SCH_2-$or $-SCH_2CH_2-$, and one or more H atom(s) in the above groups can be substituted with D atom(s); alternatively, E is $-CH_2CH_2CH_2-$;

m is 0, 1, 2 or 3; alternatively, the chain length of $-L_1-(E)_m-L_2-$ is 4 to 14 bond lengths, still alternatively 5, 6, 7, 8, 9 or 10 bond lengths;

$R_{s1}$ is selected from H, CN, halogen, OH, $NH_2$, $C_{2-6}$ alkenyl, $C_{2-6}$ alkynyl, $-O-C_{1-6}$ alkyl, $-O-C_{1-6}$ haloalkyl, $-NH-C_{1-6}$ alkyl, $C_{1-6}$ alkyl or $C_{1-6}$ haloalkyl; alternatively, $R_{s1}$ is H, CN or halogen;

s1 is 0, 1 or 2.

In a more specific embodiment, the present disclosure provides a compound of formula (I-1-G), (I-1-H), (I-1-H'), (I-1-H"), or a pharmaceutically acceptable salt, enantiomer, diastereomer, racemate, solvate, hydrate, polymorph, prodrug, or isotopic variant thereof, or mixture thereof:

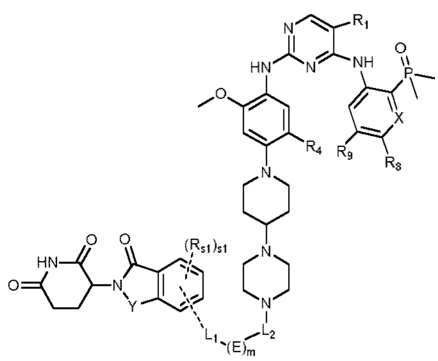

(I-1-G),

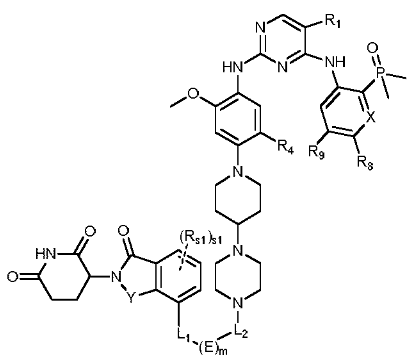

(I-1-H),

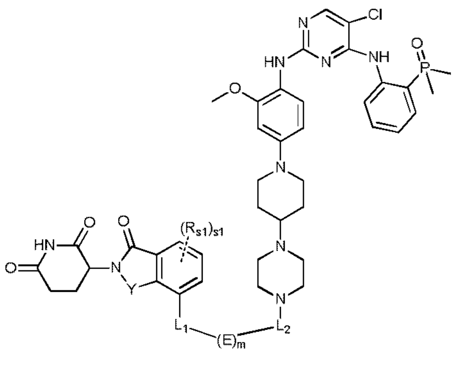

(I-1-H'),

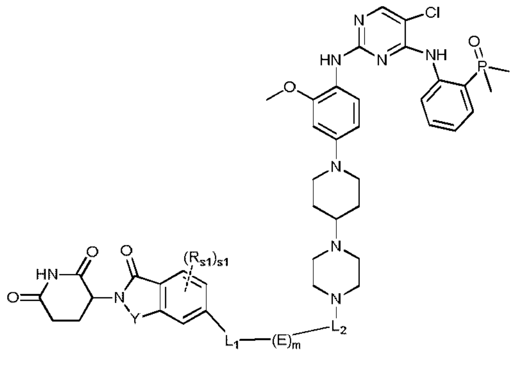

(I-1-H"),

wherein

X is -CH=;

Y is C atom, which is optionally substituted with one or two Rzi, wherein Rzi is H, CN or halogen; or two Rzi are taken together with Y to form C=O; alternatively, Y is $CH_2$;

$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is halogen;

$R_4$ is selected from H, halogen, $C_{1-6}$ alkyl or $C_{1-6}$ haloalkyl; alternatively, $R_4$ is H;

$R_8$ is H;

$R_9$ is selected from H, halogen, $C_{1-6}$ alkyl, -CN or $C_{1-6}$ haloalkyl; alternatively, $R_9$ is H;

$L_1$ is selected from $-CH_2CH_2-$, -CH=CH-, -C=C-, $-OCH_2-$, $-SCH_2-$, $-S(O)CH_2-$,$-S(O)_2CH_2-$, $-NHCH_2-$, $-N(Me)CH_2-$, $-C(O)CH_2-$, $-CH_2C(O)-$, -OC(O)-, -SC(O)-, -NHC(O)-or -N(Me)C(O)-, and one or more H atom(s) in the above groups can be substituted with D atom(s); alternatively, $L_1$ is selected from $-CH_2CH_2-$, -CH=CH-, -C=C-, $-OCH_2-$ or $-NHCH_2-$; alternatively, $L_1$ is selected from $-CH_2CH_2-$, -C=C-, $-OCH_2-$ or $-NHCH_2-$;

$L_2$ is selected from bond, $-CH_2-$ or $-CH_2CH_2-$, and one or more H atom(s) in the above groups can be substituted with D atom(s);

E is $-CH_2CH_2CH_2-$, $-CH_2CH_2O-$, $-CH_2OCH_2-$, $-OCH_2CH_2-$, $-CH_2CH_2S-$, $-CH_2SCH_2-$or $-SCH_2CH_2-$, and one or more H atom(s) in the above groups can be substituted with D atom(s); alternatively, E is $-CH_2CH_2CH_2-$;

m is 0, 1, 2 or 3; alternatively, the chain length of $-L_1-(E)_m-L_2-$ is 4 to 14 bond lengths, still alternatively 5, 6, 7, 8, 9 or 10 bond lengths;

$R_{s1}$ is selected from H, CN, halogen, OH, $NH_2$, $C_{2-6}$ alkenyl, $C_{2-6}$ alkynyl, $-O-C_{1-6}$ alkyl, $-O-C_{1-6}$ haloalkyl, $-NH-C_{1-6}$ alkyl, $C_{1-6}$ alkyl or $C_{1-6}$ haloalkyl; alternatively, $R_{s1}$ is H or halogen;

s1 is 0, 1 or 2.

In a more specific embodiment, the present disclosure provides a compound of formula (I-1-G), (I-1-H), (I-1-H'), (I-1-H"), or a pharmaceutically acceptable salt, enantiomer, diastereomer, racemate, solvate, hydrate, polymorph, prodrug, or isotopic variant thereof, or mixture thereof:

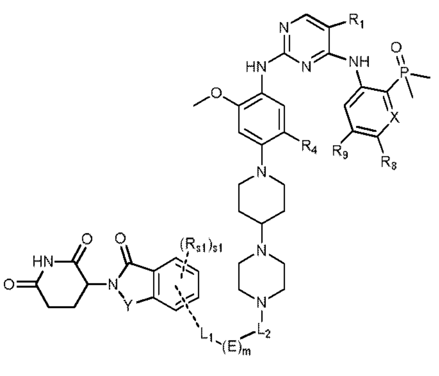

(I-1-G),

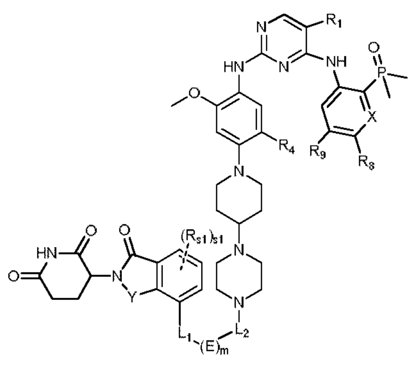

(I-1-H),

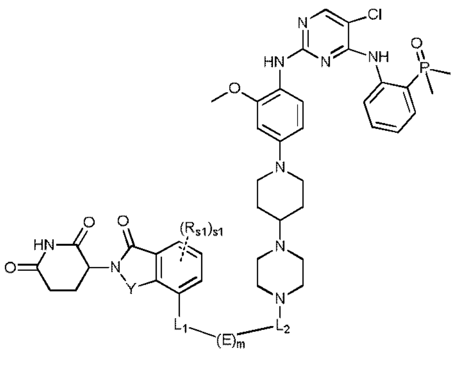

(I-1-H'),

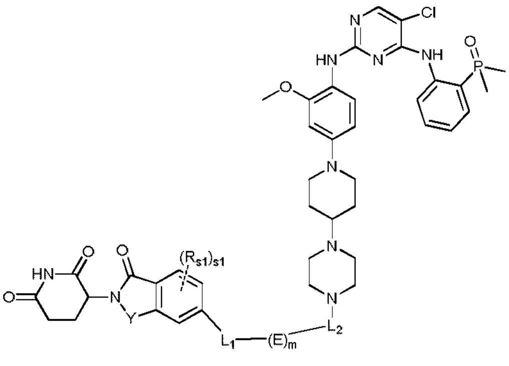

(I-1-H"),

wherein

X is -CH=;
Y is C atom, which is optionally substituted with one or two Rzi, wherein Rzi is H, CN or halogen; or two Rzi are taken together with Y to form C=O; alternatively, Y is $CH_2$ or C=O;
$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is halogen;
$R_4$ is selected from H, halogen, $C_{1-6}$ alkyl or $C_{1-6}$ haloalkyl; alternatively, $R_4$ is H;
$R_8$ is H;
$R_9$ is selected from H, halogen, $C_{1-6}$ alkyl, -CN or $C_{1-6}$ haloalkyl; alternatively, $R_9$ is H;
$L_1$ is selected from $-CH_2CH_2-$, -CH=CH-, -C=C-, $-OCH_2-$, $-SCH_2-$, $-S(O)CH_2-$,$-S(O)_2CH_2-$, $-NHCH_2-$, $-N(Me)CH_2-$, $-C(O)CH_2-$, $-CH_2C(O)-$, -OC(O)-, -SC(O)-, -NHC(O)-or -N(Me)C(O)-, and one or more H atom(s) in the above groups can be substituted with D atom(s); alternatively, $L_1$ is selected from $-CH_2CH_2-$, -CH=CH-, -C=C-, $-OCH_2-$ or $-NHCH_2-$; alternatively, $L_1$ is selected from -C=C-, $-OCH_2-$ or $-NHCH_2-$;
$L_2$ is selected from bond, $-CH_2-$ or $-CH_2CH_2-$, and one or more H atom(s) in the above groups can be substituted with D atom(s);
E is $-CH_2CH_2CH_2-$, $-CH_2CH_2O-$, $-CH_2OCH_2-$, $-OCH_2CH_2-$, $-CH_2CH_2S-$, $-CH_2SCH_2-$or $-SCH_2CH_2-$, and one or more H atom(s) in the above groups can be substituted with D atom(s); alternatively, E is $-CH_2CH_2CH_2-$;
m is 1, 2 or 3; alternatively, the chain length of $-L_1-(E)_m-L_2-$ is less than 14 bond lengths; alternatively, the chain length is 5-10 bond lengths, alternatively 5, 6, 7, 8, 9 or 10 bond lengths;
$R_{s1}$ is selected from H, CN, halogen, OH, $NH_2$, $C_{2-6}$ alkenyl, $C_{2-6}$ alkynyl, $-O-C_{1-6}$ alkyl, $-O-C_{1-6}$ haloalkyl, $-NH-C_{1-6}$ alkyl, $C_{1-6}$ alkyl or $C_{1-6}$ haloalkyl; alternatively, $R_{s1}$ is H or halogen;
s1 is 0, 1 or 2.

In a more specific embodiment, the present disclosure provides a compound of formula (I-1-I), (I-1-I'), (I-1-I"), (I-1-I'"), or a pharmaceutically acceptable salt, enantiomer, diastereomer, racemate, solvate, hydrate, polymorph, prodrug, or isotopic variant thereof, or mixture thereof:

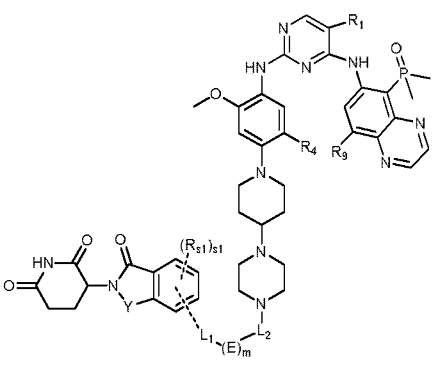

(I-1-I),

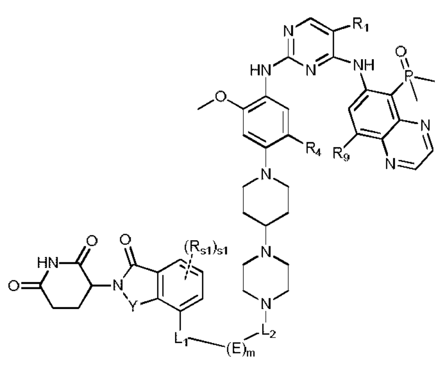

(I-1-I'),

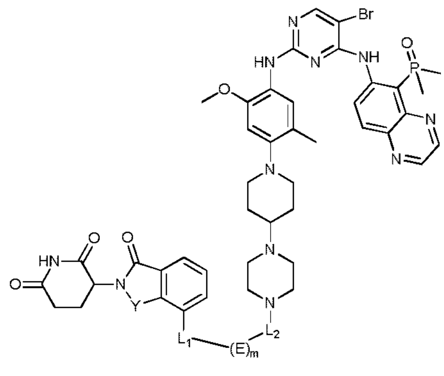 (I-1-I"),

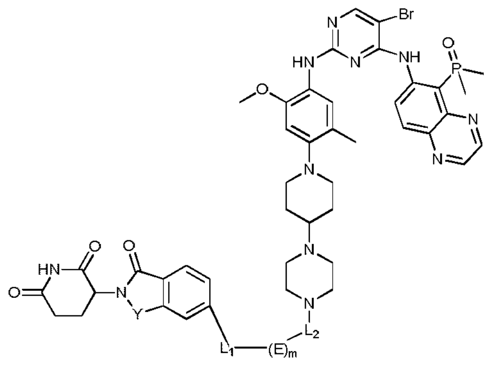 (I-1-I'''),

wherein

Y is C atom, which is optionally substituted with one or two Rzi, wherein Rzi is H, CN or halogen; or two Rzi are taken together with Y to form C=O; alternatively, Y is $CH_2$ or C=O;

$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is halogen;

$R_4$ is selected from H, halogen, $C_{1-6}$ alkyl or $C_{1-6}$ haloalkyl; alternatively, $R_4$ is selected from $C_{1-6}$ alkyl or $C_{1-6}$ haloalkyl;

$R_9$ is selected from H, halogen, $C_{1-6}$ alkyl, -CN or $C_{1-6}$ haloalkyl; alternatively, $R_9$ is H;

$L_1$ is selected from $-CH_2CH_2-$, -CH=CH-, -C=C-, $-OCH_2-$, $-SCH_2-$, $-S(O)CH_2-$,$-S(O)_2CH_2-$, $-NHCH_2-$, $-N(Me)CH_2-$, $-C(O)CH_2-$, $-CH_2C(O)-$, -OC(O)-, -SC(O)-, -NHC(O)-or -N(Me)C(O)-; alternatively, $L_1$ is selected from $-CH_2CH_2-$, -CH=CH-, -C=C- or $-OCH_2-$; alternatively, $L_1$ is selected from $-CH_2CH_2-$, -C=C- or $-OCH_2-$;

$L_2$ is selected from bond, $-CH_2-$ or $-CH_2CH_2-$;

E is $-CH_2CH_2CH_2-$, $-CH_2CH_2O-$, $-CH_2OCH_2-$, $-OCH_2CH_2-$, $-CH_2CH_2S-$, $-CH_2SCH_2-$or $-SCH_2CH_2-$; alternatively, E is $-CH_2CH_2CH_2-$;

m is 1 or 2; alternatively, the chain length of $-L_1-(E)_m-L_2-$ is 4 to 14 bond lengths, still alternatively 5, 6, 7, 8, 9 or 10 bond lengths;

$R_{s1}$ is selected from H, CN, halogen, OH, $NH_2$, $C_{2-6}$ alkenyl, $C_{2-6}$ alkynyl, $-O-C_{1-6}$ alkyl, $-O-C_{1-6}$ haloalkyl, $-NH-C_{1-6}$ alkyl, $C_{1-6}$ alkyl or $C_{1-6}$ haloalkyl; alternatively, $R_{s1}$ is H, halogen or CN;

s1 is 0, 1 or 2.

In a more specific embodiment, the present disclosure provides a compound of formula (I-1-I), (I-1-I'), (I-1-I"), (I-1-I'''), or a pharmaceutically acceptable salt, enantiomer, diastereomer, racemate, solvate, hydrate, polymorph, prodrug, or isotopic variant thereof, or mixture thereof:

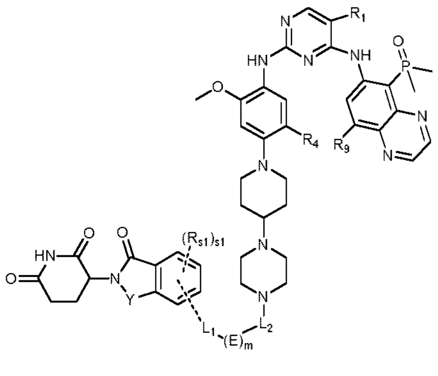 (I-1-I),

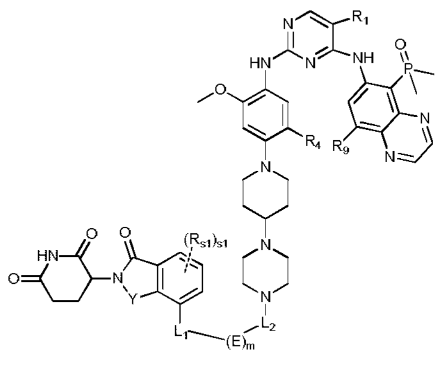 (I-1-I'),

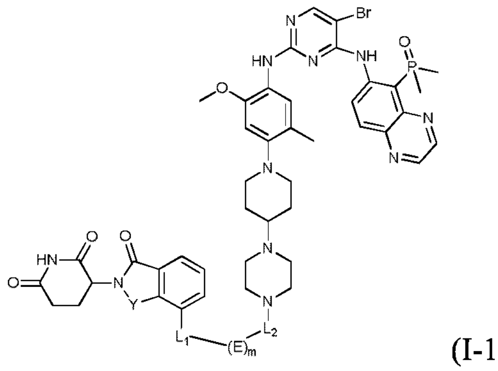

(I-1-I"),

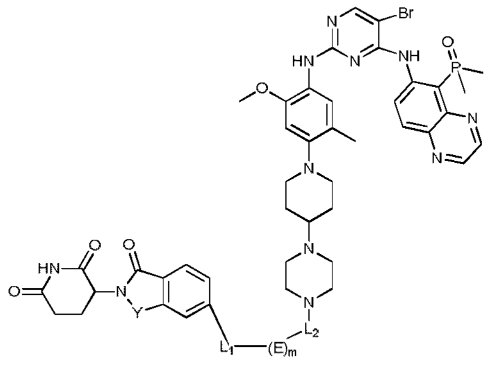

(I-1-I'''),

wherein

Y is C atom, which is optionally substituted with one or two Rzi, wherein Rzi is H, CN or halogen; or two Rzi are taken together with Y to form C=O; alternatively, Y is $CH_2$;

$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is halogen;

$R_4$ is selected from H, halogen, $C_{1-6}$ alkyl or $C_{1-6}$ haloalkyl; alternatively, $R_4$ is selected from $C_{1-6}$ alkyl or $C_{1-6}$ haloalkyl;

$R_9$ is selected from H, halogen, $C_{1-6}$ alkyl, -CN or $C_{1-6}$ haloalkyl; alternatively, $R_9$ is H;

$L_1$ is selected from $-CH_2CH_2-$, -CH=CH-, -C=C-, $-OCH_2-$, $-SCH_2-$, $-S(O)CH_2-$,$-S(O)_2CH_2-$, $-NHCH_2-$, $-N(Me)CH_2-$, $-C(O)CH_2-$, $-CH_2C(O)-$, -OC(O)-, -SC(O)-, -NHC(O)-or -N(Me)C(O)-; alternatively, $L_1$ is selected from $-CH_2CH_2-$, -CH=CH-, -C=C- or $-OCH_2-$; alternatively, $L_1$ is selected from -C=C- or $-OCH_2-$;

$L_2$ is selected from bond, $-CH_2-$ or $-CH_2CH_2-$;

E is $-CH_2CH_2CH_2-$;

m is 1 or 2; alternatively, the chain length of $-L_1-(E)_m-L_2-$ is less than 14 bond lengths; still alternatively 5, 6, 7, 8, 9 or 10 bond lengths;

$R_{s1}$ is selected from H, CN, halogen, OH, $NH_2$, $C_{2-6}$ alkenyl, $C_{2-6}$ alkynyl, -O-$C_{1-6}$ alkyl, -O-$C_{1-6}$ haloalkyl, -NH-$C_{1-6}$ alkyl, $C_{1-6}$ alkyl or $C_{1-6}$ haloalkyl; alternatively, $R_{s1}$ is H or halogen;

s1 is 0, 1 or 2.

In a more specific embodiment, the present disclosure provides a compound of formula (I-2-I), (I-2-I'), (I-2-I"), (I-2-I'''), or a pharmaceutically acceptable salt, enantiomer, diastereomer, racemate, solvate, hydrate, polymorph, prodrug, or isotopic variant thereof, or mixture thereof:

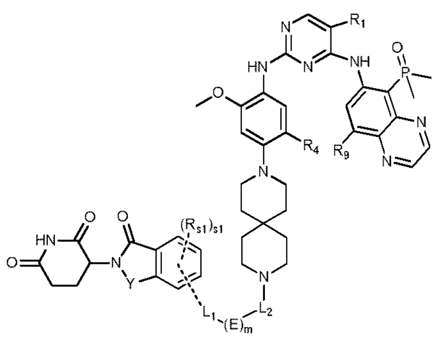

(I-2-I),

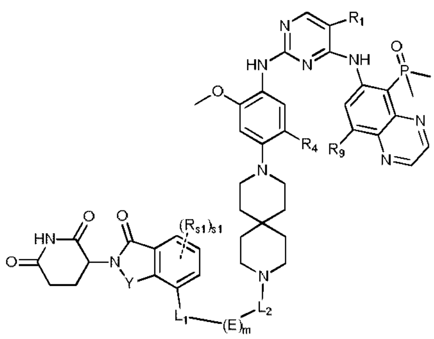

(I-2-I'),

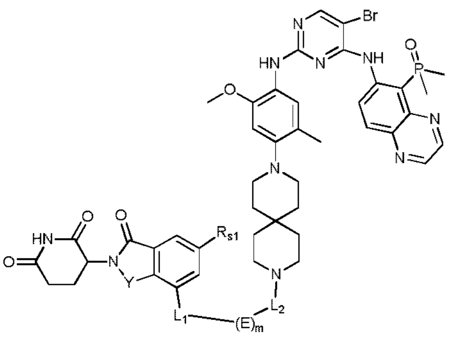

(I-2-I"),

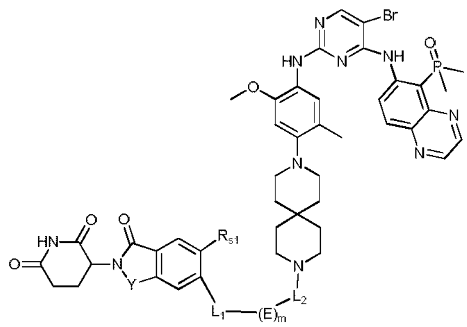

(I-2-I'"),

wherein

Y is C atom, which is optionally substituted with one or two Rzi, wherein Rzi is H, CN or halogen; or two Rzi are taken together with Y to form C=O; alternatively, Y is $CH_2$;
$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is halogen;
$R_4$ is selected from H, halogen, $C_{1-6}$ alkyl or $C_{1-6}$ haloalkyl; alternatively, $R_4$ is selected from $C_{1-6}$ alkyl or $C_{1-6}$ haloalkyl;
$R_9$ is selected from H, halogen, $C_{1-6}$ alkyl, -CN or $C_{1-6}$ haloalkyl; alternatively, $R_9$ is H;
$L_1$ is selected from $-CH_2CH_2-$, -CH=CH-, -C=C-, $-OCH_2-$, $-SCH_2-$, $-S(O)CH_2-$,$-S(O)_2CH_2-$, $-NHCH_2-$, $-N(Me)CH_2-$, $-C(O)CH_2-$, $-CH_2C(O)-$, -OC(O)-, -SC(O)-, -NHC(O)-or -N(Me)C(O)-; alternatively, $L_1$ is selected from $-CH_2CH_2-$, -CH=CH-, -C=C- or $-OCH_2-$; alternatively, $L_1$ is selected from -C=C- or $-OCH_2-$;
$L_2$ is selected from bond, $-CH_2-$ or $-CH_2CH_2-$;
E is $-CH_2CH_2CH_2-$, $-CH_2CH_2O-$, $-CH_2OCH_2-$, $-OCH_2CH_2-$, $-CH_2CH_2S-$, $-CH_2SCH_2-$or $-SCH_2CH_2-$; alternatively, E is $-CH_2CH_2CH_2-$;
m is 1 or 2; alternatively, the chain length of $-L_1-(E)_m-L_2-$ is less than 14 bond lengths; alternatively 5, 6, 7, 8, 9 or 10 bond lengths;
$R_{s1}$ is selected from H, CN, halogen, $C_{1-6}$ alkyl or $C_{1-6}$ haloalkyl; alternatively, $R_{s1}$ is H or halogen;
s1 is 0, 1 or 2.

In a more specific embodiment, the present disclosure provides a compound of formula (I-2), (I-2-A), (I-2-B), (I-2-C), (I-2-D), (I-2-E), (I-2-F), (I-2-G), (I-2-H) or (I-2-I), or a pharmaceutically acceptable salt, enantiomer, diastereomer, racemate, solvate, hydrate, polymorph, prodrug, or isotopic variant thereof, or mixture thereof, wherein

$R_1$ is -Cl, -Br or $-CH=CH_2$;
$R_4$ is H or -Me;
$R_8$ is H, -NHMe, $-NHC(O)CH_2CH_3$ or -NH-cyclopropyl;
$R_9$ is H, -F, -Cl, -Br, -Me, $-CF_3$, -OMe, $-OCF_3$, -CN, $-NHC(O)CH_2CH_3$, - $NHS(O)_2CH_2CH_3$ or $-NHC(O)CH=CH_2$;
X is $-C(R_x)=$;
$R_x$ is H, or $R_x$ and $R_8$ are taken together with the C atoms to which they are attached to form pyrazinyl;
and other groups are as defined in above.

In a more specific embodiment, the present disclosure provides a compound of formula (I-3), (I-3-A), (I-3-B), (I-3-C), (I-3-D), (I-3-E), (I-3-F), (I-3-G) or (I-3-H), or a pharmaceutically acceptable salt, enantiomer, diastereomer, racemate, solvate, hydrate, polymorph, prodrug, or isotopic variant thereof, or mixture thereof, wherein

$R_1$ is H, -Cl or $-CH=CH_2$;
$R_4$ is $-NHC(O)CH=CH_2$;
$R_5$ is -Me;
$R_6$ is -Me;
or $R_5$ and $R_6$ are connected to form $-CH_2CH_2-$;
$R_7$ is -Me or cyclopropyl;
and other groups are as defined in above.

In a more specific embodiment, the present disclosure provides a compound of formula (1-4), (I-4-A), (I-4-B), (I-4-C), (I-4-D), (I-4-E), (I-4-F), (I-4-G) or (I-4-H), or a pharmaceutically acceptable salt, enantiomer, diastereomer, racemate, solvate, hydrate, polymorph, prodrug, or isotopic variant thereof, or mixture thereof, wherein

$R_1$ is $-CF_3$;
$R_9$ is H, -F, -Cl, -Br, -Me, $-CF_3$, -OMe, $-OCF_3$, -CN, $-NHC(O)CH=CH_2$ or -NH-cyclopropyl;
and other groups are as defined in above.

In a more specific embodiment, the present disclosure provides a compound of formula (I-5), (I-5-A), (I-5-B), (I-5-C), (I-5-D), (I-5-E), (I-5-F), (I-5-G) or (I-5-H), or a pharmaceutically acceptable salt, enantiomer, diastereomer, racemate, solvate, hydrate, polymorph, prodrug, or isotopic variant thereof, or mixture thereof, wherein
ring A is the following optionally substituted groups: $C_{3-7}$ cycloalkyl or $C_{6-10}$ aryl, wherein the substituent is selected from -F, -Cl, -Br, -Me, -OMe, $-CF_3$, $-OCF_3$, -CN, -NHMe, $-P(O)Me_2$, $-NHC(O)CH_2CH_3$, $-C(O)CH=CH_2$, $-NHS(O)_2CH_2CH_3$, -NH-cyclopropyl, - $NHC(O)CH=CH_2$ or $-C(O)CH_2CH_3$; ring A is alternatively the following groups:

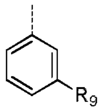 or 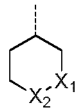 ;

wherein

$R_9$ is H, -F, -Cl, -Br, -Me, $-CF_3$, -OMe, $-OCF_3$, -CN, $-NHC(O)CH_2CH_3$,$-NHS(O)_2CH_2CH_3$ or $-NHC(O)CH=CH_2$;
$X_1$ is $-CH_2-$ or $-N(R_{X1})-$;
$X_2$ is $-CH(R_{X2})-$ or $-N(R_{X2})-$;
wherein
$R_{X1}$ is $-C(O)CH_2CH_3$ or $-C(O)CH=CH_2$;
$R_{X2}$ is H, -Me, -OMe, -NHMe, $-NHS(O)_2CH_2CH_3$, $-C(O)CH_2CH_3$ or$-NHC(O)CH_2CH_3$;
----- represents the point of attachment;
$R_1$ and R are taken together with the atoms to which they are attached to form optionally substituted 5- to 6-membered heterocyclyl, wherein the substituent is selected from halogen, oxo, -iPr, -Et, or phenyl, wherein the phenyl is mono- or poly-substituted with halogen; $R_1$ and R are alternatively taken together to form the following groups:$-C(O)N(R_N)C(O)-$ or $-C(CH_3)=C(R_N)C(O)-$;
wherein

$R_N$ is selected from -iPr, -Et or

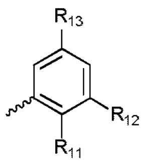 ;

$R_{11}$ is -Cl, -Br;
$R_{12}$ is H or -F;
$R_{13}$ is H or -F;



represents the point of attachment;
$R_3$ is H or -OMe;
$R_4$ is H or -Me;
and other groups are as defined in above.

In a more specific embodiment, the present disclosure provides a compound of formula (I-5-1) or (I'-5-1), or a pharmaceutically acceptable salt, enantiomer, diastereomer, racemate, solvate, hydrate, polymorph, prodrug, or isotopic variant thereof, or mixture thereof:

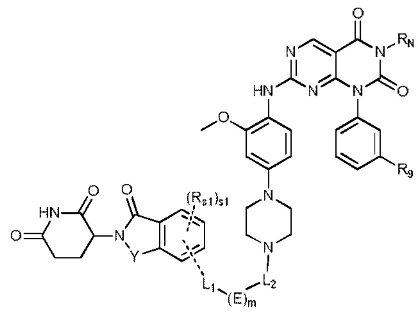

(I-5-1) or

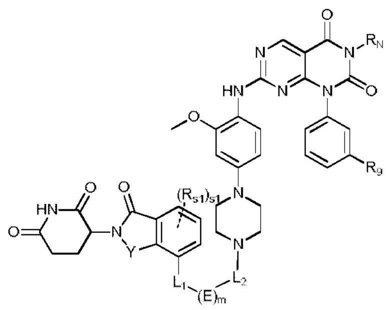

(I'-5-1)

wherein

$R_9$ is $-NHC(O)CH_2CH_3$ or $-NHC(O)CH=CH_2$;
$R_N$ is -iPr or -Et;
and other groups are as defined in above.

In a more specific embodiment, the present disclosure provides a compound of formula (I-5-2) or (I'-5-2), or a pharmaceutically acceptable salt, enantiomer, diastereomer, racemate, solvate, hydrate, polymorph, prodrug, or isotopic variant thereof, or mixture thereof:

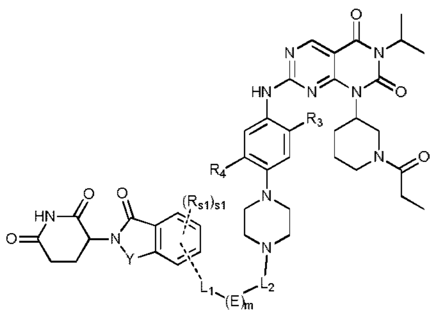

(I-5-2) or

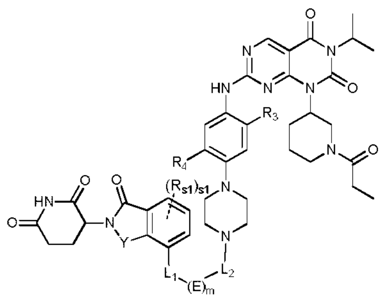

(I'-5-2)

wherein

$R_3$ is H or -OMe;
$R_4$ is H or -Me;
and other groups are as defined in above.

In a more specific embodiment, the present disclosure provides a compound of formula (I-5-3) or (I'-5-3), or a pharmaceutically acceptable salt, enantiomer, diastereomer, racemate, solvate, hydrate, polymorph, prodrug, or isotopic variant thereof, or mixture thereof:

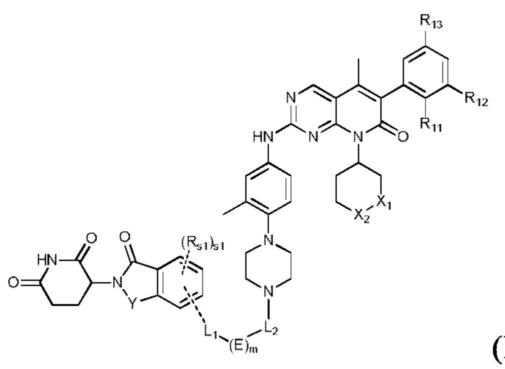

(I-5-3) or

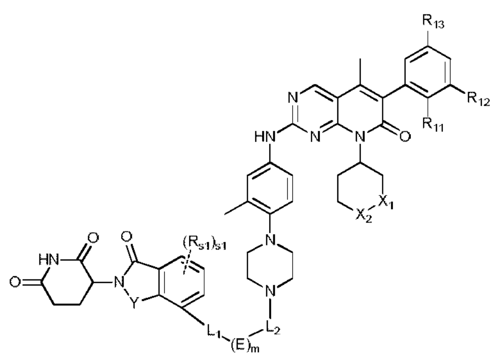

(I'-5-3)

wherein

$X_1$ is $-CH_2-$ or $-N(R_{X1})-$;
$X_2$ is $-CH(R_{X2})-$ or $-N(R_{X2})-$;
wherein
Rxi is $-C(O)CH_2CH_3$;
$R_{X2}$ is H, -Me, -OMe, -NHMe, $-C(O)CH_2CH_3$, $-NHS(O)_2CH_2CH_3$ or - $NHC(O)CH_2CH_3$;
$R_{11}$ is -Cl or -Br;
$R_{12}$ is H or -F;
$R_{13}$ is H or -F;
and other groups are as defined in above.

In a more specific embodiment, the present disclosure provides a compound of formula (I-6), (I-6-A), (I-6-B), (I-6-C), (I-6-D), (I-6-E), (I-6-F), (I-6-G) or (I-6-H), or a pharmaceutically acceptable salt, enantiomer, diastereomer, racemate, solvate, hydrate, polymorph, prodrug, or isotopic variant thereof, or mixture thereof, wherein

L is -O- or -NH-;
$R_3$ is H or -OMe;
$R_4$ is H or -F;
$R_9$ is $-CF_3$, -OMe, $-OCF_3$, -CN, $-NHC(O)CH_2CH_3$ or $-NHC(O)CH=CH_2$;
and other groups are as defined in above.

In a more specific embodiment, the present disclosure relates to a compound of all of the above formulas, or a pharmaceutically acceptable salt, enantiomer, diastereomer, racemate, solvate, hydrate, polymorph, prodrug, or isotopic variant thereof, or mixture thereof,
wherein

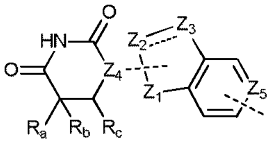

is selected from the following groups:

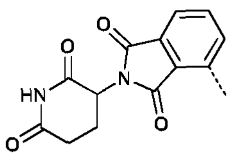 , 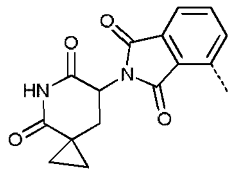 , 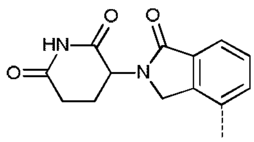 , 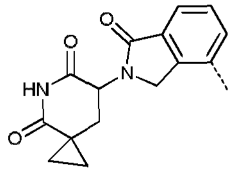 ,

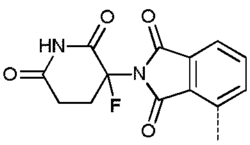 , 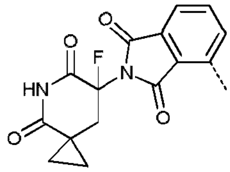 , 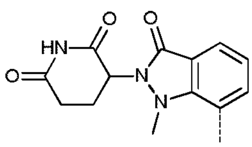 , 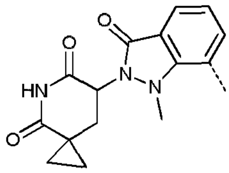 ,

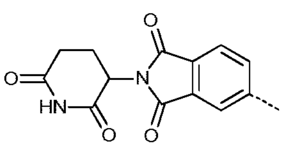 , 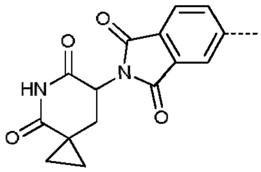 , 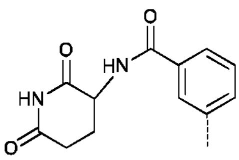 , 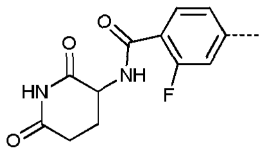 ,

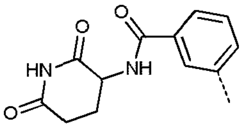, 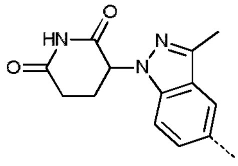, 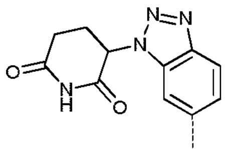, 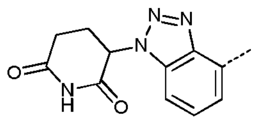,

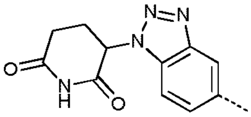, 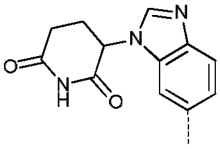, 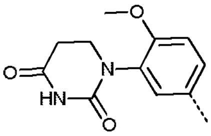, 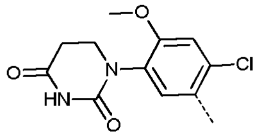,

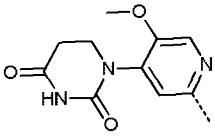, 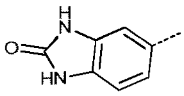, 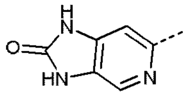, 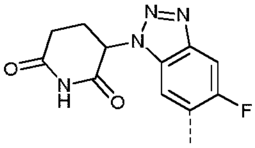, 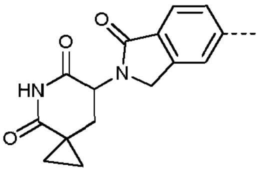,

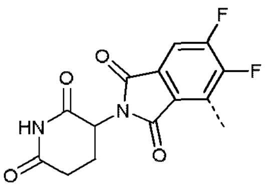, 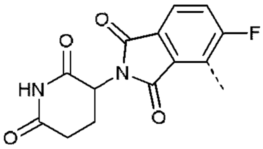, 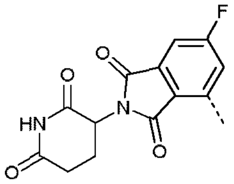, 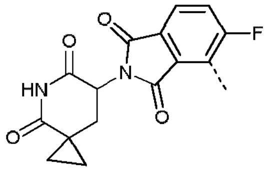,

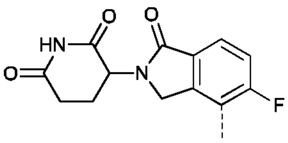, 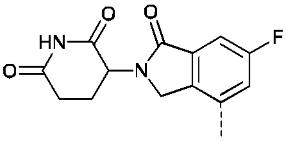, 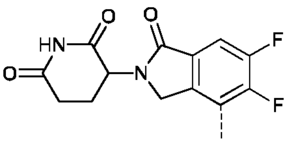,

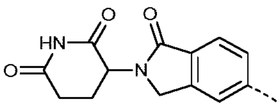 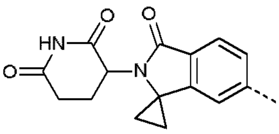, 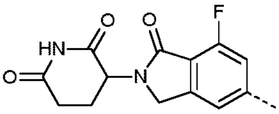, 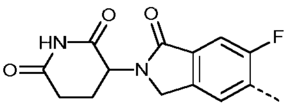,

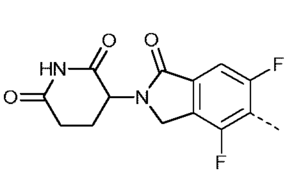, 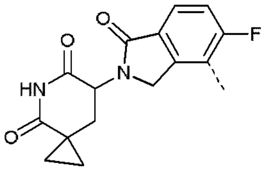, 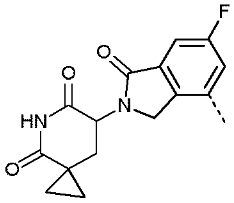, 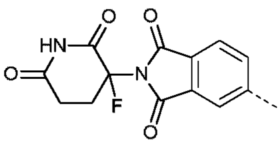,

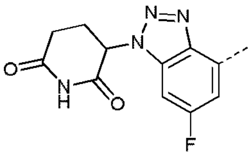, 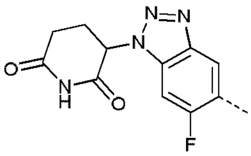, 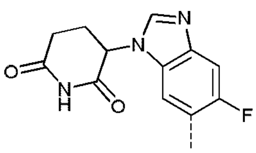, 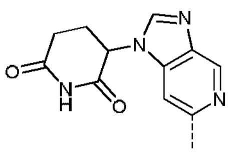,

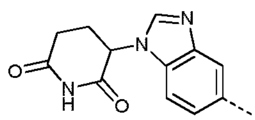, 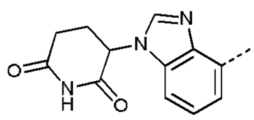, 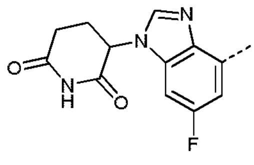, 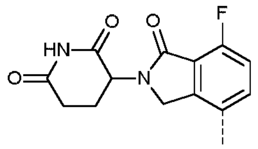,

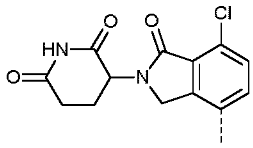, 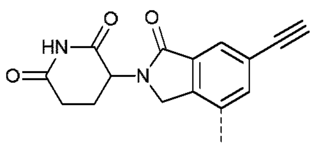, 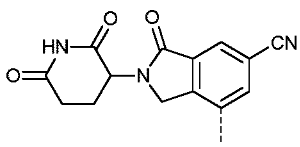, 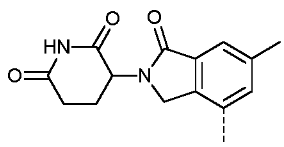,

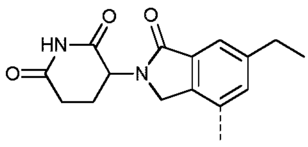, 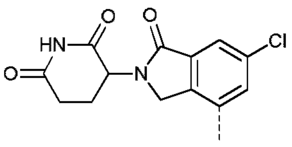 or 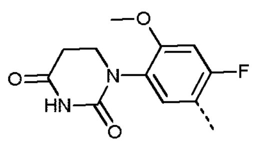;

and one or more H atom(s) in the above groups can be substituted with D atom(s).

In a more specific embodiment, the present disclosure relates to a compound of all of the above formulas, or a pharmaceutically acceptable salt, enantiomer, diastereomer, racemate, solvate, hydrate, polymorph, prodrug, or isotopic variant thereof, or mixture thereof,

wherein $L_1$ and $L_2$ are independently selected from bond, -O-, -S-, -S(O)-, $-S(O)_2-$, - S(O)(=NH)-, -S(O)(=NMe)-,

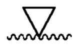, 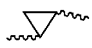,

-NH-, -N(Me)-,

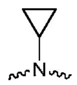,

$-N(CF_3)-$, $-CH_2-$,-CH(OMe)-, -CH(Cl)-, -CH(F)-, $-CF_2-$, $-CH(CF_3)-$, -C(O)-, $-CH_2CH_2-$, -CH=CH-, -C=C-,$-OCH_2-$, $-CH_2O-$, $-SCH_2-$, $-CH_2S-$, $-S(O)CH_2-$, $-CH_2S(O)-$, $-S(O)_2CH_2-$, $-CH_2S(O)_2-$,$-NHCH_2-$, $-N(Me)CH_2-$, $-CH_2NH-$, $-CH_2N(Me)-$, $-C(O)CH_2-$, $-CH_2C(O)-$, $-C(O)CMe_2-$,$-CMe_2C(O)-$, -OC(O)-, -C(O)O-, -SC(O)-, -C(O)S-, -NHC(O)-, -N(Me)C(O)-, -C(O)NH-,-C(O)N(Me)-, -S(O)=NH-, -NH=S(O)-, -N=S(O)Me-, -S(O)Me=N-,

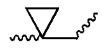 or 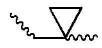;

and one or more H atom(s) in the above groups can be substituted with D atom(s).

In a more specific embodiment, the present disclosure relates to a compound of all of the above formulas, or a pharmaceutically acceptable salt, enantiomer, diastereomer, racemate, solvate, hydrate, polymorph, prodrug, or isotopic variant thereof, or mixture thereof,

wherein E is selected from bond, $-CH_2CH_2CH_2-$, $-CH_2CH=CH-$, $-CH=CHCH_2-$, - $CH_2C{\equiv}C-$, $-C{\equiv}CCH_2-$, $-CH_2CH_2C(O)-$, $-CH_2C(O)CH_2-$, $-C(O)CH_2CH_2-$, $-CH_2CH_2S(O)_2-$, - $CH_2S(O)_2CH_2-$, $-S(O)_2CH_2CH_2-$, -C(O)CH=CH-, $-C(O)C{\equiv}C-$, $-CH_2CH_2O-$, $-CH_2OCH_2-$, - $OCH_2CH_2-$, $-CH_2CH_2S-$, $-CH_2SCH_2-$, $-SCH_2CH_2-$, $-C(O)CH_2O-$, $-OCH_2C(O)-$, $-CH_2C(O)O-$, $-C(O)CH_2S-$, $-SCH_2C(O)-$, $-CH_2C(O)S-$, $-OC(O)CH_2-$, $-C(O)OCH_2-$, $-CH_2OC(O)-$, - $SC(O)CH_2-$, $-C(O)SCH_2-$, $-CH_2SC(O)-$, $-CH_2CH_2NH-$, $-CH_2NHCH_2-$, $-NHCH_2CH_2-$, - $CH_2CH_2NMe-$, $-CH_2NMeCH_2-$, $-NMeCH_2CH_2-$, $-C(O)CH_2NH-$, $-NHCH_2C(O)-$, - $CH_2C(O)NH-$, $-NHC(O)CH_2-$, $-C(O)NHCH_2-$, $-CH_2NHC(O)-$,

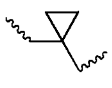, 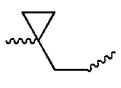, 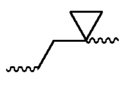,

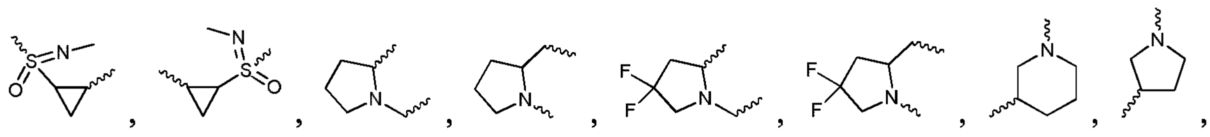

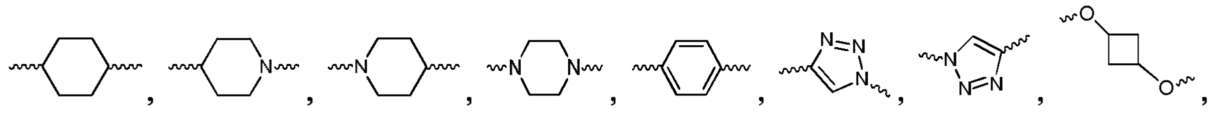

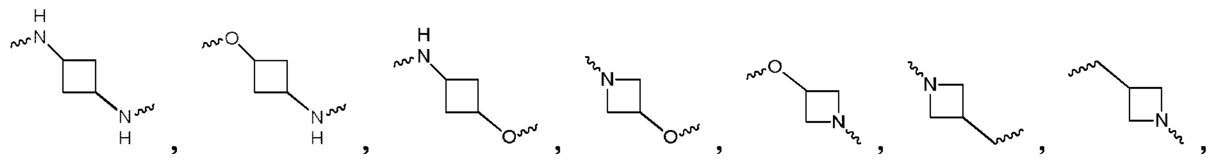

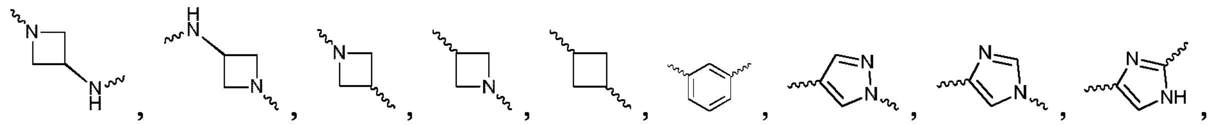

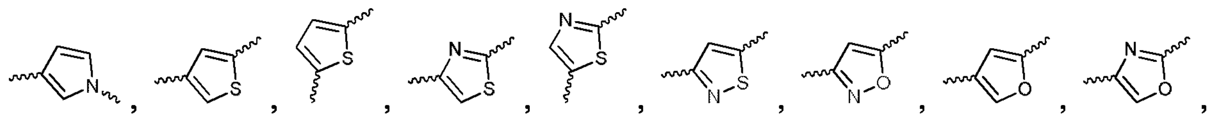

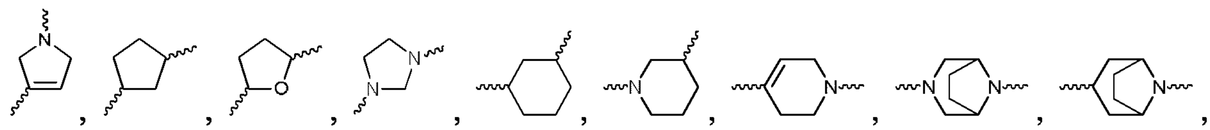

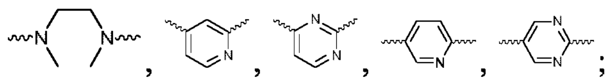

or two E moieties, or two E' moieties can be taken together to form - $CH_2CH_2OCH_2CH_2$-, -$OCH_2CH_2CH_2CH_2$-, -$CH_2CH_2CH_2CH_2O$-,

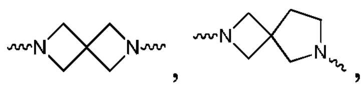

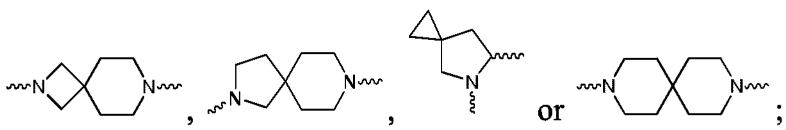

and one or more H atom(s) in the above groups can be substituted with D atom(s).

In a more specific embodiment, the present disclosure relates to a compound of all of the above formulas, or a pharmaceutically acceptable salt, enantiomer, diastereomer, racemate, solvate, hydrate, polymorph, prodrug, or isotopic variant thereof, or mixture thereof, wherein m is 0, 1, 4, 5, 6, 7, 8, 9 or 10; alternatively, m is 0, 1, 4, 5, 6, 7 or 8; alternatively, m is 0, 1, 4, 5 or 6; alternatively, m is 0, 1, 4 or 5.

In a more specific embodiment, the present disclosure relates to a compound of all of the above formulas, or a pharmaceutically acceptable salt, enantiomer, diastereomer, racemate, solvate, hydrate, polymorph, prodrug, or isotopic variant thereof, or mixture thereof, wherein the chain length of $-L_1-(E)_m-L_2-$ is 4 to 14 bond lengths; alternatively, the chain length is less than 12 bond lengths; alternatively, the chain length is 5-10 bond lengths; alternatively, the chain length is 5, 6, 7, 8, 9 or 10 bond lengths.

In a more specific embodiment, the present disclosure relates to a compound, or a pharmaceutically acceptable salt, enantiomer, diastereomer, racemate, solvate, hydrate, polymorph, prodrug, or isotopic variant thereof, or mixture thereof, and the compound is selected from:

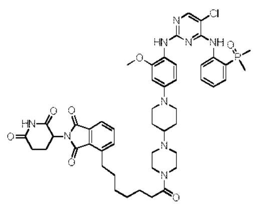,
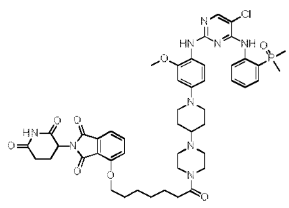,
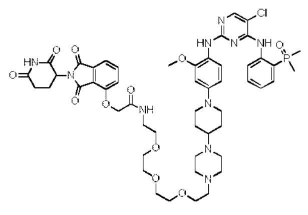,
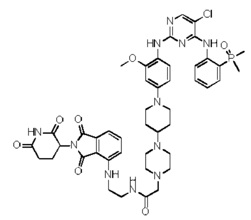,

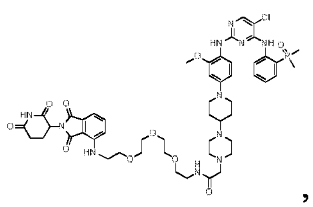,
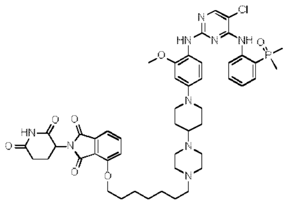,
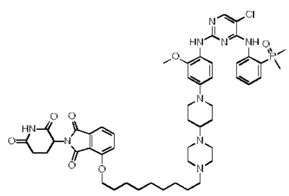,
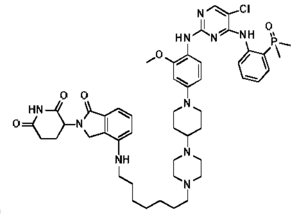,

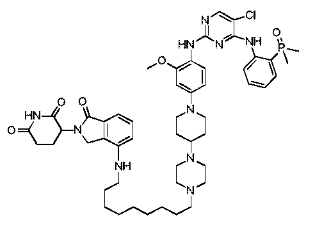,
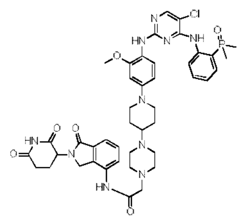,
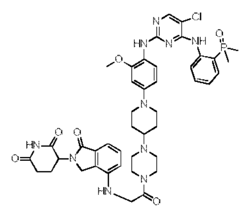,
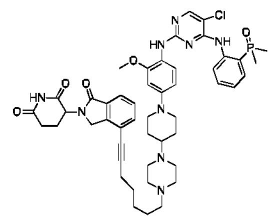,

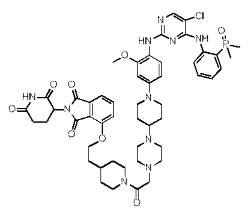,
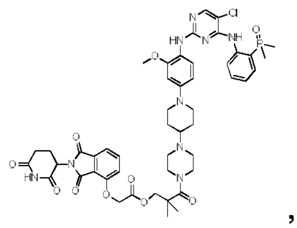,
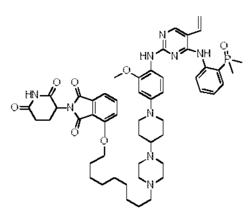,
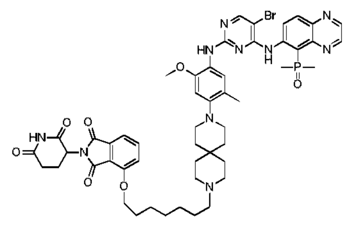,

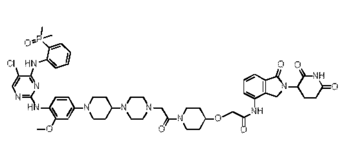,
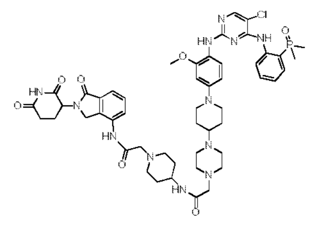,
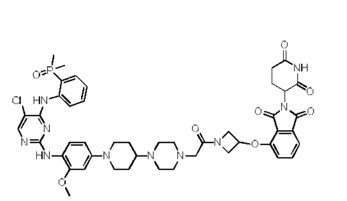,

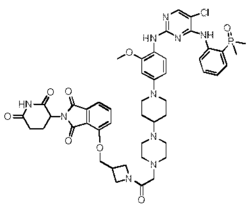 , 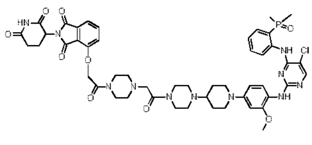 , 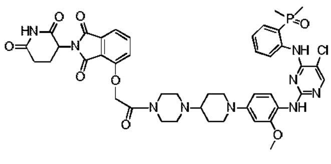 , 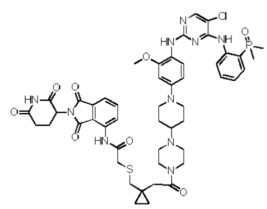 ,

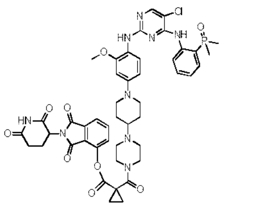 , 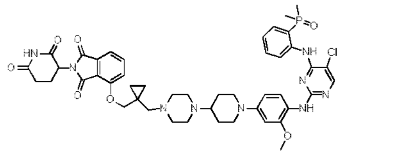 , 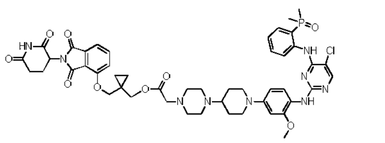 ,

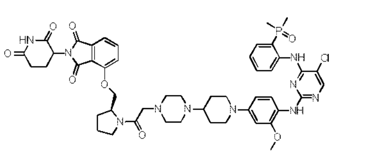 , 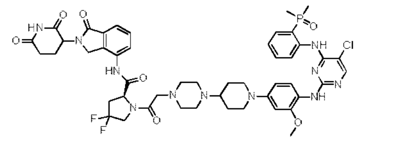 , 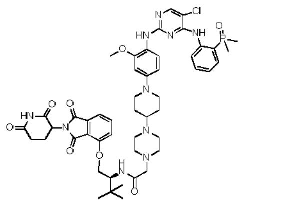 ,

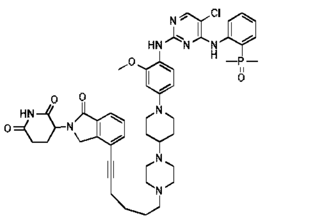 , 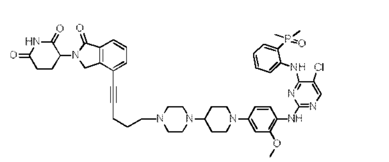 , 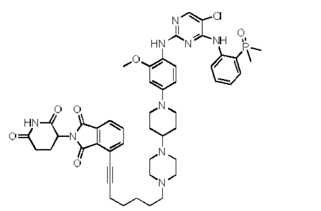 ,

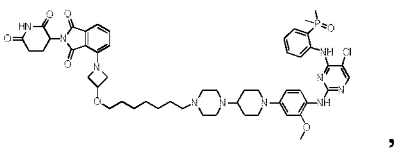 , 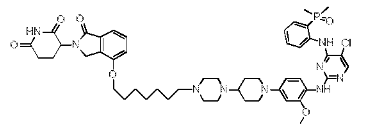 , 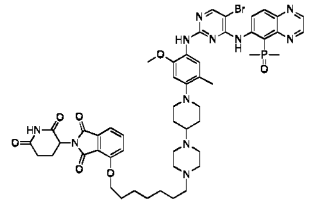 ,

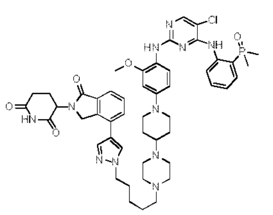 , 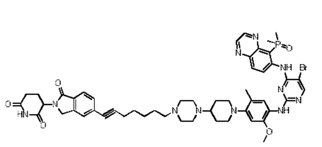 , 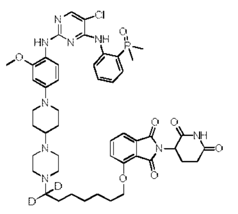 , 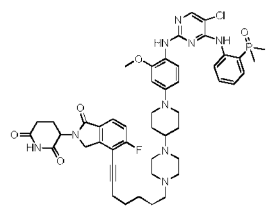 ,

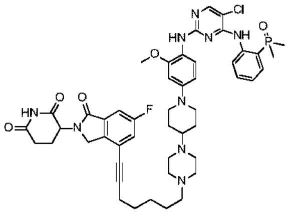 , 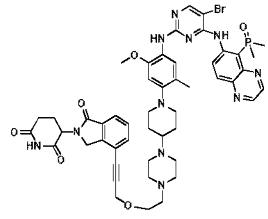 , 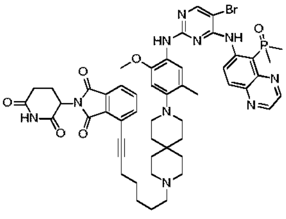 , 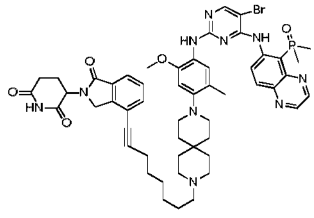 ,

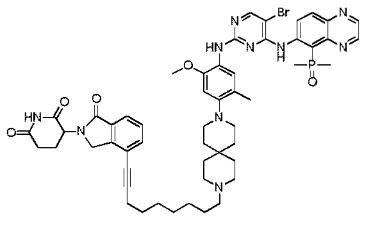 , 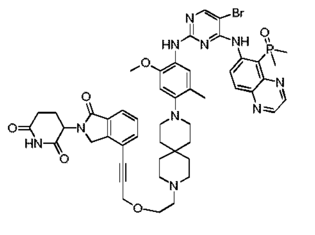 , 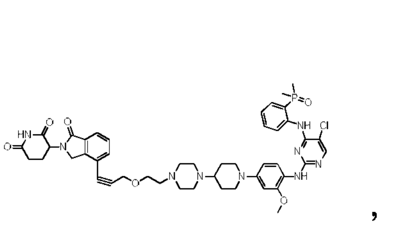 ,

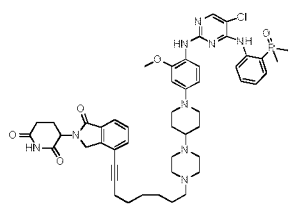 , 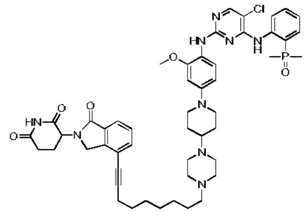 , 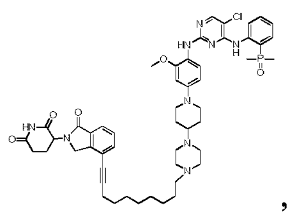 , 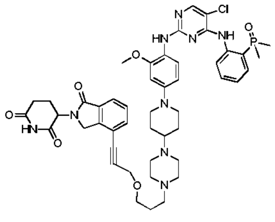 ,

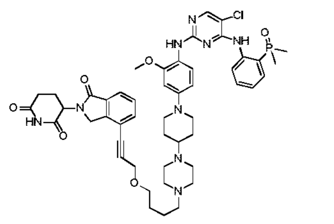 , 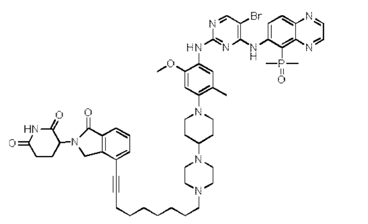 , 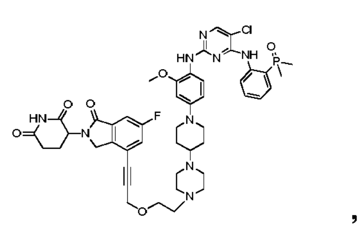 ,

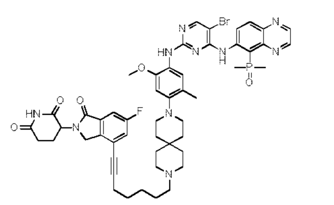 , 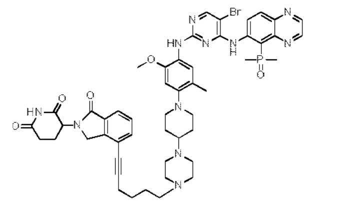 , 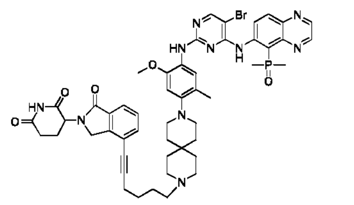 ,

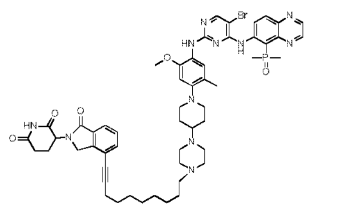 , 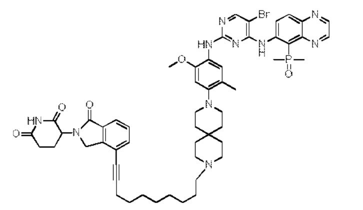 , 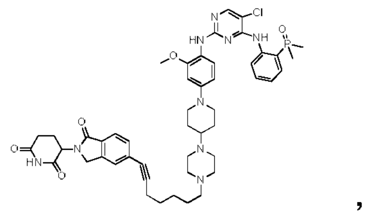 ,

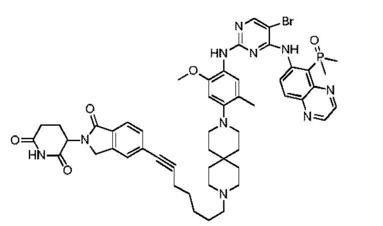 , 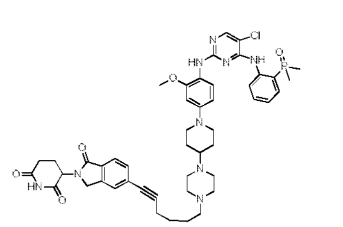 , 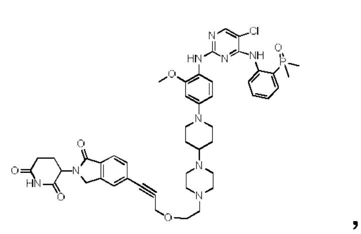 ,

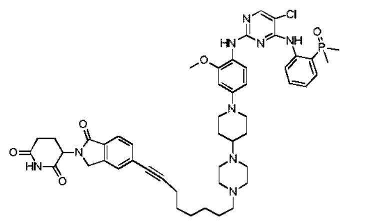,
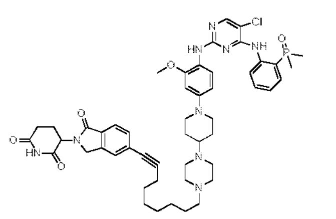,
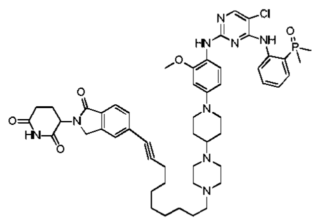,

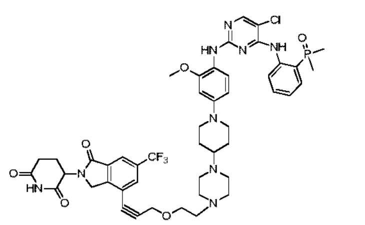,
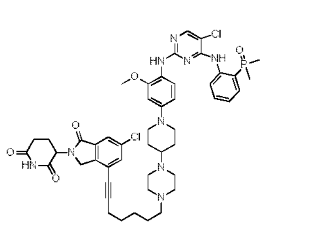,
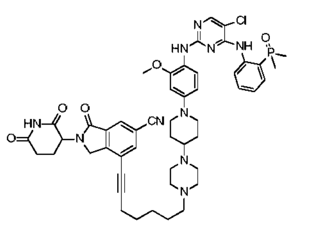,

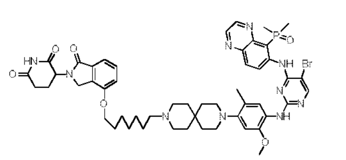,
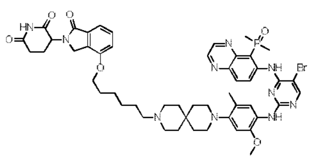,
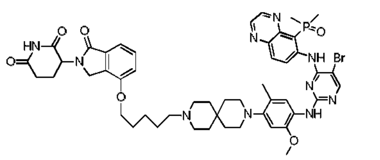,

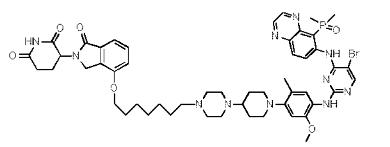,
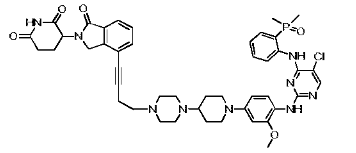,
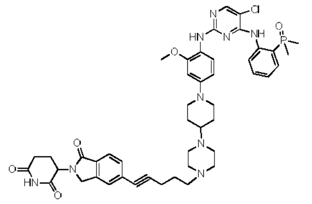,

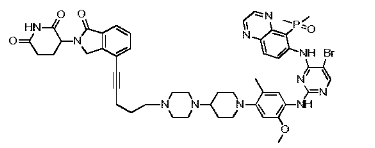,
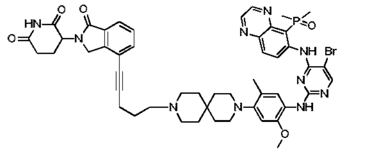,
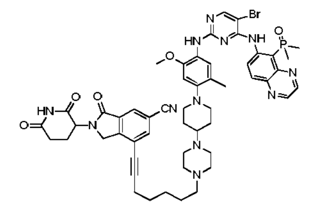,

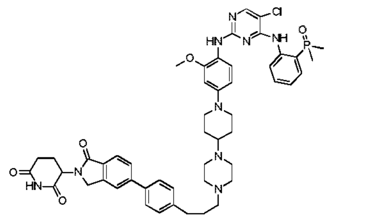,
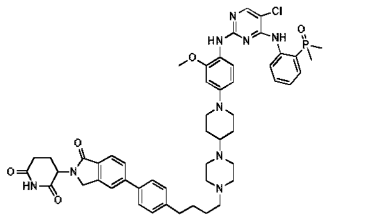,
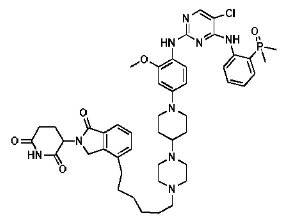,

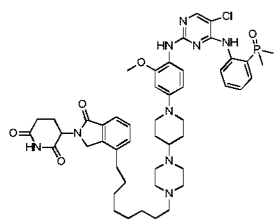,
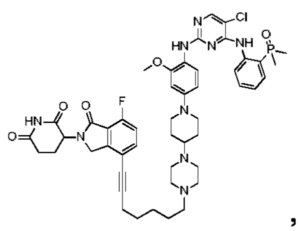,
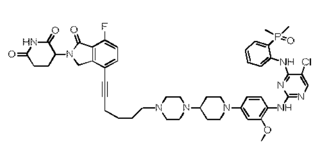,
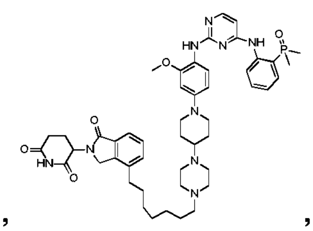,

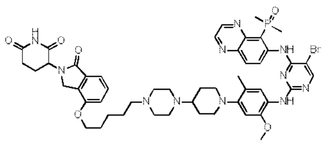 , 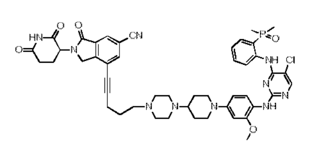 , 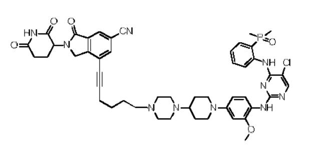 ,

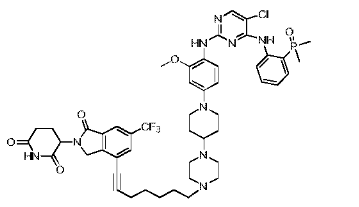 , 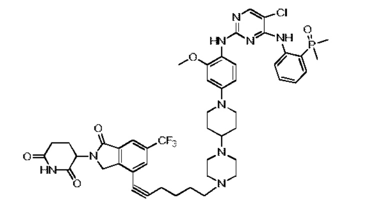 , 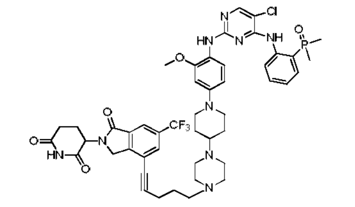 ,

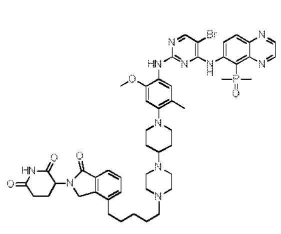 , 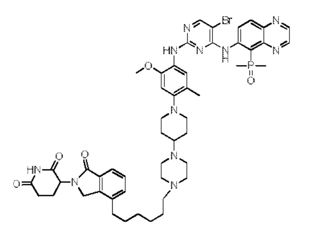 , 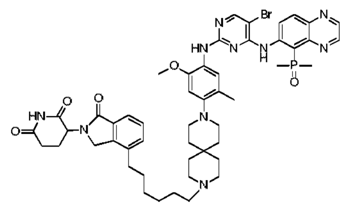 ,

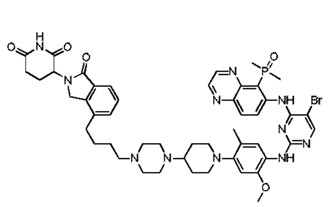 , 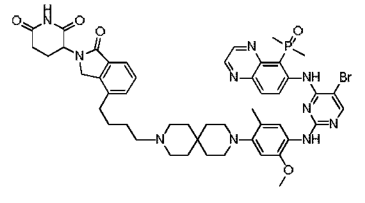 , 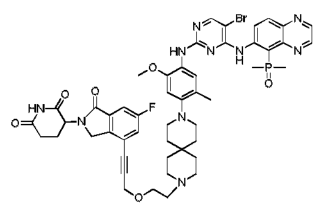 ,

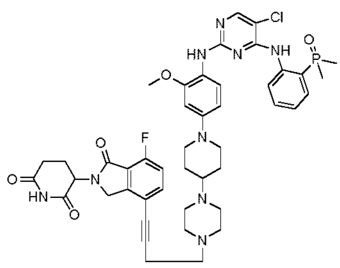 , 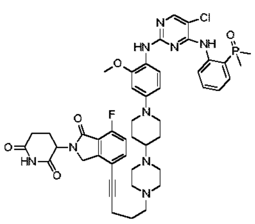 , 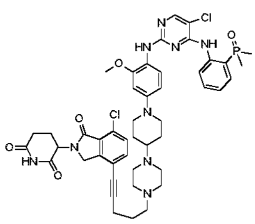 , 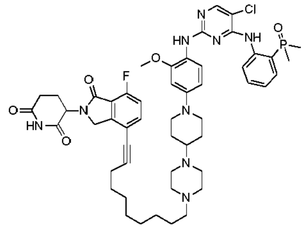 ,

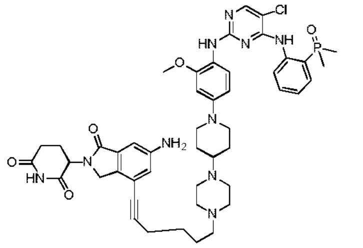 , 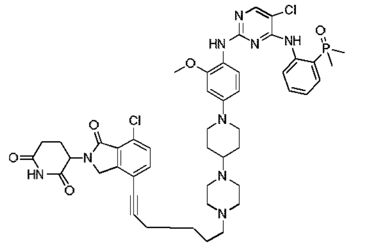 , 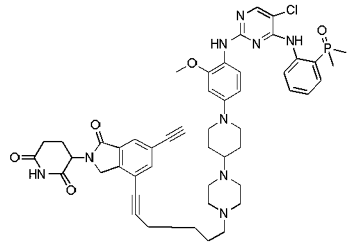 ,

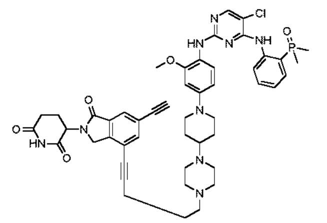,
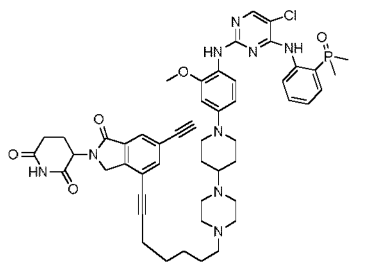,
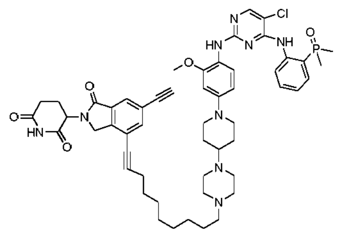,
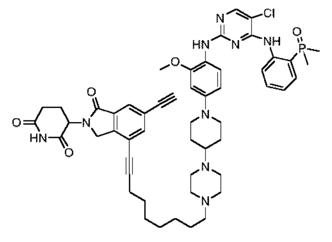,
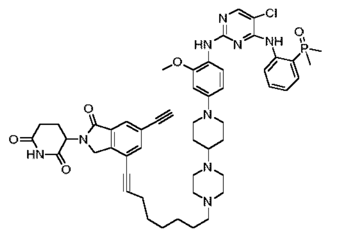,
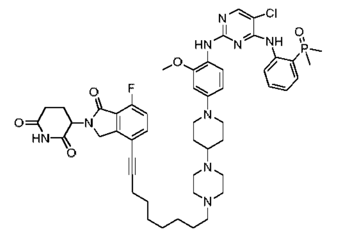,
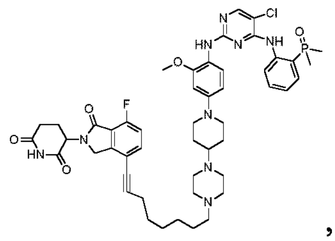,
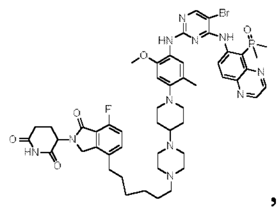,
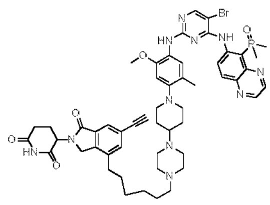,
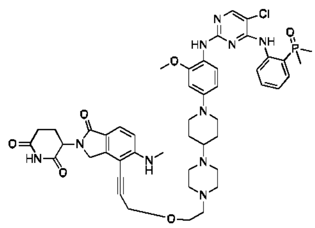,
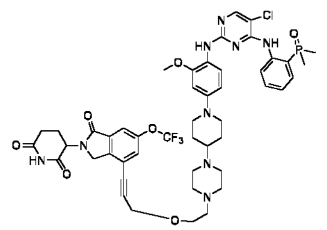,
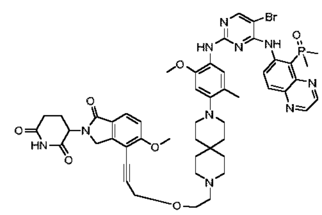,
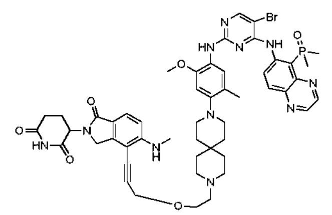,
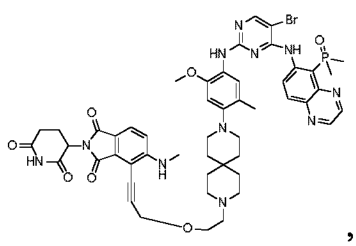,
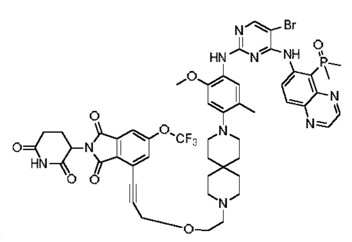
or
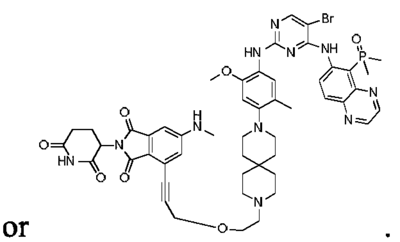.

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and transisomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

It will be understood by those skilled in the art that the organic compounds can form complexes with solvents in which they are reacted or from which they are precipitated or crystallized. These complexes are known as "solvates." Where the solvent is water, the complex is known as "hydrate." The present disclosure encompasses all solvates of the compounds of the present disclosure.

The term "solvate" refers to forms of a compound or a salt thereof, which are associated with a solvent, usually

by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula $R \cdot x\ H_2O$, wherein R is the compound, and x is a number greater than 0. Given compounds can form more than one type of hydrates, including, for example, monohydrates (x is 1), lower hydrates (x is a number greater than 0 and smaller than 1, for example, hemihydrates ($R \cdot 0.5\ H_2O$)) and polyhydrates (x is a number greater than 1, for example, dihydrates ($R \cdot 2\ H_2O$) and hexahydrates ($R \cdot 6\ H_2O$)).

Compounds of the present disclosure may be in an amorphous or a crystalline form (polymorph). Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes (isotope variants), which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., $^{3}H$ and $^{14}C$), can be used for the measurement of the distribution of drug and/or substrate in tissue. Tritium, which is $^{3}H$ and carbon-14, which is $^{14}C$ isotope, are yet alternative, because they are easy to prepare and detect. Furthermore, replaced by heavier isotopes, such as deuterium, which is $^{2}H$, may provide therapeutic benefits due to the higher metabolic stability, such as prolonging the half-life *in vivo* or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted into an active form that has medical effects *in vivo* by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

The prodrugs are any covalently bonded compounds of the present disclosure, which release the parent compound *in vivo* when the prodrug is administered to a patient. Prodrugs are typically prepared by modifying functional groups in such a way that the modifications can be cleaved either by routine manipulation or decompose *in vivo* to yield the parent compound. Prodrugs include, for example, compounds of the present disclosure wherein the hydroxyl, amino or sulfhydryl groups are bonded to any group that, when administered to a patient, cleaves to form the hydroxyl, amino or sulfhydryl groups. Thus, representative examples of prodrugs include (but are not limited to) the acetate/acetamide, formate/formamide and benzoate/benzamide derivatives of the hydroxyl, amino or sulfhydryl functional groups of the compounds of formula (I). Furthermore, in the case of carboxylic acid (-COOH), esters such as methyl esters and ethyl esters, etc. can be employed. The ester itself may be active in their own and/or hydrolyzable under *in vivo* conditions in the human body. Suitable pharmaceutically acceptable *in vivo* hydrolysable ester groups include those groups that can readily break down in the human body to release the parent acids or salts thereof.

The present disclosure also provides a pharmaceutical formulation comprising a therapeutically effective amount of a compound of formula (I), or therapeutically acceptable salts thereof, and pharmaceutically acceptable carriers,

diluents or excipients thereof. All of these forms belong to the present disclosure.

Pharmaceutical compositions and kits

In another aspect, the present disclosure provides a pharmaceutical composition comprising a compound of the present disclosure (also referred to as the "active ingredient") and a pharmaceutically acceptable excipient. In certain embodiments, the pharmaceutical composition comprises an effective amount of the compound of the present disclosure. In certain embodiments, the pharmaceutical composition comprises a therapeutically effective amount of the compound of the present disclosure. In certain embodiments, the pharmaceutical composition comprises a prophylactically effective amount of the compound of the present disclosure.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulosebased materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other materials) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

Administration

The pharmaceutical composition provided by the present disclosure can be administered by a variety of routes including, but not limited to, oral administration, parenteral administration, inhalation administration, topical administration, rectal administration, nasal administration, oral administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, e.g., for example, over 3 months, 6 months, 1 year, 2 years, 3 years, 5 years, *etc.,* or may be continued indefinitely, for example, for the rest of the subject's life. In certain embodiments, the chronic administration is intended to provide a constant level of the compound in the blood, *e.g.,* within the therapeutic window over the extended period of time.

The pharmaceutical compositions of the present disclosure may be further delivered using a variety of dosing methods. For example, in certain embodiments, the pharmaceutical composition may be given as a bolus, e.g., in order to raise the concentration of the compound in the blood to an effective level. The placement of the bolus dose depends on the systemic levels of the active ingredient desired throughout the body, e.g., an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV

drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with alternative doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and still alternatively from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oilin-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington 's Pharmaceutical Sciences, 17th edition, 1985, Mack Publishing Company, Easton, Pennsylvania, which is incorporated herein by reference.

The compounds of the present disclosure can also be administered in sustained release forms or from sustained release drug delivery systems. A description of representative sustained release materials can be found in Remington's Pharmaceutical Sciences.

The present disclosure also relates to the pharmaceutically acceptable formulations of a compound of the present disclosure. In one embodiment, the formulation comprises water. In another embodiment, the formulation comprises a cyclodextrin derivative. The most common cyclodextrins are α-, β- and γ- cyclodextrins consisting of 6, 7 and 8 α-1,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether β-cyclodextrin, e.g., for example, sulfobutyl ether β-cyclodextrin, also known as Captisol. See, *e.g.,* U.S. 5,376,645. In certain embodiments, the formulation comprises hexapropyl-β-cyclodextrin *(e.g.,* 10-50% in water).

Treatment

As stated herein, it is known that EGFR kinase have roles in tumourigenesis as well as numerous other diseases. We have found that the compounds of the present disclosure possess potent anti-tumour activity which it is believed is afforded by way of inhibition of EGFR kinase.

The compounds of the present disclosure are of value as anti-tumour agents. Particularly, the compounds of the present disclosure are of value as anti-proliferative, apoptotic and/or anti-invasive agents in the containment and/or treatment of solid and/or liquid tumour disease. Particularly, the compounds of the present disclosure are expected to be useful in the prevention or treatment of those tumours which are sensitive to inhibition of EGFR. Further, the compounds of the present disclosure are expected to be useful in the prevention or treatment of those tumours which are mediated alone or in part by EGFR. The compounds may thus be used to produce an EGFR enzyme inhibitory effect in a warmblooded animal in need of such treatment.

As stated herein, inhibitors of EGFR kinase should be of therapeutic value for the treatment of cancer, such as ovarian cancer, cervical cancer, colorectal cancer, breast cancer, pancreatic cancer, glioma, glioblastoma, melanoma, prostate cancer, leukemia, lymphoma, non-Hodgkin's lymphoma, gastric cancer, lung cancer, hepatocellular cancer, stomach cancer, gastrointestinal stromal tumor (GIST), thyroid cancer, cancer of bile duct, endometrial cancer, kidney cancer, anaplastic large cell lymphoma, acute myeloid leukemia (AML), multiple myeloma, melanoma, and mesothelioma.

Anti-cancer effects which are accordingly useful in the treatment of cancer in a patient include, but are not limited to, anti-tumour effects, the response rate, the time to disease progression and the survival rate. Anti-tumour effects of a method of treatment of the present disclosure include but are not limited to, inhibition of tumour growth, tumour growth delay, regression of tumour, shrinkage of tumour, increased time to regrowth of tumour on cessation of treatment, slowing of disease progression. Anti-cancer effects include prophylactic treatment as well as treatment of existing disease.

A EGFR kinase inhibitor, or a pharmaceutically acceptable salt thereof, may also be useful for the treatment patients with cancers, including, but not limited to, haematologic malignancies such as leukaemia, multiple myeloma, lymphomas such as Hodgkin's disease, non-Hodgkin's lymphomas (including mantle cell lymphoma), and myelodysplastic syndromes, and also solid tumours and their metastases such as breast cancer, lung cancer (non-small cell lung cancer (NSCL), small cell lung cancer (SCLC), squamous cell carcinoma), endometrial cancer, tumours of the central nervous system such as gliomas, dysembryoplastic neuroepithelial tumour, glioblastoma multiforme, mixed gliomas, medulloblastoma, retinoblastoma, neuroblastoma, germinoma and teratoma, cancers of the gastrointestinal tract such as gastric cancer, oesophagal cancer, hepatocellular (liver) carcinoma, cholangiocarcinomas, colon and rectal carcinomas, cancers of the small intestine, pancreatic cancers, cancers of the skin such as melanomas (in particular metastatic melanoma), thyroid cancers, cancers of the head and neck and cancers of the salivary glands, prostate, testis, ovary, cervix, uterus, vulva, bladder, kidney (including renal cell carcinoma, clear cell and renal oncocytoma), squamous cell carcinomas, sarcomas such as osteosarcoma, chondrosarcoma, leiomyosarcoma, soft tissue sarcoma, Ewing's sarcoma, gastrointestinal stromal tumour (GIST), Kaposi's sarcoma, and paediatric cancers such as rhabdomyosarcomas and neuroblastomas.

The effective dose of the compound of the present disclosure is usually at an average daily dose of 0.01 mg to 50 mg compound/kg of patient weight, alternatively 0.1 mg to 25 mg compound/kg of patient weight, in single or multiple administrations. Generally, the compound of the present disclosure can be administered to the patient who needs this treatment in the daily dose range of about 1 mg to about 3500 mg per patient, alternatively 10 mg to 1000 mg. For example, the daily dose per patient can be 10, 20, 30, 40, 50, 60, 70, 80, 90, 100, 150, 200, 250, 300, 350, 400, 500, 600, 700, 800, 900 or 1000 mg. It can be administered once or several times a day, weekly (or several days apart) or on an intermittent schedule. For example, on a weekly basis (e.g. every Monday), the compound can be administered one or more times a day, variably for several weeks, for example 4-10 weeks. Or, the compound may be administered daily for several days (e.g. 2-10 days), and then a few days (e.g. 1-30 days) without administering the compound, repeating the cycle arbitrarily or repeating a given number of times, e.g. 4-10 cycles. For example, the compound of the present disclosure can be administered daily for 5 days, and then interrupted for 9 days, and then administered daily for 5 days, then interrupted for 9 days, and so on, repeating the cycle arbitrarily or repeating 4-10 times in total.

Combination therapy

The treatment defined herein may be applied as a sole therapy or may involve, in addition to the compounds of the present disclosure, conventional surgery or radiotherapy or chemotherapy. Accordingly, the compounds of the present disclosure can also be used in combination with existing therapeutic agents for the treatment of cancer.

In addition to the compound disclosed herein, conventional surgery, radiotherapy, chemotherapy, or immunotherapy can be used for the treatment. Such chemotherapy can be administered simultaneously, sequentially or separately with the compound disclosed herein and may include one or more of the following categories of anti-tumour agents:

(i) antiproliferative/antineoplastic drugs and combinations thereof, as used in medical oncology, such as alkylating agents (for example czs-platin, oxaliplatin, carboplatin, cyclophosphamide, nitrogen mustard, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, cytosine arabinoside, and hydroxyurea); antitumour antibiotics (for example anthracyclines like adriamycin, bleomycin, doxorubicin, daunomycin, epirubicin, idarubicin, mitomycin- C, dactinomycin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, topotecan and camptothecin);

(ii) cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5a-reductase such as finasteride;

(iii) anti-invasion agents [for example c-Src kinase family inhibitors like 4-(6-chloro-2,3-methylenedioxyanilino)-7-[2-(4-methylpiperazin-1-yl)ethoxy]-5-tetrahydropyran-4-yloxyquinazoline [AZD0530 (saracatinib)], N-(2-chloro-6-methylphenyl)-2-{6-[4-(2-hydroxyethyl)piperazin-1-yl]-2-methylpyrimidin-4-ylamino } thiazole-5 -carboxamide (dasatinib, BMS-354825) and bosutinib (SKI-606), and metalloproteinase inhibitors like marimastat, inhibitors of urokinase plasminogen activator receptor function or antibodies to heparanase] ;

(iv) inhibitors of growth factor function: for example such inhibitors include growth factor antibodies and growth factor receptor antibodies (for example the anti-erbB2 antibody trastuzumab [Herceptin], the anti-EGFR antibody panitumumab and the anti-erbB1 antibody cetuximab [Erbitux, C225]); such inhibitors also include tyrosine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)-quinazolin-4-amine (gefitinib, ZD 1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)-quinazolin-4-amine (CI 1033), erbB2 tyrosine kinase inhibitors such as lapatinib); inhibitors of the hepatocyte growth factor family; inhibitors of the insulin growth factor family; inhibitors of the platelet-derived growth factor family such as imatinib and/or nilotinib (AMN107); inhibitors of serine/threonine kinases (for example Ras/Raf signaling inhibitors such as farnesyl transferase inhibitors, for example sorafenib (BAY 43-9006), tipifarnib (R1 15777) and lonafarnib (SCH66336)), inhibitors of cell signaling through MEK and/or AKT kinases, c-kit inhibitors, abl kinase inhibitors, PI3 kinase inhibitors, Plt3 kinase inhibitors, CSF-1R kinase inhibitors, IGF receptor (insulinlike growth factor) kinase inhibitors; aurora kinase inhibitors (for example AZD1152, PH739358, VX-680, MLN8054, R763, MP235, MP529, VX-528 and AX39459) and cyclin dependent kinase inhibitors such as CDK2 and/or CDK4 inhibitors;

(v) antiangiogenic agents such as those which inhibit the effects of vascular endothelial growth factor, [for example the anti-human vascular endothelial cell growth factor antibody bevacizumab (Avastin) and for example, a VEGF receptor tyrosine kinase inhibitor such as vandetanib (ZD6474), vatalanib (PTK787), sunitinib (SUI 1248), axitinib (AG-013736), pazopanib (GW 786034) and 4-(4-fiuoro-2-methylindol-5-yloxy)-6-methoxy-7-(3-pyrrolidin-1-ylpropoxy)quinazoline (AZD2171) and compounds that work by other mechanisms (for example linomide, inhibitors of integrin $\alpha\nu\beta3$ function and angiostatin)];

(vi) vascular damaging agents such as combretastatin A4;

(vii) an endothelin receptor antagonist, for example zibotentan (ZD4054) or atrasentan;

(viii) antisense therapies, for example those which are directed to the targets listed above, such as ISIS 2503, an anti-ras antisense;

(ix) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy; and

(x) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines, approaches using anti-idiotypic antibodies, approaches to decrease the function of immune suppressive cells such as regulatory T cells, myeloid-derived suppressor cells or IDO (indoleamine 2,3,-deoxygenase)-expressing dendritic cells, and approaches using cancer vaccines consisting of proteins or peptides derived from tumour-associated antigens such as NY-ESO-1, MAGE-3, WTI or Her2/neu.

## Examples

The materials or reagents used herein are commercially available or are prepared by synthetic methods generally known in the art.

Preparation of intermediates

**Preparation of 2-(2,6-dioxopiperidin-3-yl)-4-hydroxyisoindoline-1,3-dione (A2-1)**

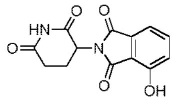

4-Hydroxyisobenzofuran-1,3-dione (5.0 g, 30.5 mmol), 3-aminopiperidine-2,6-dione (6.85 g, 41.8 mmol) and sodium acetate (4.1 g, 50.0 mmol) were dispersed in 100 mL of $CH_3COOH$, and the mixture was stirred and refluxed at 140°C under the protection of nitrogen for 8h. The mixture was cooled to room temperature and concentrated under reduced pressure to remove $CH_3COOH$, and then 200 mL of water was added to the residue. The mixture was then slurried with stirring for 2h. The mixture was filtrated with suction. The solid product was rinsed twice with water, and dried with baking to afford 7.4 g of off-white solid product. Yield: 89.15%. LCMS: $[M + H]^+ = 275$. $^1H$-NMR: (400 MHz, DMSO-$d_6$) $\delta$ 11.164 (s, 1H), 11.080 (s, 1H), 7.675 (dd, *J* = 7.2 Hz, 8.4 Hz, 1H), 7.329 (dd, *J* = 7.2 Hz, 26.4 Hz, 2H), 5.094 (dd, *J* = 5.2 Hz, 12.8 Hz, 1H), 2.919-2.840 (m, 1H), 2.619-2.530 (m, 2H), 2.048-1.989 (m, 1H).

**Preparation of 2-(2,6-dioxopiperidin-3-yl)-4-fluoroisoindoline-1,3-dione (A6-1)**

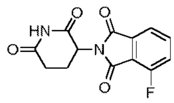

The preparation method was the same as the preparation of 2-(2,6-dioxopiperidin-3-yl)-4-hydroxyisoindoline-1,3-dione, and 4-fluoroisobenzofuran-1,3-dione was used as raw material to afford a white solid. LCMS: $[M + H]^+ = 277$.

**Preparation of 4-bromo-2-(2,6-dioxopiperidin-3-yl)isoindoline-1,3-dione (A1-1)**

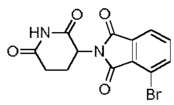

The preparation method was the same as the preparation of 2-(2,6-dioxopiperidin-3-yl)-4-hydroxyisoindoline-1,3-dione, and 4-bromoisobenzofuran-1,3-dione was used as raw material to afford a white solid. LCMS: $[M + H]^+ = 337$, 339.

**Preparation** of **2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)acetic acid (A6-5)**

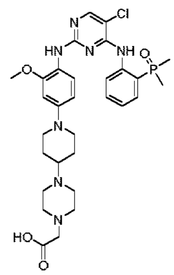

(2-((5-Chloro-2-((2-methoxy-4-(4-(piperazin-1-yl)piperidin-1-yl)phenyl)amino)pyrimidin-4-yl)amino)phenyl)dimethyl phosphorus oxide (300 mg, 0.527 mmol), potassium carbonate (145 mg, 1.054 mmol) and tert-butyl bromoacetate (113.04 mg, 0.527 mmol) were added successively to a 100mL three-necked flask equipped with a condenser and dissolved in DMF (5 mL). The mixture was heated to 60°C, and reacted with stirring for 3h. The mixture was cooled to room temperature, then diluted by adding 20 mL of water, and extracted with ethyl acetate for three times (20 mL for each time). The organic phases were combined, washed with saturated brine for three times, dried with anhydrous sodium sulfate, and concentrated under reduced pressure to afford a crude product, which was separated by silica gel column chromatography to afford 120 mg of yellow solid. The solid afforded above was dissolved in 1,4-dioxane (5 mL), 2 mL of HCl/1,4-dioxane solution (4 mol/L) was added, and the mixture was stirred at room temperature for 2h. The reaction result was detected by LCMS. The mixture was basically the title product, and it was concentrated under reduced pressure to afford 112 mg of yellow solid. Yield: 34%. LCMS: $[M + H]^+$ = 628.

**Preparation of 3-(4-bromo-1-oxoisoindolin-2-yl)piperidine-2,6-dione (C1-1)**

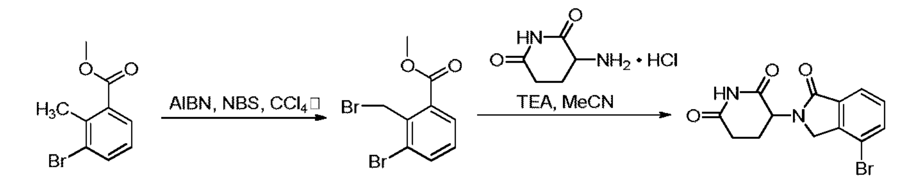


Methyl 3-bromo-2-methylbenzoate (1.14 g, 5.0 mmol) was dissolved in 20.0 mL of $CCl_4$. NBS (1.34 g, 7.5 mmol) and AIBN (164 mg, 1.0 mmol) were added under nitrogen protection. The mixture was heated to 85°C, and reacted under reflux for 20 h. TLC showed that there was no raw material remaining. The mixture was cooled to room temperature, then filtered with suction and concentrated under reduced pressure to afford a crude product, which was purified by Flash to afford 1.35g of light-yellow oily product. The oily compound (1.35 g, 4.41 mmol) and 3-aminopiperidine-2,6-dione hydrochloride (941 mg, 5.74 mmol) were dispersed in 25.0 mL of anhydrous MeCN, and TEA (580 mg, 5.74 mmol) was added. The mixture was heated to 80°C, and reacted under reflux for 16h. LCMS showed that the reaction was completed. The mixture was cooled to room temperature, and then filtrated with suction. The filter cake was washed with MeCN for three times, and dried with baking to afford compound C1-1(1.31 g, yield: 92.3%). LCMS: $[M + H]^+$ = 323, 325.

**Preparation** of **2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-N-(piperidin-4-yl)acetamide (C88-4)**

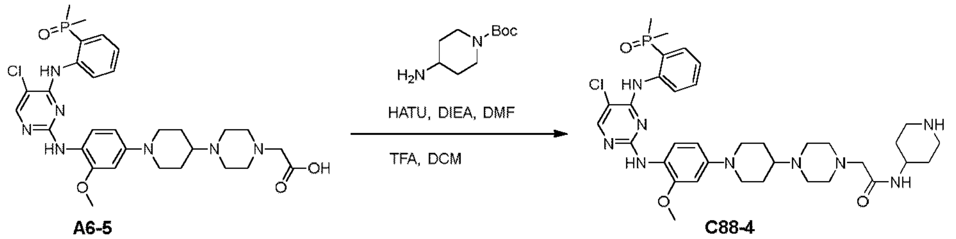


**A6-5** **C88-4**

Compound **A6-5** (75mg, 0.12mmol), HATU(45mg, 0.118mmol), and DIEA(77mg, 0.60mmol) were dissolved in 2.5mL of DMF, and the mixture was stirred at room temperature under nitrogen protection for 1h. Then, tert-butyl 4-aminopiperidine-1-carboxylate (26mg, 0.13mmol) was added and the mixture was further stirred for 2.5h. The solvent was removed under reduced pressure, and the residue was dissolved in dichloromethane (4.0 mL). TFA (1.0 mL) was added dropwise, and the mixture was stirred at room temperature for 2h. The solvent was removed under reduced pressure, and the residue was separated by thin layer chromatography to afford compound **C88-4**. LCMS: $[M + H]^+ = 710$.

**Preparation of 3-(5-bromo-1-oxoisoindolin-2-yl)piperidine-2,6-dione (C192-1)**

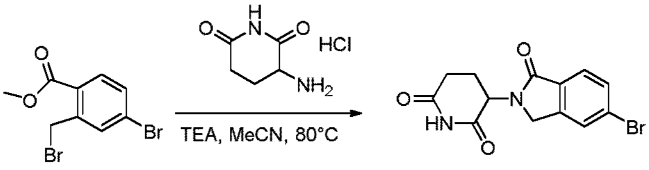


**C192-1**

Methyl 4-bromo-2-(bromomethyl)benzoate (300mg, 0.974mmol), 3-aminopiperidine-2,6-dione hydrochloride (208mg, 1.266mmol), and triethylamine (128mg, 1.266mmol) were dissolved in 5mL of acetonitrile, and the mixture was stirred at 80°C under nitrogen protection overnight. The mixture was cooled to room temperature. The solvent was removed under reduced pressure, and the crude product was purified by Flash to afford white solid compound **C192-1**(180mg, yield 57.0%). LCMS: $[M + H]^+ = 323, 325$.

**Preparation** of **4-(7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-7-oxoheptyl)-2-(2,6-dioxopiperidin-3-yl)isoindoline-1,3-dione (A1)**

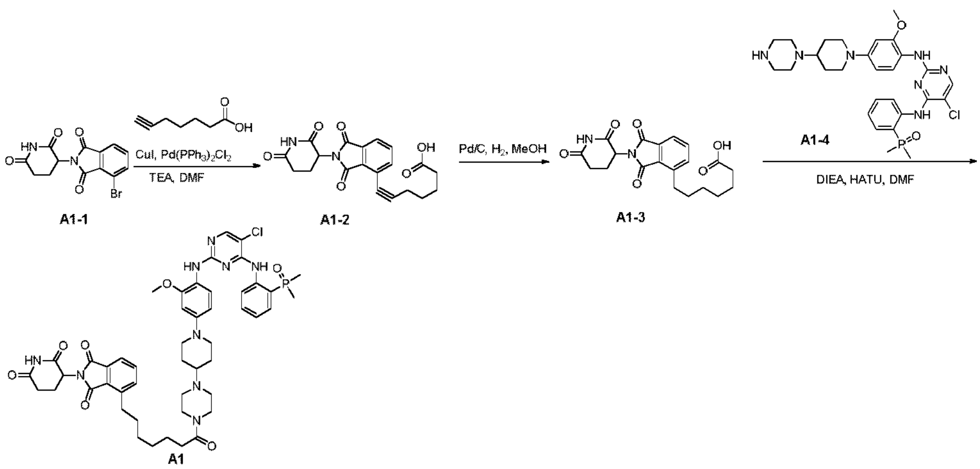


A1-1 A1-2 A1-3 A1

Step 1:

A1-1(600 mg, 1.79 mmol), 6-alkynylheptanoic acid (450 mg, 3.58 mmol), CuI (69 mg, 0.36 mmol), $Pd(PPh_3)_2Cl_2$ (501 mg, 0.71 mmol) and triethylamine (903 mg, 8.94 mmol) were added to DMF (15 mL), and the mixture was stirred at 70°C under nitrogen protection for 5h. TLC showed that the raw materials were consumed and LCMS showed that the title product was produced. The mixture was concentrated under reduced pressure, and the crude product was separated by column chromatography to afford solid **A1-2** (680 mg, yield: 99.7%). LCMS: $[M + H]^+ = 383$.

Step 2:

**A1-2** (340 mg, 0.89 mmol) and Pd/C (10%, 74 mg) were added to methanol (8 mL), and the mixture was hydrogenated at room temperature at 3MPa for 7h. The mixture was filtered to remove solids, and the solvent was

removed under reduced pressure to afford solid A1-3 (300 mg, yield: 87.5%). LCMS: $[M + H]^+ = 387$.

Step 3:

**A1-3** (85 mg, 0.22 mmol), DIEA (85 mg, 0.66 mmol) and HATU (84 mg, 0.22 mmol) were dissolved in DMF, and the mixture was stirred at room temperature under nitrogen protection for 0.5h. Then, A1-4(175 mg, 0.31 mmol) was added and the mixture was further stirred for 3h. TLC showed that the raw materials were consumed and LCMS showed that the title product was produced. The mixture was directly purified by Flash chromatography to afford white solid A1 (50 mg, yield: 24.3%). LCMS: $[M + H]^+ = 938$.

**Preparation of 4-((7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-7-oxoheptyl)oxy)-2-(2,6-dioxopiperidin-3-yl)isoindoline-1,3-dione (A2)**

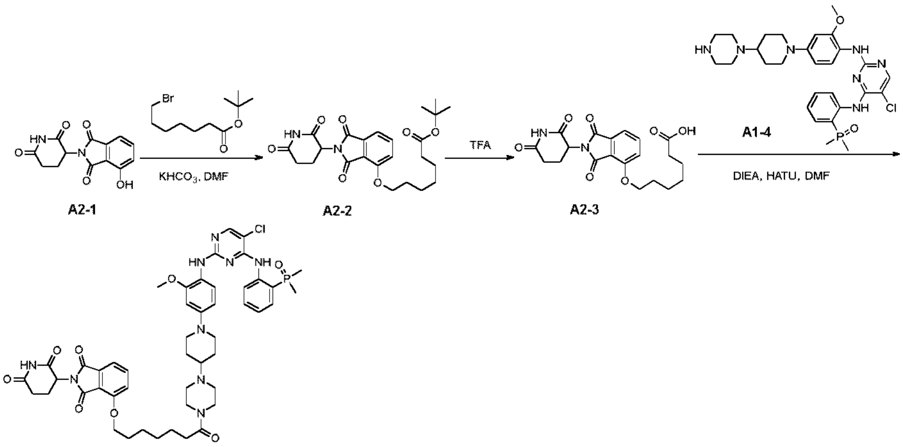


A2-1 A2-2 A2-3

A2

Step 1:

A2-1(200 mg, 0.73 mmol), tert-butyl 7-bromoheptanoate (232 mg, 0.87 mmol), KI (12 mg, 0.072 mmol) and potassium bicarbonate (110 mg) were dispersed in DMF (5 mL), and the mixture was stirred at 60°C under nitrogen protection overnight. The mixture was filtered. The solvent was removed under reduced pressure, and the residue was purified by **Flash** chromatography to afford 160 mg of white solid A2-2.

Step 2:

**A2-2** (160 mg) was dissolved in 2 mL of TFA, and the mixture was stirred at room temperature for 1h. The solvent was removed under reduced pressure to afford crude A2-3, which was directly used in the next step.

Step 3:

**A2-3** (140 mg, 0.348 mmol), DIEA (225 mg, 1.744 mmol), and HATU (133 mg, 0.35 mmol) were dissolved in DMF (7 mL), and the mixture was stirred at room temperature under nitrogen protection for 0.5h. Then, **A1-4** (237 mg, 0.417 mmol) was added and the mixture was further stirred for 2h. The mixture was directly purified by Flash chromatography to afford **A2** (25 mg, 7.5%). LCMS: $[M + H]^+ = 954$.

**Preparation of N-(2-(2-(2-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)ethoxy)ethoxy)ethoxy)ethyl)-2-((2-(2,6-dioxopiperidin-3-yl)-1,3-dioxoisoindolin-4-yl)oxy)acetamide (A3)**

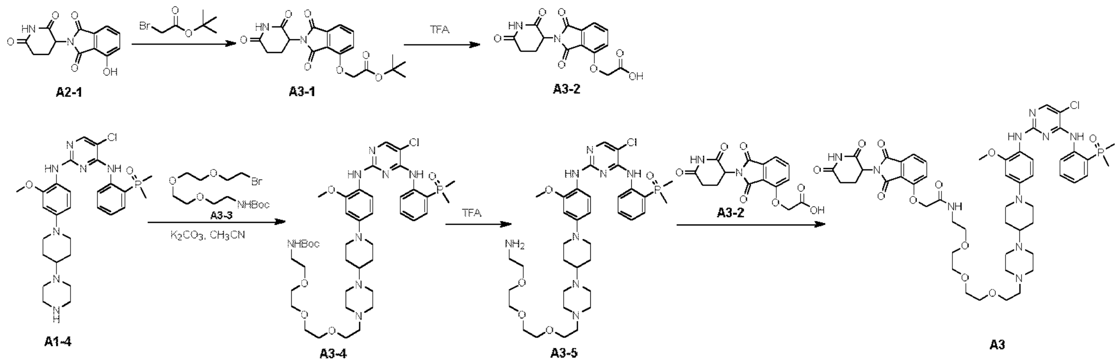


Step 1:

**A2-1**(300 mg, 1.15 mmol), tert-butyl bromoacetate (467 mg, 1.79 mmol), KI (18 mg, 0.109 mmol) and potassium bicarbonate (164 mg, 1.64 mmol) were dispersed in DMF, and the mixture was stirred at 60°C under nitrogen protection overnight. The mixture was filtered. The solvent was removed under reduced pressure, and the residue was purified by Flash chromatography to afford 116 mg of product **A3-1.** Yield: 26.9%.

Step 2:

**A3-1** (116 mg) was dissolved in TFA (1 mL), and the mixture was stirred at room temperature for 1h. The solvent was evaporated to afford crude **A3-2,** which was directly used in the next step.

Step 3:

Under nitrogen protection, **A1-4** (150 mg, 0.21 mmol), **A3-3** (94 mg, 0.26 mmol), and $K_2CO_3$ (73 mg, 0.53 mg) were added to acetonitrile, and the mixture was stirred at 90°C overnight. The mixture was filtered to remove solids. The solvent was removed under reduced pressure, and the residue was purified by Flash chromatography to afford compound **A3-4** (110 mg, yield: 49.5%).

Step 4:

**A3-4** (110 mg, 0.13 mmol) and TFA (1 mL) were dissolved in dichloromethane, and the mixture was stirred at room temperature for 1h. The solvent was removed under reduced pressure to afford crude **A3-5,** which was directly used in the next step.

Step 5:

**A3-2** (99 mg, 0.31 mmol), DIEA (192 mg, 1.49 mmol), and HATU (118 mg, 0.31 mmol) were dissolved in DMF, and the mixture was stirred at room temperature under nitrogen protection for 0.5h. Then, **A3-5** (222 mg, 0.30 mmol) was added and the mixture was further stirred for 3h. The mixture was directly purified by Flash chromatography to afford white solid **A3** (25 mg, yield: 18.1%). LCMS: $[M + H]^+ = 1059$.

**Preparation of 2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-N-(2-((2-(2,6-dioxopiperidin-3-yl)-1,3-dioxoisoindolin-4-yl)amino)ethyl)acetamide (A6)**

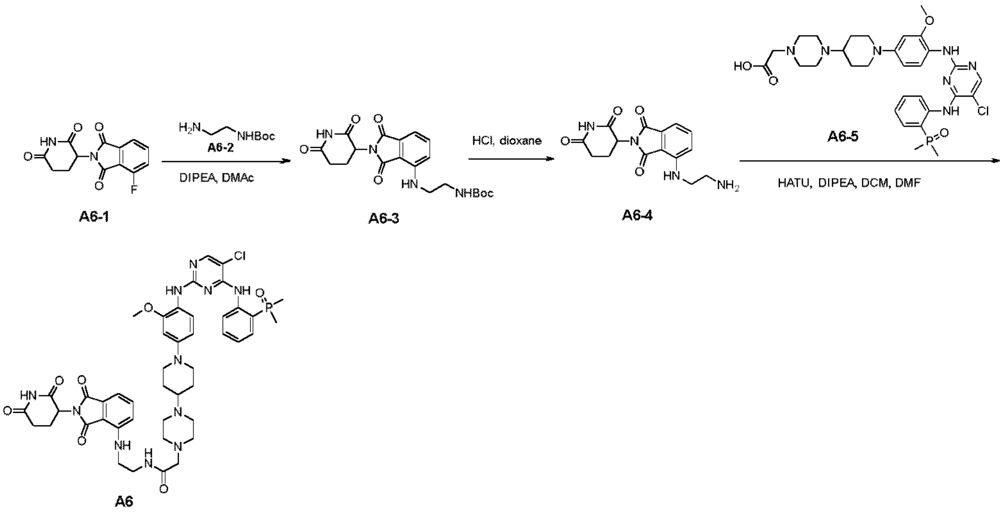


A6-1 A6-3 A6-4

A6

Step 1:

Compound **A6-1**(276.2 mg, 1.0 mmol), compound **A6-2** (160.2 mg, 1.0 mmol), DIPEA (387 mg, 3.0 mmol) and N,N-dimethylacetamide (5 mL) were added successively to a three-necked flask equipped with a condenser. The mixture was heated to 90°C and reacted with stirring under nitrogen protection overnight. LCMS showed that the raw materials were basically consumed, and the title product was produced. The mixture was cooled, then diluted with 50 mL of water, and extracted with ethyl acetate for three times (25 mL for each time). The organic phases were combined, and washed with saturated brine for three times (20 mL for each time). The organic phase was dried with anhydrous sodium sulfate for 2h, and filtered. The filtrate was concentrated under reduced pressure to afford a crude product, which was separated by silica gel column chromatography to afford yellow solid A6-3(200 mg, yield: 48%).

Step 2:

Compound **A6-3** (100 mg, 0.24 mmol) and 1,4-dioxane (5 mL) were added to a 50 mL single-necked flask and dissolved with stirring. HCl/1,4-dioxane solution (2 mL, 4M) was added, and the mixture was reacted with stirring at room temperature for 2h. TLC showed that the raw materials were consumed and LCMS showed that the title product was produced. The mixture was concentrated under reduced pressure to afford a hydrochloride of compound **A6-4,** which was yellow solid (80 mg, yield: 95%). LCMS: $[M + H]^+$ = 317.

Step 3:

Compound **A6-5(31.4** mg, 0.05 mmol), HATU (23 mg, 0.06 mmol) and dichloromethane (3 mL) were added to a 50 mL three-necked flask. The mixture was dissolved with stirring at room temperature under nitrogen protection. DIPEA (64 mg, 0.5 mmol) was then added, and the mixture was reacted with stirring at room temperature for 30 minutes. Then, a solution of compound **A6-4(22** mg, 0.05 mmol) in DMF (2.0 mL) was added. The mixture was stirred at room temperature overnight. LCMS showed that the title product was produced. The mixture was concentrated under reduced pressure to remove dichloromethane, and separated by Flash preparative column chromatography to afford a light-yellow powder, which was compound **A6** (14 mg, yield: 30.4%). LCMS: $[M + H]^+$ = 926.

**Preparation of 2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-N-(2-(2-(2-(2-((2-(2,6-dioxopiperidin-3-yl)-1,3-dioxoisoindolin-4-yl)amino)ethoxy)ethoxy)ethoxy)ethyl)acetamide (A8)**

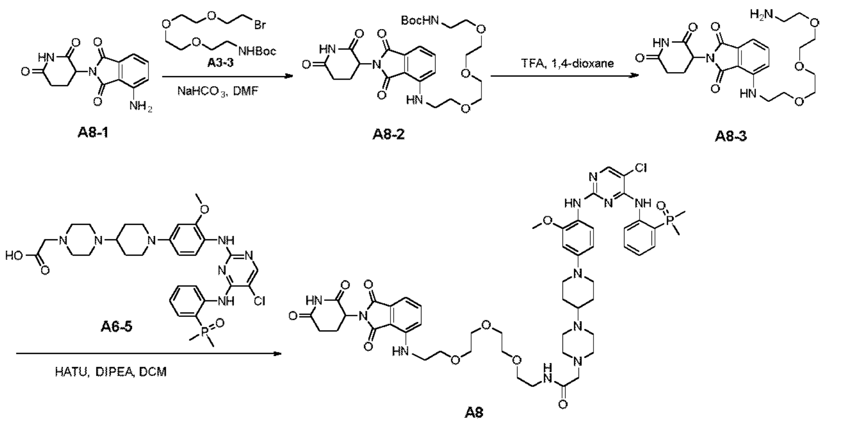


A8-1 A8-2 A8-3 A6-5 A8

Step 1:

Compound **A3-3** (260.7 mg, 0.732 mmol), commercially purchased compound **A8-1** (200 mg, 0.732 mmol), sodium bicarbonate (232 mg, 2.196 mmol) and N,N-dimethylformamide (5 mL) were added successively to a 20 mL microwave reaction tube, and the mixture was heated to 90°C in a microwave reactor under nitrogen protection for 2h (reactor: CEM, power: 100W). Thin layer chromatography showed that compound **A8-1** basically disappeared, and LCMS showed that the title product was produced. The mixture was cooled and filtered to remove inorganic salts, diluted by adding 20 mL of water, and extracted with ethyl acetate for three times (25 mL for each time). The organic phases were combined, washed twice with saturated brine (10 mL for each time), then dried with anhydrous sodium sulfate, and filtered. The filtrate was concentrated under reduced pressure to afford a crude product, which was separated by silica gel column chromatography to afford a compound **A8-2**(100 mg, yield: 24.9%).

Step 2:

Compound **A8-2**(100 mg, 0.182 mmol) and 1,4-dioxane (4 mL) were added to a 50 mL single-necked flask and dissolved with stirring. Trifluoroacetate (2 mL) was then added, and the mixture was reacted with stirring at room temperature for 2h. TLC showed that the reaction was completed. The mixture was concentrated under reduced pressure to afford brown oily product. The brown oily product was dissolved with 20 mL of dichloromethane, washed twice with saturated sodium bicarbonate solution (5 mL for each time) and once with 10 mL of water, dried with anhydrous sodium sulfate, and filtered. The filtrate was concentrated under reduced pressure to afford 55 mg of yellow green solid **A8-3.** Yield: 68%. LCMS: $[M + H]^+ = 449$.

Step 3:

Compound **A6-5** (70 mg, 0.1mmol), HATU (45.6 mg, 0.12 mmol) and dichloromethane (3 mL) were added to a 50 mL three-necked flask. The mixture was dissolved with stirring at room temperature under nitrogen protection. DIPEA (64.5 mg, 0.5 mmol) was then added, and the mixture was reacted with stirring at room temperature for 30 minutes. Then compound **A8-3(**45 mg, 0.1 mmol) was added, and the mixture was stirred at room temperature overnight. LCMS showed that the title product was produced. The mixture was diluted with 20 mL of dichloromethane, washed twice with water (5 mL for each time), and concentrated under reduced pressure to afford a crude product. The crude product was separated by a silica gel preparative plate to afford light-yellow powder **A8** (22 mg, yield: 20.8%). LCMS: $[M + H]^+ = 1058$.

**Preparation of 4-((7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)heptyl)oxy)-2-(2,6-dioxopiperidin-3-yl)isoindoline-1,3-dione (A10)**

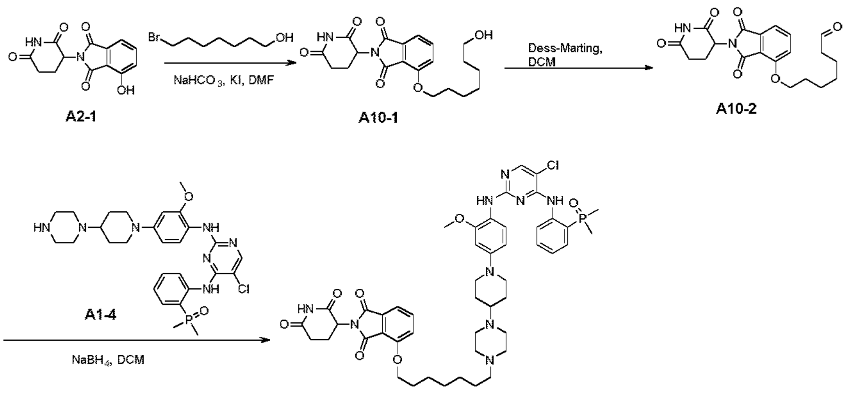


A2-1 A10-1 A10-2 A1-4 A10

Step 1:

**A2-1** (274 mg, 1.0 mmol), $NaHCO_3$ (252 mg, 3.0 mmol) and KI (83 mg, 0.5 mmol) were placed in a 20 mL reaction flask. 5.5 mL of anhydrous DMF was added under nitrogen protection, and 7-bromo-1-heptanol (292.5 mg, 1.5 mmol) was added dropwise with stirring. After the dropwise addition, the mixture was reacted at 70°C for 20 h. The mixture was diluted with 100 mL of ethyl acetate, and then washed successively with saturated $NH_4Cl$ solution (30 mL x 1), $H_2O$ (30 mL x 3) and saturated brine (30 mL x 2). The organic layer was dried with anhydrous $Na_2SO_4$, and filtered with suction to remove the desiccant. The filtrate was concentrated under reduced pressure to afford a crude product, which was purified by Prep-TLC to afford 300 mg of white solid product **A10-1.** Yield: 77.3%. LCMS: $[M + H]^+$ = 389. $^1$H-NMR: (400 MHz, DMSO-$d_6$) δ 11.092 (s, 1H), 7.827 (dd, *J*= 7.6 Hz, 8.8 Hz, 1H), 7.523 (dd, *J* = 8.4 Hz, 29.6 Hz, 2H), 5.098-5.052 (m, 1H), 4.358 (t, *J* = 5.2 Hz, 1H), 4.218 (t, *J* = 6.4 Hz, 2H), 2.926-2.836 (m, 1H), 2.045-1.995 (m, 1H), 1.791-1.722 (m, 2H), 1.492-1.237 (m, 12H).

Step 2:

**A10-1** (80 mg, 0.206 mmol) was dissolved in 15 mL of anhydrous DCM, and Dess-Martin reagent (262 mg, 0.618 mmol) was added under nitrogen protection. After the addition, the mixture was reacted at 55°C for 2h. The mixture was diluted with 30 mL of DCM, and then 15 mL of saturated $NaHCO_3$ solution and 15 mL of saturated $Na_2S_2O_3$ solution were added. The mixture was stirred for 5 min. The organic layer was separated, dried with anhydrous $Na_2SO_4$, and filtered with suction to remove the desiccant. The filtrate was concentrated under reduced pressure to afford crude **A10-2** (75 mg). LCMS: $[M + H]^+$ = 387. The crude product was directly used in the next step.

Step 3:

**A10-2** (75 mg, 0.194 mmol) and **A1-4** (110.3 mg, 0.194 mmol) were dissolved in 12.0 mL of anhydrous DCM. 2 drops of $CH_3COOH$ was added under nitrogen protection. The mixture was then stirred at room temperature for 5 min. $NaBH_4$ (73.7 mg, 1.94 mmol) was then added, and the mixture was reacted with stirring at room temperature for 2h. The mixture was diluted with 30 mL of DCM, and then 15 mL saturated $NH_4Cl$ solution was added and stirred for 5 min. The organic layer was separated, washed once with 15 mL of saturated brine, then dried with anhydrous $Na_2SO_4$, and filtered with suction to remove the desiccant. The filtrate was concentrated under reduced pressure to afford a crude product. The crude product was purified by RP-Flash chromatography to afford 65 mg of white solid with a purity of about 60%. The product was further purified by Prep-TLC to afford 30 mg of white solid product with a purity of 90%. The product was purified again by RP-Flash chromatography to afford 15 mg of white solid pure product. Yield: 8.2%. LCMS: $[M + H]^+$ = 940.5. $^1$H-NMR: (400 MHz, DMSO-$d_6$) δ 11.164 (s, 1H), 11.106 (s, 1H), 8.480 (s, 1H), 8.063-8.046 (m, 2H), 7.827 (t, J = 8.0 Hz, 1H), 7.555-7.502 (m, 2H), 7.450-7.432 (m, 1H), 7.391-7.336 (m, 2H), 7.111 (t, *J*= 7.2 Hz, 1H), 6.625 (d, *J*= 2.4 Hz, 1H), 6.474 (d, *J*= 8.0 Hz, 1H), 5.085 (dd, *J* = 5.6, 12.8 Hz, 1H), 4.204 (t, *J* = 6.4 Hz, 2H), 3.757 (s, 3H), 3.724-3.695 (m, 2H), 2.884-2.851 (m, 1H), 2.688-2.661 (m, 2H), 2.630-2.566 (m, 3H), 2.333-2.246 (m, 6H), 2.227-1.990 (m, 2H), 1.858-1.830 (m, 2H), 1.779-1.745 (m, 9H), 1.524-1.239 (m, 12H).

**Preparation of 4-((9-(4-(1-(4-((5-chloro-4-((2-(dimethylphosphoryl)phenyl)amino)pyrimidin-2-yl)amino)-3-methoxyphenyl)piperidin-4-yl)piperazin-1-yl)nonyl)oxy)-2-(2,6-dioxopiperidin-3-yl)isoindoline-1,3-dione (A11)**

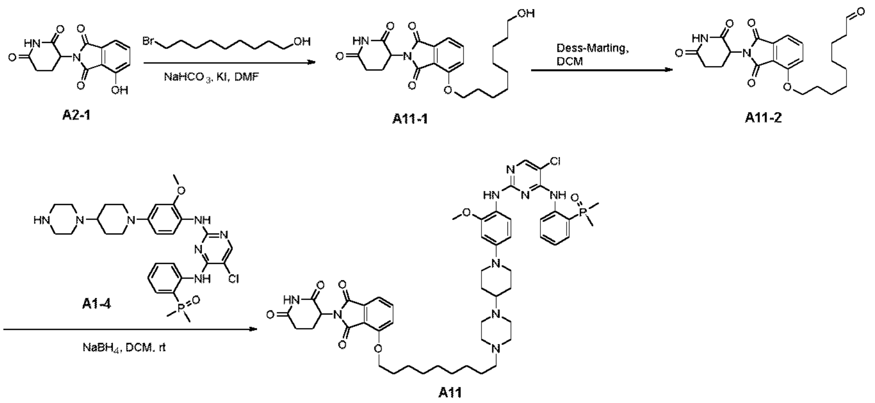


Step 1:

**A2-1** (137 mg, 0.5 mmol), $NaHCO_3$ (126 mg, 1.5 mmol) and KI (41.5 mg, 0.25 mmol) were placed in a 20 mL reaction flask, and 5.0 mL of anhydrous DMF was added under the protection of nitrogen. 9-bromo-1-nonanol (167.3 mg, 0.75 mmol) was added dropwise with stirring. After the dropwise addition was completed, the mixture was reacted at 70°C for 20 h. The mixture was diluted with 80 mL of ethyl acetate and then washed successively with saturated $NH_4Cl$ solution (20 mL × 1), $H_2O$ (20 mL x 3) and saturated brine (20 mL x 2). The organic layer was dried with anhydrous $Na_2SO_4$, and filtered with suction to remove the desiccant. The filtrate was concentrated under reduced pressure to afford a crude product, which was purified by Prep-TLC to afford 70 mg of white solid product **A11-1.** Yield: 33.6%. LCMS: $[M + H]^+$ = 417.

Step 2:

**A11-1** (60 mg, 0.144 mmol) was dissolved in 12 mL of anhydrous DCM, and Dess-Martin reagent (305.8 mg, 0.721 mmol) was added under nitrogen protection. After the addition was completed, the mixture was reacted at 55°C for 3h. The mixture was diluted with 30 mL of DCM, and then 15 mL of saturated $NaHCO_3$ solution and 15 mL of saturated $Na_2S_2O_3$ solution were added and stirred for 5 min. The organic layer was separated, dried with anhydrous $Na_2SO_4$, and filtered with suction to remove the desiccant. The filtrate was concentrated under reduced pressure to afford crude **A11-2** (55 mg). LCMS: $[M + H]^+$ = 415. The crude product was directly used in the next step.

Step 3:

**A11-2** (50 mg, 0.121 mmol) and **A1-4**(68.7 mg, 0.121 mmol) were dissolved in 10.0 mL of anhydrous DCM. 2 drops of $CH_3COOH$ was added under nitrogen protection. The mixture was then stirred at room temperature for 5 min, and $NaBH_4$ (46 mg, 1.21 mmol) was then added. The mixture was reacted with stirring at room temperature for 2h. The mixture was diluted with 20 mL of DCM, and then 15 mL of saturated $NH_4Cl$ solution was added and stirred for 5 min. The organic layer was separated, washed once with 15 mL of saturated brine, then dried with anhydrous $Na_2SO_4$, and filtered with suction to remove the desiccant. The filtrate was concentrated under reduced pressure to afford a crude product, which was purified by RP-Flash chromatography to afford 20 mg of white solid product. Yield: 17%. LCMS: $[M + H]^+$ = 968.5. $^1H$-NMR: (400 MHz, DMSO-$d_6$) δ 11.169 (s, 1H), 11.114 (s, 1H), 8.475 (s, 1H), 8.047 (d, *J*= 6.4 Hz, 2H), 7.825-7.860 (m, 1H), 7.555-7.502 (m, 3H), 7.448-7.317 (m, 2H), 7.092 (t, *J* = 7.6 Hz, 1H), 6.624-6.450 (m, 2H), 5.085 (dd, *J* = 5.2, 12.8 Hz, 1H), 4.201 (t, *J*= 6.4 Hz, 2H), 3.725 (t, *J*= 12.8 Hz, 6H), 3.287 (s, 2H), 2.688-2.540 (m, 6H), 2.328-2.195 (m, 6H), 2.029-1.991 (m, 2H), 1.861-1.830 (m, 2H), 1.779-1.746 (m, 10H), 1.661-1.274 (m, 4H), 1.275-1.237 (m, 8H).

**Preparation of 3-(4-((7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)heptyl)amino)-1-oxoisoindolin-2-yl)piperidine-2,6-dione (A12)**

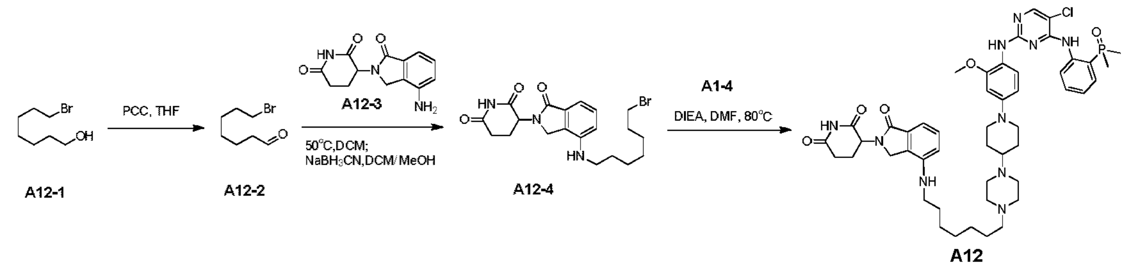


Step 1:

7-Bromo-1-heptanol (200 mg, 1.03 mmol) was added to a solution of pyridinium chlorochromate (333 mg, 1.54 mmol) in THF (5 mL). The mixture was reacted with stirring at room temperature overnight. The solvent was removed under reduced pressure, and the residue was dissolved by adding 20 mL of diethyl ether. The mixture was filtered, and concentrated to afford crude **A12-2** (160 mg, yield: 80.8%). LCMS: $[M + H]^+$ = 193, 195.

Step 2:

**A12-2** (200 mg, 1.04 mmol) and 3-(4-amino-1-oxoisoindolin-2-yl)piperidine-2,6-dione (A12-3)(270 mg, 1.04 mmol) were dissolved in DCM (10 mL) and MeOH (5 mL). The mixture was stirred at 50°C under nitrogen protection for 1h. The mixture was cooled to room temperature, and sodium cyanobohydride (98 mg, 1.56 mmol) and 2 drops of glacial acetic acid were added. The mixture was heated to 50°C and further stirred for 1.5h. The mixture was cooled to room temperature, quenched with aqueous saturated $NH_4Cl$ solution, extracted with dichloromethane, and layered. The organic phases were combined, dried with anhydrous sodium sulfate, allowed to stand, and filtered. The filtrate was concentrated, and the crude product was purified by Flash chromatography to afford **A12-4** (100 mg, yield: 22.1%). LCMS: $[M + H]^+$ = 436, 438.

Step 3:

**A12-4** (10 mg, 0.023 mmol), **A1-4** (13 mg, 0.023 mmol) and DIEA (16 mg, 0.124 mmol) were dissolved in DMF (0.5 mL). The mixture was stirred at 80°C under nitrogen protection for 7h. The solvent was removed under reduced pressure, and the residue was purified by Flash chromatography to afford **A12** (2.97 mg, yield: 14.0%). LCMS: $[M + H]^+$ = 925. $^1$H NMR (400 MHz, DMSO-$d_6$) $\delta$ 11.17 (s, 1H), 11.00 (s, 1H), 8.48 (s, 1H), 8.06 (d, *J* = 5.3 Hz, 1H), 7.53 (dd, *J* = 13.9, 7.4 Hz, 1H), 7.43-7.24 (m, 4H), 7.18 (s, 1H), 7.09 (t, *J* = 7.4 Hz, 1H), 6.92 (d, *J* = 7.4 Hz, 1H), 6.74 (d, *J* = 8.1 Hz, 1H), 6.62 (d, *J* = 2.5 Hz, 1H), 6.47 (d, *J*= 8.3 Hz, 1H), 5.55 (s, 1H), 5.11 (dd, J = 13.1, 5.1 Hz, 1H), 4.23 (d, *J*= 17.0 Hz, 1H), 4.12 (d, *J* = 17.2 Hz, 1H), 3.76 (s, 3H), 3.73 (m, 2H), 3.52 (m, 1H), 3.10(m,2H), 2.93 (m,1H), 2.63(m, 3H), 2.33 (m, 3H), 2.29-2.27 (m, 2H), 2.03-1.98 (m, 4H), 1.85(m, 2H), 1.78(s, 3H), 1.75(s, 3H), 1.57-1.50 (m, 6H), 1.42 (m, 2H), 1.34 (m, 2H), 1.26 (m, 2H).

**Preparation of 3-(4-((9-(4-(1-(4-((5-chloro-4-((2-(dimethylphosphoryl)phenyl)amino)pyrimidin-2-yl)amino)-3-methoxyphenyl)piperidin-4-yl)piperazin-1-yl)nonyl)amino)-1-oxoisoindolin-2-yl)piperidine-2,6-dione (A13)**

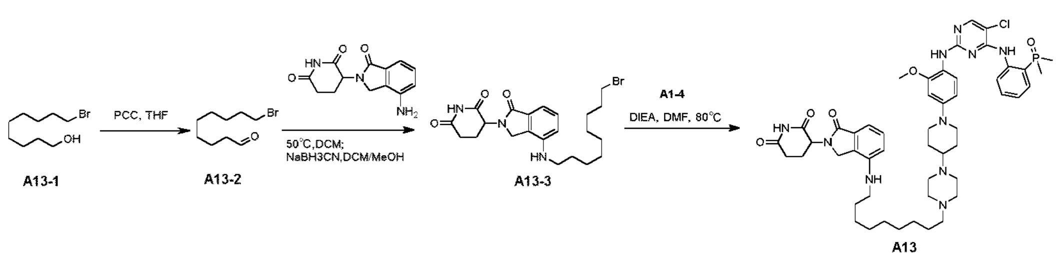


Step 1:

9-Bromo-1-nonanol (222 mg, 1.03 mmol) was added to a solution of pyridinium dichlorochromate (333 mg, 1.54 mmol) in THF (5 mL). The mixture was reacted with stirring at room temperature overnight. The solvent was removed

under reduced pressure, and the residue was dissolved by adding 20 mL of diethyl ether. The mixture was filtered, and concentrated to afford crude **A13-2** (150 mg, yield: 68.2%). LCMS: $[M + H]^+$ = 221, 223.

Step 2:

**A13-2** (228 mg, 1.04 mmol) and 3-(4-amino-1-oxoisoindolin-2-yl)piperidine-2,6-dione (A12-3) (270 mg, 1.04 mmol) were dissolved in DCM (10 mL) and MeOH (5 mL). The mixture was stirred at 50°C under nitrogen protection for 1h. The mixture was cooled to room temperature, and sodium cyanobohydride (98 mg, 1.56 mmol) and 2 drops of glacial acetic acid were added. The mixture was heated to 50°C and further stirred for 1.5h. The mixture was cooled to room temperature, quenched with aqueous saturated $NH_4Cl$ solution, extracted with dichloromethane, and layered. The organic phases were combined, dried with anhydrous sodium sulfate, allowed to stand, and filtered. The filtrate was concentrated, and the crude product was purified by Flash chromatography to afford **A13-3** (100 mg, yield: 20.9%). LCMS: $[M + H]^+$ = 464, 466.

Step 3:

**A13-3** (11 mg, 0.023 mmol), **A1-4** (13 mg, 0.023 mmol) and DIEA (16 mg, 0.124 mmol) were dissolved in DMF (0.5 mL). The mixture was stirred at 80°C under nitrogen protection for 7h. The solvent was removed under reduced pressure, and the residue was purified by Flash chromatography to afford **A13** (2.49 mg, yield: 11.0%). LCMS: $[M + H]^+$ = 953. $^1$H-NMR (400 MHz, DMSO-$d_6$) δ 11.17 (s, 1H), 11.01 (s, 1H), 8.48 (s, 1H), 8.06 (s, 1H), 7.56-7.50 (m, 1H), 7.40-7.25 (m, 4H), 7.18 (s, 1H), 7.09 (t, *J* = 7.5 Hz, 1H), 6.90 (dd, *J* = 15.3, 7.3 Hz, 1H), 6.77-6.67 (m, 1H), 6.62 (d, *J* = 2.6 Hz, 1H), 6.47 (d, *J* = 9.2 Hz, 1H), 5.54 (t, 1H), 5.11 (dd, *J* = 13.2, 5.0 Hz, 1H), 4.23 (d, *J* = 17.2 Hz, 1H), 4.12 (d, *J* = 17.2 Hz, 1H), 3.76 (s, 3H), 3.73 (m, 1H), 3.70 (m, 1H), 3.52 (s, 1H), 3.11 (d, *J* = 6.1 Hz, 2H), 2.99 -2.86 (m, 1H), 2.66 (t, *J*= 11.7 Hz, 3H), 2.32 (m, 3H), 2.26 (m, 2H), 2.01 (m, 4H), 1.85 (d, *J*= 12.2 Hz, 2H), 1.78 (s, 3H), 1.75 (s, 3H), 1.59-1.49 (m, 4H), 1.44-1.38 (m, 4H), 1.34 (m, 2H), 1.30 (m, 2H), 1.27 (m, 2H), 1.26 (m, 2H), 1.25 (m, 2H).

**Preparation** of **2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-N-(2-(2,6-dioxopiperidin-3-yl)-1-oxoisoindolin-4-yl)acetamide (A17)**

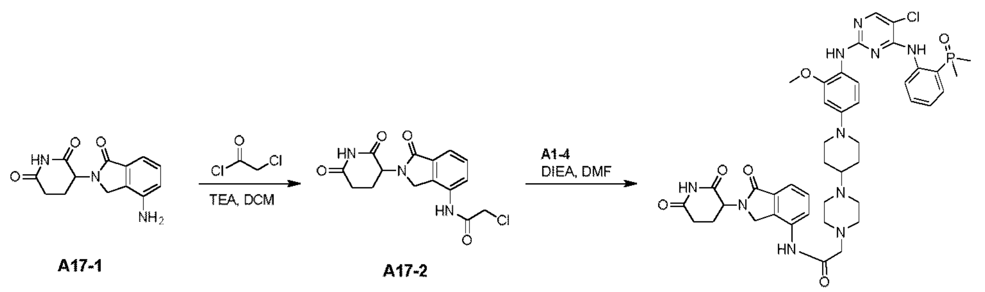


A17-1 A17-2 A17

Step 1:

**A17-1** (or **A12-3)** (200 mg, 0.772 mmol) and TEA(234 mg, 2.32 mmol) were dissolved in DCM (3 mL), and chloroacetyl chloride (78 mg, 0.697 mmol) was added dropwise at 0°C under nitrogen protection. After the dropwise addition, the mixture was further stirred at 0°C for 2h. LCMS showed that the reaction was completed. 1 mL of methanol was added to quench the reaction. The solvent was removed at 40°C under reduced pressure, and the crude product was purified by Flash chromatography to afford white solid **A17-2(170** mg, yield: 73.0%). LCMS: $[M + H]^+$ = 336.

Step 2:

**A17-2** (30 mg, 0.090 mmol), **A1-4** (51 mg, 0.090 mmol) and DIEA (59 mg, 0.457 mmol) were added to DMF (2 mL). The mixture was reacted with stirring at 60°C under nitrogen protection for 2h. LCMS showed that the reaction was completed. The mixture was purified by Flash chromatography to afford off-white solid **A17** (15 mg, yield: 19.5%). LCMS: $[M + H]^+$ = 869. $^1$H-NMR (400 MHz, DMSO-$d_6$) δ 11.17 (s, 1H), 11.03 (s, 1H), 8.49 (s, 1H), 8.06 (m, *J* = 4.4 Hz, 2H), 7.87-7.72 (m, 1H), 7.58-7.49 (m, 3H), 7.38 (dd, *J*= 15.7, 8.0 Hz,1H), 7.10 (t, *J* = 7.6 Hz, 1H), 6.71-6.60 (m, 1H), 6.56-6.45 (m, 1H), 5.15 (dd, *J* = 13.3, 5.2 Hz, 2H), 4.45-4.33 (m, 3H), 3.97-3.89 (m, 1H), 3.80-3.69 (m,4H), 3.49 (d, *J* =

15.9 Hz, 1H), 3.38 (s, 1H), 3.19 (s, 1H), 3.02 (s, 4H), 2.65 (q, *J*= 17.0, 15.0 Hz, 8H), 2.42-2.22 (m, 2H), 1.90 (d, *J*= 12.9 Hz, 3H), 1.78 (s, 3H), 1.75 (s, 2H), 1.28 (d, *J* = 15.9 Hz, 2H).

**Preparation of 3-(4-((2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-2-oxoethyl)amino)-1-oxoisoindolin-2-yl)piperidine-2,6-dione (A18)**

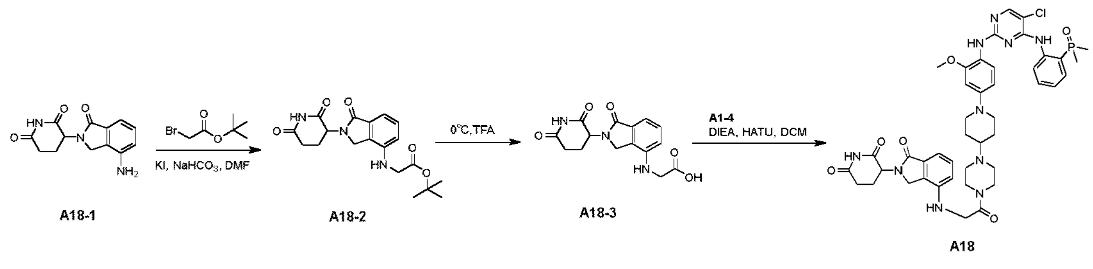


Step 1:

**A18-1** (or **A12-3)** (259.1 mg, 1 mmol), 2-tert-butyl bromoacetate (232.8 mg, 1.2 mmol), KI (16.6 mg, 0.1 mmol) and $NaHCO_3$ (126.0 mg, 1.5 mmol) were added to DMF (7 mL). The mixture was stirred at 60°C under nitrogen protection overnight. The solvent was removed under reduced pressure. The crude product was separated by column chromatography to afford white solid compound **A18-2**(150 mg, yield: 40.2%).

Step 2:

At 0°C, **A18-2(44** mg, 0.118 mmol) was dissolved in 1 mL of TFA. The mixture was stirred at room temperature for 1h. The solvent was removed at 40°C under reduced pressure to afford crude **A18-3.** LCMS: $[M + H]^+$ = 318.

Step 3:

The above crude **A18-3** was dispersed in dichloromethane (2 mL) at room temperature, and DIEA (81 mg, 0.628 mmol) and HATU (44 mg, 0.116 mmol) were added. The mixture was stirred at room temperature under nitrogen protection for 0.5h. **A1-4**(51 mg, 0.090 mmol) was then added and further stirred at room temperature for 3h. LCMS showed that the reaction was completed. The solvent was removed at 40°C under reduced pressure. The crude product was purified by Flash chromatography to afford off-white solid **A18** (25 mg, yield: 32.1%). LCMS: $[M + H]^+$ = 869. $^1$H-NMR (400 MHz, DMSO-$d_6$) δ 11.16 (s, 1H), 8.48 (s, 1H), 8.05 (d, *J*= 7.4 Hz, 2H), 7.56-7.48 (m, 1H), 7.40-7.33 (m, 3H), 7.19 (t, *J*= 7.6 Hz, 1H), 7.09 (t, *J* = 7.4 Hz, 1H), 6.92 (d, *J* = 7.4 Hz, 1H), 6.80 (d, *J* = 7.9 Hz, 1H), 6.68-6.60 (m, 1H), 6.52-6.44 (m, 1H), 5.45 (s, 1H), 5.27 (dd, *J* = 13.3, 5.2 Hz, 1H), 4.51 (d, *J* = 6.1 Hz, 3H), 4.24 (d, *J* = 16.9 Hz, 1H), 4.02 (d, *J* = 16.8 Hz, 1H), 3.75 (s, 4H), 3.46 (d, J = 26.8 Hz, 3H), 3.09 (s, 1H), 2.84 (d, *J* = 17.2 Hz, 1H), 2.66 (t, *J* = 12.1 Hz, 8H), 2.30 (d, *J* = 13.6 Hz, 1H), 2.15 (s, 1H), 1.84 (d, *J*= 11.8 Hz, 1H), 1.76 (d, *J* = 13.5 Hz, 6H), 1.55 (d, *J* = 11.8 Hz, 1H), 1.28 (d, *J*= 15.9 Hz, 2H), 0.85 (t, *J*= 6.6 Hz, 1H).

**Preparation of 3-(4-(7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hept-1-yn-1-yl)-1-oxoisoindolin-2-yl)piperidine-2,6-dione (C1)**

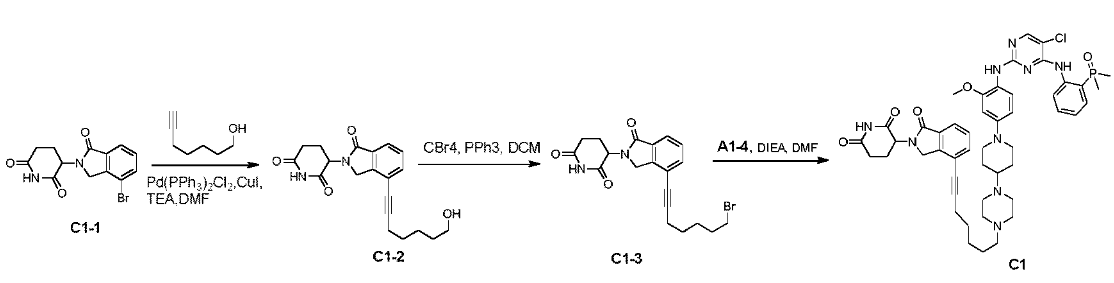

Step 1:

**C1-1** (322 mg, 1 mmol), hept-6-yn-1-ol (280 mg, 2.50 mmol), CuI (38 mmol, 0.2 mmol), $Pd(PPh_3)_2Cl_2$ (280 mg, 0.4 mmol) and TEA (303 mg, 3 mmol) were added to DMF (10 mL). The mixture was reacted with stirring at 70°C under nitrogen protection overnight. The mixture was cooled to room temperature, and 30 mL of water was added. The mixture was extracted with ethyl acetate (20 mL x 3). The organic phases were combined, dried with anhydrous sodium sulfate, and filtered. The filtrate was concentrated, and the crude product was purified by Flash chromatography to afford C1-2, 200 mg, yield: 56.5%. LCMS: $[M + H]^+$ = 355.

Step 2:

**C1-2** (40 mg, 0.11mmol) and $CBr_4$ (75.5 mg, 0.22 mmol) were dissolved in DCM (15 mL). The mixture was stirred at room temperature under nitrogen protection for 1h. The mixture was cooled to 0°C. A solution of $PPh_3$ (59.2 mg, 0.22 mmol) in DCM (2 mL) was added dropwise under nitrogen protection and stirred for 0.5h. The mixture was heated to 50°C and further stirred for 3h. The solvent was removed under reduced pressure, and the crude product was purified by Flash chromatography to afford **Cl-3,** 200 mg, yield: 63.8%. LCMS: $[M + H]^+$ = 417, 419.

Step 3:

**C1-3** (10 mg, 0.024 mmol), **A1-4** (15 mg, 0.026 mmol) and DIEA (16 mg, 0.124 mmol) were dissolved in DMF (0.5 mL). The mixture was stirred at 80°C under nitrogen protection for 7h. The solvent was removed under reduced pressure, and the crude product was purified by Flash chromatography to afford compound C1, 15 mg, yield: 69.1%. LCMS: $[M + H]^+$ = 906.

**Preparation of 4-(2-(1-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)acetyl)piperidin-4-yl)ethoxy)-2-(2,6-dioxopiperidin-3-yl)isoindoline-1,3-dione (C4)**

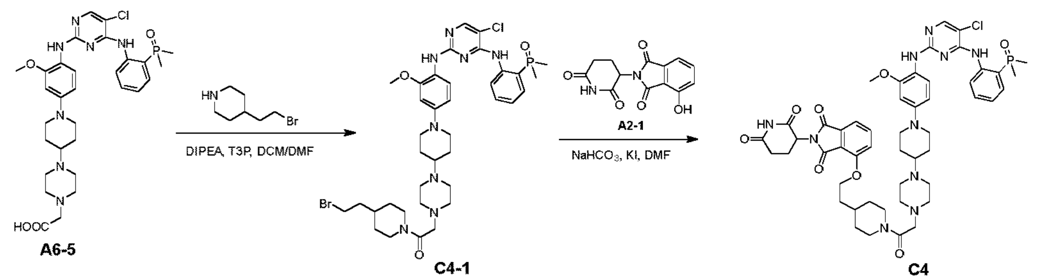


A6-5 C4-1 C4

Step 1:

**A6-5** (66.3 mg, 0.1 mmol) and 4-(2-bromoethyl)piperidine trifluoroacetate (30.5 mg, 0.1 mmol) were dispersed in 5.0 mL of anhydrous DCM, and T3P (159 mg, 0.25 mmol) and DIPEA (64.5 mg, 0.5 mmol) were added successively. After the addition was completed, the mixture was reacted at room temperature for 2h. The mixture was diluted with 20 mL of DCM, and then washed successively with saturated $NH_4Cl$ solution (10 mL × 1) and saturated NaCl (10 mL x 1). The organic layer was dried with anhydrous $MgSO_4$, and filtered with suction. The filtrate was concentrated under reduced pressure to afford a crude product. The crude product was purified by Prep-TLC to afford light-yellow solid product **C4-1,** 35 mg. Yield: 43.8 %. LCMS: $[M + H]^+$ = 801, 803.

Step 2:

**A2-1** (11.3 mg, 0.041 mmol), C4-1 (30.0 mg, 0.038 mmol), KI (3.1 mg, 0.019 mmol) and $NaHCO_3$ (9.45 mg, 0.113 mmol) were dispersed in 2.0 mL of anhydrous DMF. The mixture was reacted at 70°C under nitrogen protection for 20 h. LCMS showed that the reaction was completed. The mixture was directly purified by Prep-HPLC to afford light-yellow solid pure product C4, 13 mg. Yield: 35.1%. LCMS: $[M + H]^+$ = 995.

**Preparation of 2-((2-(2,6-dioxopiperidin-3-yl)-1,3-dioxoisoindolin-4-yl)oxy)acetic acid 3-(4-(1-(4-((5-Chloro-4-((2-(dimethylphosphoryl)phenyl)amino)pyrimidin-2-yl)amino)-3-methoxyphenyl)piperidin-4-yl)piperazin-1-yl)-2,2-dimethyl-3-oxopropyl ester (C6)**

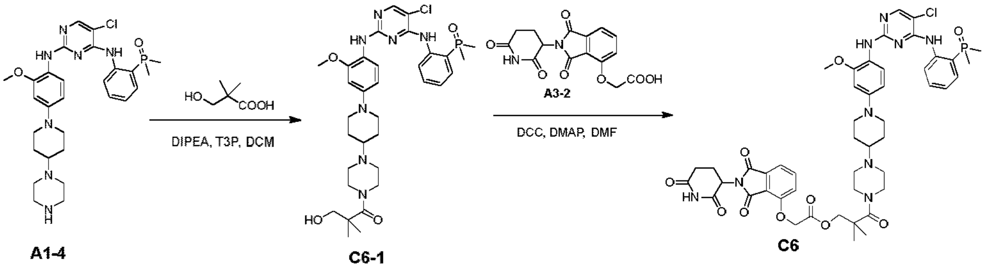


A1-4 C6-1 C6

Step 1:

**A1-4** (56.9 mg, 0.1 mmol), 3-hydroxy-2,2-dimethylpropionic acid (11.8 mg, 0.1 mmol) and T3P (49.4 mg, 0.13 mmol) were dispersed in 3.0 mL of anhydrous DCM, and DIPEA (51.6 mg, 0.4 mmol) was added with stirring. After the addition was completed, the mixture was reacted at room temperature for 2h. LCMS showed that the reaction was completed. The mixture was diluted with 30 mL of ethyl acetate, and then washed successively with saturated $NH_4Cl$ solution (20 mL $\times$ 1) and saturated brine (20 mL $\times$ 1). The organic layer was dried with anhydrous $Na_2SO_4$, and filtered with suction. The filtrate was concentrated under reduced pressure to afford a crude product, which was purified by Prep-TLC to afford white solid product **C6-1,** 65 mg. Yield: 97.0%. LCMS: $[M + H]^+ = 670$.

Step 2:

**C6-1** (80.0 mg, 0.1144 mmol), **A3-2** (57.0mg, 0.1716 mmol), DCC (28.3 mg, 0.1373 mmol) and DMAP (14.0 mg, 0.1144 mmol) were dispersed in 6.0 mL of anhydrous DMF. The mixture was stirred at room temperature under nitrogen protection for 8h. DCC (35.3 mg, 0.1716 mmol) and DMAP (14.0 mg, 0.1144 mmol) were added to the mixture and the reaction was continued at room temperature for 12h. LCMS showed that the reaction was completed. The mixture was diluted with 50 mL of ethyl acetate, and then washed successively with saturated $NH_4Cl$ solution (20 mL $\times$ 1), $H_2O$ (20 mL x 3) and saturated brine (20 mL x 2). The organic layer was dried with anhydrous $Na_2SO_4$, and filtered with suction. The filtrate was concentrated under reduced pressure to afford a crude product. The crude product was purified by Prep-HPLC to afford 20 mg of light-yellow solid pure product. Yield: 17.8 %. LCMS: $[M + H]^+ = 984$. $^1$H-NMR: (400 MHz, DMSO-$d_6$) $\delta$ 11.168 (s, 1H), 11.099 (s, 1H), 8.477 (s, 1H), 8.064 (d, *J*= 7.6 Hz, 2H), 8.064 (t, *J*= 8.0 Hz, 1H), 7.553-7.504 (m, 2H), 7.499-7.321 (m, 3H), 7.111 (t, *J* = 7.6 Hz, 1H), 6.628 (d, *J* = 2.4 Hz, 1H), 6.484 (dd, *J* = 2.4, 8.8 Hz, 1H), 5.123 (s, 2H), 4.164 (s, 2H), 3.760-3.710 (m, 5H), 3.504 (s, 4H), 2.938-2.846 (m, 1H), 2.665-2.455 (m, 8H), 2.373-2.324 (m, 1H), 2.054-1.989 (m, 1H), 1.857-1.746 (m, 8H), 1.565-1.475 (m, 3H), 1.192 (s, 6H).

**Preparation of 4-((9-(4-(1-(4-((4-((2-(dimethylphosphoryl)phenyl)amino)-5-vinylpyrimidin-2-yl)amino)-3-methoxyphenyl)piperidin-4-yl)piperazin-1-yl)nonyl)oxy)-2-(2,6-dioxopiperidin-3-yl)isoindoline-1,3-dione (C25)**

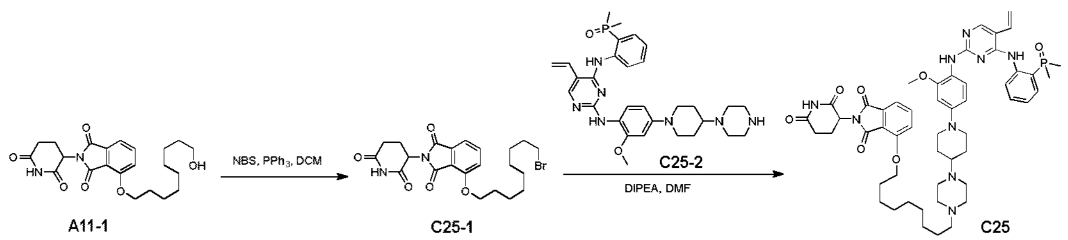


A11-1 C25-1 C25

Step 1:

**All-1** (120 mg, 0.288 mmol), $PPh_3$ (113.4 mg, 0.432 mmol) and NBS (77.0 mg, 0.432 mmol) were dissolved in

8.0 mL of anhydrous DCM. The mixture was reacted at room temperature under $N_2$ protection for 2h. TLC showed that there was no raw material remaining. The mixture was diluted with 40 mL of ethyl acetate, and then washed successively with $H_2O$ (20 mL x 1) and saturated brine (20 mL x 1). The organic layer was dried with anhydrous $Na_2SO_4$, and filtered with suction. The filtrate was concentrated under reduced pressure to afford a crude product, which was purified by Prep-TLC to afford white solid product **C25-1,** 100 mg. Yield: 72.6%. LCMS: $[M + H]^+$ = 479, 481.

Step 2:

**C25-1** (15 mg, 0.0314 mmol) and **C25-2** (17.6 mg, 0.0314 mmol) were dissolved in 2.0 mL of anhydrous DMF. DIPEA (20.3 mg, 0.157 mmol) was added under $N_2$ protection. After the addition was completed, the mixture was reacted at 80°C for 8h. LCMS showed that there was no raw material remaining. The mixture was diluted with 20 mL of ethyl acetate, and then washed successively with saturated $NH_4Cl$ (10 mL x 1), $H_2O$ (10 mL x 3) and saturated brine (10 mL × 1). The organic layer was dried with anhydrous $Na_2SO_4$, and filtered with suction. The filtrate was concentrated under reduced pressure to afford a crude product. The crude product was purified by Prep-HPLC to afford light-yellow solid product **C25,** 3.0 mg. Yield: 10.0%. LCMS: $[M + 1]^+$ = 960.

**Preparation of 4-((7-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)heptyl)oxy)-2-(2,6-dioxopiperidin-3-yl)isoindoline-1,3-dione (C47)**

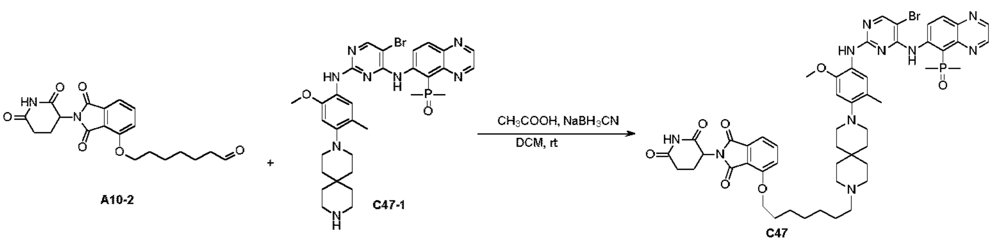


**A10-2** (46.3 mg, 0.12 mmol) and **C47-1** (66.4 mg, 0.1 mmol) were dissolved in 10.0 mL of anhydrous DCM. $CH_3COOH$ (6.0 mg, 0.1 mmol, dissolved in 0.5 mL of DCM) was added under nitrogen protection. The mixture was stirred at room temperature for 1h. Then solid $NaBH_3CN$ (15.7 mg, 0.25 mmol) was added and the mixture was further reacted at room temperature for 2h. LCMS showed that the reaction was completed. The mixture was diluted with 20 mL of DCM, and then washed successively with saturated $NH_4Cl$ (1 × 15 mL) and saturated brine (1 × 15 mL). The organic layer was dried with anhydrous $Na_2SO_4$, and filtered with suction. The filtrate was concentrated under reduced pressure to afford a crude product. The crude product was purified by Prep-HPLC to afford 40 mg of yellow solid pure product. Yield: 38.7%. LCMS: $[M + H]^+$ = 1035, 1037. $^1$H-NMR: (400 MHz, DMSO-$d_6$) δ 12.763 (s, 1H), 11.102 (s, 1H), 9.017 (br s, 1H), 8.886 (d, *J*= 1.6 Hz, 1H), 8.856 (d, *J* = 2.0 Hz, 1H), 8.401 (s, 1H), 8.292 (s, 1H), 7.951 (d, *J* = 9.6 Hz, 1H), 7.841 (t, *J* = 8.0Hz, 1H), 7.532 (d, *J* = 8.4 Hz, 1H), 7.466 (d, *J* = 7.2 Hz, 1H), 7.380 (s, 1H), 6.868 (s, 1H), 5.102 (dd, *J* = 5.6, 12.8 Hz, 1H), 4.834 (s, 4H), 4.238 (t, *J* = 6.0 Hz, 2H), 3.797 (s, 3H), 3.398-3.369 (m, 2H), 3.097-2.857 (m, 8H), 2.575-2.542 (m, 1H), 2.110 (s, 3H), 2.048-2.012 (m, 7H), 1.804-1.678 (m, 6H), 1.572-1.371 (m, 10H).

**Preparation of 2-((1-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)acetyl)piperidin-4-yl)oxy)-N-(2-(2,6-dioxopiperidin-3-yl)-1-oxoisoindolin-4-yl)acetamide (C78)**

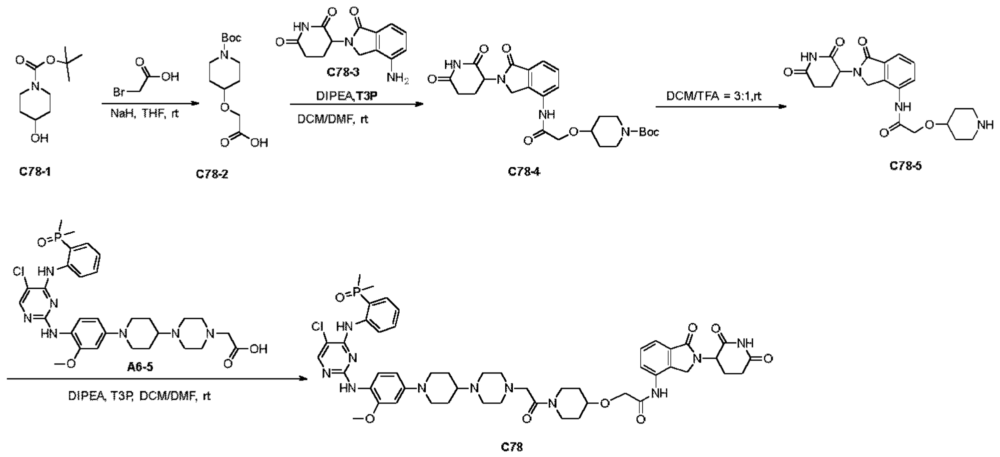


C78-1 C78-2 C78-4 C78-5

C78

Step 1:

**C78-1** (560 mg, 2.78 mmol) was dissolved in 5.0 mL of anhydrous THF, and NaH (222.4 mg, 5.56 mmol) was added at 0°C. The mixture was reacted with stirring at room temperature for 2h. The mixture was cooled to 0°C, and bromoacetic acid (386 mg, 2.78 mmol, dissolved in 5.0 mL of anhydrous THF) was added dropwise to the mixture. After the dropwise addition, the mixture was warmed to room temperature and reacted for 20 h. The mixture was quenched with water (10 mL) at 0°C. The pH of the mixture was adjusted to 11-12 with 1 M NaOH, and then the mixture was extracted twice with diethyl ether. The pH of remaining aqueous phase was adjusted to 3-4 with 2 N HCl, and then the mixture was extracted with diethyl ether (50 mL × 3). The ethyl acetate layers were combined, and washed with saturated brine (50 mL × 1). The organic layer was dried with anhydrous $Na_2SO_4$, and filtered with suction to remove the desiccant. The filtrate was concentrated under reduced pressure to afford crude **C78-2** (520 mg, yield: 72.2%), and the crude product was directly used in the next step.

Step 2:

**C78-2** (104 mg, 0.4 mmol) and **C78-3** (or **A12-3**) (104 mg, 0.4 mmol) were dispersed in a mixture of 8.0 mL of anhydrous DCM and 1.6 mL of anhydrous DMF, and T3P (508.8 mg, 0.8 mmol) and DIPEA (258 mg, 2.0 mmol) were added successively under nitrogen protection. The mixture was reacted at room temperature for 2h. The mixture was diluted with 30 mL of ethyl acetate, and then washed successively with saturated $NH_4Cl$ (20 mL × 1), $H_2O$ (20 mL × 2) and saturated NaCl (20 mL × 2). The organic layer was dried with anhydrous $MgSO_4$, and filtered with suction. The filtrate was concentrated under reduced pressure to afford a crude product, which was purified by Flash chromatography to afford light-yellow oily product **C78-4** (120 mg, yield: 60%).

Step 3:

**C78-4** (120 mg, 0.24 mmol) was dissolved in 3.0 mL of anhydrous DCM, and 1.0 mL of TFA was added with stirring. The mixture was reacted at room temperature for 2.0 h. LCMS showed that the reaction was completed. The mixture was concentrated under reduced pressure to afford a crude product, which was purified by RP-Flash chromatography to afford white solid product **C78-5** (39 mg, yield: 52.1%). LCMS: $[M + H]^+ = 401$.

Step 4:

**C78-5** (50 mg, 0.125 mmol) and **A6-5** (78.4 mg, 0.125 mmol) were dispersed in a mixture of 5.0 mL of anhydrous DCM and 1.0 mL of anhydrous DMF. T3P (159 mg, 0.25 mmol) and DIPEA (80.6 mg, 0.625 mmol) were added under nitrogen protection. The mixture was stirred at room temperature for 2h. The mixture was concentrated under reduced pressure to afford a crude product, which was purified by RP-Flash chromatography to afford yellow solid pure product **C78** (20 mg, yield: 15.9%). LCMS: $[M + H]^+ = 1010$.

**Preparation of 2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-N-(1-(2-((2-(2,6-dioxopiperidin-3-yl)-1-oxoisoindolin-4-yl)amino)-2-oxoethyl)piperidin-4-yl)acetamide (C88)**

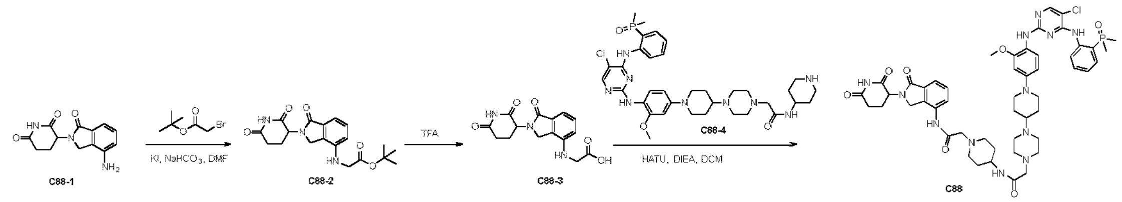


C88-1 C88-2 C88-3 C88

Step 1:

Compound **C88-1** (or **A12-3**) (259.1 mg, 1 mmol), tert-butyl bromoacetate (232.8mg, 1.2mmol), potassium iodide (16.6mg, 0.1mmol) and sodium bicarbonate (126.0mg, 1.5mmol) were added to dry DMF (7 mL). The mixture was stirred at 60°C under nitrogen protection overnight. The solvent was removed under reduced pressure. The crude product was separated by column chromatography to afford white solid compound **C88-2** (150mg, yield: 40.2%).

Step 2:

Compound **C88-2** (44mg, 0.118mmol) was dissolved in 1 mL of TFA at 0°C. The mixture was stirred at room temperature for 1h. The solvent was removed at 40°C under reduced pressure, and 3 mL of toluene was added. The solvent was evaporated to afford crude **C88-3,** which was directly used in the next step. LCMS: $[M + H]^+ = 318$.

Step 3:

The above crude **C88-3** was dispersed in dichloromethane (2 mL) at room temperature, and HATU (9.2mg, 0.024mmol) was added. The mixture was stirred at room temperature under nitrogen protection for 0.5h. DIEA (15mg, 0.116mmol) and compound **C88-4** (51mg, 0.072mmol) were then added and the mixture was further stirred at room temperature for 4.5h. After the reaction was completed, the solvent was removed at 40°C under reduced pressure. The crude product was purified by Flash chromatography to afford off-white solid **C88** (5mg, yield: 19.7%). LCMS: $[M + H]^+ = 1009$.

**Preparation of 4-((1-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)acetyl)azetidin-3-yl)oxy)-2-(2,6-dioxopiperidin-3-yl)isoindoline-1 ,3-dione (C93)**

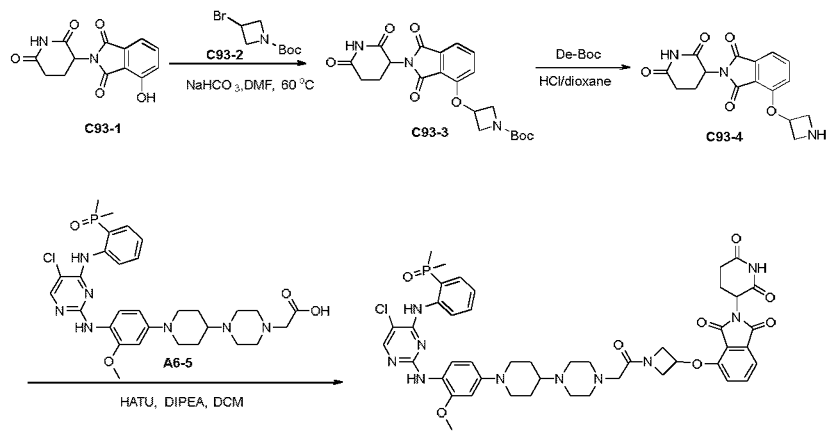


C93-1 C93-3 C93-4 C93

Step 1:

Compound **C93-1** (or **A2-1**) (116 mg, 0.42 mmol), compound **C93-2** (100 mg, 0.42mmol) and sodium bicarbonate (106 mg, 1.26 mmol) were added to a 50 mL single-necked flask, and then DMF (3 mL) was added. The mixture was heated to 60°C and reacted for 12h. After the mixture was cooled, water (30 mL) was added. The mixture was extracted with ethyl acetate for three times (20 mL for each time). The organic phases were combined, washed with brine (15 mL × 3), dried with anhydrous sodium sulfate, and filtered. The filtrate was concentrated under reduced pressure to afford a crude product. The crude product was separated by column chromatography to afford light white solid **C93-3**, 110 mg, yield: 61%.

Step 2:

Compound **C93-3** (110 mg, 0.256 mmol) and HCl/1,4-dioxane solution (5 mL, 4 mol/L) were added to a 25 mL single-necked flask. The mixture was reacted with stirring at room temperature for 2h. The reaction process was monitored by TLC. After the reaction was completed, the solvent was concentrated under reduced pressure, and the residue was dissolved with dichloromethane (20 mL). Sodium bicarbonate solution was added to adjust the pH to 8-9. After extraction and separation, the dichloromethane solution was dried with anhydrous sodium sulfate, and filtered. The filtrate was concentrated under reduced pressure to afford light-yellow solid **C93-4,** 51 mg, yield: 60.7%. LCMS: $[M + H]^+$ = 330.

Step 3:

Compound **C93-4** (20 mg, 0.06 mmol), compound **A6-5** (38 mg, 0.06mmol), HATU (27.3 mg, 0.072mmol) and DIPEA (38.7 mg, 0.03 mmol) were added to a 25 mL single-necked flask, and dichloromethane (2 mL) was added. The reaction was stirred for 3h. After the reaction was completed, dichloromethane (10 mL) was added, and the mixture was washed with water (5 mL × 2). The organic phases were combined, washed with brine (5 mL × 2), dried with anhydrous sodium sulfate, and filtered. The filtrate was concentrated under reduced pressure to afford a crude product, which was separated by Prep-HPLC to afford light white solid **C93,** 5.6 mg, yield: 10%. LCMS: $[M + H]^+$ = 939.

**Preparation of 4-((1-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)acetyl)azetidin-3-yl)methoxy)-2-(2,6-dioxopiperidin-3-yl)isoindoline-1,3-dione (C94)**

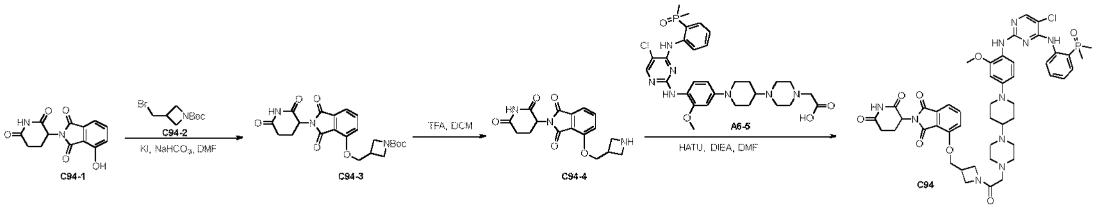

C94-1 C94-3 C94-4 C94

Step 1:

Compound **C94-1** (or **A2-1**) (100mg, 0.365mmol), compound **C94-2** (109mg, 0.438mmol), potassium iodide (6mg, 0.036mmol) and sodium bicarbonate (61mg, 0.726mmol) were added to dry DMF (3 mL). The mixture was stirred at 60°C under nitrogen protection for 24h. The solvent was removed under reduced pressure. The crude product was separated by column chromatography to afford white solid compound **C94-3** (50mg, yield: 31.6%).

Step 2:

Compound **C94-3** (50mg, 0.113mmol) was dissolved in a solution of trifluoroacetate in dichloromethane (1 mL). The mixture was stirred at room temperature for 2h. The solvent was removed at 40°C under reduced pressure to afford crude **C94-4,** which was directly used in the next step. LCMS: $[M + H]^+$ = 344.

Step 3:

The above crude **C94-4** was dispersed in DMF (2 mL) at room temperature, and DIEA (53mg, 0.411mmol) and

HATU (24mg, 0.063mmol) were added. The mixture was stirred at room temperature under nitrogen protection for 1h. Compound **A6-5** (40mg, 0.064mmol) was then added and the mixture was further stirred at room temperature for 2.5h. After the reaction was completed, the solvent was removed at 40°C under reduced pressure, and the crude product was purified by Flash chromatography to afford off-white solid **C94** (20mg, yield: 36.0%). LCMS: $[M + H]^+ = 953$. $^1H$ NMR (400 MHz, DMSO-$d_6$) $\delta$ 11.16 (s, 1H), 11.08 (s, 1H), 8.47 (d, 1H), 8.04 (d, 2H), 7.87-7.81 (m, 1H), 7.57-7.47 (m, 3H), 7.43-7.30 (m, 2H), 7.09 (t, 1H), 6.62 (t, 1H), 6.47 (d, 1H), 5.08 (m, 1H), 4.37 (d, 2H), 4.32 (t, 1H), 4.14 (m, 1H), 3.98 (t, 1H), 3.76 (s, 4H), 3.71 (d, 2H), 3.10-3.04 (m, 1H), 2.97 (s, 2H), 2.95-2.83 (m, 2H), 2.70-2.60 (m, 4H), 2.57 (s, 1H), 2.44 (s, 3H), 2.08-1.94 (m, 2H), 1.83 (d, 2H), 1.78 (s, 3H), 1.74 (s, 3H), 1.51 (d, 2H), 1.25(m, 3H).

**Preparation of 4-(2-(4-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-2-oxoethyl)piperazin-1-yl)-2-oxoethoxy)-2-(2,6-dioxopiperidin-3-yl)isoindoline-1,3-dione (C99)**

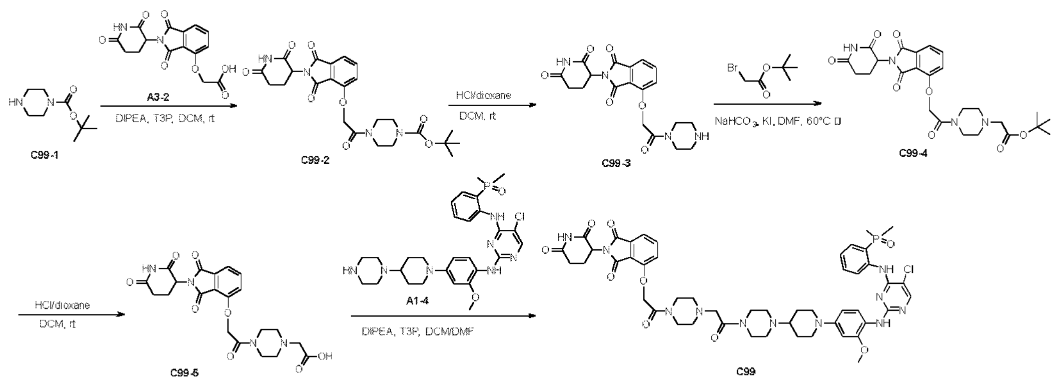


Step 1:

**A3-2** (223 mg, 0.5 mmol) and **C99-1** (93 mg, 0.5 mmol) were dispersed in 10.0 mL of anhydrous DCM, and T3P (636 mg, 1.0 mmol) and DIPEA (322.5 mg, 2.5 mmol) were added successively under nitrogen protection. The mixture was reacted at room temperature for 2h. The mixture was diluted with 30 mL of ethyl acetate, and then washed successively with saturated $NH_4Cl$ (20 mL $\times$ 1) and saturated NaCl (20 mL $\times$ 2). The organic layer was dried with anhydrous $MgSO_4$, and filtered with suction. The filtrate was concentrated under reduced pressure to afford a crude product. The crude product was purified by Flash chromatography to afford white solid product **C99-2** (230 mg, yield: 92%).

Step 2:

**C99-2** (230 mg, 0.46 mmol) was dissolved in 6.0 mL of anhydrous DCM, and 2.0 mL of HCl/dioxane (4.0 M) was added with stirring. The mixture was reacted at room temperature for 2.0 h. LCMS showed that the reaction was completed. The mixture was concentrated under reduced pressure to afford white solid product **C99-3** (250 mg, yield: 100%). LCMS: $[M + H]^+ = 401$.

Step 3:

**C99-3** (109 mg, 0.25 mmol), KI (20.8 mg, 0.125 mmol) and $NaHCO_3$ (84 mg, 1.0 mmol) were dispersed in 5.0 mL of anhydrous DMF. Tert-butyl bromoacetate (48.8 mg, 0.25 mmol) was added under nitrogen protection. The mixture was reacted at 60°C for 20 h. TLC showed that the reaction was completed. The mixture was diluted with 30 mL of ethyl acetate, and then washed successively with saturated $NH_4Cl$ (10 mL $\times$ 1), $H_2O$ (10 mL $\times$ 2) and saturated NaCl (10 mL $\times$ 2). The organic layer was dried with anhydrous $Na_2SO_4$, and filtered with suction. The filtrate was concentrated under reduced pressure to afford a crude product. The crude product was purified by Prep-TLC to afford light-yellow solid product **C99-4** (95 mg, yield: 74.2%). LCMS: $[M + H]^+ = 515$.

Step 4:

**C99-4** (95 mg, 0.185 mmol) was dissolved in 3.0 mL of anhydrous DCM, and 1.5 mL of HCl/dioxane (4.0 M)

was added with stirring. The mixture was reacted at room temperature for 2.0 h, and then filtered with suction to afford light-yellow solid product **C99-5** (70 mg, yield: 82.6%). LCMS: $[M + H]^+$ = 459.

Step 5:

**C99-5** (70 mg, 0.142 mmol) and **A1-4** (64.6 mg, 0.114 mmol) were dispersed in 5.0 mL of anhydrous DCM and 1.0 mL of anhydrous DMF. T3P (180.6 mg, 0.284 mmol) and DIPEA (91.6 mg, 0.71 mmol) were added under nitrogen protection. The mixture was stirred at room temperature for 2h. The mixture was concentrated under reduced pressure to afford a crude product, which was purified by Prep-HPLC to afford yellow solid pure product **C99** (25 mg, yield: 17.5%). LCMS: $[M + H]^+$ = 1010.

**Preparation of 4-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-2-oxoethoxy)-2-(2,6-dioxopiperidin-3-yl)isoindoline-1,3-dione (C101)**

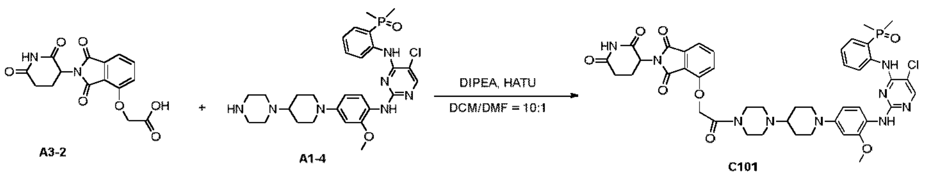


**A3-2** (16.6 mg, 0.05 mmol), **A1-4** (28.5 mg, 0.05 mmol) and HATU (28.5 mg, 0.075 mmol) were dispersed in a mixture of 2.0 mL of anhydrous DCM and 0.2 mL of anhydrous DMF. DIPEA (32.3 mg, 0.25 mmol) was added dropwise under nitrogen protection. The mixture was stirred at room temperature for 2h. The mixture was concentrated under reduced pressure to afford a crude product. The crude product was purified by RP-Flash chromatography to afford 30 mg of crude product, which was further purified by Prep-HPLC to afford light-yellow solid pure product **C101** (15 mg, yield: 34.1%). LCMS: $[M + H]^+$ = 884.

**Preparation of 2-(((1-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-2-oxoethyl)cyclopropyl)methyl)thio)-N-(2-(2,6-dioxopiperidin-3-yl)-1,3-dioxoisoindolin-4-yl)acetamide (C103)**

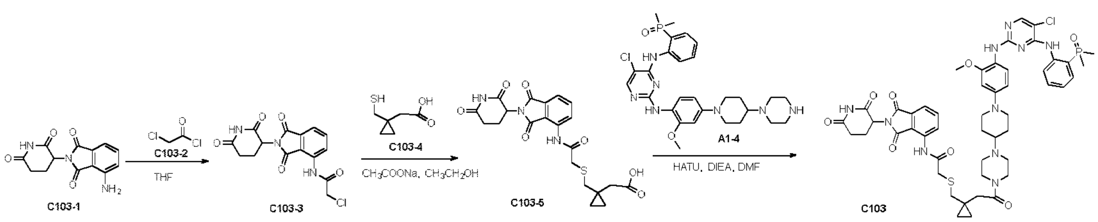


Step 1:

Compound **C103-2** (or **A8-1**) (226mg, 2.02mmol) was slowly added to a solution of compound **C103-1** (500mg, 1.83mmol) in dry THF (10 mL) at 0°C under nitrogen protection. The mixture was heated and refluxed for 4h. The mixture was then cooled to room temperature. The solvent was removed under reduced pressure, and the crude product was separated by column chromatography to afford white solid compound **C103-3** (300mg, yield: 46.9%). LCMS: $[M + H]^+$ = 350.

Step 2:

Compound **C103-3** (250mg, 0.716mmol), compound **C103-4** (105mg, 0.716mmol) and sodium acetate (70mg, 0.854mmol) were added to anhydrous ethanol (5 mL). The mixture was stirred and refluxed for 3h. The solvent was removed at 40°C under reduced pressure, and the crude product was separated by column chromatography to afford compound **C103-5** (100mg, 30.4%). LCMS: $[M + H]^+$ = 460.

Step 3:

Compound **C103-5** (40mg, 0.087mmol), compound **A1-4** (50mg, 0.088mmol), DIEA (34mg, 0.264mmol) and HATU (33mg, 0.087mmol) were dissolved in 2 mL of DMF at room temperature. The mixture was stirred at room temperature under nitrogen protection for 7h. LCMS showed that the reaction was completed. The solvent was removed at 40°C under reduced pressure, and the crude product was purified by Flash chromatography to afford off-white solid **C103** (28mg, yield: 31.8%). LCMS: $[M + H]^+$ = 1011, $^1H$ NMR (400 MHz, DMSO-$d_6$) δ 11.16 (s, 1H), 10.45 (s, 1H), 8.63 (d, 1H), 8.48 (s, 1H), 8.05 (d, 2H), 7.86 (t, 1H), 7.63 (d, 1H), 7.53 (dd, 1H), 7.42-7.31 (m, 2H), 7.09 (t, 1H), 6.62 (d, 1H), 6.47 (d, 1H), 5.75 (s, 1H), 5.17 (dd, 1H), 3.76 (s, 3H), 3.71 (d, 2H), 3.57 (s, 2H), 3.38 (s, 4H), 2.79 (d, 2H), 2.65 (s, 4H), 2.43 (s, 5H), 2.32 (s, 1H), 2.13-2.05 (m, 1H), 2.00 (d, 1H), 1.82 (s, 1H), 1.76 (d, 7H), 1.48 (s, 3H), 0.43 (d, 4H).

**Preparation of 1-(4-(1-(4-((5-chloro-4-((2-(dimethylphosphoryl)phenyl)amino)pyrimidin-2-yl)amino)-3-methoxyphenyl)piperidin-4-yl)piperazine-1-carbonyl)cyclopropane-1-carboxylate 2-(2,6-dioxopiperidin-3-yl)-1,3-dioxoisoindolin-4-yl ester (C104)**

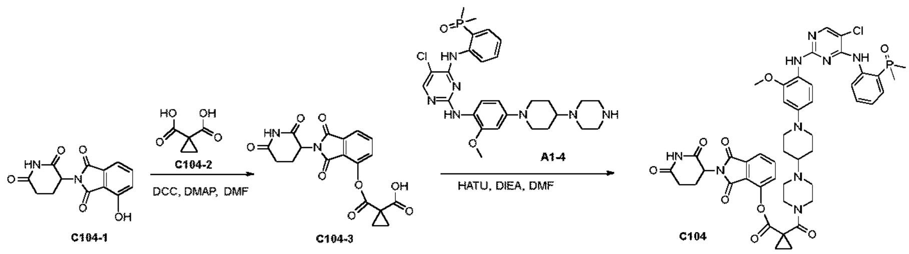


Step 1:

Compound **C104-1** (or **A2-1**) (100mg, 0.365mmol), compound **C104-2** (119mg, 0.915mmol), DCC (90mg, 0.436mmol) and DMAP (45mg, 0.368mmol) were dissolved in DMF (4 mL). The mixture was stirred at room temperature under nitrogen protection for 5h. The solvent was removed under reduced pressure, and the crude product was separated by column chromatography to afford white solid compound **C104-3** (70mg, yield: 49.6%).

Step 2:

Compound **C104-3** (40mg, 0.104mmol), compound **A1-4** (56mg, 0.098mmol), HATU (40mg, 0.105mmol) and DIEA (40mg, 0.310mmol) were added to DMF (2 mL). The mixture was stirred at room temperature under nitrogen protection for 3h. The solvent was removed at 40°C under reduced pressure, and the crude product was purified by Flash chromatography to afford off-white solid **C104** (50mg, yield: 51.5%). LCMS: $[M + H]^+$ = 938. $^1H$ NMR (400 MHz, DMSO-$d_6$) δ 11.14 (d, 2H), 8.48 (s, 1H), 8.06 (d, 2H), 7.96 (t, 1H), 7.87 (d, 1H), 7.70 (d, 1H), 7.56-7.49 (m, 1H), 7.39 (d, 1H), 7.34 (t, 1H), 7.09 (t, 1H), 6.63 (d, 1H), 6.47 (dd, 1H), 5.14 (dd, 1H), 3.76 (s, 3H), 3.72 (d, 4H), 3.51 (s, 2H), 2.89 (dt, 1H), 2.66 (t, 3H), 2.39 (s, 1H), 2.10-1.96 (m, 2H), 1.83 (d, 2H), 1.79-1.73 (m, 9H), 1.55-1.50 (m, 3H), 1.24(m, 3H).

**Preparation of 4-((1-((4-(1-(4-((5-chloro-4-((2-(dimethylphosphoryl)phenyl)amino)pyrimidin-2-yl)amino)-3-methoxyphenyl)piperidin-4-yl)piperazin-1-yl)methyl)cyclopropyl)methoxy)-2-(2,6-dioxopiperidin-3-yl)isoindoline-1,3-dione (C106)**

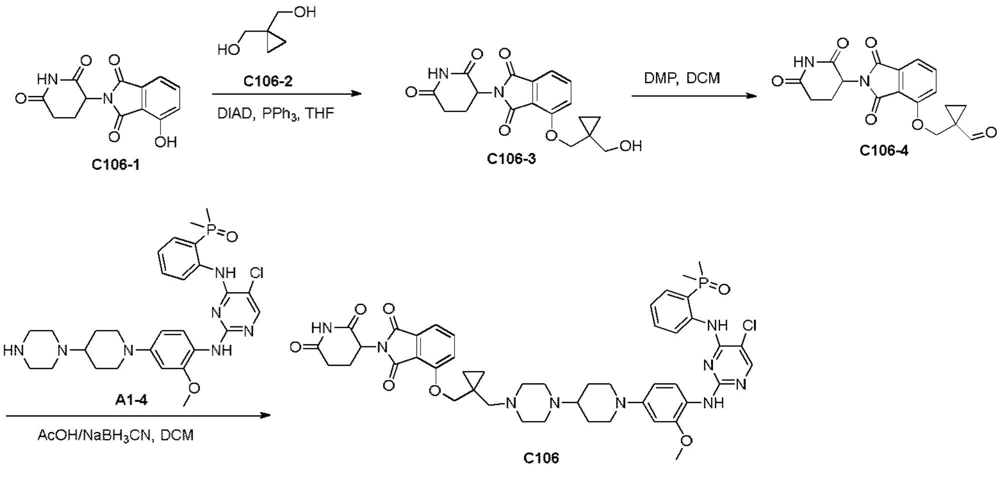


Step 1:

Compound **C106-1** (or **A2-1**) (274 mg, 1 mmol), compound **C106-2** (102 mg, 1 mmol), and triphenyl phosphine (393 mg, 1.5 mmol) were added to a 50 mL three-necked flask, and then anhydrous tetrahydrofuran (5 mL) was added. The atmosphere was replaced with nitrogen gas to remove oxygen. The mixture was stirred under nitrogen protection and cooled to 0°C. DIAD (303 mg, 1.5 mmol) was added dropwise. After the addition was completed, the mixture was warmed to room temperature and reacted with stirring for 3h. The reaction process was monitored by LCMS. The raw materials were basically transformed into the title product. The mixture was concentrated under reduced pressure to afford a crude product, which was separated by column chromatography to afford a title product **C106-3**, 210 mg, yield: 58.6%. LCMS: $[M + H]^+$ = 359.

Step 2:

Compound **C106-3** (100 mg, 0.28 mmol) was added to a 50mL single-necked flask and dissolved in dichloromethane (15 mL). Dess-Martin reagent (305 mg, 0.72mmol) was then added. The mixture was reacted with stirring at room temperature overnight. The reaction process was monitored by LCMS. The raw materials were basically consumed and the title product was produced. 5 mL of saturated sodium bicarbonate solution and 5 mL of saturated sodium thiosulfate solution were successively added to the mixture and stirred for 10 minutes. After the mixture became clear, the layers were separated. The organic phase was dried with anhydrous sodium sulfate, and filtered. The filtrate was concentrated under reduced pressure to afford an off-white solid product, which was separated by Prep-TLC to afford light white solid **C106-4,** 45 mg, yield: 45%. LCMS: $[M + H]^+$ = 357.

Step 3:

Compound **C106-4** (41 mg, 0.115 mmol) and compound **A1-4** (66mg, 0.115 mmol) were added to a 50mL single-necked flask and dissolved in dichloromethane (3 mL). $CH_3COOH$ (0.05 mL) was then added. The mixture was reacted with stirring at room temperature for 0.5h. Then, sodium cyanobohydride (22 mg, 0.345 mmol) was added and reacted overnight. The reaction process was monitored by LCMS. The raw materials were basically consumed and the title product was produced. Saturated $NH_4Cl$ solution (3 mL) was added to the mixture and stirred for 5 minutes. The layers were separated. The organic phase was dried with anhydrous sodium sulfate, and filtered. The filtrate was concentrated under reduced pressure to afford a crude product, which was separated by Prep-TLC to afford white powder **C106,** 10 mg, yield: 9.6%. LCMS: $[M + H]^+$ = 910.

**Preparation of 2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)acetate (1-(((2-(2,6-dioxopiperidin-3-yl) -1,3-dioxoisoindolin-4-yl)oxy)methyl)cyclopropyl)methyl ester (C107)**

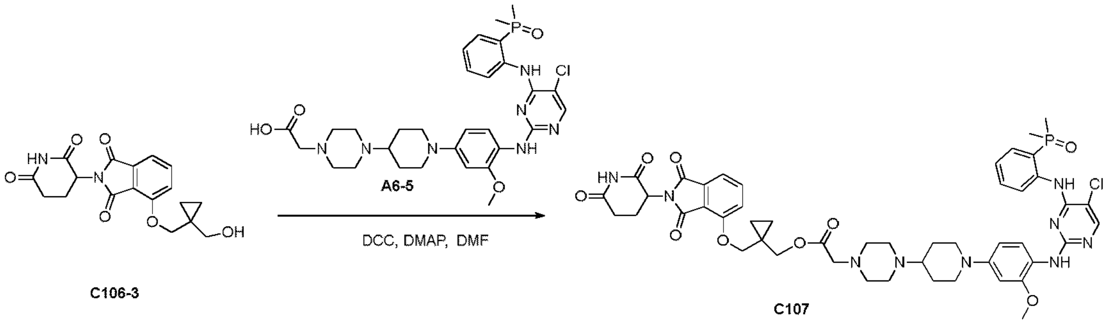


Compound **C106-3** (28.5 mg, 0.08 mmol), compound A6-5 (50 mg, 0.08 mmol), DCC (20 mg, 0.096 mmol) and DMAP (10 mg, 0.08 mmol) were added to a 50 mL single-necked flask and dissolved in DMF (3 mL). The mixture was reacted with stirring at room temperature overnight. The reaction process was monitored by LCMS. The reaction was stopped until the raw materials were basically transformed. The mixture was concentrated under reduced pressure to afford a crude product, which was separated by Prep-TLC to afford light-yellow solid product, which was further separated by prep-HPLC to afford white powder **C107,** 10 mg, yield: 12.98%. LCMS: $[M + H]^+ = 968$.

**Preparation of 4-(((S)-1-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)acetyl)pyrrolidin-2-yl)methoxy)-2-(2,6-dioxopiperidin-3-yl)isoindoline-1,3-dione (C109)**

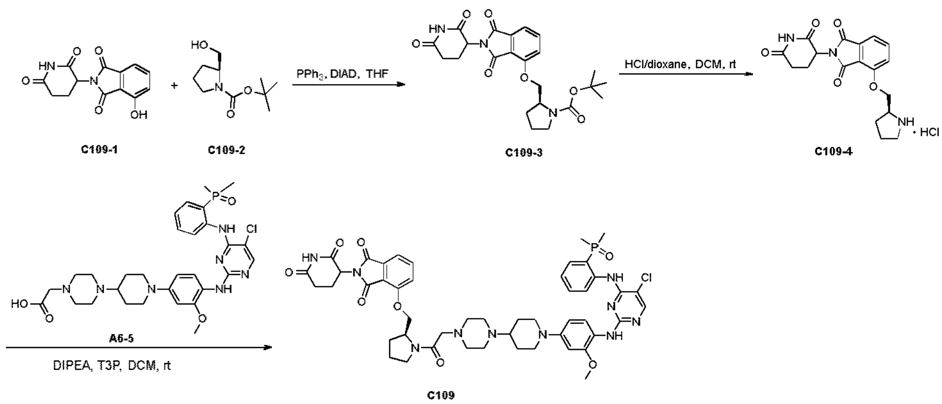


Step 1:

**C109-1** (or **A2-1**) (82.2 mg, 0.3 mmol), **C109-2** (66.3 mg, 0.33 mmol) and $PPh_3$ (94.3 mg, 0.36 mmol) were dispersed in 7.0 mL of anhydrous THF. DIAD (78.8 mg, 0.39 mmol) was added dropwise under nitrogen protection. After the dropwise addition, the mixture was reacted at room temperature for 5.0 h. The mixture was concentrated under reduced pressure to afford a crude product, which was purified by Prep-HPLC to afford white solid product **C109-3,** 85 mg, yield: 70.0%. LCMS: $[M + H]^+ = 408$.

Step 2:

**C109-3** (85 mg, 0.186 mmol) was dissolved in 4.0 mL of anhydrous DCM, and 2.0 mL of 4.0 M HCl/dioxane solution was added. The mixture was sealed and reacted at room temperature for 20 h. LCMS showed that the reaction was completed. The mixture was concentrated under reduced pressure to afford white solid crude **C109-4,** 100 mg, yield: 100%. The crude product was directly used in the next step. LCMS: $[M + H]^+ = 358$.

Step 3:

**C109-4** (42.8 mg, 0.12 mmol) and **A6-5** (62.7 mg, 0.1 mmol) were dispersed in 4.0 mL of anhydrous DCM, and T3P (127.2 mg, 0.2 mmol) and DIPEA (64.5 mg, 0.5 mmol) were added successively. The mixture was reacted at room

temperature for 2h. LCMS showed that there was no raw material remaining. The mixture was concentrated under reduced pressure to afford a crude product, which was purified by Prep-HPLC to afford light-yellow solid pure product **C109,** 18 mg, yield: 18.6%. LCMS: $[M + H]^+$ = 967. $^1$H-NMR: (400 MHz, DMSO-$d_6$) δ 11.160 (s, 1H), 11.093 (d, *J* = 3.6 Hz, 1H), 8.476 (s, 1H), 8.062 (d, *J* = 13.2 Hz, 2H), 7.861-7.788 (m, 1H), 7.577-7.320 (m, 5H), 7.112 (t, *J* = 7.2 Hz, 1H), 6.622 (d, *J* = 2.8 Hz, 1H), 6.476 (t, *J* = 2.0, 1H), 5.135-5.079 (m, 1H), 4.411-4.238 (m, 3H), 3.761 (s, 3H), 3.707 (d, *J* = 12.0 Hz, 3H), 3.550 (d, *J* = 5.2 Hz, 1H), 3.179-3.122 (m, 1H), 3.005-2.858 (m, 2H), 2.674-2.615 (m, 3H), 2.424-2.202 (m, 9H), 1.997 (s, 4H), 1.813-1.745 (m, 10H), 1.509-1.453 (m, 2H).

**Preparation of (2S)-1-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)acetyl)-N-(2-(2,6-dioxopiperidin-3-yl)-1-oxoisoindolin-4-yl)-4,4-difluoropyrrolidine-2-carboxamide (C110)**

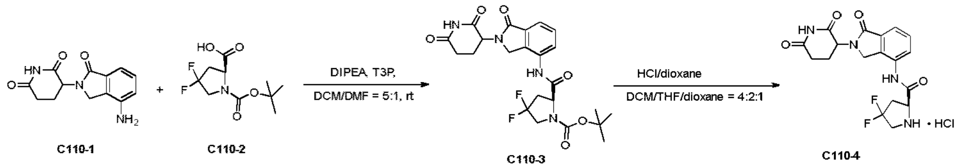


C110-1 C110-2 C110-3 C110-4

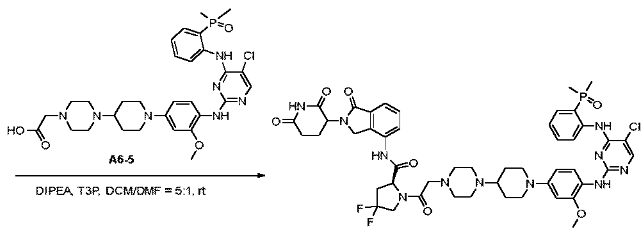


A6-5 C110

Step 1:

**C110-1** (or **A12-3**) (130 mg, 0.5 mmol) and **C110-2** (125.5 mg, 0.5 mmol) were dispersed in a mixture of 6.0 mL of anhydrous DCM and 1.2 mL of anhydrous DMF. T3P (636 mg, 1.0 mmol) and DIPEA (258 mg, 2.0 mmol) were added successively under $N_2$ protection. The mixture was reacted at room temperature for 2h. LCMS showed that there was no raw material remaining. The mixture was concentrated under reduced pressure to remove DCM. The residue was diluted with 50 mL of ethyl acetate, and then washed successively with saturated $NH_4Cl$ solution (20 mL × 1), $H_2O$ (20 mL × 3) and saturated brine (20 mL × 1). The organic layer was dried with anhydrous $Na_2SO_4$, and filtered with suction. The filtrate was concentrated under reduced pressure to afford a crude product, which was purified by Prep-TLC to afford light-yellow oily product **C110-3,** 100 mg, yield: 40.7%. $^1$H-NMR: (400 MHz, DMSO-$d_6$) δ 11.021 (s, 1H), 10.080 (s, 1H), 7.781-7.726 (m, 1H), 7.575-7.523 (m, 2H), 5.182-5.136 (m, 1H), 4.574-4.255 (m, 2H), 3.853 (m, 2H), 2.962-2.890 (m, 2H), 2.639-2.585 (m, 1H), 2.341-2.253 (m, 1H), 2.051-2.028 (m, 1H), 1.416 (s, 3H), 1.344 (s, 6H).

Step 2:

**C110-3** (100 mg, 0.203 mmol) was dissolved in a mixture of 4.0 mL of anhydrous DCM, 2.0 mL of anhydrous THF and 1.0 mL of anhydrous 1,4-dioxane, and 3.0 mL of 4.0 M HCl/dioxane solution was added. The mixture was sealed and reacted at room temperature for 2h. LCMS showed that the reaction was completed. The mixture was concentrated under reduced pressure to afford white solid crude **C110-4,** 130 mg, yield: 100%. The crude product was directly used in the next step. LCMS: $[M + H]^+$ = 393.

Step 3:

**A6-5** (51.3 mg, 0.0818 mmol) and C110-4 (35 mg, 0.0818 mmol) were dispersed in a mixture of 5.0 mL of anhydrous DCM and 1.0 mL of anhydrous DMF. T3P (104 mg, 0.1636 mmol) and DIPEA (63.3 mg, 0.4908 mmol) were added successively under $N_2$ protection. The mixture was reacted at room temperature for 2h. LCMS showed that there was no raw material remaining. The mixture was concentrated under reduced pressure to afford a crude product, which was purified by Prep-HPLC to afford white solid pure product **C110,** 25 mg, yield: 30.6%. LCMS: $[M + H]^+$ = 1002. $^1$H-

NMR: (400 MHz, DMSO-$d_6$) δ 11.164 (s, 1H), 11.037 (s, 1H), 10.052 (s, 1H), 8.477 (s, 1H), 8.155-8.043 (m, 2H), 7.776 (t, $J$ = 6.8 Hz, 1H), 7.566-7.499 (m, 3H), 7.391-7.318 (m, 2H), 7.112-7.075 (m, 2H), 6.627-6.373 (m, 2H), 5.201-5.130 (m, 1H), 4.505-4.059 (m, 5H), 3.773-3.703 (m, 5H), 3.530 (s, 1H), 3.286-3.275 (m, 2H), 3.095-2.810 (m, 10H), 2.640-2.598 (m, 1H), 2.291- 2.180 (m, 4H), 2.008-1.838 (m, 3H), 1.782-1.748 (m, 1H), 1.741 (d, $J$ = 12.0 Hz, 6H), 1.538-1.445 (m, 3H).

**Preparation of 2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)-N-(((2S)-1-((2-(2,6-dioxopiperidin-3-yl)-1,3-dioxoisoindolin-4-yl)oxy)-3,3-dimethylbutan-2-yl)acetamide (C112)**

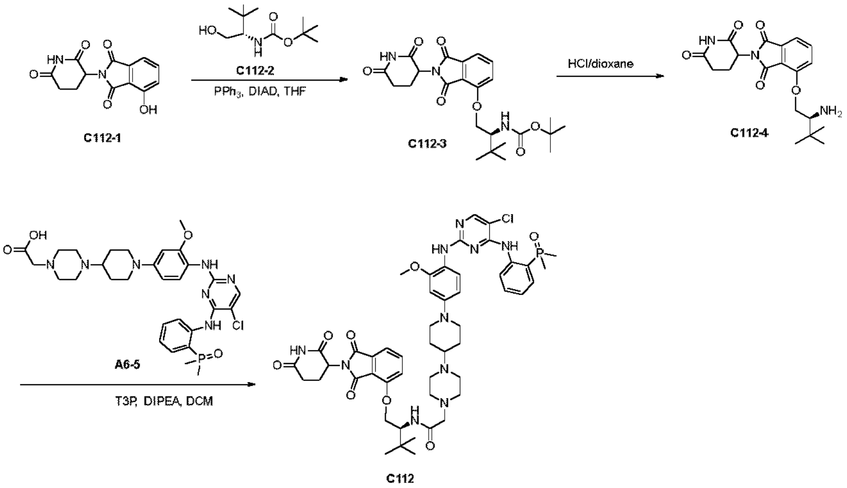


Step 1:

Compound **C112-1** (or **A2-1**) (274 mg, 1mmol), compound **C112-2** (217 mg, 1 mmol), and triphenyl phosphine (393 mg, 1.5 mmol) were added to a 50 mL three-necked flask, and anhydrous tetrahydrofuran (5 mL) was then added. The atmosphere was replaced with nitrogen gas to remove oxygen. The mixture was stirred under nitrogen protection and cooled to 0°C. DIAD (303 mg, 1.5 mmol) was added dropwise. After the addition was completed, the mixture was warmed to room temperature and reacted with stirring for 3h. The reaction process was monitored by LCMS. After the reaction was completed, the mixture was concentrated under reduced pressure to afford a crude product, which was separated by column chromatography to afford a title product **C112-3,** 240 mg, yield: 50.7%. LCMS: $[M + H]^+$ = 475.

Step 2:

Compound **C112-3** (100 mg, 0.21 mmol) and HCl/1,4-dioxane solution (5 mL, 4 mol/L) were added to a 25 mL single-necked flask. The mixture was reacted with stirring at room temperature for 2h. The reaction process was monitored by TLC. After the reaction was completed, the mixture was concentrated under reduced pressure to remove HCl and most of the 1,4-dioxane, and dissolved with dichloromethane (20 mL). Sodium bicarbonate solution was added to adjust the pH to 8-9. After separation, the dichloromethane solution was dried with anhydrous sodium sulfate, and filtered. The filtrate was concentrated under reduced pressure to afford light-yellow solid **C112-4,** 40 mg, yield: 51%. LCMS: $[M + H]^+$ = 374.

Step 3:

Compound **C112-4** (40 mg, 0.107 mmol) and **A6-5** (67 mg, 0.107 mmol) were added to a 50 mL single-necked flask and dissolved in dichloromethane (3 mL). $T_3P$ (136 mg, 0.214 mmol) was then added. The mixture was reacted with stirring at room temperature for 3h. The reaction process was monitored by LCMS. After the reaction was completed, dichloromethane (10 mL) was added to the mixture, and the mixture was washed with water (5 mL). The layers were separated. The organic phase was dried with anhydrous sodium sulfate, and filtered. The filtrate was concentrated under reduced pressure to afford a crude product, which was purified by prep-TLC to afford light-yellow solid, which was further separated by prep-HPLC to afford light white powder **C112,** 34 mg. yield 32.3%. LCMS: $[M + H]^+$ = 983.

**Preparation of 3-(4-(6-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hex-1-yn-1-yl)-1-oxoisoindolin-2-yl)piperidine-2,6-dione (C116)**

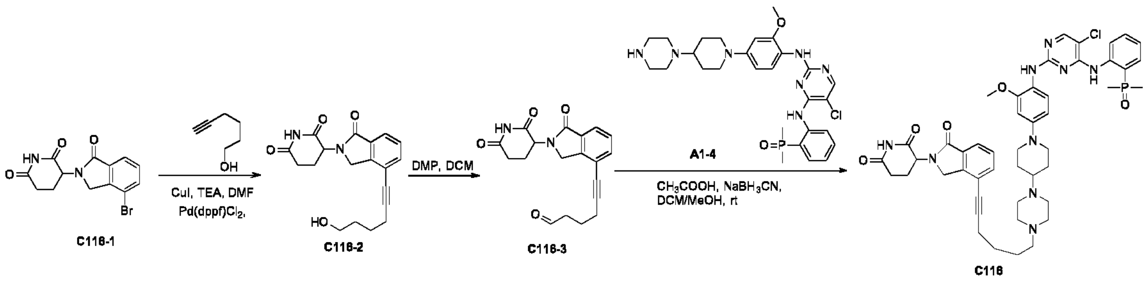


Step 1:

**C116-1** (or **C1-1**) (500 mg, 1.553 mmol), CuI (59 mg, 0.3106 mmol) and Pd(dppf)$Cl_2$ (454 mg, 0.6212 mmol) were dispersed in 25 mL of anhydrous DMF. 5-alkynyl-1-hexanol (380 mg, 3.883 mmol) and TEA (470 mg, 4.659 mmol) were added successively under $N_2$ protection. The mixture was heated to 70°C and reacted for 20 h. LCMS showed that the reaction was completed. The mixture was cooled to room temperature, diluted with 150 mL of ethyl acetate, and then washed successively with saturated $NH_4Cl$ (2 × 50 mL), $H_2O$ (2 × 50 mL) and saturated brine (2 × 50 mL). The organic layer was dried with anhydrous sodium sulfate, and filtered with suction. The filtrate was concentrated under reduced pressure to afford a crude product, which was purified by RP-Flash chromatography to afford light-yellow solid product **C116-2** (255 mg, 48.3%). LCMS: $[M + H]^+$ = 341.

Step 2:

**C116-2** (90 mg, 0.265 mmol) was dispersed in 18.0 mL of anhydrous DCM, and the mixture was heated to 40°C to make the mixture clear. Dess-Martin reagent (168.5 mg, 0.3975 mmol) was added under $N_2$ protection. The mixture was heated to 50°C and refluxed for 2.0 h. TLC showed that the reaction was completed. The mixture was cooled to room temperature, and then 10 mL of saturated $NaHCO_3$ solution and 10 mL of saturated $Na_2S_2O_3$ solution were added. The mixture was stirred vigorously at room temperature for 5 min. The organic layer was separated, dried with anhydrous sodium sulfate, and filtered with suction. The filtrate was concentrated under reduced pressure to afford a crude product. The crude product was purified by Prep-TLC to afford light-yellow solid product **C116-3** (35 mg, 39.1%). LCMS: $[M + H]^+$ = 339.

Step 3:

**C116-3** (30 mg, 0.0888 mmol) and **A1-4** (45.5 mg, 0.0799 mmol) were dissolved in a mixture of 4.0 mL of anhydrous DCM and 0.4 mL of anhydrous methanol. $CH_3COOH$ (5.3 mg, 0.0888 mmol, dissolved in 0.5 mL of DCM) was added under nitrogen protection. The mixture was stirred at room temperature for 0.5h. Then solid $NaBH_3CN$ (8.36 mg, 0.1332 mmol) was added and the mixture was further reacted at room temperature for 2h. LCMS and TLC showed that the reaction was completed. The mixture was purified by Prep-HPLC to afford white solid product **C116,** 25 mg, yield: 31.6%. LCMS: $[M + H]^+$ = 892. $^1$H-NMR: (400 MHz, DMSO-$d_6$) δ 12.683 (s, 1H), 11.119 (s, 1H), 8.869 (dd, $J$ = 2.0 Hz, 9.2 Hz, 3H), 8.281 (s, 1H), 8.263 (s, 1H), 7.721 (d, $J$ = 6.8 Hz, 1H), 7.646 (d, $J$ = 0.8 Hz, 1H), 7.643 (d, $J$ = 6.8 Hz, 1H), 7.539 (d, $J$ = 7.6 Hz, 1H), 7.321 (s, 1H), 6.809 (s, 1H), 5.188 (dd, $J$ = 5.2 Hz, 13.6 Hz, 1H), 4.477 (d, $J$ = 17.6 Hz, 1H), 4.325 (d, $J$ = 17.6 Hz, 1H), 3.784 (s, 3H), 2.513-2.495 (m, 10H), 2.494-2.103 (m, 6H), 2.075 (s, 3H), 2.040 (s, 3H), 2.004 (s, 3H), 1.557-1.429 (m, 18H).

**Preparation of 3-(4-(5-(4-(1-(4-((5-chloro-4-((2-(dimethylphosphoryl)phenyl)amino)pyrimidin-2-yl)amino)-3-methoxyphenyl)piperidin-4-yl)piperazin-1-yl)pent-1-yn-1-yl)-1-oxoisoindolin-2-yl)piperidine-2,6-dione (C126)**

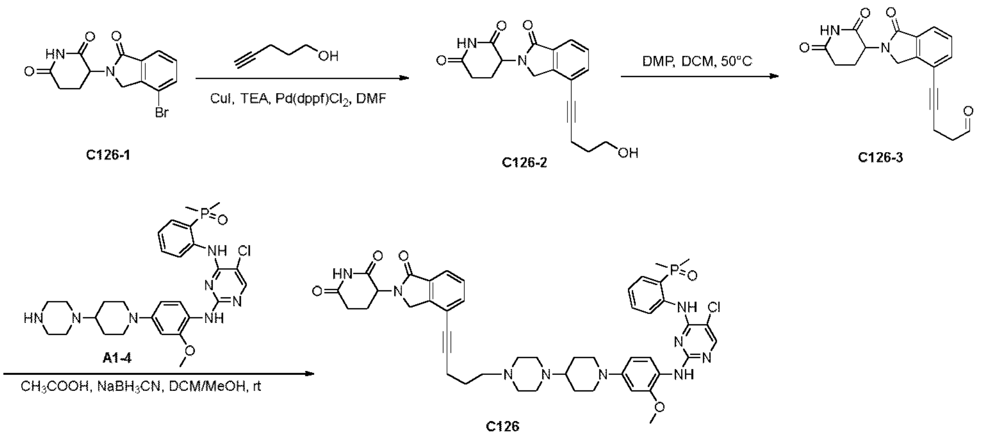


C126-1 C126-2 C126-3

C126

Step 1:

**C126-1** (or **C1-1**) (322 mg, 1.0 mmol), CuI (19 mg, 0.1 mmol) and $Pd(dppf)Cl_2$ (73.1 mg, 0.1 mmol) were dispersed in 10.0 mL of anhydrous DMF. 4-alkynyl-1-pentanol (210 mg, 2.5 mmol) and TEA (303 mg, 3.0 mmol) were added successively under $N_2$ protection. The mixture was heated to 70°C and reacted for 20 h. LCMS showed that the reaction was completed. The mixture was cooled to room temperature and purified by RP-Flash chromatography to afford white solid crude **C126-2** (410mg, yield: 94.1%). LCMS: $[M + H]^+ = 327$.

Step 2:

**C126-2** (400 mg, 0.920 mmol) was dissolved in a mixture of 150 mL of anhydrous DCM and 10 mL of anhydrous THF, and Dess-Martin reagent (1.04 g, 2.454 mmol) was added under $N_2$ protection. The mixture was heated to 50°C and refluxed for 2.0 h. TLC showed that the reaction was completed. The mixture was cooled to room temperature, and then 20 mL of saturated $NaHCO_3$ solution and 20 mL of saturated $Na_2S_2O_3$ solution were added. The mixture was stirred vigorously at room temperature for 5 min. The organic layer was separated, dried with anhydrous sodium sulfate, and filtered with suction. The filtrate was concentrated under reduced pressure to afford a crude product. The crude product was purified by Flash chromatography to afford light-yellow solid product **C126-3** (280 mg, 93.3%). LCMS: $[M + H]^+ = 325$.

Step 3:

**C126-3** (48.6 mg, 0.15 mmol) and **A1-4** (76.8 mg, 0.135 mmol) were dissolved in a mixture of 5.0 mL of anhydrous DCM and 0.5 mL of anhydrous MeOH. $CH_3COOH$ (13.5 mg, 0.225 mmol) was added under nitrogen protection. The mixture was stirred at room temperature for 0.5h. Then solid $NaBH_3CN$ (14.1 mg, 0.225 mmol) was added and the mixture was further reacted at room temperature for 2h. LCMS and TLC showed that the reaction was completed. The mixture was purified by Prep-HPLC to afford white solid pure product **C126** (35 mg, yield: 26.7%). LCMS: $[M + H]^+ = 878$.

**Preparation of 4-(7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hept-1-yn-1-yl)-2-(2,6-dioxopiperidin-3-yl)isoindoline-1,3-dione (C148)**

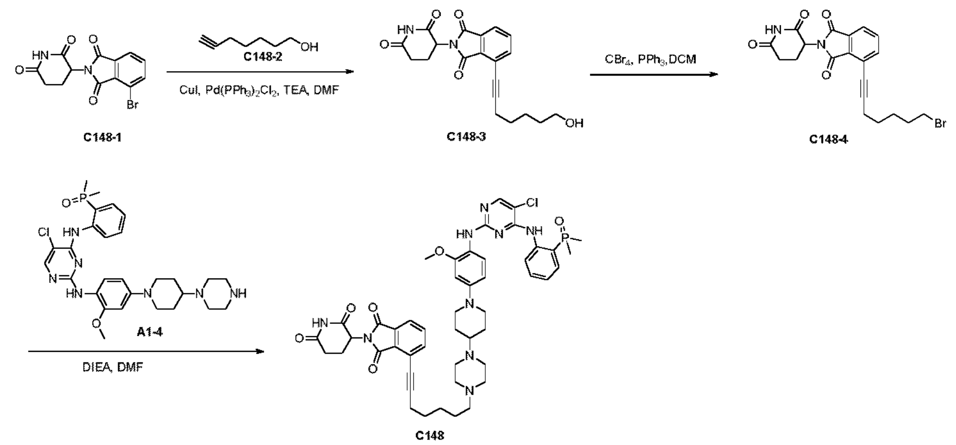


C148-1 C148-3 C148-4 A1-4 C148

Step 1:

Compound **C148-1** (or **A1-1**) (330mg, 0.98mmol), compound **C148-2** (220mg, 1.96mmol), cuprous iodide (38mg, 0.20mmol), $Pd(PPh_3)_2Cl_2$ (274mg, 0.39mmol) and triethylamine (297mg, 2.94mmol) were dissolved in dry DMF (10 mL). The mixture was stirred at 70°C under nitrogen protection overnight. The mixture was cooled to room temperature. The solvent was removed under reduced pressure, and the crude product was separated by column chromatography to afford compound **C148-3** (90mg, yield: 24.9%). LCMS: $[M + H]^+$ = 369.

Step 2:

Compound **C148-3** (60mg, 0.163mmol) and $CBr_4$ (108mg, 0.326mmol) were dissolved in dichloromethane (20 mL). The mixture was stirred at room temperature for 2h. The mixture was cooled to 0°C. A solution of triphenyl phosphine (86mg, 0.326mmol) in dichloromethane (2 mL) was added dropwise. The mixture was naturally warmed to room temperature, and reacted with stirring at 55°C for 2h. The solvent was removed under reduced pressure, and the crude product was separated by column chromatography to afford compound **C148-4** (40mg, 57.1%). LCMS: $[M + H]^+$ = 431, 433.

Step 3:

Compound **C148-4** (40mg, 0.093mmol), compound **A1-4** (58mg, 0.102mmol), and DIEA (60mg, 0.465mmol) were dissolved in 2 mL of DMF. The mixture was stirred at 80°C under nitrogen protection for 5h. LCMS showed that the reaction was completed. The solvent was removed at 40°C under reduced pressure. The crude product was purified by Flash chromatography to afford **C148** (22mg, yield: 25.9%). LCMS: $[M + H]^+$ = 920. $^1H$ NMR (400 MHz, DMSO-$d_6$) $\delta$ 11.13 (d, 2H), 8.47 (s, 1H), 8.04 (d, 2H), 7.87 (dd, 3H), 7.52 (dd, 1H), 7.36 (dd, 2H), 7.09 (t, 1H), 6.62 (d, 1H), 6.46 (dd, 1H), 5.75 (s, 1H), 5.14 (dd, 1H), 3.76 (s, 3H), 3.71 (d, 2H), 3.26 (s, 1H), 2.92-2.83 (m, 1H), 2.69-2.60 (m, 4H), 2.55 (d, 4H), 2.34 (d, 5H), 2.03 (m, 2H), 1.84 (d, 2H), 1.76 (d, 6H), 1.60 (m, 3H), 1.47 (s, 6H).

**Preparation of 4-(3-((7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)heptyl)oxy)azetidin-1-yl)-2-(2,6-dioxopiperidin-3-yl)isoindoline-1,3-dione (C150)**

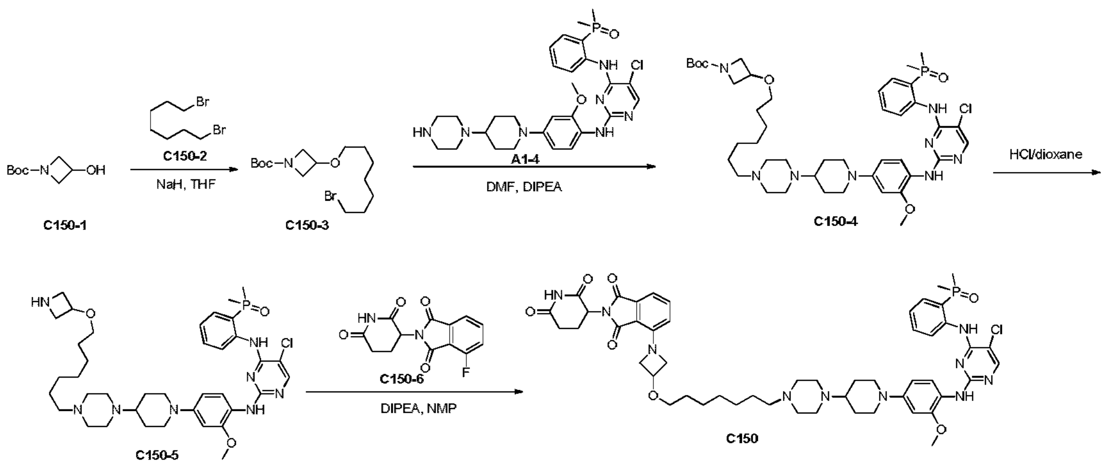


Step 1:

Compound **C150-1** (470 mg, 2.72 mmol) was added to a 100 mL three-necked flask and dissolved in anhydrous tetrahydrofuran (6 mL). The mixture was cooled to 0°C under nitrogen protection, and sodium hydride (120 mg, 2.98 mmol) was then added. The mixture was warmed to room temperature and reacted with stirring for 30 minutes. The mixture was cooled to 0°C again, and compound **C150-2** (842 mg, 3.26 mmol) was added. The mixture was reacted at room temperature for 3h. The reaction process was monitored by TLC. The reaction was stopped until the raw materials were basically transformed. Ice water (3g) was added to the mixture. The mixture was extracted twice with ethyl acetate (10 mL for each time). The organic phases were combined, washed with saturated brine (5 mL × 2), dried with anhydrous sodium sulfate, and filtered. The filtrate was concentrated under reduced pressure to afford a crude product, which was separated by column chromatography to afford light-yellow solid **C150-3,** 100 mg, yield: 10.6%.

Step 2:

Compound **C150-3** (35 mg, 0.1 mmol), compound **A1-4** (57 mg, 0.1 mmol) and DIPEA (38.7 mg, 0.3 mmol) were added to a 50 mL single-necked flask and dissolved in DMF (2 mL). The mixture was heated to 70°C and reacted for 2h. The reaction process was monitored by LCMS. After the reaction was completed, the mixture was cooled, and ethyl acetate (10 mL) and water (5 mL) were added to the mixture. The layers were separated, and extracted once with ethyl acetate. The organic phases were combined, washed with saturated brine (5 mL × 2), dried with anhydrous sodium sulfate, and filtered. The filtrate was concentrated under reduced pressure to afford a crude product, which was separated by Prep-TLC to afford light white solid **C150-4,** 35 mg, yield: 41.7%.

Step 3:

Compound **C150-4** (30 mg, 0.035mmol) and HCl/1,4-dioxane solution (5 mL, 4 mol/L) were added to a 25 mL single-necked flask. The mixture was reacted with stirring at room temperature for 2h. The reaction process was monitored by TLC. After the reaction was completed, the solvent was concentrated under reduced pressure, and the residue was dissolved with dichloromethane (20 mL). Sodium bicarbonate solution was added to adjust the pH to 8-9. After separation, the dichloromethane solution was dried with anhydrous sodium sulfate, and filtered. The filtrate was concentrated under reduced pressure to afford light-yellow solid **C150-5,** 25 mg, yield: 99%. LCMS: $[M + H]^+ = 739$.

Step 4:

Compound **C150-5** (20 mg, 0.027 mmol), **C150-6** (or **A6-1**) (8.8 mg, 0.032 mmol), DIPEA (10.4 mg, 0.081 mmol) and NMP (2 mL) were added to a 25 mL single-necked flask. The mixture was heated to 90°C and reacted under nitrogen protection for 3h. LCMS showed that a small amount of product was produced. The mixture was directly separated by prep-HPLC to afford white powder **C150,** 0.7 mg, yield: 2.6%. LCMS: $[M + H]^+ = 995$.

**Preparation of 3-(4-((7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)heptyl)oxy)-1-oxoisoindolin-2-yl)piperidine-2,6-dione (C153)**

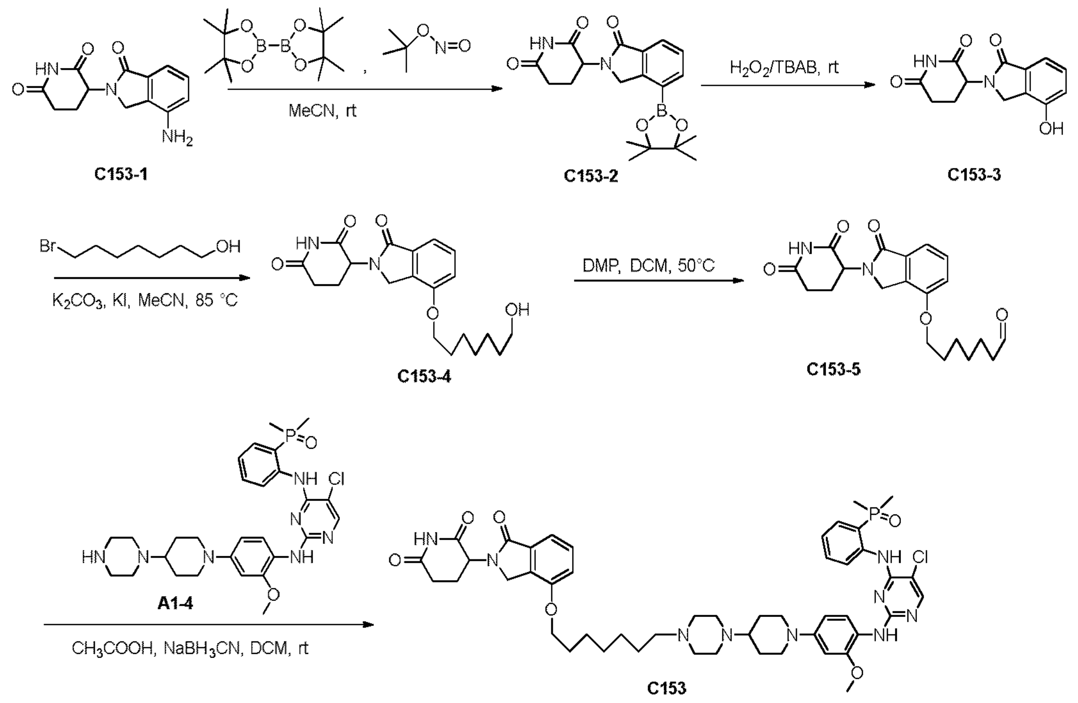


C153-1 C153-2 C153-3 C153-4 C153-5 A1-4 C153

Step 1:

**C153-1** (or **A12-3**) (2.59 g, 10.0 mmol) and bis(pinacolato)diboron (2.794 g, 11.0 mmol) were dispersed in 80.0 mL of anhydrous MeCN, and tert-butyl nitrite (1.545 g, 15.0 mmol) was added dropwise under nitrogen protection. After the dropwise addition, the mixture was reacted at room temperature for 4.0 h. The mixture was diluted with 80 mL of ethyl acetate, stirred for 5 minutes, and filtered with suction. The filtrate was concentrated under reduced pressure to afford a crude product, which was purified by Flash chromatography to afford light-yellow solid product **C153-2** (2.1 g, 56.7%). LCMS: $[M + H]^+ = 371$.

Step 2:

**C153-2** (2.1 g, 5.68 mmol) was dispersed in 63.0 $H_2O_2$ (30%), and TBAB (548 mg, 1.70 mmol) was added with stirring. The mixture was reacted in air for 1h. LCMS showed that the reaction was completed. The mixture was diluted with 150 mL of ethyl acetate, and stirred for 5 minutes. The organic layer was separated, and then the aqueous phase was extracted once. The organic layers were combined, and washed with saturated brine (50 mL × 2). The organic layer was dried with anhydrous $Na_2SO_4$, and filtered with suction to remove the desiccant. The filtrate was concentrated under reduced pressure to afford a crude product, which was purified by Flash chromatography to afford light-yellow solid product **C153-3** (0.71 g, 48.2%). LCMS: $[M + H]^+ = 261$.

Step 3:

**C153-3** (130 mg, 0.5 mmol), $K_2CO_3$ (103.5 mg, 0.75 mmol) and KI (41.5 mg, 0.25 mmol) were dispersed in 13.0 mL of anhydrous MeCN, and 7-bromo-1-heptanol (195 mg, 1.0 mmol) was added. The mixture was reacted at 85°C for 1.5h. The mixture was cooled to room temperature, and then filtered with suction to remove solid residue. The filtrate was concentrated under reduced pressure to afford a crude product. The crude product was purified by Prep-TLC to afford light-yellow solid product **C153-4** (115 mg, 61.5%). LCMS: $[M + H]^+ = 375$.

Step 4:

**C153-4** (75 mg, 0.2 mmol) was dissolved in 10.0 mL of anhydrous DCM, and Dess-Martin reagent (127.5 mg, 0.3 mmol) was added. The mixture was reacted under reflux at 50°C for 2h. The mixture was cooled to room temperature, and diluted with 10 mL of DCM. 5 mL of saturated $NaHCO_3$ solution and 5 mL of saturated $Na_2S_2O_3$ solution were then added, and the mixture was stirred for 5 min. The organic layer was separated, washed with saturated NaCl solution (10 mL × 1), dried with anhydrous $Na_2SO_4$, and filtered with suction to remove the desiccant. The filtrate was concentrated

under reduced pressure to afford a crude product, which was purified by Prep-TLC to afford light-yellow solid product **C153-5** (40 mg, 53%). LCMS: $[M + H]^+$ = 373.

Step 5:

**C153-5** (40 mg, 0.1075 mmol) and **A1-4** (55.1 mg, 0.0968 mmol) were dissolved in 8.0 mL of anhydrous DCM. $CH_3COOH$ (6.45 mg, 0.1075 mmol) was added dropwise under nitrogen protection. The mixture was stirred at room temperature for 30 min. Then $NaBH_3CN$ (62.8 mg, 0.1613 mmol) was added. The mixture was reacted with stirring at room temperature for 2h. The mixture was diluted with 30 mL of DCM, and then washed successively with saturated $NH_4Cl$ solution (15 mL × 1) and saturated NaCl solution (10 mL × 1). The organic layer was dried with anhydrous $Na_2SO_4$, and filtered with suction to remove the desiccant. The filtrate was concentrated under reduced pressure to afford a crude product, which was purified by Prep-HPLC to afford white solid pure product **C153** (16 mg, yield: 16.2%). LCMS: $[M + H]^+$ = 926. $^1$H-NMR: (400 MHz, DMSO-$d_6$) δ 11.163 (s, 1H), 10.962 (s, 1H), 8.480 (brs, 1H), 8.061 (d, *J* = 13.2 Hz, 1H), 7.534-7.492 (m, 2H), 7.473-7.292 (m, 3H), 7.241-7.221 (m, 1H), 7.110 (t, *J* = 7.2 Hz, 1H), 6.623 (d, *J* = 2.4 Hz, 1H), 6.477 (dd, *J* = 2.4, 8.8 Hz, 1H), 5.127 (dd, *J* = 5.2, 13.6 Hz, 1H), 4.390 (d, *J* = 17.6 Hz, 1H), 4.244 (d, *J* = 17.2 Hz, 1H), 4.128 (t, *J* = 6.4 Hz, 2H), 3.758 (s, 3H), 3.720-3.690 (m, 2H), 3.300-3.277 (m, 1H), 2.954-2.864 (m, 1H), 2.690-2.558 (m, 4H), 2.328-2.204 (m, 7H), 2.010-1.974 (m, 2H), 1.855-1.745 (m, 10H), 1.549-1.237 (m, 12H).

**Preparation of 4-((7-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)heptyl)oxy)-2-(2,6-dioxopiperidin-3-yl)isoindoline-1,3-dione (C158)**

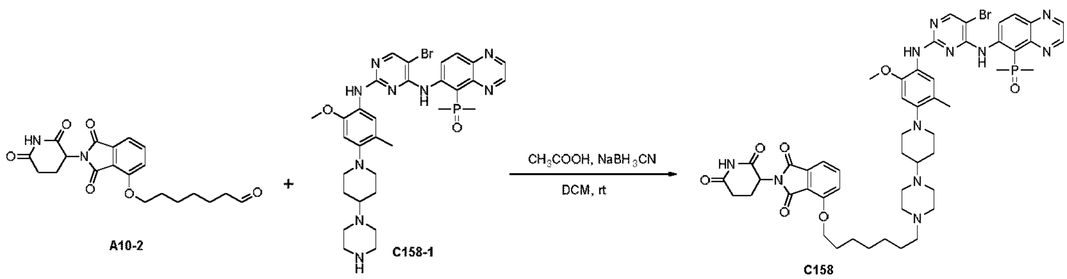


A10-2 C158-1 C158

A10-2 (46.3 mg, 0.12 mmol) and **C158-1** (67.9 mg, 0.1 mmol) were dissolved in 10.0 mL of anhydrous DCM. $CH_3COOH$ (6.0 mg, 0.1 mmol, dissolved in 0.5 mL of DCM) was added under nitrogen protection. The mixture was stirred at room temperature for 1h. Then solid $NaBH_3CN$ (15.7 mg, 0.25 mmol) was added and the mixture was further reacted at room temperature for 2h. LCMS showed that the reaction was completed. The mixture was diluted with 20 mL of DCM, and then washed successively with saturated $NH_4Cl$ (1 × 15 mL) and saturated brine (1 × 15 mL). The organic layer was dried with anhydrous $Na_2SO_4$, and filtered with suction. The filtrate was concentrated under reduced pressure to afford a crude product. The crude product was purified by Prep-HPLC to afford yellow solid pure product **C158,** 36 mg, yield: 34.3%. LCMS: $[M + H]^+$ = 1050. $^1$H-NMR: (400 MHz, DMSO-$d_6$) δ 11.749 (s, 1H), 11.096 (s, 1H), 8.887-8.849 (m, 3H), 8.341 (s, 1H), 8.288 (s, 1H), 7.948 (d, *J* = 9.6 Hz, 1H), 7.841 (t, *J* = 7.2 Hz, 1H), 7.531 (d, *J* = 8.8 Hz, 1H), 7.467 (d, *J* = 7.2 Hz, 1H), 7.406 (s, 1H), 6.767 (s, 1H), 5.102 (dd, *J* = 5.2, 12.8 Hz, 1H), 4.583 (s, 6H), 4.236 (t, *J* = 6.4 Hz, 3H), 3.789 (s, 3H), 3.198-2.700 (m, 9H), 2.625-32.532 (m, 3H), 2.111 (s, 3H), 2.049 (s, 4H), 2.013 (s, 3H), 1.800-1.647 (m, 5H), 1.495-1.376 (m, 6H), 1.238 (s, 2H).

**Preparation of 3-(4-(1-(5-(4-(1-(4-((5-chloro-4-((2-(dimethylphosphoryl)phenyl)amino)pyrimidin-2-yl)amino)-3-methoxyphenyl)piperidin-4-yl)piperazin-1-yl)pentyl)-1H-pyrazol-4-yl)-1-oxoisoindolin-2-yl)piperidine-2,6-dione (C162)**

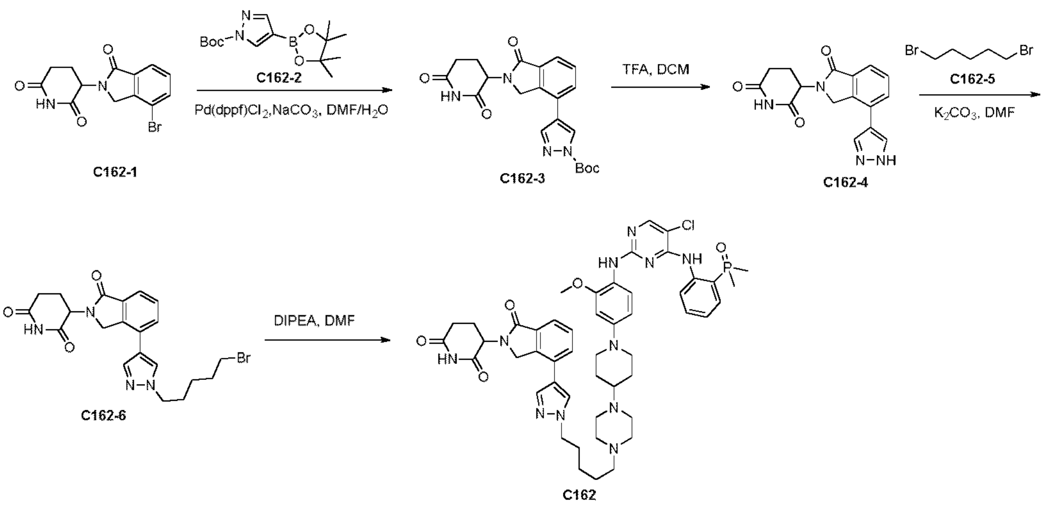


C162-1 C162-3 C162-4 C162-6 C162

Step 1:

Compound **C162-1** (or **C1-1**) (300 mg, 1 mmol), compound **C162-2** (440 mg, 1.5 mmol), $Pd(dppf)Cl_2$ (73.1 mg, 0.1 mmol) and anhydrous sodium carbonate (414 mg, 3 mmol) were added to a 50 mL single-necked flask, and then DMF (10 mL) and water (2 mL) were added. The mixture was degassed and deoxidized with nitrogen gas, and heated to 90°C in a microwave reactor under nitrogen protection for 1h. After the mixture was cooled, water (30 mL) was added and the mixture was extracted with ethyl acetate for three times (20 mL for each time). The organic phases were combined, washed with brine (25 mL × 3), dried with anhydrous sodium sulfate, and filtered. The filtrate was concentrated under reduced pressure to afford a crude product. The crude product was separated by column chromatography to afford light white solid **C162-3,** 140 mg, yield: 34%.

Step 2:

Compound **C162-3** (70 mg, 0.17 mmol) and dichloromethane (3 mL) were added to a 25 mL single-necked flask and dissolved with stirring. Then, trifluoroacetate (2 mL) was added. The mixture was reacted with stirring at room temperature for 1h. The reaction process was monitored by TLC. After the reaction was completed, the mixture was concentrated under reduced pressure to remove dichloromethane and most of trifluoroacetate, and dissolved with dichloromethane (10 mL). Sodium bicarbonate solution was added to adjust the pH to 8-9. After separation, the dichloromethane solution was dried with anhydrous sodium sulfate, and filtered. The filtrate was concentrated under reduced pressure to afford light-yellow solid **C162-4,** 51 mg, yield: 96%. LCMS: $[M + H]^+$ = 311.

Step 3:

Compound **C162-4** (50 mg, 0.16 mmol), compound **C162-5** (36.8 mg, 0.16 mmol) and potassium carbonate (66.24 mg, 0.48 mmol) were added to a 25 mL single-necked flask, and then DMF (3 mL) was added. The mixture was stirred and heated to 45°C to react overnight. The reaction process was monitored by LCMS. After the reaction was completed, the mixture was cooled. Water (10 mL) was added and the mixture was extracted with ethyl acetate for three times (5 mL for each time). The organic phases were combined, washed with brine (5 mL × 3), dried with anhydrous sodium sulfate, and filtered. The filtrate was concentrated under reduced pressure to afford a crude product. The crude product was separated by preparative thin layer chromatography to afford light-yellow waxy solid **C162-6,** 22 mg, yield: 30.1%. LCMS: $[M + H]^+$ = 459, 461.

Step 4:

Compound **C162-6** (20 mg, 0.053 mmol), compound **A1-4** (30.5 mg, 0.053 mmol) and diisopropylethylamine (20.5 mg, 0.159 mmol) were added to a 25 mL single-necked flask, and then DMF (2 mL) was added. The mixture was dissolved with stirring, heated to 70°C and reacted for 2h. The reaction process was monitored by LCMS. After the reaction was completed, the mixture was cooled and concentrated under reduced pressure to afford a crude product. The crude product was separated by preparative high performance liquid chromatography to afford white solid **C162,** 9 mg, yield: 17.9%. LCMS: $[M + H]^+$ = 948.

**Preparation of 3-(5-(7-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)hept-1-yn-1-yl)-1-oxoisoindolin-2-yl)piperidine-2,6-dione (C163)**

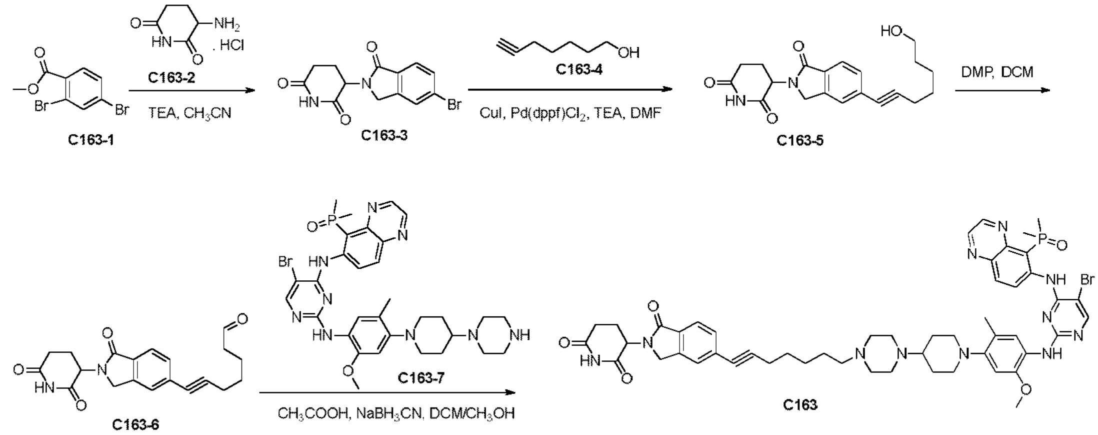


Step 1:

Compound **C163-1** (5g, 16.35 mmol), compound **C163-2** (3.49g, 21.25mmol), and triethylamine (2.15g, 21.25 mmol) were dissolved in 80 mL of acetonitrile. The mixture was stirred at 80°C under nitrogen protection overnight. The mixture was cooled to room temperature, and filtered. Solids were rinsed with acetonitrile. Mother liquor was concentrated, and washed with a small amount of acetonitrile. Solids were combined and dried to afford white solid compound **C163-3,** which was directly used in the next step (7.03g, yield: 84.4%). LCMS: $[M + H]^+$ = 323, 325.

Step 2:

Compound **C163-3** (322mg, 1mmol), compound **C163-4** (280mg,2.5mmol), cuprous iodide (38mg, 0.2mmol), $Pd(dppf)Cl_2$ (280mg, 0.4mmol) and triethylamine (30.3mg, 3mmol) were dissolved in 10 mL of anhydrous DMF. The mixture was reacted with stirring at 70°C under nitrogen protection overnight. The solvent was removed under reduced pressure. The crude product was purified by thin layer chromatography to afford off-white solid compound **C163-5** (135mg, yield: 38.1%). LCMS: $[M + H]^+$ = 355.

Step 3:

Compound **C163-5** (45mg, 0.127mmol) and Dess-Martin reagent (162mg, mmol) were dissolved in 20 mL of dichloromethane and stirred at 50°C for 2h. The mixture was cooled to room temperature, and quenched with saturated sodium bicarbonate aqueous solution. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C163-6** (26.7 mg, yield: 60.7%). LCMS: $[M + H]^+$ = 353.

Step 4:

Compound **C163-6** (26.7mg, 0.076mmol) and **C163-7** (or **C158-1)** (51.5mg, 0.076mmol) were dissolved in 3 mL of dichloromethane/methanol (2/1), and 1 drop of glacial acetic acid was added dropwise. The mixture was stirred at room temperature under nitrogen protection for 1h. Then sodium cyanobohydride (4.8mg, 0.076mmol) was added and the mixture was further stirred for 1h. The solvent was removed under reduced pressure below 40°C, and the crude product was purified by Flash chromatography to afford off-white solid compound **C163** (15mg, yield: 19.5%). LCMS: $[M + H]^+$ = 1016, 1018.

**Preparation of 4-((7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)heptyl-7,7-dideuterium)oxy)-2-(2,6-dioxopiperidin-3-yl)isoindoline-1,3-dione (C164)**

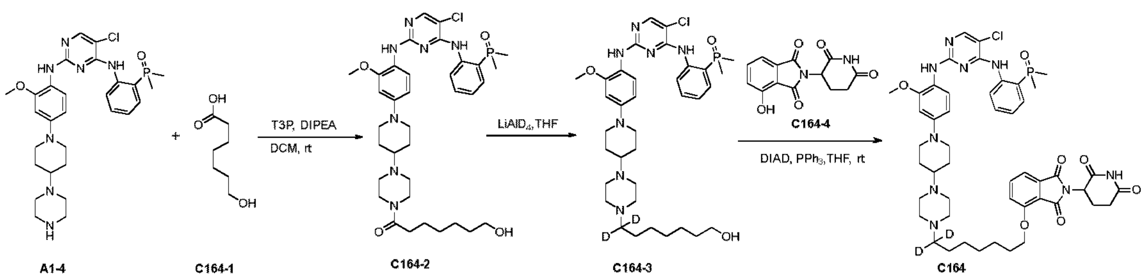


A1-4 C164-1 C164-2 C164-3 C164

Step 1:

**A1-4** (100 mg, 0.176 mmol) and **C164-1** (30.8 mg, 0.211 mmol) were dispersed in 10.0 mL of anhydrous DCM, and T3P (224 mg, 0.352 mmol) and DIPEA (90.8 mg, 0.704 mmol) were added successively under nitrogen protection. The mixture was reacted at room temperature for 2h. The mixture was diluted with 20 mL of dichloromethane, and then washed successively with saturated $NH_4Cl$ (10 mL × 1) and saturated NaCl (10 mL × 1). The organic layer was dried with anhydrous $MgSO_4$, and filtered with suction. The filtrate was concentrated under reduced pressure to afford a crude product. The crude product was purified by Prep-TLC to afford yellow solid product **C164-2** (45 mg, 36.7%). LCMS: $[M + H]^+$ = 698.

Step 2:

**C164-2** (45 mg, 0.065 mmol) was dissolved in 10.0 mL of anhydrous THF. $LiAlD_4$ (27.1 mg, 0.65 mmol) was added under nitrogen protection. The mixture was reacted at 70°C for 2.0 h. LCMS showed that the reaction was completed. The mixture was cooled to 0°C, quenched with 1.0 mL of $H_2O$, and then 5 drops of 1 mol/L NaOH solution was added to the mixture and stirred for 5 min. The organic layer was separated and concentrated under reduced pressure to afford crude **C164-3** (35 mg, 77.7%). The crude product was directly used in the next step. LCMS: $[M + H]^+$ = 686.

Step 3:

**C164-3** (35 mg, 0.051 mmol), **C164-4** (or **A2-1**) (15.4 mg, 0.056 mmol) and $PPh_3$ (20 mg, 0.077 mmol) were dissolved in 6.0 mL of anhydrous THF. DIAD (15.4 mg, 0.077 mmol) was added under nitrogen protection. The mixture was reacted at room temperature for 2h. LCMS showed that the reaction was completed. The mixture was concentrated under reduced pressure to afford a crude product, which was purified by Prep-HPLC to afford light-yellow solid product **C164** (10 mg, yield: 20.8%). LCMS: $[M + H]^+$ = 942.

**Preparation of 3-(4-(7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hept-1-yn-1-yl)-5-fluoro-1-oxoisoindolin-2-yl)piperidine-2,6-dione (C166)**

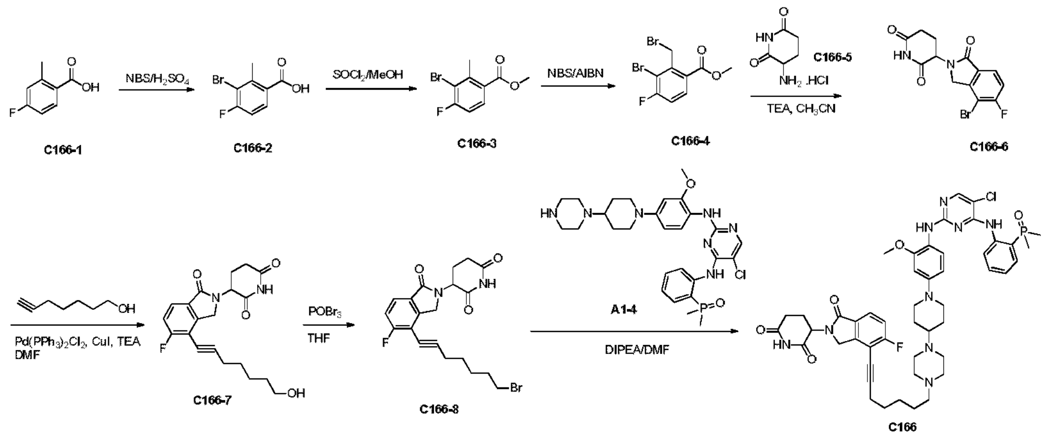


C166-1 C166-2 C166-3 C166-4 C166-6 C166-7 C166-8 C166

Step 1:

Compound **C166-1** (10 g, 64.87 mmol) was added to a 500 mL single-necked flask, and then sulfuric acid (200 mL) was added. The mixture was dissolved with stirring, and then NBS (10.4 g, 58.44 mmol) was added. The mixture was reacted with stirring at room temperature for 5h. The reaction process was monitored by LCMS. After the reaction was completed, the mixture was added to ice water (1000g) to quench the reaction. White solids were precipitated, and collected by filtration. The filter cake was washed three times with water, 200 mL for each time, to afford a white solid containing water, which was vacuum-dried at 50°C to afford 12 g of a mixture of white powder **C166-2,** which was directly used in the next step.

Step 2:

The mixture of compound **C166-2** (6 g, 25.75 mmol) was added to a 250 mL single-necked flask and dissolved in methanol (100 mL). The mixture was cooled to 0°C, and then thionyl chloride (7.67 g, 64.37 mmol) was added dropwise. After the dropwise addition, the mixture was heated to reflux and reacted for 2h. The reaction process was monitored by TLC. The reaction was stopped until the raw materials were basically transformed. The mixture was concentrated under reduced pressure to remove most of solvent and thionyl chloride, and then ice water was added to the concentrated solution. The mixture was extracted with ethyl acetate (100 mL × 3). The organic phases were combined, washed successively with saturated sodium bicarbonate solution, water and brine (50 mL × 2), dried with anhydrous sodium sulfate, and filtered. The filtrate was concentrated under reduced pressure to afford 5.2 g of light-yellow oily product, yield: 81.7%, which was directly used in the next step.

Step 3:

The mixture of compound **C166-3** (3 g, 12.1mmol) was added to a 250 mL single-necked flask, and dissolved with chloroform (100 mL). NBS (2.16 g, 12.1 mmol) and AIBN (196.8 mg, 1.21 mmol) were then added successively. The mixture was stirred and heated to reflux for 5h. The reaction process was monitored by TLC. The reaction was stopped until the raw materials were basically transformed into the product. The mixture was cooled, and then the insolubles were removed by filtration. The mother liquor was washed with water for three times (20 mL for each time), dried with anhydrous sodium sulfate, and filtered. The filtrate was concentrated under reduced pressure to afford a crude product, which was separated by column chromatography to afford 2.2 g of colorless oily product **C166-4.**

Step 4:

Compound **C166-4** (2 g, 12.24 mmol), compound **C166-5** (4 g, 12.24 mmol), acetonitrile (100 mL) and triethylamine (2.56 mL, 36.72 mmol) were successively added to a 250 mL single-necked flask. The mixture was heated to reflux and reacted overnight. The mixture was cooled, and then solids were precipitated. The precipitation was further continued for 5h. Then the mixture was filtered to afford a light white solid, which was washed with acetonitrile (20 mL × 2), and vacuum-dried at 40 °C to afford 700 mg of light white powder **C166-6.** Yield: 16.8%. LCMS: $[M + H]^+$ = 341, 343.

Step 5:

Compound **C166-6** (700 mg, 2.05 mmol), hept-6-yn-1-ol (392 mg, 3.5 mmol), $Pd(PPh_3)_2Cl_2$ (490 mg, 0.35

mmol), cuprous iodide (66.8 mg, 0.35 mmol) and triethylamine (530 mg, 5.25 mmol) were added to a 100 mL three-necked flask, and then DMF (20 mL) was added. The mixture was dissolved, then heated to 70°C and reacted under nitrogen protection for 5h. The reaction process was monitored by LCMS. After the reaction was completed, the mixture was cooled and concentrated under reduced pressure to afford a crude product, which was separated by column chromatography to afford 170 mg of light-yellow solid **C166-7.** Yield: 22.3%. LCMS: $[M + H]^+$ = 373.2.

Step 6:

Compound **C166-7** (170 mg, 0.46 mmol) was added to a 50 mL three-necked flask equipped with a condenser and dissolved in THF (3 mL). Phosphorus oxybromide (170 mg, 0.59 mmol) was then added. The mixture was heated to 70°C and reacted for 2h. The reaction process was monitored by LCMS. After the reaction was completed, the mixture was cooled, and ethyl acetate (10 mL) was added. The mixture was washed twice with saturated sodium bicarbonate solution, 5 mL for each time, then washed with brine (5 mL × 2), dried with anhydrous sodium sulfate, and filtered. The filtrate was concentrated under reduced pressure to afford a crude product. The crude product was separated by preparative thin layer chromatography to afford 39 mg of light white solid **C166-8.** Yield: 19.5%. LCMS: $[M + H]^+$ = 435, 437.

Step 7:

Compound **C166-8** (40 mg, 0.11mmol), **A1-4** (61.2 mg, 0.11 mmol), diisopropylethylamine (41.7 mg, 0.33 mmol) and DMF (2 mL) were added to a 25mL single-necked flask equipped with a condenser. The mixture was heated to 70°C and reacted for 3h. The reaction process was monitored by LCMS. The reaction was stopped until the raw materials were basically transformed into the title product. The mixture was cooled and concentrated under reduced pressure to afford a crude product, which was separated by prep-HPLC to afford 10 mg of white powder **C166.** Yield: 10%. LCMS: $[M + H]^+$ = 924.

**Preparation of 3-(4-(7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hept-1-yn-1-yl)-6-fluoro-1-oxoisoindolin-2-yl)piperidine-2,6-dione (C167)**

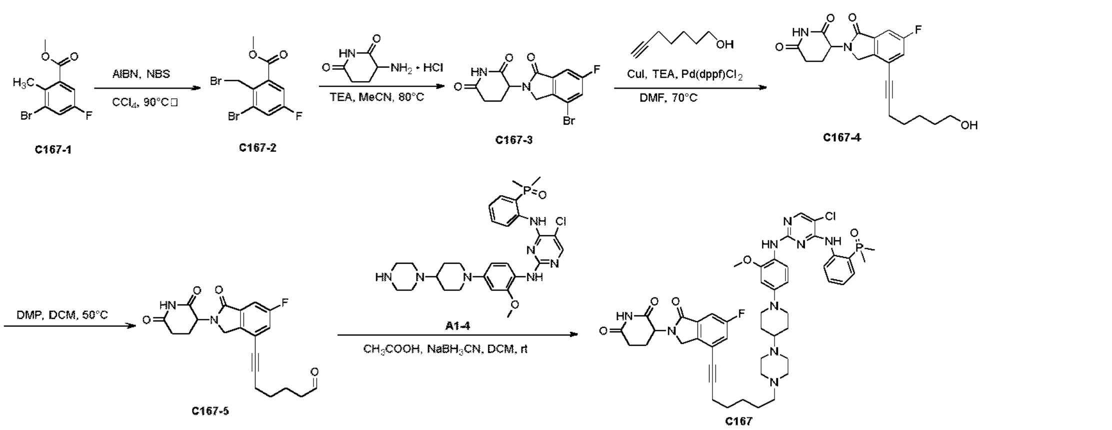


Step 1:

**C167-1** (1.0 g, 4.06 mmol) was dissolved in 10.0 mL of $CCl_4$. NBS (1.1 g, 6.1 mmol) and AIBN (266.5 mg, 1.624 mmol) were added under nitrogen protection. The mixture was heated to 90°C, and reacted under reflux for 20 h. LCMS showed that there was no raw material remaining. The mixture was cooled to room temperature, and then filtered with suction. The filtrate was concentrated under reduced pressure to afford a crude product, which was purified by Flash chromatography to afford 1.2 g of light-yellow oily product **C167-2** (which became solid upon standing).

Step 2:

**C167-2** (1.2 g, 3.69 mmol) and 3-aminopiperidine-2,6-dione hydrochloride (787 mg, 4.80 mmol) were dispersed in 25.0 mL of anhydrous MeCN, and TEA (485 mg, 4.80 mmol) was added. The mixture was heated to 80°C, and reacted

under reflux for 16h. LCMS showed that the reaction was completed. The mixture was cooled to room temperature, and then filtrated with suction. The filter cake was washed with MeCN for three times, and dried with baking to afford product **C167-3** (1.0 g, yield: 80%). LCMS: $[M + H]^+$ = 341, 343.

Step 3:

**C167-3** (340 mg, 1.0 mmol), CuI (38 mg, 0.2 mmol) and Pd(dppf)$Cl_2$ (292.4 mg, 0.4 mmol) were dispersed in 17.0 mL of anhydrous DMF. Hept-6-yn-1-ol (281 mg, 2.5 mmol) and TEA (303 mg, 3.0 mmol) were added successively under $N_2$ protection. The mixture was heated to 70°C and reacted for 16h. LCMS showed that the reaction was completed. The mixture was cooled to room temperature, diluted with 80 mL of ethyl acetate, and then washed successively with saturated $NH_4Cl$ (2 × 40 mL), $H_2O$ (2 × 40 mL) and saturated brine (2 × 40 mL). The organic layer was dried with anhydrous sodium sulfate, and filtered with suction. The filtrate was concentrated under reduced pressure to afford a crude product, which was purified by RP-Flash chromatography to afford light-yellow solid product **C167-4** (95 mg, 25.5%). LCMS: $[M + H]^+$ = 373.

Step 4:

**C167-4** (90 mg, 0.242 mmol) was dispersed in 25.0 mL of anhydrous DCM. Dess-Martin reagent (154 mg, 0.363 mmol) was added under $N_2$ protection. The mixture was heated to 50°C, and reacted under reflux for 2.0 h. TLC showed that the reaction was completed. The mixture was cooled to room temperature, and then 10 mL of saturated $NaHCO_3$ solution and 10 mL of saturated $Na_2S_2O_3$ solution were added. The mixture was stirred at room temperature for 5 min. The organic layer was separated, dried with anhydrous sodium sulfate, and filtered with suction. The filtrate was concentrated under reduced pressure to afford a crude product. The crude product was purified by Prep-TLC to afford light-yellow solid product **C167-5** (55 mg, 60%). LCMS: $[M + H]^+$ = 371.

Step 5:

**C167-5** (30.0 mg, 0.081 mmol) and **A1-4** (46.1 mg, 0.081 mmol) were dissolved in 5.5 mL of anhydrous DCM. $CH_3COOH$ (4.86 mg, 0.081 mmol, dissolved in 0.5 mL of DCM) was added under nitrogen protection. The mixture was stirred at room temperature for 0.5h. Then solid $NaBH_3CN$ (10.2 mg, 0.162 mmol) was added. The mixture was further reacted at room temperature for 2h. LCMS and TLC showed that the reaction was completed. The mixture was diluted with 20 mL of DCM, and then washed successively with saturated $NH_4Cl$ (1 × 15 mL) and saturated brine (1 × 15 mL). The organic layer was dried with anhydrous $Na_2SO_4$, and filtered with suction. The filtrate was concentrated under reduced pressure to afford a crude product, which was purified by Prep-HPLC to afford 25 mg of white solid pure product. Yield: 33.5%. LCMS: $[M + H]^+$ = 924. $^1$H-NMR: (400 MHz, DMSO-$d_6$) δ 11.190 (s, 1H), 11.051 (s, 1H), 8.490 (br s, 1H), 8.098 (s, 1H), 8.066 (s, 1H), 7.563-7.507 (m, 3H), 7.397-7.315 (m, 2H), 7.110 (t, *J* = 6.4 Hz, 1H), 6.628 (d, *J* = 2.4 Hz, 1H), 6.479 (dd, *J* = 2.4 Hz, 8.4 Hz, 1H), 5.182 (dd, *J* = 5.2 Hz, 13.2 Hz, 1H), 4.462 (dd, *J* = 17.2 Hz, 60.0 Hz, 2H), 3.758 (s, 3H), 3.725 (d, *J* = 12.8 Hz, 2H), 2.967-2.876 (m, 2H), 2.672-2.503 (m, 3H), 2.501-2.397 (m, 6H), 2.398-2.247 (m, 3H), 2.032-1.974 (m, 1H), 1.832-1.748 (m, 12H), 1.5615-1.527 (m, 2H), 1.498-1.436 (m, 6H).

**Preparation of 3-(4-(3-(2-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)ethoxy)prop-1-yn-1-yl)-1-oxoisoindolin-2-yl)piperidine-2,6-dione (C169)**

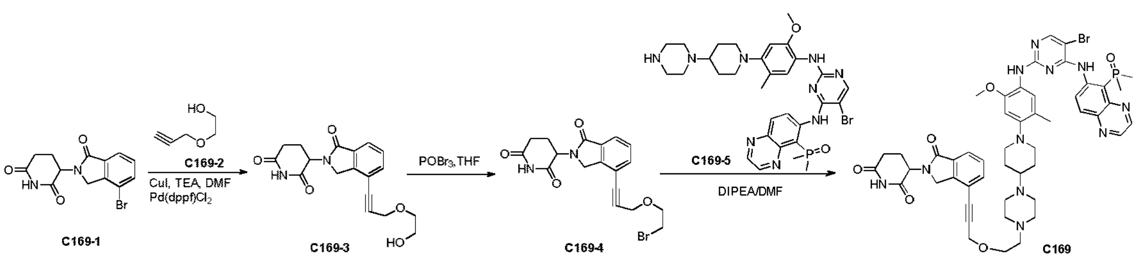


C169-1 C169-3 C169-4 C169

Step 1:

Compound **C169-1** (or **C1-1**) (309 mg, 1 mmol), compound **C169-2** (200 mg, 2 mmol), Pd(dppf)$Cl_2$ (280 mg,

0.4 mmol), cuprous iodide (38.2 mg, 0.2 mmol) and triethylamine (303 mg, 3 mmol) were added to a 100 mL single-necked flask, and then DMF (20 mL) was added. After the mixture was dissolved, the atmosphere was replaced with nitrogen gas for three times to remove oxygen. The mixture was heated to 70°C and reacted under nitrogen protection for 5h. The reaction process was monitored by LCMS. After the reaction was completed, the mixture was cooled and concentrated under reduced pressure to afford a crude product, which was separated by column chromatography to afford light-yellow solid **C169-3,** 70 mg, yield: 20.3%. LCMS: $[M + H]^+$ = 343.

Step 2:

Compound **C169-3** (70 mg, 0.2 mmol) was added to a 50 mL three-necked flask equipped with a condenser and dissolved in THF (2 mL). Phosphorus oxybromide (170 mg, 0.59 mmol) was then added. The mixture was heated to 70°C and reacted for 2h. The reaction process was monitored by LCMS. After the reaction was completed, the mixture was cooled, and ethyl acetate (10 mL) was added. The mixture was washed with saturated sodium bicarbonate solution (5 mL × 2), then washed with brine (5 mL × 2), dried with anhydrous sodium sulfate, and filtered. The filtrate was concentrated under reduced pressure to afford a crude product. The crude product was separated by preparative thin layer chromatography to afford 24 mg of light white solid **C169-4.** Yield: 29.7%. LCMS: $[M + H]^+$ = 405, 407.

Step 3:

Compound **C169-4** (12 mg, 0.029 mmol), compound **C169-5** (or **C158-1**) (19.3 mg, 0.029 mmol), diisopropylethylamine (11.2 mg, 0.087 mmol) and DMF (1 mL) were added to a 25 mL single-necked flask equipped with a condenser. The mixture was heated to 70°C and reacted for 3h. The reaction process was monitored by LCMS. After the reaction was completed, the mixture was cooled and concentrated under reduced pressure to afford a crude product, which was separated by prep-HPLC to afford 10 mg of white powder **C169.** Yield: 34.5%. LCMS: $[M + H]^+$ = 1004.

**Preparation of 4-(7-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)hept-1-yn-1-yl)-2-(2,6-dioxopiperidin-3-yl)isoindoline-1,3-dione (C171)**

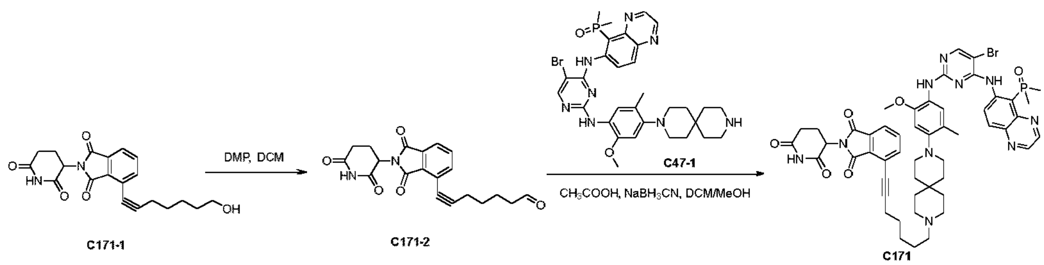


C171-1 C171-2 C171

Step 1:

Compound **C171-1** (or **C148-3**) (56.6 mg, 0.154 mmol) and Dess-Martin reagent (196mg, 0.461mmol) were dissolved in dichloromethane (20 mL). The mixture was reacted with stirring at 50°C for 2h. The mixture was cooled to room temperature, and quenched with saturated sodium bicarbonate aqueous solution. The layers were separated, and the aqueous phase was extracted with dichloromethane for three times. The organic phases were combined, washed with saturated brine (15 mL × 2), dried with anhydrous sodium sulfate, and filtered. The solvent was removed under reduced pressure. The crude product was separated by column chromatography to afford compound **C171-2** (45mg, yield: 80.4%). LCMS: $[M + H]^+$ = 367.

Step 2:

Compound **C171-2** (45mg, 0.12mmol) and compound **C47-1** (82mg, 0.12mmol) were dissolved in dichloromethane and methanol (2/1) (3 mL), and 1 drop of glacial acetic acid was added. The mixture was stirred at room temperature under nitrogen protection for 1h. Then sodium cyanobohydride (7.8mg, 0.12mmol) was added. The mixture was further stirred for 1h. The mixture was concentrated to remove the solvent. The crude product was purified by Flash chromatography to afford compound **C171** (15mg, yield: 14.7%). LCMS: $[M + H]^+$ = 1015, 1017.

**Preparation of 3-(4-(8-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)oct-1-yn-1-yl)-1-oxoisoindolin-2-yl)piperidine-2,6-dione (C172)**

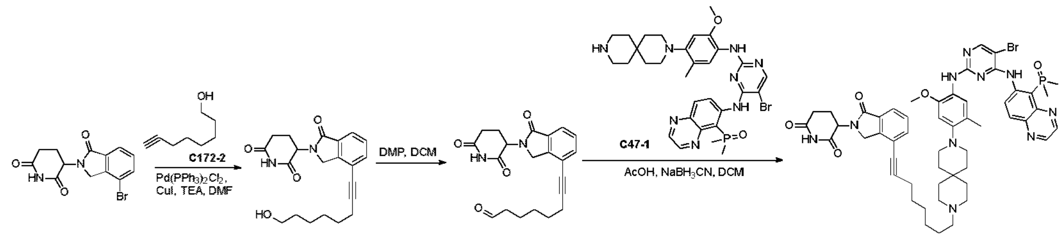


C172-1 C172-3 C172-4 C172

Step 1:

Compound **C172-1** (or **C1-1**) (309 mg, 1 mmol), compound **C172-2** (252 mg, 2 mmol), bistriphenylphosphine palladium dichloride (280 mg, 0.4 mmol), cuprous iodide (38.4 mg, 0.2 mmol) and triethylamine (303 mg, 3 mmol) were added to a 100 mL single-necked flask, and then DMF (20 mL) was added. After the mixture was dissolved, the atmosphere was replaced with nitrogen gas for three times to remove oxygen. The mixture was heated to 70°C and reacted under nitrogen protection for 5h. The reaction process was monitored by LCMS. After the reaction was completed, the mixture was cooled and concentrated under reduced pressure to afford a crude product, which was separated by column chromatography to afford 200 mg of light-yellow solid **C172-3.** Yield: 54.3%. LCMS: $[M + H]^+$ = 369.4.

Step 2:

Compound **C172-3** (200 mg, 0.543 mmol) and dichloromethane (40 mmol) were added to a 100 mL single-necked flask and dissolved with stirring. Dess-Martin reagent (345 mg, 0.814 mmol) was then added. The mixture was heated to 40°C and reacted for 2h. The reaction process was monitored by LCMS. The reaction was stopped until the raw materials were basically transformed into the product. The mixture was cooled to 0-5°C with an ice bath. Saturated sodium bicarbonate solution (10 mL) and sodium thiosulfate solution (10 mL) were successively added and stirred for about 10 minutes. After separation, extracted twice with dichloromethane (20 mL for each time). The organic phases were combined, washed with saturated brine (10 mL × 2), dried with anhydrous sodium sulfate, and concentrated under reduced pressure to afford 180 mg of white viscous solid **C172-4,** which was directly used in the next step. LCMS: $[M + H]^+$ = 367.2.

Step 3:

Compound **C172-4** (40 mg, 0.11mmol) and compound C47-1 (59.7 mg, 0.09 mmol) were added to a 25 mL single-necked flask and dissolved in dichloromethane (5 mL). $CH_3COOH$ (10 mg, 0.16 mmol) was then added. The mixture was reacted with stirring at room temperature for 30 minutes. Then sodium cyanobohydride (9.5 mg, 0.15 mmol) was added and reacted at room temperature for 3h. The reaction process was monitored by LCMS, and the reaction was stopped until the raw materials were basically transformed into the product. Saturated $NH_4Cl$ (5 mL) was added to the mixture and stirred for 5 minutes. The layers were separated, and extracted twice with dichloromethane, 10 mL for each time. The organic phases were combined, washed with saturated brine (5 mL × 2), dried with anhydrous sodium sulfate, and concentrated under reduced pressure. The residue was separated by Prep-HPLC to afford 20 mg of yellow powder **C172.** Yield: 18%. LCMS: $[M + H]^+$ = 1001.

**Preparation of 3-(4-(9-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)non-1-yn-1-yl)-1-oxoisoindolin-2-yl)piperidine-2,6-dione (C173)**

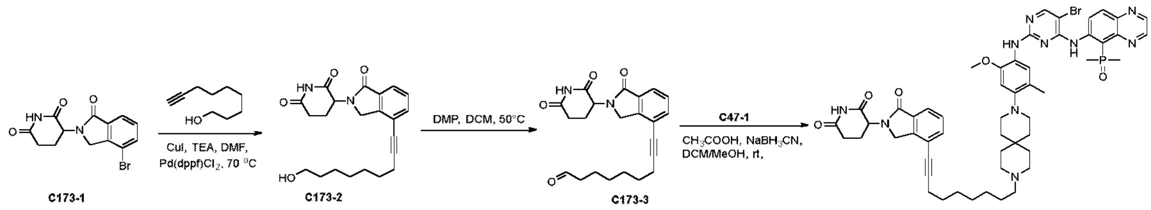


C173-1 C173-2 C173-3 C173

Step 1:

**C173-1** (or **C1-1**) (644 mg, 2.0 mmol), CuI (76 mg, 0.4 mmol) and Pd(dppf)$Cl_2$ (585 mg, 0.8 mmol) were dispersed in 34 mL of anhydrous DMF. 8-alkynyl-1-nonanol (700 mg, 5.0 mmol) and TEA (606 mg, 6.0 mmol) were added successively under $N_2$ protection. The mixture was heated to 70°C and reacted for 20 h. LCMS showed that the reaction was completed. The mixture was cooled to room temperature, diluted with 150 mL of EA, and washed successively with saturated $NH_4Cl$ (2 × 50 mL), $H_2O$ (2 × 50 mL) and saturated brine (2 × 50 mL). The organic layer was dried with anhydrous sodium sulfate, and filtered with suction. The filtrate was concentrated under reduced pressure to afford a crude product, which was purified by RP-Flash chromatography to afford light-yellow solid product **C173-2** (210 mg, 27.5%). LCMS: $[M + H]^+$ = 383.

Step 2:

**C173-2** (200 mg, 0.524 mmol) was dispersed in 30.0 mL of anhydrous DCM. Dess-Martin reagent (333 mg, 0.785 mmol) was added under $N_2$ protection. The mixture was heated to 50°C, and reacted under reflux for 2.0 h. TLC showed that the reaction was completed. The mixture was cooled to room temperature, and then 10 mL of saturated $NaHCO_3$ solution and 10 mL of saturated $Na_2S_2O_3$ solution were added. The mixture was stirred at room temperature for 5 min. The organic layer was separated, dried with anhydrous sodium sulfate, and filtered with suction. The filtrate was concentrated under reduced pressure to afford a crude product, which was purified by Prep-TLC to afford light-yellow solid product **C173-3** (150 mg, 75%). LCMS: $[M + H]^+$ = 381.

Step 3:

**C173-3** (45 mg, 0.1184 mmol) and **C47-1** (70.8 mg, 0.1066 mmol) were dissolved in a mixture of 8.0 mL of anhydrous DCM and 0.8 mL of anhydrous methanol. $CH_3COOH$ (7.1 mg, 0.1184 mmol, dissolved in 0.5 mL of DCM) was added under nitrogen protection. The mixture was stirred at room temperature for 0.5h. Then solid $NaBH_3CN$ (11.2 mg, 0.1776 mmol) was added. The mixture was further reacted at room temperature for 2h. LCMS and TLC showed that the reaction was completed. The mixture was diluted with 20 mL of DCM, and then washed successively with saturated $NH_4Cl$ (1 × 15 mL) and saturated brine (1 × 15 mL). The organic layer was dried with anhydrous $Na_2SO_4$, and filtered with suction. The filtrate was concentrated under reduced pressure to afford a crude product, which was purified by Prep-HPLC to afford 45 mg of yellow solid pure product. Yield: 40.0%. LCMS: $[M + H]^+$ = 1029, 1031.

$^1$H-NMR: (400 MHz, DMSO-$d_6$) δ 12.683 (s, 1H), 11.119 (s, 1H), 8.869 (dd, *J* = 2.0 Hz, 9.2 Hz, 3H), 8.281 (s, 1H), 8.263 (s, 1H), 7.721 (d, *J*= 6.8 Hz, 1H), 7.646 (d, *J* = 0.8 Hz, 1H), 7.643 (d, *J* = 6.8 Hz, 1H), 7.539 (d, *J* = 7.6 Hz, 1H), 7.321 (s, 1H), 6.809 (s, 1H), 5.188 (dd, *J* = 5.2 Hz, 13.6 Hz, 1H), 4.477 (d, *J* = 17.6 Hz, 1H), 4.325 (d, *J* = 17.6 Hz, 1H), 3.784 (s, 3H), 2.513-2.495 (m, 10H), 2.494-2.103 (m, 6H), 2.075 (s, 3H), 2.040 (s, 3H), 2.004 (s, 3H), 1.557-1.429 (m, 18H).

**Preparation of 3-(4-(3-(2-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)ethoxy)prop-1-yn-1-yl)-1-oxoisoindolin-2-yl)piperidine-2,6-dione (C174)**

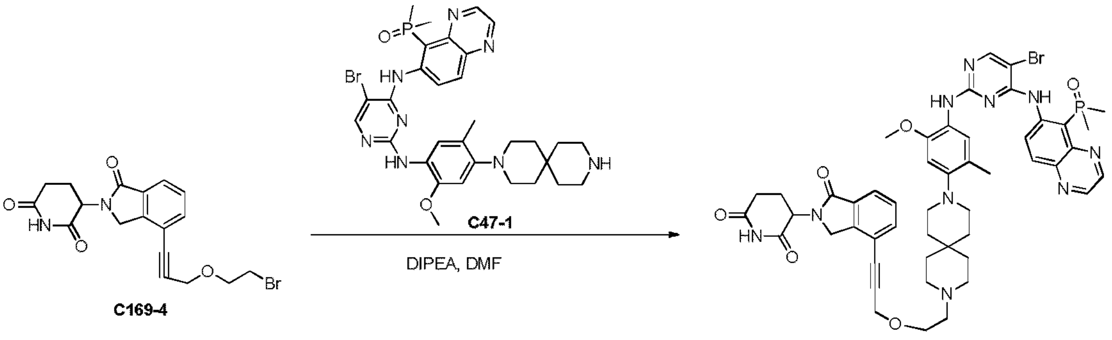


C169-4 C47-1 C174

Compound **C169-4** (2 mg, 0.029 mmol), compound **C47-1** (1 mg, 0.029 mmol), diisopropylethylamine (11.2 mg, 0.087 mmol) and DMF (1 mL) were added to a 25 mL single-necked flask equipped with a condenser. The mixture was heated to 70°C and reacted for 3h. The reaction process was monitored by LCMS. After the reaction was completed, the mixture was cooled and concentrated under reduced pressure to afford a crude product, which was separated by Prep-HPLC to afford 9 mg of white powder **C174.** Yield: 31.2%. LCMS: $[M + H]^+$ = 989, 991.

**Preparation of 3-(4-(3-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)ethoxy)prop-1-yn-1-yl)-1-oxoisoindolin-2-yl)piperidine-2,6-dione (C176)**

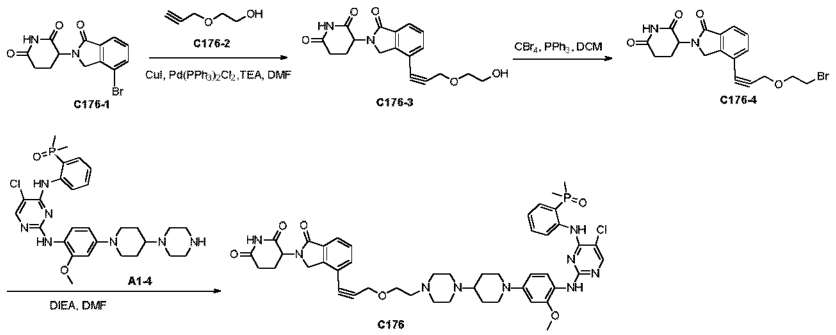


C176-1 C176-2 C176-3 C176-4 A1-4 C176

Step 1:

Compound **C176-1** (or **C1-1**) (322mg, 1.00mmol), compound **C176-2** (200mg, 2.00mmol), cuprous iodide (38mg, 0.20mmol), $Pd(PPh_3)_2Cl_2$ (280mg, 0.40mmol) and triethylamine (303mg, 3.00mmol) were dissolved in dry DMF (10 mL). The mixture was stirred at 70°C under nitrogen protection overnight. The mixture was cooled to room temperature. The solvent was removed under reduced pressure, and the crude product was separated by column chromatography to afford compound **C176-3** (80mg, yield: 23.4%). LCMS: $[M + H]^+$ = 343.

Step 2:

Compound **C176-3** (52mg, 0.152mmol) and carbon tetrabromide (101mg, 0.305mmol) were dissolved in dichloromethane (20 mL). The mixture was stirred at room temperature for 2h. The mixture was cooled to 0°C. A solution of $PPh_3$ (80mg, 0.305mmol) in dichloromethane (2 mL) was added dropwise. The mixture was naturally warmed to room temperature, and then stirred at 55 °C for 2h. The solvent was removed under reduced pressure. The crude product was separated by column chromatography to afford compound **C176-4** (20mg, yield: 32.8%). LCMS: $[M + H]^+$ = 405, 407.

Step 3:

Compound **C176-4** (20mg, 0.050mmol), compound **A1-4** (31mg, 0.054mmol), and DIEA (32mg, 0.248mmol) were dissolved in 1 mL of DMF. The mixture was stirred at 80°C under nitrogen protection for 5h. LCMS showed that the reaction was completed. The solvent was removed at 40°C under reduced pressure, and the crude product was

purified by Flash chromatography to afford **C176** (18mg, yield: 40.9%). LCMS: $[M + H]^+$ = 894. $^1$H NMR (400 MHz, DMSO-$d_6$) δ 11.17 (s, 1H), 11.01 (s, 1H), 8.48 (s, 1H), 8.05 (d, 2H), 7.75 (dd, 2H), 7.54 (dt, 2H), 7.36 (dd, 2H), 7.09 (t, 1H), 6.62 (d, 1H), 6.47 (dd, 1H), 5.75 (s, 1H), 5.16 (dd, 1H), 4.50 (d, 1H), 4.35 (d, 1H), 3.76 (s, 3H), 3.71 (d, 2H), 3.65 (t, 2H), 3.28 (s, 1H), 3.00-2.87 (m, 2H), 2.66 (d, 4H), 2.57 (s, 1H), 2.44 (d, 4H), 2.29 (s, 2H), 2.01 (p, 3H), 1.84 (d, 2H), 1.76 (d, 6H), 1.57-1.42 (m, 3H).

**Preparation of 3-(4-(8-(4-(1-(4-((5-chloro-4-((2-(dimethylphosphoryl)phenyl)amino)pyrimidin-2-yl)amino)-3-methoxyphenyl)piperidin-4-yl)piperazin-1-yl)oct-1-yn-1-yl)-1-oxoisoindolin-2-yl)piperidine-2,6-dione (C177)**

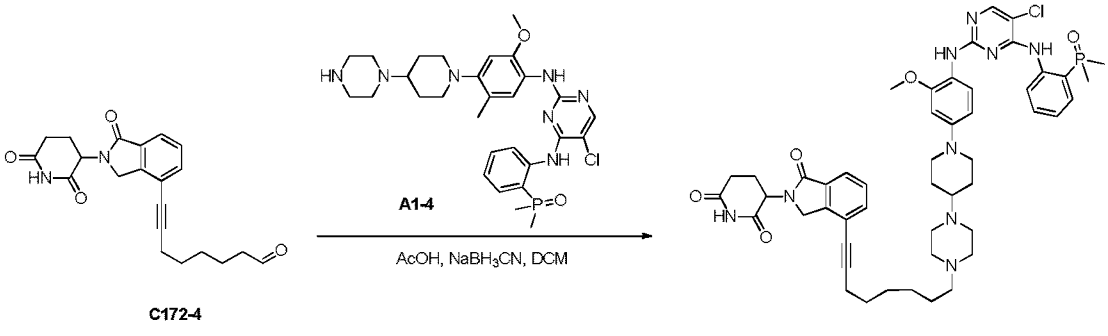


C172-4

C177

Compound **C172-4** (40 mg, 0.11mmol) and **A1-4** (51.2 mg, 0.09 mmol) were added to a 25 mL single-necked flask and dissolved in dichloromethane (5 mL). $CH_3COOH$ (10 mg, 0.16 mmol) was then added. The mixture was reacted with stirring at room temperature for 30 minutes. Then sodium cyanobohydride (9.5 mg, 0.15 mmol) was added and reacted at room temperature for 3h. The reaction process was monitored by LCMS. After the reaction was completed, saturated $NH_4Cl$ (5 mL) was added to the mixture and stirred for 5 minutes. The layers were separated, and extracted with dichloromethane (10 mL × 2). The organic phases were combined, washed with saturated brine (5 mL × 2), dried with anhydrous sodium sulfate, and concentrated under reduced pressure. The residue was separated by Prep-HPLC to afford 18 mg of yellow powder. Yield: 19.5%. LCMS: $[M + H]^+$ = 920.

**Preparation of 3-(4-(9-(4-(1-(4-((5-chloro-4-((2-(dimethylphosphoryl)phenyl)amino)pyrimidin-2-yl)amino)-3-methoxyphenyl)piperidin-4-yl)piperazin-1-yl)non-1-yn-1-yl)-1-oxoisoindolin-2-yl)piperidine-2,6-dione (C178)**

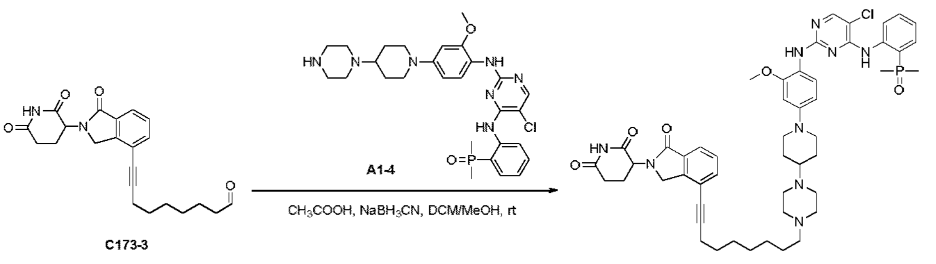


C173-3

C178

**C173-3** (48 mg, 0.1263 mmol) and **A1-4** (64.7 mg, 0.1137 mmol) were dissolved in a mixture of 8.0 mL of anhydrous DCM and 0.8 mL of anhydrous methanol. $CH_3COOH$ (7.6 mg, 0.1263 mmol, dissolved in 0.5 mL of DCM) was added under nitrogen protection. The mixture was stirred at room temperature for 0.5h. Then solid $NaBH_3CN$ (11.9 mg, 0.1895 mmol) was added. The mixture was further reacted at room temperature for 2h. LCMS and TLC showed that the reaction was completed. The mixture was diluted with 20 mL of DCM, and then washed successively with saturated $NH_4Cl$ (1 × 15 mL) and saturated brine (1 × 15 mL). The organic layer was dried with anhydrous $Na_2SO_4$, and filtered with suction. The filtrate was concentrated under reduced pressure to afford a crude product. The crude product was purified by Prep-TLC to afford 55 mg of crude product, which was further purified by Prep-HPLC to afford 35 mg of white solid pure product. Yield: 29.7%. LCMS: $[M + H]^+$ = 934. $^1$H-NMR:(400 MHz, DMSO-$d_6$) δ 11.190 (s, 1H), 11.089 (s, 1H), 8.481 (brs, 1H), 8.092 (d, *J* = 10.0 Hz, 2H), 7.721 (d, *J* = 7.6 Hz, 1H), 7.644 (d, *J* = 7.6 Hz, 1H), 7.539-7.502

(m, 2H), 7.340 (dd, *J* = 8.4 Hz, 17.2 Hz, 2H), 7.070 (t, *J* = 14.4 Hz, 1H), 6.621 (s, 1H), 6.476 (d, *J* = 8.8 Hz, 1H), 5.184 (dd, *J* = 4.8 Hz, 13.2 Hz, 1H), 4.474 (d, *J* = 17.6 Hz, 1H), 4.321 (d, *J* = 17.6 Hz, 1H), 3.757-3.694 (m, 5H), 2.932-2.842 (m, 2H), 2.636-2.530 (m, 3H), 2.444-2.380 (m, 4H), 2.357-2.160 (m, 7H), 1.989-1.933 (m, 2H), 1.809-780 (m, 2H), 1.743 (s, 3H), 1.709 (s, 3H), 1.566-1.355 (m, 8H), 1.319-1.157 (m, 5H).

**Preparation of 3-(4-(10-(4-(1-(4-((5-chloro-4-((2-(dimethylphosphoryl)phenyl)amino)pyrimidin-2-yl)amino)-3-methoxyphenyl)piperidin-4-yl)piperazin-1-yl)dec-1-yn-1-yl)-1-oxoisoindolin-2-yl)piperidine-2,6-dione (C179)**

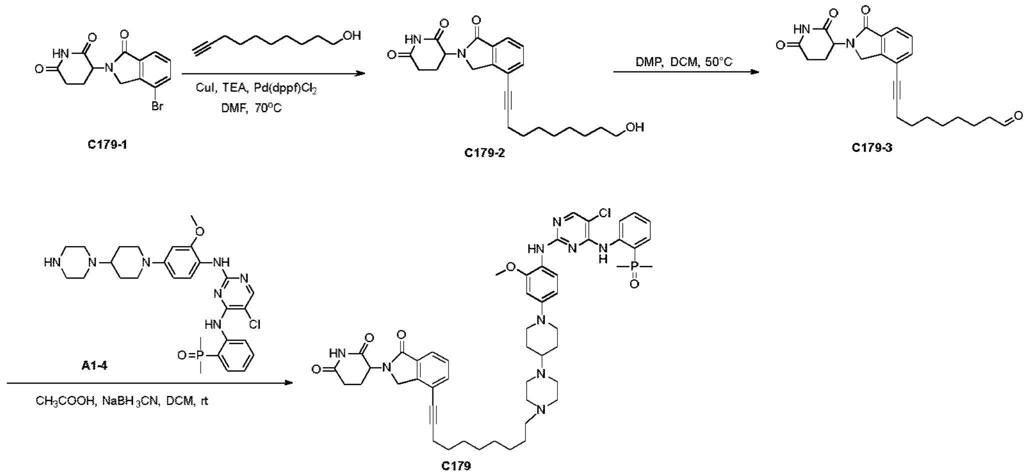


Step 1:

**C179-1** (or **C1-1)** (322 mg, 1.0 mmol), CuI (38 mg, 0.2 mmol) and Pd(dppf)$Cl_2$ (292.4 mg, 0.4 mmol) were dispersed in 15 mL of anhydrous DMF. 9-alkynyl-1-decanol (385 mg, 2.5 mmol) and TEA (303 mg, 3.0 mmol) were added successively under $N_2$ protection. The mixture was heated to 70°C and reacted for 20 h. LCMS showed that the reaction was completed. The mixture was cooled to room temperature, diluted with 80 mL of EA, and then washed successively with saturated $NH_4Cl$ (2 × 50 mL), $H_2O$ (2 × 50 mL) and saturated brine (2 × 50 mL). The organic layer was dried with anhydrous sodium sulfate, and filtered with suction. The filtrate was concentrated under reduced pressure to afford a crude product, which was purified by RP-Flash chromatography to afford light-yellow solid product **C179-2** (105 mg, 26.5%). LCMS: $[M + H]^+$ = 397.

Step 2:

**C179-2** (35 mg, 0.088 mmol) was dissolved in 10.0 mL of anhydrous DCM. Dess-Martin reagent (75 mg, 0.176 mmol) was added under $N_2$ protection. The mixture was heated to 50°C, and reacted under reflux for 2.0 h. TLC showed that the reaction was completed. The mixture was cooled to room temperature, and then 10 mL of saturated $NaHCO_3$ solution and 10 mL of saturated $Na_2S_2O_3$ solution were added. The mixture was stirred vigorously at room temperature for 5 min. The organic layer was separated, dried with anhydrous sodium sulfate, and filtered with suction. The filtrate was concentrated under reduced pressure to afford a crude product, which was purified by Prep-TLC to afford light-yellow solid product **C179-3** (15 mg, 42.8%). LCMS: $[M + H]^+$ = 395.

Step 3:

**C179-3** (15 mg, 0.0381 mmol) and **A1-4** (19.5 mg, 0.0343 mmol) were dissolved in 3.0 mL of anhydrous DCM. $CH_3COOH$ (2.3 mg, 0.0381 mmol, dissolved in 0.5 mL of DCM) was added under nitrogen protection. The mixture was stirred at room temperature for 0.5h. Then solid $NaBH_3CN$ (3.59 mg, 0.0572 mmol) was added. The mixture was further reacted at room temperature for 2h. LCMS and TLC showed that the reaction was completed. The mixture was directly purified by Prep-TLC to afford 15 mg of crude product, which was further purified by Prep-HPLC to afford white solid pure product **C179** (7.0 mg, yield: 19.5%). LCMS: $[M + H]^+$ = 948.

**Preparation of 3-(4-(3-(3-(4-(1-(4-((5-chloro-4-((2-(dimethylphosphoryl)phenyl)amino)pyrimidin-2-yl)amino)-3-methoxyphenyl)piperidin-4-yl)piperazin-1-yl)propoxy)prop-1-yn-1-yl)-1-oxoisoindolin-2-ylpiperidine-2,6-dione (C181)**

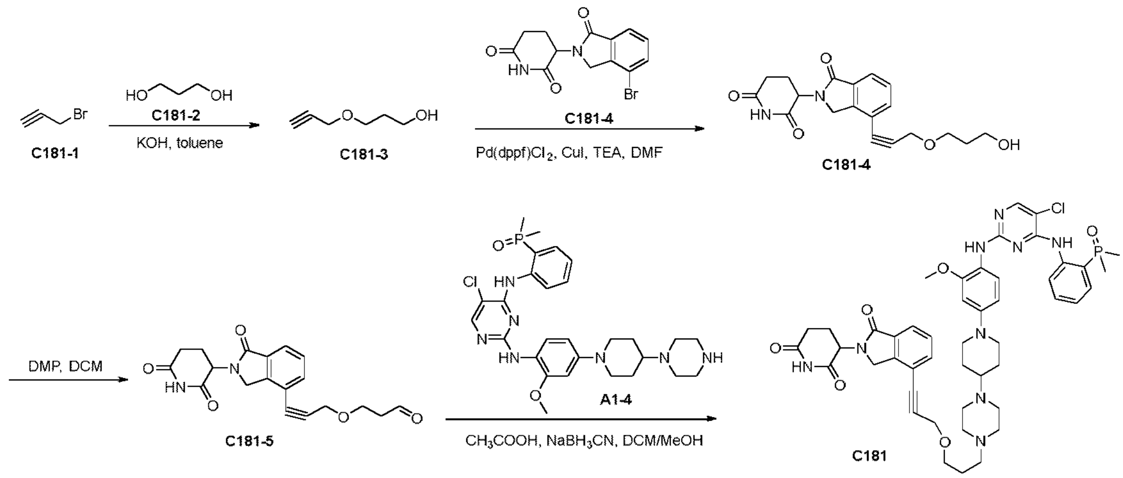


Step 1:

At 0°C, potassium hydroxide (3.75g, 66.8mmol) was added portionwise to a solution of a mixture of compound **C181-1** (3.68g, 25.0mmol) and compound **C181-2** (1.99g, 26.2mmol) in toluene. The mixture was stirred vigorously, then warmed to room temperature, and further stirred for 1h. The mixture was diluted with water, and extracted with 100 mL of ethyl acetate. The organic phase was dried with anhydrous sodium sulfate, and filtered. The solvent was removed under reduced pressure, and the crude product was separated by column chromatography to afford 1.2 g of liquid. Yield: 42.9%.

Step 2:

Compound **C181-4** (or **C1-1**) (200mg, 0.62mmol), compound **C181-3** (566mg, 4.97mmol), cuprous iodide (24mg, 0.12mmol), Pd(dppf)$Cl_2$ (174mg, 0.25mmol) and triethylamine (188mg, 1.86mmol) were dissolved in 5 mL of anhydrous DMF. The mixture was reacted with stirring at 70°C under nitrogen protection for 5h. The solvent was removed under reduced pressure, and the crude product was purified by thin layer chromatography to afford off-white solid compound **C181-4** (60mg, yield: 27.1%). LCMS: $[M + H]^+$ = 357.

Step 3:

Compound **C181-4** (20mg, 0.056mmol) and Dess-Martin reagent (36mg, 0.084 mmol) were dissolved in 20 mL of dichloromethane and stirred at 50°C for 2h. The mixture was cooled to room temperature, and quenched with saturated sodium bicarbonate aqueous solution. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C181-5** (18 mg, yield: 90.0%). LCMS: $[M + H]^+$ = 355.

Step 4:

Compound **C181-5** (18mg, 0.051mmol) and compound **A1-4** (32mg, 0.056mmol) were dissolved in 3 mL of dichloromethane/methanol (2/1, v/v), and 1 drop of glacial acetic acid was added dropwise. The mixture was stirred at room temperature under nitrogen protection for 1h. Then sodium cyanobohydride (3.2mg, 0.051mmol) was added. The mixture was further stirred for 1h. The solvent was removed under reduced pressure below 40°C. The crude product was purified by Flash chromatography to afford off-white solid compound **C181** (15mg, yield: 32.6%). LCMS: $[M + H]^+$ = 908. $^1H$ NMR (400 MHz, DMSO-$d_6$) $\delta$ 11.15 (s, 1H), 11.00 (s, 1H), 8.50(dr, 1H), 8.04 (d, 2H), 7.71 (dd, 1H), 7.63 (d, 1H),7.57-7.44 (m, 2H), 7.36(m, 2H), 7.06 (d, 1H), 6.64 (s, 1H), 6.43 (d, 1H), 5.29 (t, 1H), 5.13 (dd, 1H), 4.46 (d, 1H),

4.38 (s, 2H), 4.31 (d, 1H), 3.72 (s, 3H), 3.67 (d, 2H), 3.53 (t, 2H), 2.98(m,1H), 2.67 (m,4H),2.59 (m, 3H), 2.25 (m, 4H), 2.02 (m, 2H), 1.96 (q, 2H), 1.69 (s, 3H), 1.68 (s, 3H), 1.45 (s, 2H), 1.21 (d, 4H).

**Preparation of 3-(4-(3-(4-(4-(1-(4-((5-chloro-4-((2-(dimethylphosphoryl)phenyl)amino)pyrimidin-2-yl)amino-3-methoxyphenyl)piperidin-4-yl)piperazin-1-yl)butoxy)prop-1-yn-1-yl)-1-oxoisoindolin-2-yl)piperidine-2,6-dione (C182)**

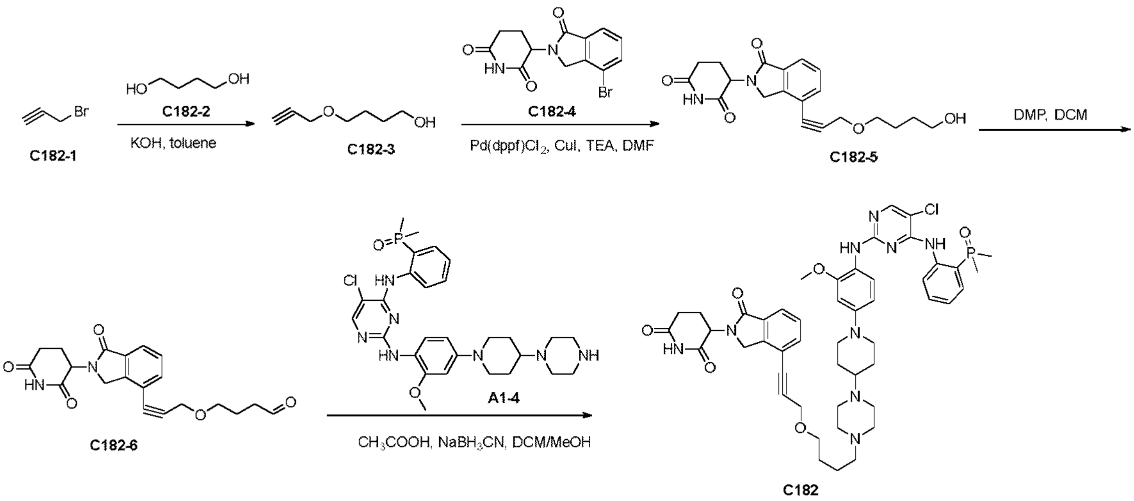


Step 1:

At 0°C, potassium hydroxide (3.75g, 66.8mmol) was added portionwise to a solution of a mixture of compound **C182-1** (3.68g, 25.0mmol) and compound **C182-2** (2.36g, 26.2mmol) in toluene. The mixture was stirred vigorously, then warmed to room temperature, and further stirred for 1h. The mixture was diluted with water, and extracted with 100 mL of ethyl acetate. The organic phase was dried with anhydrous sodium sulfate, and filtered. The solvent was removed under reduced pressure, and the crude product was separated by column chromatography to afford 1.4 g of liquid **C182-3.** Yield: 45.2%.

Step 2:

Compound **C182-4** (or **C1-1**) (200mg, 0.62mmol), compound **C182-3** (6366mg, 4.97mmol), cuprous iodide (24mg, 0.12mmol), Pd(dppf)$Cl_2$ (174mg, 0.25mmol) and triethylamine (188mg, 1.86mmol) were dissolved in 5 mL of anhydrous DMF. The mixture was reacted with stirring at 70°C under nitrogen protection for 5h. The solvent was removed under reduced pressure, and the crude product was purified by thin layer chromatography to afford off-white solid compound **C182-5** (60mg, yield: 26.1%). LCMS: $[M + H]^+$ = 371.

Step 3:

Compound **C182-5** (30mg, 0.081mmol) and Dess-Martin reagent (52mg, 0.1224 mmol) were dissolved in 20 mL of dichloromethane and stirred at 50°C for 2h. The mixture was cooled to room temperature, and quenched with saturated sodium bicarbonate aqueous solution. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C182-6** (28 mg, yield: 93.3%). LCMS: $[M + H]^+$ = 369.

Step 4:

Compound **C182-6** (20mg, 0.051mmol) and compound **A1-4** (32mg, 0.056mmol) were dissolved in 3 mL of dichloromethane/methanol (2/1), and 1 drop of glacial acetic acid was added dropwise. The mixture was stirred at room temperature under nitrogen protection for 1h. Then sodium cyanobohydride (3.2mg, 0.051mmol) was added. The mixture was further stirred for 1h. The solvent was removed under reduced pressure below 40°C. The crude product was purified

by Flash chromatography to afford off-white solid compound **C182** (10mg, yield: 20.8%). LCMS: $[M + H]^+$ = 948. $^1H$ NMR (400 MHz, DMSO-$d_6$) δ 11.18 (s, 1H), 11.05 (s, 1H), 8.50(dr, 1H), 8.08 (d, 2H), 7.78 (dd, 1H), 7.72(d, 1H),7.56-7.44 (m, 2H), 7.36(m, 2H), 7.09 (d, 1H), 6.62 (s, 1H), 6.48 (d, 1H), 5.32 (t, 1H), 5.15 (dd, 1H), 4.53 (d, 1H), 4.37 (s, 2H), 4.32 (d, 1H), 3.75 (s, 3H), 3.69 (d, 2H), 3.54 (t, 2H), 2.97(m,1H), 2.66 (m,4H),2.56 (m, 3H), 2.49 (m, 4H), 2.02 (m, 2H), 1.96 (q, 2H), 1.69 (s, 3H), 1.68 (s, 3H), 1.45 (s, 3H), 1.23(d, 5H).

**Preparation of 3-(4-(9-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)non-1-yn-1-yl)-1-oxoisoindolin-2-yl)piperidine-2,6-dione (C183)**

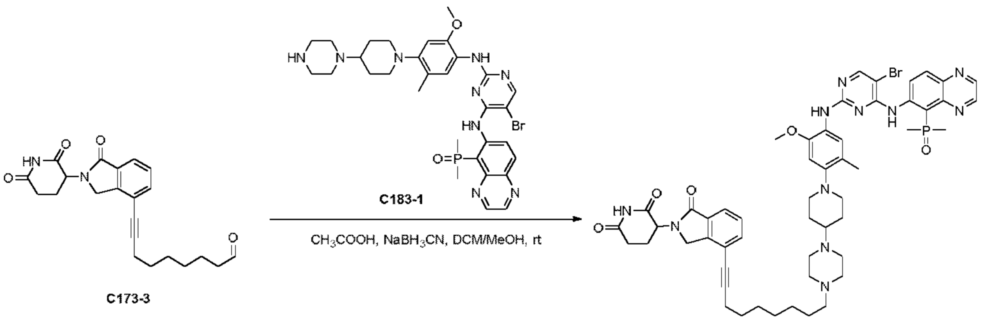


C173-3

C183

**C173-3** (30 mg, 0.079 mmol) and **C183-1** (or **C158-1**) (48.2 mg, 0.071 mmol) were dissolved in a mixture of 4.0 mL of anhydrous DCM and 0.4 mL of anhydrous methanol. $CH_3COOH$ (4.74 mg, 0.079 mmol, dissolved in 0.5 mL of DCM) was added under nitrogen protection. The mixture was stirred at room temperature for 0.5h. Then solid $NaBH_3CN$ (7.44 mg, 0.1185 mmol) was added. The mixture was further reacted at room temperature for 2h. LCMS and TLC showed that the reaction was completed. The mixture was diluted with 20 mL of DCM, and then washed successively with saturated $NH_4Cl$ (1 × 15 mL) and saturated brine (1 × 15 mL). The organic layer was dried with anhydrous $Na_2SO_4$, and filtered with suction. The filtrate was concentrated under reduced pressure to afford a crude product, which was purified by Prep-HPLC to afford 25 mg of yellow solid pure product. Yield: 30.4%. LCMS: $[M + H]^+$ = 1044, 1046. $^1H$-NMR: (400 MHz, DMSO-$d_6$) δ 12.696 (s, 1H), 11.089 (s, 1H), 8.876 (dd, *J* = 2.0 Hz, 7.6 Hz, 3H), 8.272 (d, *J*= 1.6Hz, 2H), 7.722 (d, *J* = 7.6 Hz, 1H), 7.646 (d, *J* = 6.8 Hz, 1H), 7.629 (t, *J* = 0.8 Hz, 1H), 7.347 (s, 1H), 6.748 (s, 1H), 5.187 (dd, *J* = 5.2 Hz, 13.2 Hz, 1H), 4.478 (d, *J* = 17.6 Hz, 1H), 4.325 (d, *J* = 17.6 Hz, 1H), 3.775 (s, 3H), 3.345-2.831 (m, 4H), 2.522-2.469 (m, 4H), 2.468-2.185 (m, 9H), 2.087 (s, 3H), 2.043 (s, 3H), 2.007 (s, 3H), 1.574-1.560 (m, 2H), 1.432-1.230 (m, 16H).

**Preparation of 3-(4-(3-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)ethoxy)prop-1-yn-1-yl)-6-fluoro-1-oxoisoindolin-2-yl)piperidine-2,6-dione (C184)**

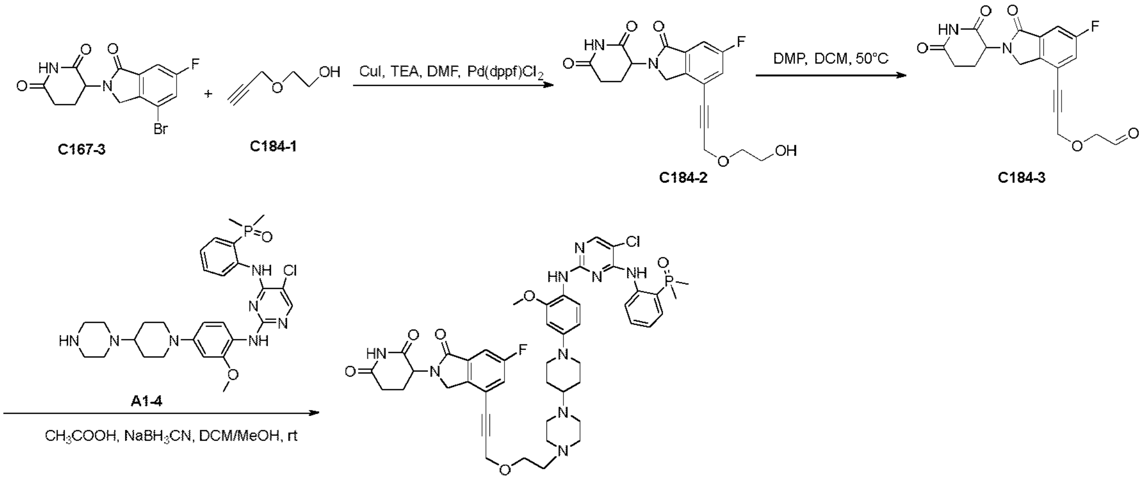

C167-3 C184-1 C184-2 C184-3 A1-4 C184

Step 1:

**C167-3** (340 mg, 1.0 mmol), CuI (19.0 mg, 0.10 mmol) and Pd(dppf)$Cl_2$ (73.1 mg, 0.10 mmol) were dispersed in 17.0 mL of anhydrous DMF. **C184-1** (200.2 mg, 2.0 mmol) and TEA (303 mg, 3.0 mmol) were added successively under $N_2$ protection. The mixture was heated to 70°C and reacted for 20 h. LCMS showed that the reaction was completed. The mixture was cooled to room temperature, diluted with 100 mL of ethyl acetate, and then washed successively with saturated $NH_4Cl$ (2 × 50 mL), $H_2O$ (2 × 50 mL) and saturated brine (2 × 50 mL). The organic layer was dried with anhydrous sodium sulfate, and filtered with suction. The filtrate was concentrated under reduced pressure to afford a crude product, which was purified by RP-Flash chromatography to afford white solid product **C184-2** (175 mg, 48.6%). LCMS: $[M + H]^+$ = 361.

Step 2:

**C184-2** (70 mg, 0.195 mmol) was dissolved in 14.0 mL of anhydrous DCM, and Dess-Martin reagent (124 mg, 0.293 mmol) was added under $N_2$ protection. The mixture was heated to 50°C, and reacted under reflux for 2.0 h. TLC showed that the reaction was completed. The mixture was cooled to room temperature, and then 10 mL of saturated $NaHCO_3$ solution and 10 mL of saturated $Na_2S_2O_3$ solution were added. The mixture was stirred vigorously at room temperature for 5 min. The organic layer was separated, dried with anhydrous sodium sulfate, and filtered with suction. The filtrate was concentrated under reduced pressure to afford a crude product. The crude product was purified by Prep-TLC to afford light-yellow solid product **C184-3** (40 mg, 57.2%). LCMS: $[M + H]^+$ = 359.

Step 3:

**C184-3** (40 mg, 0.112 mmol) and **A1-4** (51 mg, 0.0896 mmol) were dissolved in a mixture of 5.0 mL of anhydrous DCM and 0.5 mL of anhydrous methanol. $CH_3COOH$ (10.08 mg, 0.168 mmol) was added under nitrogen protection. The mixture was stirred at room temperature for 0.5h. Then solid $NaBH_3CN$ (14.1 mg, 0.224 mmol) was added. The mixture was further reacted at room temperature for 2h. LCMS and TLC showed that the reaction was completed. The mixture was purified by Prep-HPLC to afford white solid pure product **C184** (20 mg, yield: 19.6%). LCMS: $[M + H]^+$ = 912.

**Preparation of 3-(4-(7-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)hept-1-yn-1-yl)-6-fluoro-1-oxoisoindolin-2-yl)piperidine-2,6-dione (C185)**

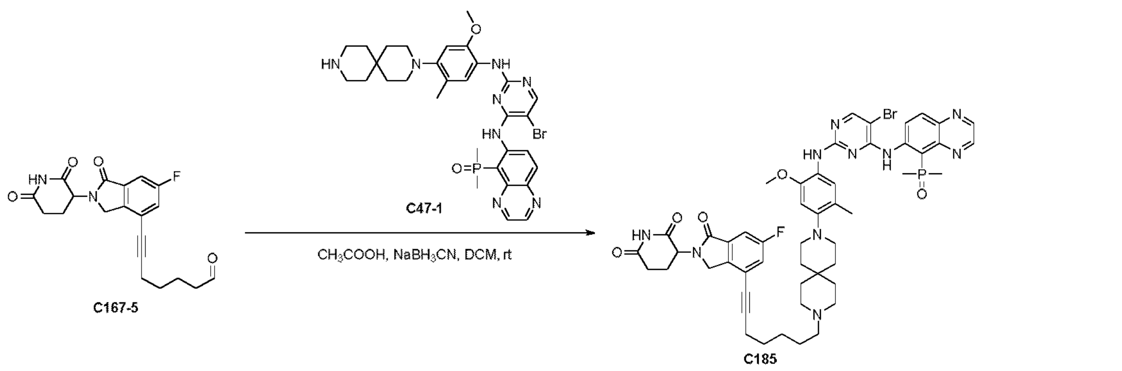


Step 1:

**C167-5** (18.0 mg, 0.0486 mmol) and **C47-1** (32.3 mg, 0.0486 mmol) were dissolved in 4.0 mL of anhydrous DCM. $CH_3COOH$ (2.9 mg, 0.0486 mmol, dissolved in 0.5 mL of DCM) was added under nitrogen protection. The mixture was stirred at room temperature for 0.5h. Then solid $NaBH_3CN$ (6.1 mg, 0.0972 mmol) was added. The mixture was further reacted at room temperature for 2h. LCMS and TLC showed that the reaction was completed. The mixture was diluted with 20 mL of DCM, and washed successively with saturated $NH_4Cl$ (1 × 15 mL) and saturated brine (1 × 15 mL). The organic layer was dried with anhydrous $Na_2SO_4$, and filtered with suction. The filtrate was concentrated under reduced pressure to afford a crude product, which was purified by Prep-HPLC to afford 13 mg of yellow solid pure product. Yield: 26.3%. LCMS: $[M + H]^+$ = 1019, 1021. $^1$H-NMR: (400 MHz, DMSO-$d_6$) δ 11.688 (s, 1H), 11.051 (s, 1H), 8.877 (dd, *J* = 2.0 Hz, 11.2 Hz, 3H), 8.307 (s, 1H), 8.272 (d, *J* = 2.0 Hz, 1H), 7.936 (d, *J* = 7.6 Hz, 1H), 7.586-7.541 (m, 2H), 7.328 (s, 1H), 6.820 (s, 1H), 5.191 (dd, *J* = 5.2 Hz, 13.2 Hz, 1H), 4.474 (dd, *J* = 18.8 Hz, 60.0 Hz, 2H), 3.787 (s, 3H), 3.505 (s, 1H), 3.347 (s, 3H), 2.942-2.619 (m, 6H), 2.570 (s, 4H), 2.081-2.004 (m, 9H), 1.639-1.432 (m, 11H), 1.232 (s, 5H).

**Preparation of 3-(4-(6-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)hex-1-yn-1-yl)-1-oxoisoindolin-2-yl)piperidine-2,6-dione (C186)**

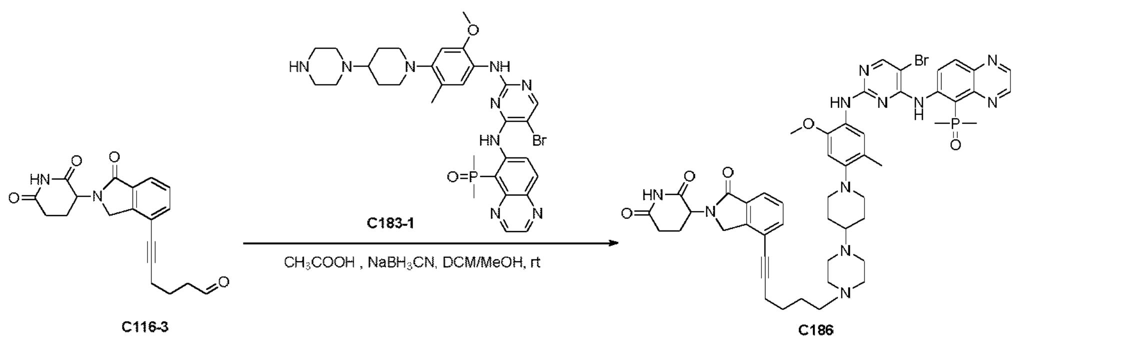


**C116-3** (25 mg, 0.0740 mmol) and **C183-1** (or **C158-1**) (50.2 mg, 0.0740 mmol) were dissolved in a mixture of 4.0 mL of anhydrous DCM and 0.4 mL of anhydrous methanol. $CH_3COOH$ (4.44 mg, 0.0740 mmol, dissolved in 0.5 DCM) was added under nitrogen protection. The mixture was stirred at room temperature for 0.5h. Then solid $NaBH_3CN$ (6.97 mg, 0.1110 mmol) was added. The mixture was further reacted at room temperature for 2h. LCMS and TLC showed that the reaction was completed. The mixture was purified by Prep-HPLC to afford yellow solid product **C186,** 23 mg, yield: 31.0%. LCMS: $[M + H]^+$ = 1002, 1004. $^1$H-NMR: (400 MHz, DMSO-$d_6$) δ 11.027 (s, 1H), 8.874-8.850 (m, 4H), 8.271-8.255 (m, 2H), 7.937 (d, *J* = 9.6 Hz, 1H), 7.728 (d, *J* = 1.2 Hz, 1H), 7.709 (d, *J* = 1.2 Hz, 1H), 7.655-7.634 (m, 1H), 7.357 (s, 1H), 6.752 (s, 1H), 5.181 (dd, *J* = 4.0 Hz, 13.6 Hz, 1H), 4.484 (d, *J* = 17.6 Hz, 1H), 4.335 (d, *J* = 17.6 Hz, 1H), 3.776 (s, 3H), 3.328-3.107 (m, 2H), 2.967-2.892 (m, 1H), 2.682-2.334 (m, 13H), 2.091-2.007 (m, 12H), 1.884-1.855 (m, 2H), 1.600-1.567 (m, 6H), 1.236 (s, 2H).

**Preparation of 3-(4-(6-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)hex-1-yn-1-yl)-1-oxoisoindolin-2-yl)piperidine-2,6-dione (C187)**

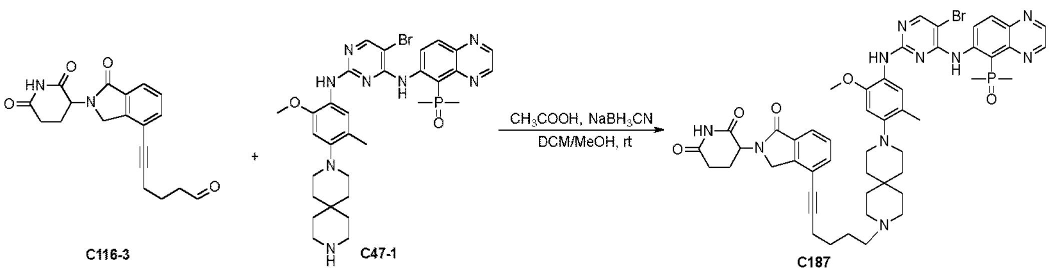


C116-3 C47-1 C187

**C116-3** (25 mg, 0.0740 mmol) and **C47-1** (49.1 mg, 0.0740 mmol) were dissolved in a mixture of 4.0 mL of anhydrous DCM and 0.4 mL of anhydrous methanol. $CH_3COOH$ (4.44 mg, 0.0740 mmol, dissolved in 0.5 mL of DCM) was added under nitrogen protection. The mixture was stirred at room temperature for 0.5h. Then solid $NaBH_3CN$ (6.97 mg, 0.1110 mmol) was added. The mixture was further reacted at room temperature for 2h. LCMS and TLC showed that the reaction was completed. The mixture was purified by Prep-HPLC to afford yellow solid pure product **C187,** 30 mg, yield: 41.1%. LCMS: $[M + H]^+$ = 987, 989. $^1$H-NMR:(400 MHz, DMSO-$d_6$) $\delta$ 11.026 (s, 1H), 8.864 (dd, *J* = 2.0 Hz, 9.6 Hz, 4H), 8.265 (s, 2H), 7.940 (d, *J* = 11.2 Hz, 1H), 7.723 (dd, *J* = 1.2 Hz, 7.6 Hz, 1H), 7.653 (dd, *J* = 1.2 Hz, 7.6 Hz, 1H), 7.544 (t, *J* = 7.6 Hz, 1H), 7.327 (s, 1H), 6.815 (s, 1H), 5.181-5.135 (m, 1H), 4.483 (d, *J* = 16.8 Hz, 1H), 4.334 (d, *J* = 17.2 Hz, 1H), 3.783 (s, 3H), 2.933-2.821 (m, 1H), 2.807-2.797 (m, 4H), 2.621-2.577 (m, 3H), 2.503-2.335 (m, 6H), 2.081 (s, 3H), 2.040 (s, 4H), 2.004 (s, 4H), 1.600-1.528 (m, 12H).

**Preparation of 3-(4-(10-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)dec-1-yn-1-yl)-1-oxoisoindolin-2-yl)piperidine-2,6-dione (C188)**

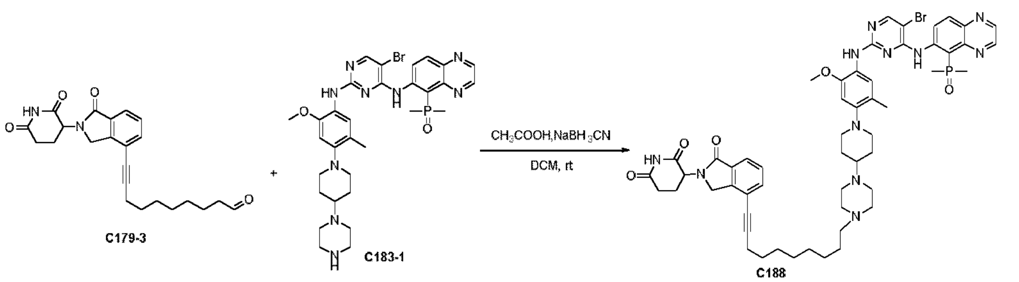


C179-3 C183-1 C188

**C179-3** (30 mg, 0.076 mmol) and **C183-1** (or **C158-1**) (43.9 mg, 0.0647 mmol) were dissolved in 8.0 mL of anhydrous DCM. $CH_3COOH$ (4.56 mg, 0.076 mmol, dissolved in 0.5 mL of DCM) was added under nitrogen protection. The mixture was stirred at room temperature for 0.5h. Then solid $NaBH_3CN$ (7.16 mg, 0.114 mmol) was added. The mixture was further reacted at room temperature for 2h. LCMS and TLC showed that the reaction was completed. The mixture was directly diluted with 20 mL of DCM, and washed successively with saturated $NH_4Cl$ (1 $\times$ 10 mL) and saturated brine (1 $\times$ 10 mL). The organic layer was dried with anhydrous sodium sulfate, and filtered with suction. The filtrate was concentrated under reduced pressure to afford a crude product, which was purified by Prep-HPLC to afford yellow solid pure product **C188** (19.5 mg, yield: 24.4%). LCMS: $[M + H]^+$ = 1058, 1060.

**Preparation of 3-(4-(10-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)dec-1-yn-1-yl)-1-oxoisoindolin-2-yl)piperidine-2,6-dione (C189)**

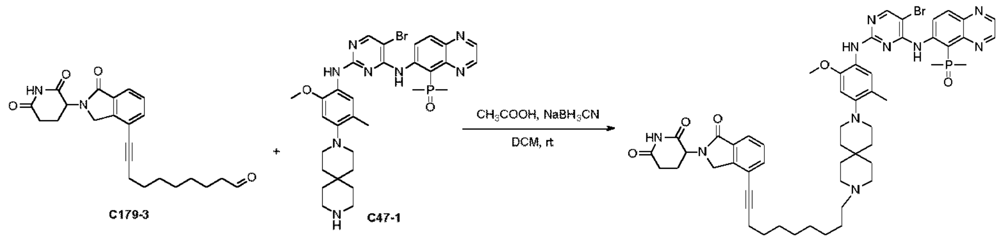

C179-3 C47-1

C189

**C179-3** (30 mg, 0.076 mmol) and **C47-1** (40.4 mg, 0.0608 mmol) were dissolved in 5.0 mL of anhydrous DCM. $CH_3COOH$ (4.56 mg, 0.076 mmol, dissolved in 0.5 mL of DCM) was added under nitrogen protection. The mixture was stirred at room temperature for 0.5h. Then solid $NaBH_3CN$ (7.16 mg, 0.114 mmol) was added. The mixture was further reacted at room temperature for 2h. LCMS and TLC showed that the reaction was completed. The mixture was purified by Prep-HPLC to afford yellow solid pure product **C189** (25 mg, yield: 31.5%). LCMS: $[M + H]^+$ = 1043, 1045.

**Preparation of 3-(5-(7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hept-1-yn-1-yl)-1-oxoisoindolin-2-yl)piperidine-2,6-dione (C190)**

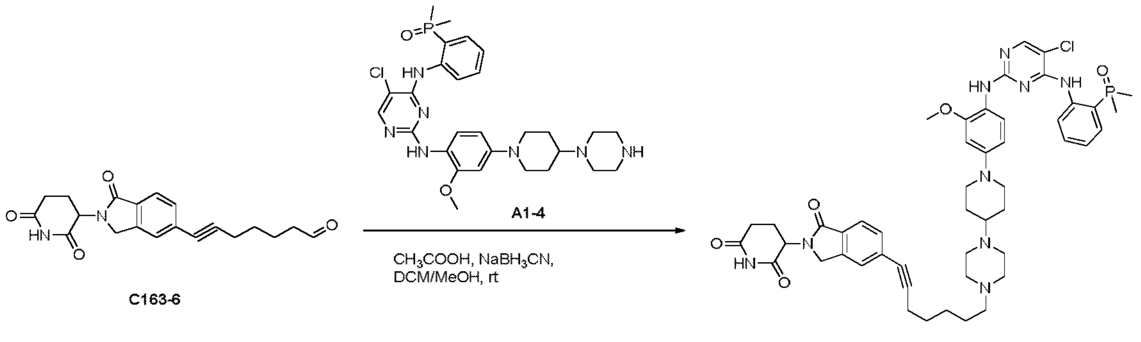

A1-4

C163-6

C190

Compound **C163-6** (35mg, 0.099mmol) and compound **A1-4** (62mg, 0.109mmol) were dissolved in 3 mL of dichloromethane/methanol (2/1), and 1 drop of glacial acetic acid was added dropwise. The mixture was stirred at room temperature under nitrogen protection for 1h. Then sodium cyanobohydride (6.2mg, 0.099mmol) was added. The mixture was further stirred for 1h. The solvent was removed under reduced pressure below 40°C, and the crude product was purified by Flash chromatography to afford off-white solid compound **C190** (15mg, yield: 16.7%). LCMS: $[M + H]^+$ = 906.

**Preparation of 3-(5-(7-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)hept-1-yn-1-yl)-1-oxoisoindolin-2-yl)piperidine-2,6-dione (C191)**

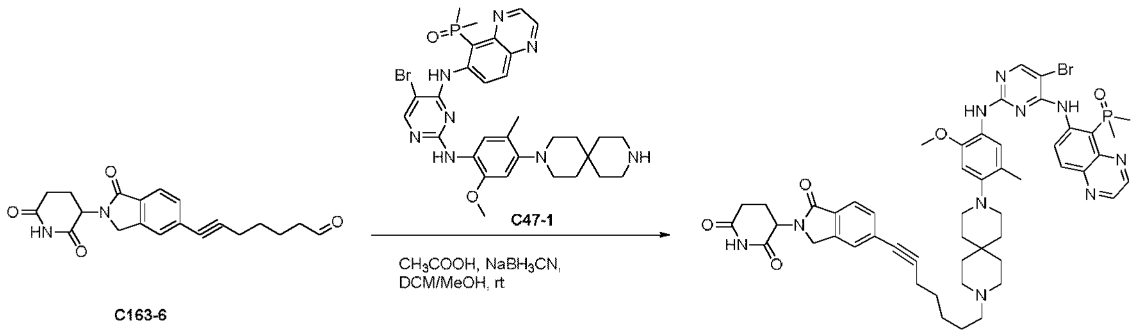

C47-1

C163-6

C191

Compound **C163-6** (35mg, 0.099mmol) and compound **C47-1** (73mg, 0.109mmol) were dissolved in 3 mL of dichloromethane/methanol (2/1), and 1 drop of glacial acetic acid was added dropwise. The mixture was stirred at room temperature under nitrogen protection for 1h. Then sodium cyanobohydride (6.2mg, 0.099mmol) was added. The mixture was further stirred for 1h. The solvent was removed under reduced pressure below 40°C. The crude product was purified by Flash chromatography to afford yellow solid compound **C191** (20mg, yield: 20.2%). LCMS: $[M + H]^+$ = 1001, 1003.

**Preparation of 3-(5-(6-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hex-1-yn-1-yl)-1-oxoisoindolin-2-yl)piperidine-2,6-dione (C192)**

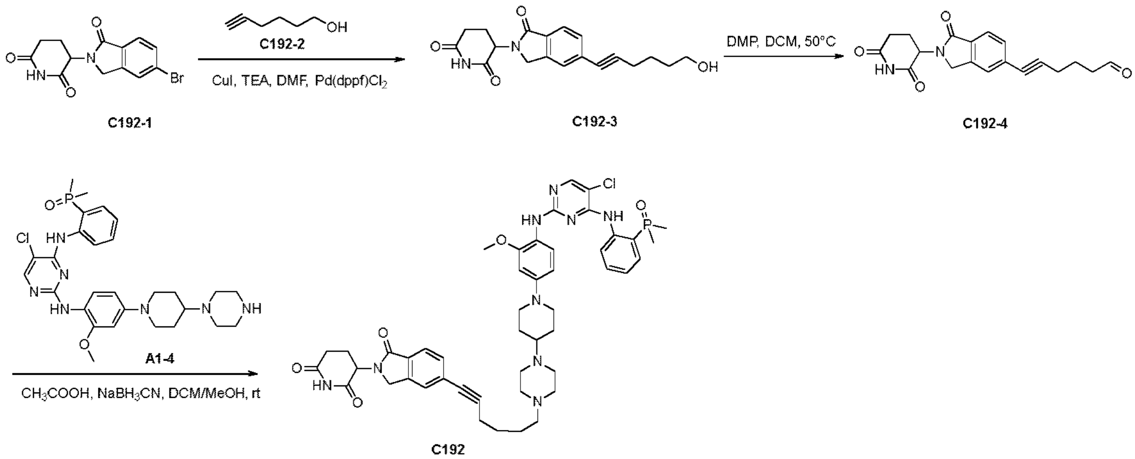


Step 1:

Compound **C192-1** (100mg, 0.31mmol), compound **C192-2** (76mg,0.78mmol), cuprous iodide (12mg, 0.06mmol), $Pd(dppf)Cl_2$ (87mg, 0.12mmol) and triethylamine (94mg, 0.93mmol) were dissolved in 4 mL of anhydrous DMF. The mixture was reacted with stirring at 70°C under nitrogen protection for 5h. The solvent was removed under reduced pressure. The crude product was purified by thin layer chromatography to afford off-white solid compound **C192-3** (78mg, yield: 72.2%). LCMS: $[M + H]^+$ = 341.

Step 2:

Compound **C192-3** (39mg, 0.115mmol) and Dess-Martin reagent (73mg, 0.172 mmol) were dissolved in 15 mL of dichloromethane and stirred at 50°C for 2h. The mixture was cooled to room temperature, and quenched with saturated sodium bicarbonate aqueous solution. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C192-4** (30 mg, yield: 77.5%). LCMS: $[M + H]^+$ = 339.

Step 3:

Compound **C192-4** (26.6mg, 0.079mmol) and compound **A1-4** (49mg, 0.087mmol) were dissolved in 3 mL of dichloromethane/methanol (2/1), and 1 drop of glacial acetic acid was added dropwise. The mixture was stirred under nitrogen protection at room temperature for 1h. Then sodium cyanobohydride (5mg, 0.079mmol) was added. The mixture was further stirred for 1h. The solvent was removed under reduced pressure below 40°C, and the crude product was purified by Flash chromatography to afford off-white solid compound **C192** (10mg, yield: 14.3%). LCMS: $[M + H]^+$ = 892.

**Preparation of 3-(5-(3-(2-(4-(1-(4-((5-chloro-4-((2-((dimethylphosphoryl)phenyl)amino)pyrimidin-2-yl)amino)-3-methoxyphenyl)piperidin-4-yl)piperazin-1-yl)ethoxy)prop-1-yn-1-yl)-1-oxoisoindolin-2-yl)piperidine-2,6-dione (C193)**

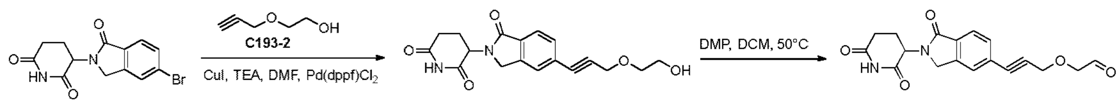


C193-1 C193-3 C193-4

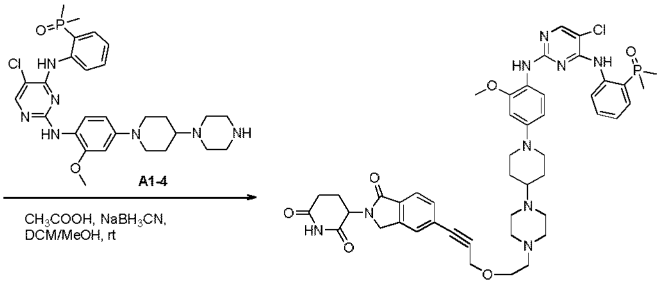


C193

Step 1:

Compound **C193-1** (or **C192-1**) (100mg, 0.31mmol), compound **C193-2** (78mg, 0.78mmol), cuprous iodide (12mg, 0.06mmol), Pd(dppf)$Cl_2$ (87mg, 0.12mmol) and triethylamine (94mg, 0.93mmol) were dissolved in 4 mL of anhydrous DMF. The mixture was reacted with stirring at 70°C under nitrogen protection for 5h. The solvent was removed under reduced pressure, and the crude product was purified by thin layer chromatography to afford off-white solid compound **C193-3** (70mg, yield: 66.0%). LCMS: $[M + H]^+$ = 343.

Step 2:

Compound **C193-3** (23.2mg, 0.068mmol) and Dess-Martin reagent (43.1mg, 0.102 mmol) were dissolved in 15 mL of dichloromethane and stirred at 50°C for 2h. The mixture was cooled to room temperature, and quenched with saturated sodium bicarbonate aqueous solution. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C193-4** (20 mg, yield: 87.0%). LCMS: $[M + H]^+$ = 341.

Step 3:

Compound **C193-4** (20mg, 0.059mmol) and compound **A1-4** (37mg, 0.065mmol) were dissolved in 3 mL of dichloromethane/methanol (2/1), and 1 drop of glacial acetic acid was added dropwise. The mixture was stirred at room temperature under nitrogen protection for 1h. Then sodium cyanobohydride (3.7mg, 0.059mmol) was added. The mixture was further stirred for 1h. The solvent was removed under reduced pressure below 40°C. The crude product was purified by Flash chromatography to afford off-white solid compound **C193** (15mg, yield: 28.8%). LCMS: $[M + H]^+$ = 894.

**Preparation of 3-(5-(8-(4-(1-(4-((5-chloro-4-((2-(dimethylphosphoryl)phenyl)amino)pyrimidin-2-yl)amino)-3-methoxyphenyl)piperidin-4-yl)piperazin-1-yl)oct-1-yn-1-yl)-1-oxoisoindolin-2-yl)piperidine-2,6-dione (C194)**

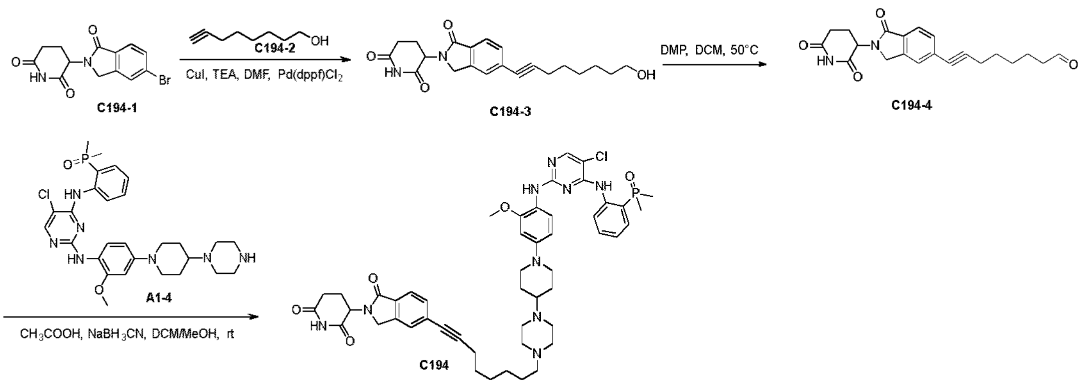


Step 1:

Compound **C194-1** (or **C192-1**) (100mg, 0.31mmol), compound **C194-2** (98mg, 0.78mmol), cuprous iodide (12mg, 0.06mmol), Pd(dppf)$Cl_2$ (87mg, 0.12mmol) and triethylamine (94mg, 0.93mmol) were dissolved in 4 mL of anhydrous DMF. The mixture was reacted with stirring at 70°C under nitrogen protection for 5h. The solvent was removed under reduced pressure. The crude product was purified by thin layer chromatography to afford off-white solid compound **C194-3** (68mg, yield: 59.6%). LCMS: $[M + H]^+$ = 369.

Step 2:

Compound **C194-3** (27mg, 0.073mmol) and Dess-Martin reagent (46.7mg, 0.110 mmol) were dissolved in 16 mL of dichloromethane and stirred at 50°C for 2h. The mixture was cooled to room temperature, and quenched with saturated sodium bicarbonate aqueous solution. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C194-4** (20.6 mg, yield: 76.6%). LCMS: $[M + H]^+$ = 367.

Step 3:

Compound **C194-4** (20.6mg, 0.056mmol) and compound **A1-4** (35mg, 0.062mmol) were dissolved in 3 mL of dichloromethane/methanol (2/1), and 1 drop of glacial acetic acid was added dropwise. The mixture was stirred at room temperature under nitrogen protection for 1h. Then sodium cyanobohydride (3.5mg, 0.056mmol) was added. The mixture was further stirred for 1h. The solvent was removed under reduced pressure below 40°C, and the crude product was purified by Flash chromatography to afford off-white solid compound **C194** (15mg, yield: 29.0%). LCMS: $[M + H]^+$ = 920.

**Preparation of 3-(5-(9-(4-(1-(4-((5-chloro-4-((2-(dimethylphosphoryl)phenyl)amino)pyrimidin-2-yl)amino)-3-methoxyphenyl)piperidin-4-yl)piperazin-1-yl)non-1-yn-1-yl)-1-oxoisoindolin-2-yl)piperidine-2,6-dione (C195)**

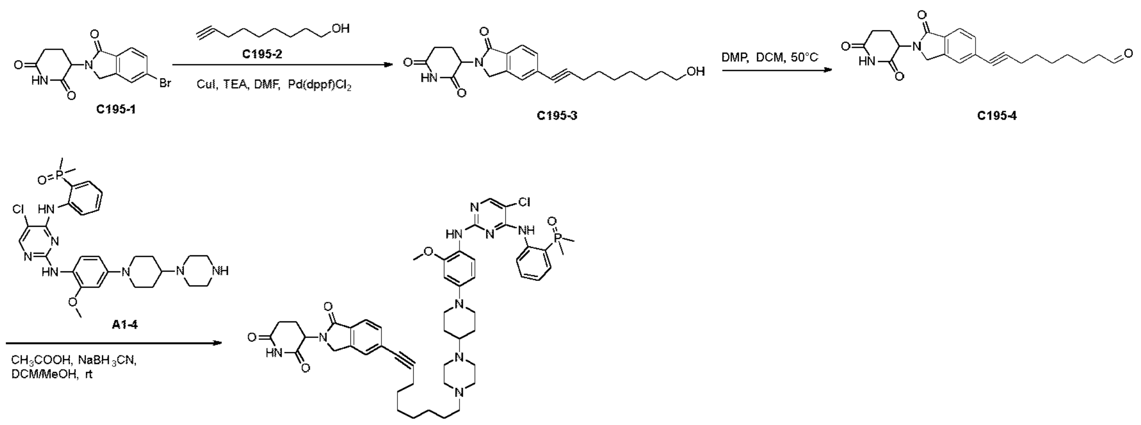


C195-1 C195-3 C195-4

C195

Step 1:

Compound **C195-1** (or **C192-1**) (100mg, 0.31mmol), compound **C195-2** (109mg, 0.78mmol), cuprous iodide (12mg, 0.06mmol), Pd(dppf)$Cl_2$ (87mg, 0.12mmol) and triethylamine (94mg, 0.93mmol) were dissolved in 4 mL of anhydrous DMF. The mixture was reacted with stirring at 70°C under nitrogen protection for 5h. The solvent was removed under reduced pressure, and the crude product was purified by thin layer chromatography to afford off-white solid compound **C195-3** (72mg, yield: 61.0%). LCMS: $[M + H]^+$ = 383.

Step 2:

Compound **C195-3** (23mg, 0.060mmol) and Dess-Martin reagent (38.3mg, 0.090 mmol) were dissolved in 16 mL of dichloromethane and stirred at 50°C for 2h. The mixture was cooled to room temperature, and quenched with saturated sodium bicarbonate aqueous solution. The organic phase was separated, and the aqueous phase was extracted with dichloromethane for three times. The organic phases were combined, dried with anhydrous sodium sulfate, and filtered. The filtrate was concentrated, and the crude product was purified by TLC to afford white solid compound **C195-4** (20 mg, yield: 87.0%). LCMS: $[M + H]^+$ = 381.

Step 3:

Compound **C195-4** (20mg, 0.053mmol) and **A1-4** (33mg, 0.058mmol) were dissolved in 3 mL of dichloromethane/methanol (2/1), and 1 drop of glacial acetic acid was added dropwise. The mixture was stirred at room temperature under nitrogen protection for 1h. Then sodium cyanobohydride (3.5mg, 0.053mmol) was added. The mixture was further stirred for 1h. The solvent was removed under reduced pressure below 40°C, and the crude product was purified by Flash chromatography to afford off-white solid compound **C195** (15mg, yield: 30.6%). LCMS: $[M + H]^+$ = 934.

**Preparation of 3-(5-(10-(4-(1-(4-((5-chloro-4-((2-(dimethylphosphoryl)phenyl)amino)pyrimidin-2-yl)amino)-3-methoxyphenyl)piperidin-4-yl)piperazin-1-yl)dec-1-yn-1-yl)-1-oxoisoindolin-2-yl)piperidine-2,6-dione (C196)**

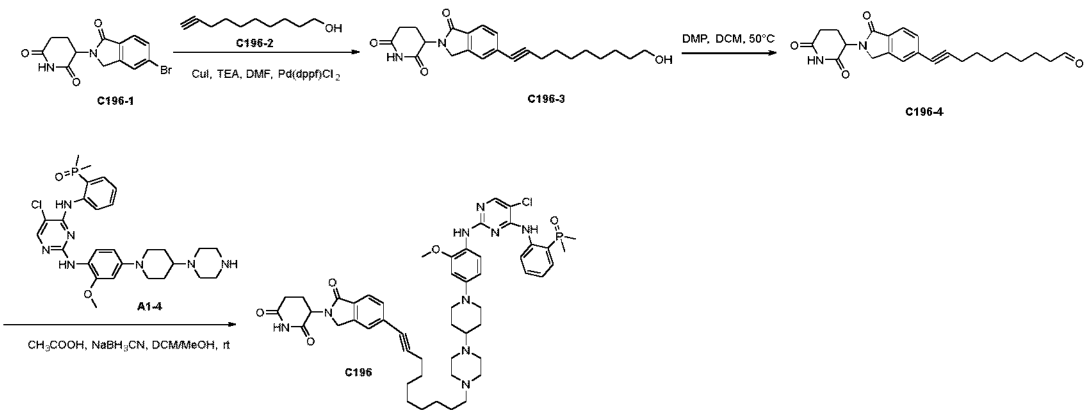


C196-1 C196-3 C196-4 C196

Step 1:

Compound **C196-1** (or **C192-1**) (100mg, 0.31mmol), compound **C196-2** (120mg, 0.78mmol), cuprous iodide (12mg, 0.06mmol), Pd(dppf)$Cl_2$ (87mg, 0.12mmol) and triethylamine (94mg, 0.93mmol) were dissolved in 4 mL of anhydrous DMF. The mixture was reacted with stirring at 70°C under nitrogen protection for 5h. The solvent was removed under reduced pressure, and the crude product was purified by thin layer chromatography to afford off-white solid compound **C196-3** (65mg, yield: 52.8%). LCMS: $[M + H]^+$ = 397.

Step 2:

Compound **C195-3** (23.9mg, 0.060mmol) and Dess-Martin reagent (38.4mg, 0.090 mmol) were dissolved in 15 mL of dichloromethane and stirred at 50°C for 2h. The mixture was cooled to room temperature, and quenched with saturated sodium bicarbonate aqueous solution. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C196-4** (20 mg, yield: 84.0%). LCMS: $[M + H]^+$ = 395.

Step 3:

Compound **C196-4** (20mg, 0.051mmol) and compound **A1-4** (32mg, 0.056mmol) were dissolved in 3 mL of dichloromethane/methanol (2/1), and 1 drop of glacial acetic acid was added dropwise. The mixture was stirred at room temperature under nitrogen protection for 1h. Then sodium cyanobohydride (3.2mg, 0.051mmol) was added. The mixture was further stirred for 1h. The solvent was removed under reduced pressure below 40°C, and the crude product was purified by Flash chromatography to afford off-white solid compound **C196** (10mg, yield: 20.8%). LCMS: $[M + H]^+$ = 948.

**Preparation of 3-(4-(3-(2-(4-(1-(4-((5-chloro-4-((2-(dimethylphosphoryl)phenyl)amino)pyrimidin-2-yl)amino)-3-methoxyphenyl)piperidin-4-yl)piperazin-1-yl)ethoxy)prop-1-yn-1-yl)-1-oxo-6-(trifluoromethyl)isoindolin-2-yl)piperidine-2,6-dione (C200)**

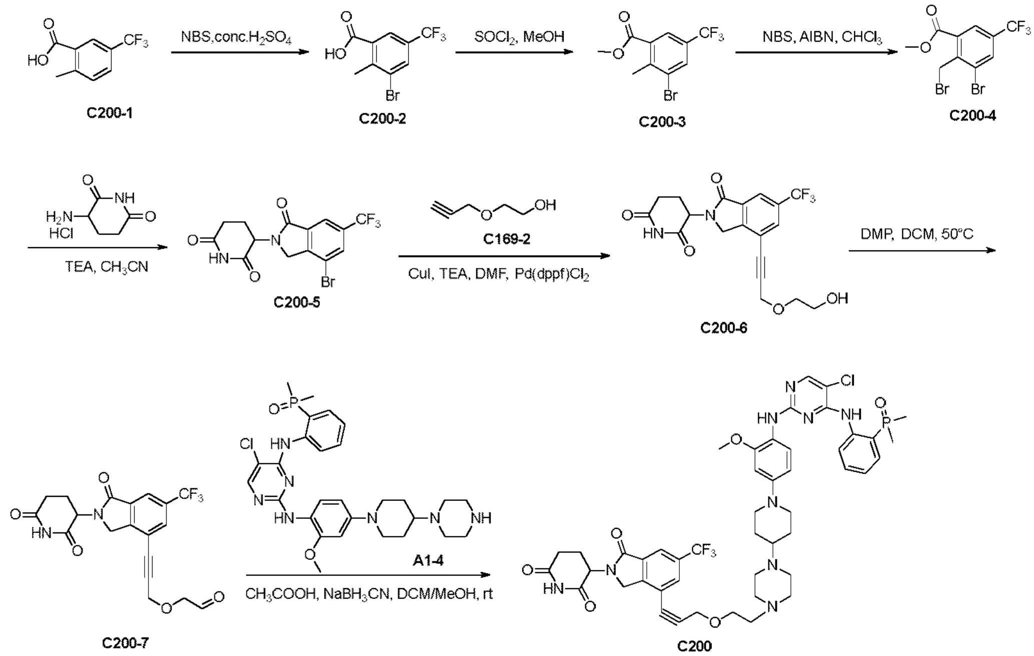


C200-1 C200-2 C200-3 C200-4 C200-5 C169-2 C200-6 C200-7 A1-4 C200

Step 1:

At 0°C, compound C200-1 (2g, 9.8mmol) and NBS (1.63g, 9.16mmol) were added portionwise with stirring to concentrated sulfuric acid (20 mL). The mixture was reacted with stirring at this temperature under nitrogen protection for 3h. The mixture was warmed to room temperature and stirred overnight. The mixture was slowly poured into ice water, and extracted with ethyl acetate for three times. The organic phases were combined, washed once with 0.5M hydrochloric acid and once with saturated brine, dried with anhydrous sodium sulfate, and filtered. The filtrate was concentrated, and the crude product was purified by Flash chromatography to afford compound C200-2 (1.3g, yield: 47.1%). LCMS: $[M + H]^+$ = 283, 285.

Step 2:

At 0°C, thionyl chloride (2 mL, 25.57mmol) was added dropwise to a solution of compound C200-2 (1.3g, 4.61mmol) in 20 mL of methanol. The mixture was heated and stirred at 70°C overnight. The mixture was cooled to room temperature, and concentrated under reduced pressure. The crude product was purified by Flash chromatography to afford compound C200-3 (1.25g, yield: 91.9%).

Step 3:

Compound **C200-3** (1.25g, 4.22mmol), NBS (857mg, 4.81mmol), and AIBN (69mg, 0.42mmol) were dissolved in 20 mL of chloroform. The mixture was refluxed at 95°C under nitrogen protection overnight. The solvent was removed under reduced pressure. The crude product was purified by Flash chromatography to afford white solid compound C200-4 (1.3g, yield: 82.3%).

Step 4:

Compound **C200-4** (1.33g, 3.56mmol), 3-aminopiperidine-2,6-dione hydrochloride (759mg, 4.62mmol), and triethylamine (467mg, 4.62mmol) were dissolved in 15 mL of acetonitrile. The mixture was stirred at 80°C under nitrogen protection overnight. The mixture was cooled to room temperature. The solvent was removed under reduced pressure, and the crude product was purified by Flash chromatography to afford white solid compound **C200-5** (790mg, yield: 56.9%). LCMS: [M + H]+ = 391, 393.

Step 5:

Compound **C200-5** (100mg, 0.256mmol), compound **C169-2** (64mg, 0.641mmol), cuprous iodide (4.9mg, 0.026mmol), $Pd(dppf)Cl_2$ (18.8mg, 0.026mmol) and triethylamine (77.7mg, 0.769mmol) were dissolved in 3 mL of anhydrous DMF. The mixture was stirred at 70°C under nitrogen protection overnight. The solvent was removed under reduced pressure, and the crude product was purified by thin layer chromatography to afford compound **C200-6** (100mg, yield: 95.2%). LCMS: $[M + H]^+$ = 411.

Step 6:

Compound **C200-6** (126mg, 0.307mmol) and Dess-Martin reagent (261mg, 0.615mmol) were dissolved in 10 mL of dichloromethane and stirred at 50°C for 2h. The mixture was cooled to room temperature, and saturated sodium bicarbonate aqueous solution and saturated sodium thiosulfate aqueous solution were added successively and stirred for 5 minutes respectively to quench the reaction. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C200-7** (44mg, yield: 35.2%). LCMS: $[M + H]^+$ = 409.

Step 7:

Compound **C200-7** (22mg, 0.054mmol) and compound **A1-4** (21.5mg, 0.038mmol) were dissolved in 1.5 mL of dichloromethane/methanol (2/1, v/v), and glacial acetic acid (3.2mg, 0.054mmol) was added dropwise. The mixture was stirred at room temperature under nitrogen protection for 1h. Then sodium cyanobohydride (2.4mg, 0.038mmol) was added. The mixture was further stirred for 1h. The solvent was removed under reduced pressure below 40°C, and the crude product was purified by Flash chromatography to afford off-white solid compound **C200** (18mg, yield: 34.7%). LCMS: $[M + H]^+$ = 962. $^1$H NMR (400 MHz, DMSO-$d_6$) δ 11.17 (s, 1H), 11.04 (s, 1H), 8.47 (s, 1H), 8.11 (d,1H), 8.06 (s, 1H), 8.05 (d,2H), 7.57-7.49 (m, 1H), 7.38 (d,1H), 7.33 (d,1H), 7.09 (t,1H), 6.62 (d, 1H), 6.47 (dd,1H), 5.34- 5.30 (m, 1H), 5.19 (dd,1H), 4.62 (d,1H), 4.48 (s, 3H), 3.76 (s, 3H), 3.73 (s, 1H), 3.67 (t, 3H), 3.29 (m, 3H), 2.93 (m, 1H), 2.70-2.61 (m, 4H), 2.52 (d,3H), 2.01 (m,4H), 1.84 (d, 2H), 1.76 (d,6H), 1.55-1.45 (m, 3H).

**Preparation of 3-(6-chloro-4-(7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hept-1-yn-1-yl)-1-oxoisoindolin-2-yl)piperidine-2,6-dione (C202)**

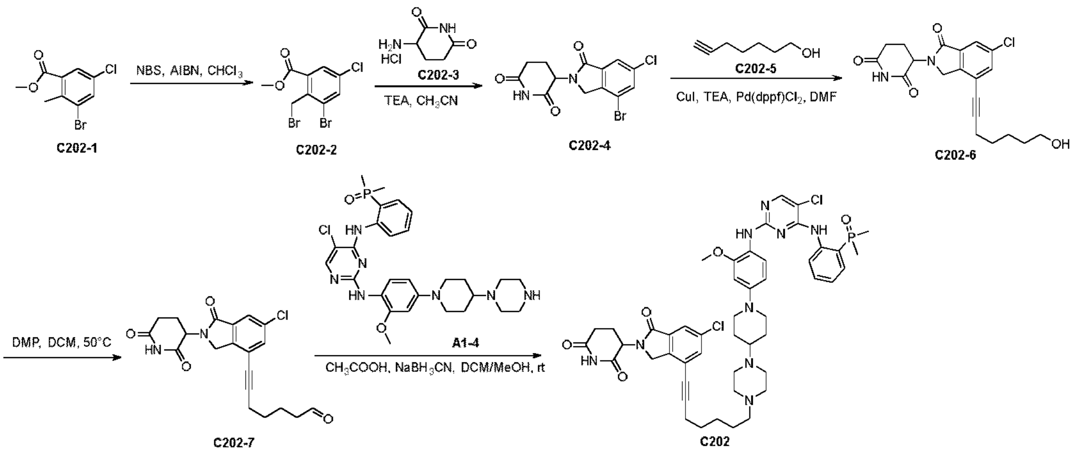


Step 1:

Compound **C202-1** (1g, 3.82 mmol), NBS (679mg, 4.35mmol), and AIBN (62mg, 0.38 mmol) were dissolved in 20 mL of chloroform. The mixture was reacted under reflux at 95°C under nitrogen protection for 5h. The solvent was removed under reduced pressure, and the crude product was purified by Flash chromatography to afford white solid compound **C202-2**(600mg, yield: 46.2%).

Step 2:

Compound **C202-2** (1.31g, 3.85mmol), compound **C202-3** (822mg, 5.01mmol), and triethylamine (506mg, 5.01

mmol) were dissolved in 10 mL of acetonitrile. The mixture was stirred at 80°C under nitrogen protection overnight. The mixture was cooled to room temperature. The solvent was removed under reduced pressure, and the crude product was purified by Flash chromatography to afford white solid compound **C202-4** (1.0g, yield: 73.0%). LCMS: $[M + H]^+$ = 357, 359.

Step 3:

Compound **C202-4** (100mg, 0.281mmol), compound **C202-5** (79mg, 0.702mmol), cuprous iodide (5.4mg, 0.028mmol), $Pd(dppf)Cl_2$ (21mg, 0.028mmol) and triethylamine (85mg, 0.843mmol) were dissolved in 2 mL of anhydrous DMF. The mixture was reacted with stirring at 70°C under nitrogen protection for 5h. The solvent was removed under reduced pressure, and the crude product was separated by preparative thin layer chromatography to afford off-white solid compound **C202-6** (94mg, yield: 87.9%). LCMS: $[M + H]^+$ = 389.

Step 4:

Compound **C202-6** (50mg, 0.129mmol) and Dess-Martin reagent (82mg, 0.193 mmol) were dissolved in 10 mL of dichloromethane and stirred at 50°C for 2h. The mixture was cooled to room temperature, and quenched with saturated sodium bicarbonate aqueous solution. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C202-7** (34 mg, yield: 68.0%). LCMS: $[M + H]^+$ = 387.

Step 5:

Compound **C202-7** (34mg, 0.0881mmol) and compound **A1-4** (35mg, 0.062mmol) were dissolved in 1.5 mL of dichloromethane/methanol (2/1, v/v), and 1 drop of glacial acetic acid was added dropwise. The mixture was stirred at room temperature under nitrogen protection for 1h. Then sodium cyanobohydride (3.9mg, 0.062mmol) was added. The mixture was further stirred for 1h. The solvent was removed under reduced pressure below 40°C, and the crude product was purified by Flash chromatography to afford off-white solid compound **C202** (10mg, yield: 12.1%). LCMS: $[M + H]^+$ = 940. $^1H$ NMR (400 MHz, DMSO-$d_6$) $\delta$ 11.18 (s, 1H), 11.03 (s, 1H), 8.48 (s, 1H), 8.06 (s, 2H), 7.72 (dd, 2H), 7.57-7.49 (m, 1H), 7.35 (dd, 2H), 7.09 (t, 1H), 6.62 (d, 1H), 6.47 (dd, 1H), 5.32(m, 1H), 5.15 (dd, 1H), 4.46 (d, 1H), 4.31 (d, 1H), 3.76 (s, 3H), 3.71 (d, 2H), 2.97-2.87 (m, 1H), 2.65 (t, 3H), 2.59 (d, 3H), 2.34-2.32 (m, 4H), 2.27 (m, 3H), 2.03-1.97 (m, 2H), 1.82 (d, 2H), 1.76 (d, 7H), 1.59 (t, 2H), 1.48 (d, 7H).

**Preparation of 7-(7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hept-1-yn-1-yl)-2-(2,6-dioxopiperidin-3-yl)-3-oxoisoindoline-5-carbonitrile (C204)**

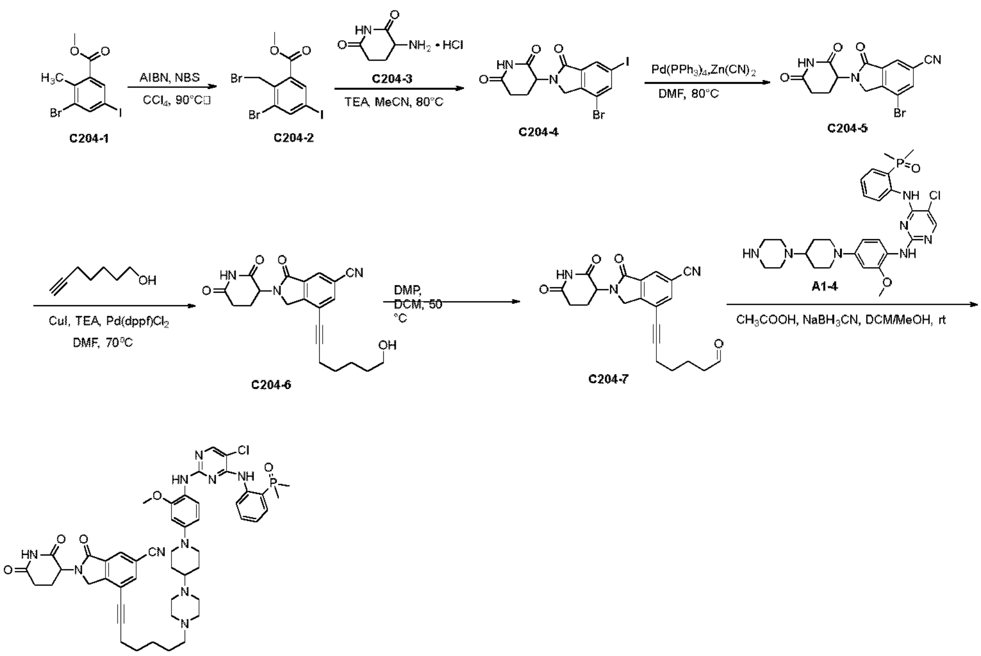


C204

Step 1:

**C204-1** (532.5 mg, 1.5 mmol) was dissolved in 10.0 mL of $CCl_4$. NBS (351 mg, 1.95 mmol) and AIBN (73.8 mg, 0.45 mmol) were added under nitrogen protection. The mixture was heated to 90°C, and reacted under reflux for 20 h. TLC showed that one third of the raw materials were left. NBS (405 mg, 2.55 mmol) and AIBN (123 mg, 0.75 mmol) were added to the mixture. After the addition was completed, the mixture was further reacted under reflux for 20 h. TLC showed that there was no raw material remaining. The mixture was cooled to room temperature, and then filtered with suction. The filtrate was concentrated under reduced pressure to afford a crude product, which was purified by Flash chromatography to afford a light-yellow oily product, which became solid upon standing, **C204-2** (690 mg, 100%).

Step 2:

**C204-2** (690 mg, 1.6 mmol) and **C204-3** (341 mg, 2.08 mmol) were dispersed in 12.0 mL of anhydrous MeCN, and TEA (210 mg, 2.08 mmol) was added. The mixture was heated to 80°C, and reacted under reflux for 16h. LCMS showed that the reaction was completed. The mixture was cooled to room temperature, and filtrated with suction. The filter cake was washed with MeCN for three times, and dried with baking to afford a title product **C204-4** (450 mg, yield: 62.8%). LCMS: $[M + H]^+$ = 449, 451.

Step 3:

**C204-4** (380 mg, 0.848 mmol) and $Pd(PPh_3)_4$ (98 mg, 0.0848 mmol) were dispersed in 20.0 mL of anhydrous DMF. $Zn(CN)_2$ (109.5 mg, 0.933 mmol) was added under $N_2$ protection. The mixture was heated to 80°C, and reacted under reflux for 2h. LCMS showed that there was raw material remaining. $Zn(CN)_2$ (149.3 mg, 1.272 mmol) and $Pd(PPh_3)_4$ (284 mg, 0.2544 mmol) were added to the mixture. After the addition was completed, the mixture was further reacted at 80°C for 18h. LCMS showed that the reaction was completed. The mixture was cooled to room temperature and directly purified by RP-Flash chromatography to afford brown solid product **C204-5** (170 mg, 57.8%). LCMS: $[M + H]^+$ = 348, 350.

Step 4:

**C204-5** (160 mg, 0.461 mmol), CuI (17.5 mg, 0.092 mmol) and $Pd(dppf)Cl_2$ (67.4 mg, 0.092 mmol) were dispersed in 20.0 mL of anhydrous DMF. 6-alkynyl-1-heptanol (129.1 mg, 1.153 mmol) and TEA (140 mg, 1.383 mmol) were added successively under $N_2$ protection. The mixture was heated to 70°C and reacted for 20h. LCMS showed that the reaction was completed. The mixture was cooled to room temperature and directly purified by RP-Flash chromatography to afford pale brown solid product **C204-6** (140 mg, 91.4%). LCMS: $[M + H]^+$ = 380.

Step 5:

**C204-6** (140 mg, 0.369 mmol) was dissolved in 28.0 mL of anhydrous DCM. Dess-Martin reagent (313 mg, 0.738 mmol) was added under $N_2$ protection. The mixture was heated to 50°C, and reacted under reflux for 2.0 h. TLC showed that the reaction was completed. The mixture was cooled to room temperature, and then 20 mL of saturated $NaHCO_3$ solution and 20 mL of saturated $Na_2S_2O_3$ solution were added. The mixture was stirred at room temperature for 5 min. The organic layer was separated, dried with anhydrous sodium sulfate, and filtered with suction. The filtrate was concentrated under reduced pressure to afford a crude product. The crude product was purified by Prep-TLC to afford light-yellow solid product **C204-7** (80 mg, yield: 57.1%). LCMS: $[M + H]^+$ = 378.

Step 6:

**C204-7** (45 mg, 0.119 mmol) and **A1-4** (61 mg, 0.107 mmol) were dissolved in a mixture of 4.0 mL of anhydrous DCM and 0.4 mL of anhydrous MeOH. $CH_3COOH$ (10.7 mg, 0.179 mmol) was added under nitrogen protection. The mixture was stirred at room temperature for 0.5h. Then solid $NaBH_3CN$ (15.0 mg, 0.238 mmol) was added. The mixture was further reacted at room temperature for 2h. LCMS and TLC showed that the reaction was completed. The mixture was purified by Prep-HPLC to afford white solid pure product **C204** (35 mg, yield: 31.8%). LCMS: $[M + H]^+$ = 931.

**Preparation of 3-(4-((7-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)heptyl)oxy)-1-oxoisoindolin-2-yl)piperidine-2,6-dione (C211)**

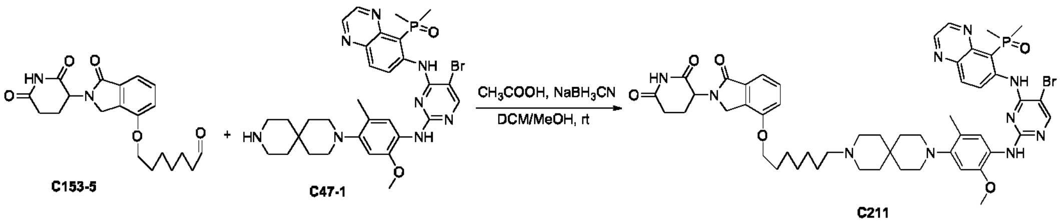


C153-5 C47-1 C211

**C153-5** (35 mg, 0.094 mmol) and **C47-1** (56.2 mg, 0.0846 mmol) were dissolved in a mixture of 4.0 mL of anhydrous DCM and 0.4 mL of anhydrous MeOH. $CH_3COOH$ (8.46 mg, 0.1410 mmol) was added dropwise under nitrogen protection. The mixture was stirred at room temperature for 30 min. Then $NaBH_3CN$ (8.8 mg, 0.1410 mmol) was added. The mixture was reacted with stirring at room temperature for 2h. After the reaction was completed, the mixture was purified by Prep-HPLC to afford a yellow solid pure product **C211** (25 mg, yield: 26.1%). LCMS: $[M + H]^+$ = 1021, 1023.

**Preparation of 3-(4-((6-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)hexyl)oxy)-1-oxoisoindolin-2-yl)piperidine-2,6-dione (C212)**

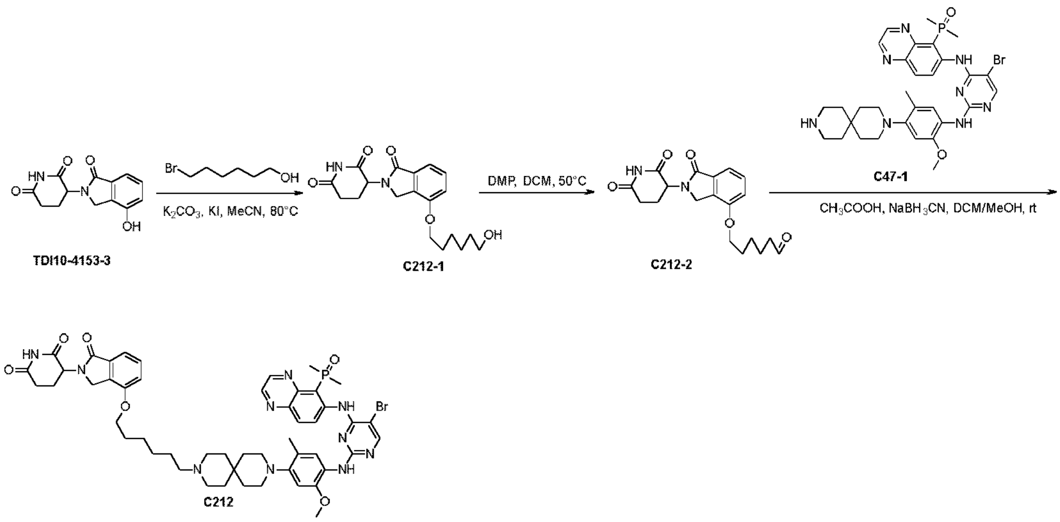


TDI10-4153-3 C212-1 C212-2 C47-1

C212

Step 1:

**C153-3** (100 mg, 0.3846 mmol), $K_2CO_3$ (79.6 mg, 0.5769 mmol) and KI (31.9 mg, 0.1923 mmol) were dispersed in 15.0 mL of anhydrous MeCN, and 6-bromohexan-1-ol (125 mg, 0.6923 mmol) was added. The mixture was reacted at 80°C for 3.0 h. The mixture was cooled to room temperature, and filtered with suction to remove solid residues. The filtrate was concentrated under reduced pressure to afford a crude product, which was purified by Prep-TLC to afford light-yellow solid product **C212-1** (40 mg, 32.0%). LCMS: $[M + H]^+ = 361$.

Step 2:

**C212-1** (40 mg, 0.111 mmol) was dissolved in 15.0 mL of anhydrous DCM, and Dess-Martin reagent (94.1 mg, 0.222 mmol) was added. The mixture was reacted under reflux at 50°C for 2h. The mixture was cooled to room temperature, and diluted with 10 mL of DCM. 8.0 mL of saturated $NaHCO_3$ solution and 8.0 mL of saturated $Na_2S_2O_3$ solution were added. The mixture was stirred for 5 min. The organic layer was separated, dried with anhydrous $Na_2SO_4$, and filtered with suction to remove the desiccant. The filtrate was concentrated under reduced pressure to afford a crude product, which was purified by Prep-TLC to afford light-yellow oily product **C212-2** (20 mg, yield: 50.4%). LCMS: $[M + H]^+ = 359$.

Step 3:

**C212-2** (20 mg, 0.056 mmol) and **C47-1** (31.5 mg, 0.048 mmol) were dissolved in a mixture of 4.0 mL of anhydrous DCM and 0.4 mL of anhydrous MeOH. $CH_3COOH$ (5.03 mg, 0.084 mmol, dissolved in 0.5 mL of DCM) was added dropwise under nitrogen protection. The mixture was stirred at room temperature for 30 min. Then $NaBH_3CN$ (7.02 mg, 0.112 mmol) was added. The mixture was reacted with stirring at room temperature for 2h. The mixture was directly purified by Prep-TLC to afford a crude product, which was further purified by Prep-TLC to afford yellow solid pure product **C212** (20 mg, yield: 35.7%). LCMS: $[M + H]^+ = 1007, 1009$.

**Preparation of 3-(4-((5-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)pentyl)oxy)-1-oxoisoindolin-2-yl)piperidine-2,6-dione (C213)**

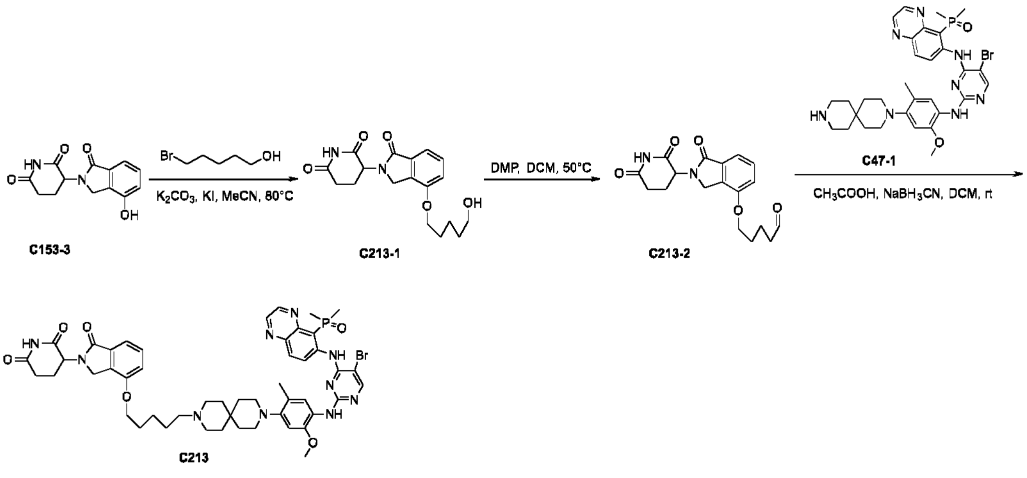


C153-3 C213-1 C213-2 C213

Step 1:

**C153-3** (130 mg, 0.5 mmol), $K_2CO_3$ (103.5 mg, 0.75 mmol) and KI (41.5 mg, 0.25 mmol) were dispersed in 15.0 mL of anhydrous MeCN, and 5-bromopentan-1-ol (125.3 mg, 0.75 mmol) was added. The mixture was reacted at 80°C for 3.0 h. The mixture was cooled to room temperature, and then filtered with suction to remove solid residues. The filtrate was concentrated under reduced pressure to afford a crude product, which was purified by Prep-TLC to afford light-yellow solid product **C213-1** (65 mg, 37.6%). LCMS: $[M + H]^+$ = 347.

Step 2:

**C213-1** (65 mg, 0.188 mmol) was dissolved in 15.0 mL of anhydrous DCM, and Dess-Martin reagent (159.4 mg, 0.376 mmol) was added. The mixture was reacted under reflux at 50°C for 2h. The mixture was cooled to room temperature, and diluted with 20 mL of DCM. 10.0 mL of saturated $NaHCO_3$ solution and 10.0 mL of saturated $Na_2S_2O_3$ solution were added. The mixture was stirred for 5 min. The organic layer was separated, dried with anhydrous $Na_2SO_4$, and filtered with suction to remove the desiccant. The filtrate was concentrated under reduced pressure to afford crude **C213-2** (57 mg, 87.7%). The crude product was directly used in the next step. LCMS: $[M + H]^+$ = 345.

Step 3:

**C213-2** (34.4 mg, 0.1 mmol) and **C47-1** (39.8 mg, 0.06 mmol) were dissolved in 4.0 mL of anhydrous DCM. $CH_3COOH$ (9.0 mg, 0.15 mmol, dissolved in 0.5 mL of DCM) was added dropwise under nitrogen protection. The mixture was stirred at room temperature for 30 min. Then $NaBH_3CN$ (12.6 mg, 0.2 mmol) was added. The mixture was reacted with stirring at room temperature for 2h. The mixture was purified by Prep-HPLC to afford yellow solid pure product **C213** (13.0 mg, yield: 13.1%). LCMS: $[M + H]^+$ = 993, 995.

**Preparation of 3-(4-((7-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)heptyl)oxy)-1-oxoisoindolin-2-yl)piperidine-2,6-dione (C214)**

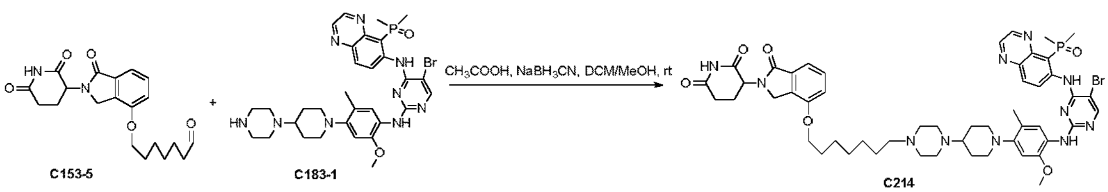


C153-5 + C183-1 C214

**C153-5** (30 mg, 0.081 mmol) and **C183-1** (or **C158-1)** (49.3 mg, 0.073 mmol) were dissolved in a mixture of 4.0 mL of anhydrous DCM and 0.4 mL of anhydrous MeOH. $CH_3COOH$ (7.29 mg, 0.1215 mmol) was added dropwise under nitrogen protection. The mixture was stirred at room temperature for 30 min. Then $NaBH_3CN$ (7.6 mg, 0.1215 mmol) was added. The mixture was reacted with stirring at room temperature for 2h. The mixture was purified by Prep-HPLC to afford yellow solid pure product **C214** (25 mg, yield: 29.9%). LCMS: $[M + H]^+$ = 1036, 1038.

**Preparation of 3-(4-(4-(4-(1-(4-((5-chloro-4-((2-(dimethylphosphoryl)phenyl)amino)pyrimidin-2-yl)amino)-3-methoxyphenyl)piperidin-4-yl)piperazin-1-yl)but-1-yn-1-yl)-1-oxoisoindolin-2-yl)piperidine-2,6-dione (C215)**

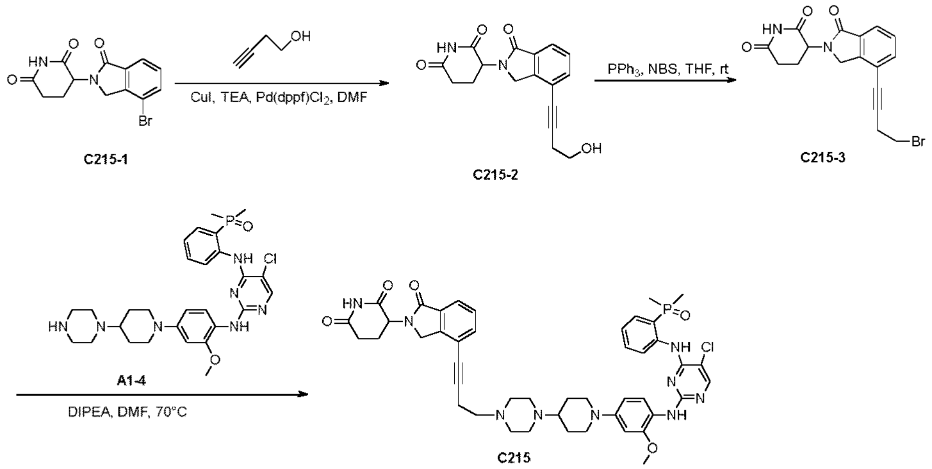


Step 1:

**C215-1** (or **C1-1**) (322 mg, 1.0 mmol), CuI (19.0 mg, 0.1 mmol) and $Pd(dppf)Cl_2$ (73.1 mg, 0.1 mmol) were dispersed in 10.0 mL of anhydrous DMF. 3-alkynyl-1-butanol (140.2 mg, 2.0 mmol) and TEA (303 mg, 3.0 mmol) were added successively under $N_2$ protection. The mixture was heated to 70°C and reacted for 20h. LCMS showed that the reaction was completed. The mixture was cooled to room temperature and directly purified by RP-Flash chromatography to afford light-yellow solid product **C215-2** (210 mg, 67.3%). LCMS: $[M + H]^+$ = 313.

Step 2:

**C215-2** (112 mg, 0.359 mmol) and $PPh_3$ (103.5 mg, 0.395 mmol) were dissolved in 33.0 mL of anhydrous THF. NBS (127.8 mg, 0.718 mmol) was added under $N_2$ protection. The mixture was reacted at room temperature for 5.0 h. The mixture was concentrated under reduced pressure to afford a crude product, which was purified by Prep-TLC to afford white solid product **C215-3** (105 mg, 78.3%). LCMS: $[M + H]^+$ = 375, 377.

Step 3:

**C215-3** (45 mg, 0.12mmol) and **A1-4** (54.8 mg, 0.096 mmol) were dissolved in 4.5 mL of anhydrous DMF. DIPEA (75.8 mg, 0.6 mmol) was added under nitrogen protection. The mixture was reacted at 70°C for 12h, and then purified by RP-Flash to afford white solid product **C215** (12 mg, yield: 9.2%). LCMS: $[M + H]^+$ = 864.

**Preparation of 3-(5-(5-(4-(1-(4-((5-chloro-4-((2-(dimethylphosphoryl)phenyl)amino)pyrimidin-2-yl)amino)-3-methoxyphenyl)piperidin-4-yl)piperazin-1-yl)pent-1-yn-1-yl)-1-oxoisoindolin-2-yl)piperidine-2,6-dione (C218)**

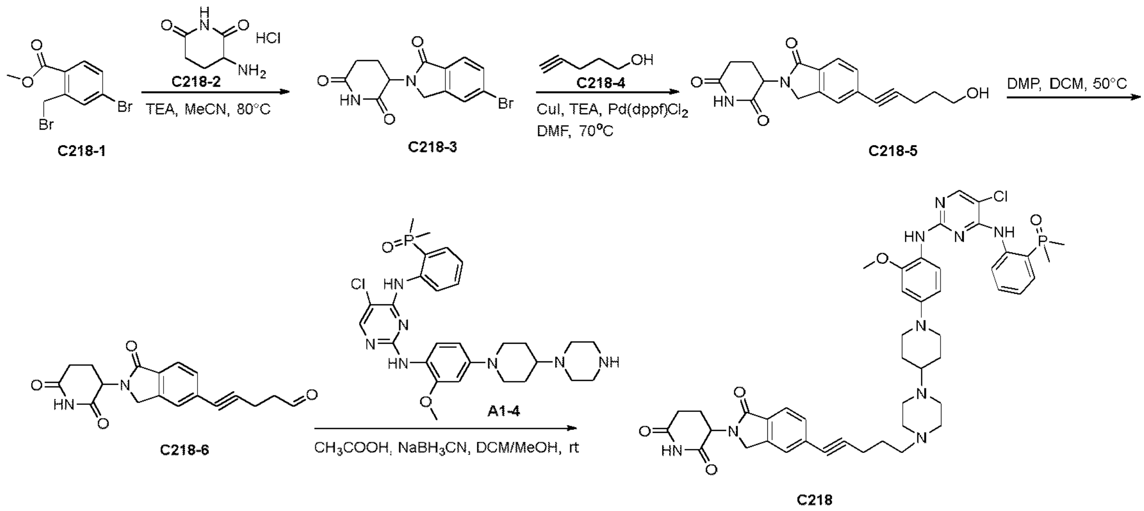


Step 1:

Compound **C218-1** (300mg, 0.974mmol), **C218-2** (208mg, 1.266mmol), and triethylamine (128mg, 1.266mmol) were dissolved in 5 mL of acetonitrile. The mixture was stirred at 80°C under nitrogen protection overnight. The mixture was cooled to room temperature. The solvent was removed under reduced pressure, and the crude product was purified by Flash chromatography to afford white solid compound **C218-3** (180mg, yield: 57.0%). LCMS: $[M + H]^+$ = 323, 325.

Step 2:

Compound **C218-3** (90mg, 0.281mmol), compound **C218-4** (59mg, 0.699mmol), cuprous iodide (5.3mg, 0.028mmol), $Pd(dppf)Cl_2$ (20.5mg, 0.028mmol) and triethylamine (85mg, 0.839mmol) were dissolved in 3 mL of anhydrous DMF. The mixture was reacted with stirring at 70°C under nitrogen protection for 5h. The solvent was removed under reduced pressure, and the crude product was purified by thin layer chromatography to afford off-white solid compound **C218-5** (83mg, yield: 91.2%). LCMS: $[M + H]^+$ = 327.

Step 3:

Compound **C218-5** (50mg, 0.153mmol) and Dess-Martin reagent (98mg, 0.230 mmol) were dissolved in 10 mL of dichloromethane and stirred at 50°C for 2h. The mixture was cooled to room temperature, and quenched with saturated sodium bicarbonate aqueous solution. The organic phase was separated, and the aqueous phase was extracted with dichloromethane for three times. The organic phases were combined, dried with anhydrous sodium sulfate, and concentrated. The crude product was purified by TLC to afford a white solid compound **C218-6** (38 mg, yield: 76.0%). LCMS: $[M + H]^+$ = 325.

Step 4:

Compound **C218-6** (38mg, 0.119mmol) and compound **A1-4** (40mg, 0.095mmol) were dissolved in 3 mL of dichloromethane/methanol (2/1, v/v), and 1 drop of glacial acetic acid was added dropwise. The mixture was stirred at room temperature under nitrogen protection for 1h. Then sodium cyanobohydride (6.0mg, 0.095mmol) was added. The mixture was further stirred for 1h. The solvent was removed under reduced pressure below 40°C, and the crude product was purified by Flash chromatography to afford off-white solid compound **C218** (25mg, yield: 24.3%). LCMS: $[M + H]^+$ = 878. $^1H$ NMR (400 MHz, DMSO-$d_6$) δ 11.17 (s, 1H), 10.99 (s, 1H), 8.48 (dr, 1H), 8.06 (d, 2H), 7.69 (dd, 1H), 7.63 (t, 1H), 7.56-7.49 (m, 2H), 7.35 (dd, 2H), 7.13-7.06 (m, 1H), 6.63 (d, 1H), 6.47 (dd, 1H), 5.32(m, 1H), 5.11 (dd, 1H), 4.48-4.28 (m, 2H), 3.76 (s, 3H), 3.72 (d, 2H), 2.91 (m, 1H), 2.72-2.60 (m, 4H), 2.57 (m, 2H), 2.40 (m, 7H), 2.03-1.98 (m, 3H), 1.86 (d, 2H), 1.78 (s, 3H), 1.74 (s, 3H), 1.72m, 2H), 1.59-1.50 (m, 2H), 1.50-1.43 (m, 1H).

**Preparation of 3-(4-(5-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)pent-1-yn-1-yl)-1-oxoisoindolin-2-yl)piperidine-2,6-dione (C227)**

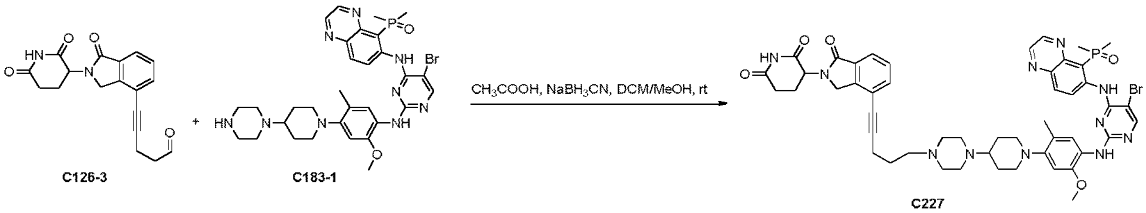


C126-3 C183-1 C227

**C126-3** (32.4 mg, 0.1 mmol) and **C183-1** (or **C158-1)** (61.1 mg, 0.09 mmol) were dissolved in a mixture of 4.0 mL of anhydrous DCM and 0.4 mL of anhydrous MeOH. $CH_3COOH$ (9.0 mg, 0.15 mmol) was added under nitrogen protection. The mixture was stirred at room temperature for 0.5h. Then solid $NaBH_3CN$ (12.6 mg, 0.2 mmol) was added. The mixture was further reacted at room temperature for 2h. LCMS and TLC showed that the reaction was completed. The mixture was purified by Prep-HPLC to afford yellow solid pure product **C227** (20 mg, yield: 20.3%). LCMS: $[M + H]^+$ = 988, 990.

**Preparation of 3-(4-(5-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)pent-1-yn-1-yl)-1-oxoisoindolin-2-yl)piperidine-2,6-dione (C228)**

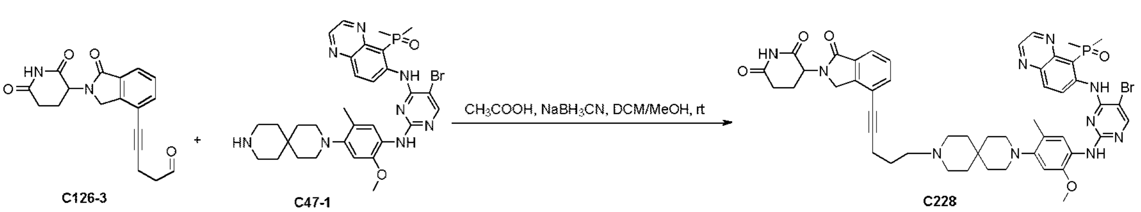


C126-3 C47-1 C228

**C126-3** (32.4 mg, 0.1 mmol) and **C47-1** (59.8 mg, 0.09 mmol) were dissolved in a mixture of 4.0 mL of anhydrous DCM and 0.4 mL of anhydrous MeOH. $CH_3COOH$ (9.0 mg, 0.15 mmol) was added under nitrogen protection. The mixture was stirred at room temperature for 0.5h. Then solid $NaBH_3CN$ (12.6 mg, 0.2 mmol) was added. The mixture was further reacted at room temperature for 2h. LCMS and TLC showed that the reaction was completed. The mixture was purified by Prep-HPLC to afford yellow solid pure product **C228** (30 mg, yield: 30.9%). LCMS: $[M + H]^+$ = 973, 975.

**Preparation of 7-(7-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)hept-1-yn-1-yl)-2-(2,6-dioxopiperidin-3-yl)-3-oxoisoindoline-5-carbonitrile (C229)**

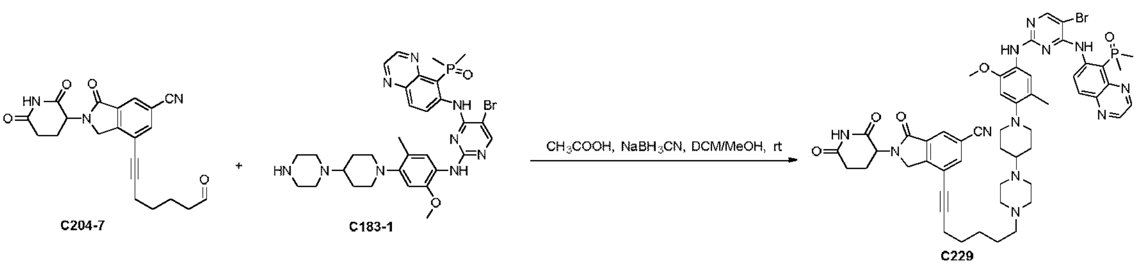


C204-7 C183-1 C229

**C204-7** (35 mg, 0.093 mmol) and **C183-1** (or **C158-1)** (56.7 mg, 0.0837 mmol) were dissolved in a mixture of

4.0 mL of anhydrous DCM and 0.4 mL of anhydrous MeOH. $CH_3COOH$ (8.36 mg, 0.1395 mmol) was added under nitrogen protection. The mixture was stirred at room temperature for 0.5h. Then solid $NaBH_3CN$ (11.7 mg, 0.1860 mmol) was added. The mixture was further reacted at room temperature for 2h. LCMS and TLC showed that the reaction was completed. The mixture was purified by Prep-HPLC to afford yellow solid pure product **C229** (25 mg, yield: 25.8%). LCMS: $[M + H]^+$ = 1041, 1043.

**Preparation of 3-(5-(4-(3-(4-(1-(4-((5-chloro-4-((2-(dimethylphosphoryl)phenyl)amino)pyrimidin-2-yl)amino)-3-methoxyphenyl)piperidin-4-yl)piperazin-1-yl)propyl)phenyl)-1-oxoisoindolin-2-yl)piperidine-2,6-dione (C235)**

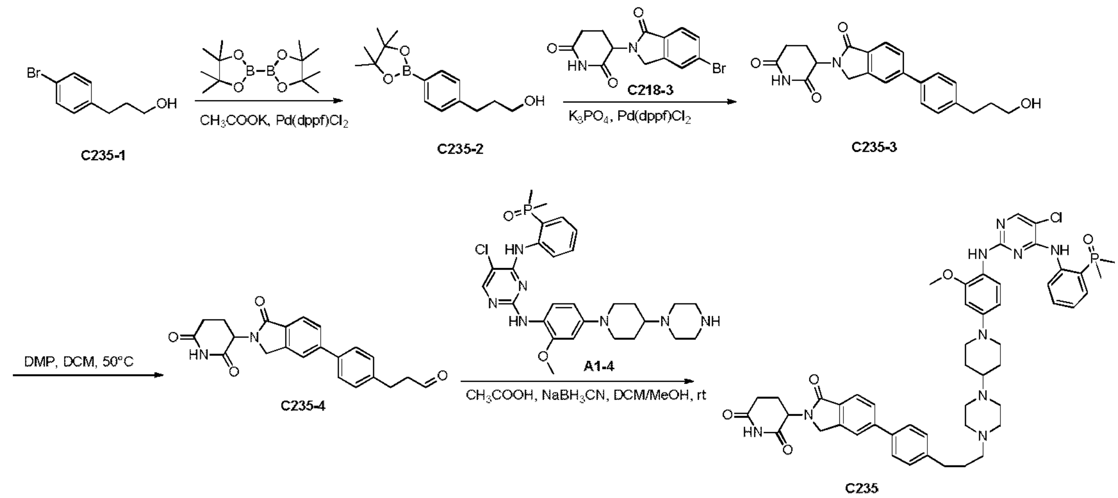


Step 1:

Compound **C235-1** (600mg, 2.84mmol), bis(pinacolato)diboron (805mg, 3.17mmol), potassium acetate (825mg, 8.41mmol), and $Pd(dppf)Cl_2$ (62mg, 0.08mmol) were dissolved in 10 mL of 1,4-dioxane. The mixture was stirred at 100°C under nitrogen protection overnight. The solvent was removed under reduced pressure, and the crude product was purified by Flash chromatography to afford compound **C235-2** (550mg, yield: 74.9%).

Step 2:

**C218-3** (or **C1-1**) (80mg, 0.248mmol), compound **C235-2** (85mg, 0.323mmol), potassium phosphate (65mg, 0.298mmol), and $Pd(dppf)Cl_2$ (18mg, 0.025mmol) were dissolved in 1.5 mL of DMF. The mixture was stirred at 90°C under nitrogen protection overnight. The solvent was removed under reduced pressure, and the crude product was purified by Flash chromatography to afford compound **C235-3** (52mg, yield: 45.2%). LCMS: $[M + H]^+$ = 379.

Step 3:

Compound **C235-3** (78mg, 0.206mmol) and Dess-Martin reagent (175mg, 0.413mmol) were dissolved in 10 mL of dichloromethane and stirred at 50°C for 2h. The mixture was cooled to room temperature, and saturated sodium bicarbonate aqueous solution and saturated sodium thiosulfate aqueous solution were added successively and stirred for 5 minutes respectively to quench the reaction. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C235-4** (44mg, yield: 57.1%). LCMS: $[M + H]^+$ = 377.

Step 4:

Compound **C235-4** (22mg, 0.058mmol) and compound **A1-4** (27mg, 0.047mmol) were dissolved in 2 mL of dichloromethane/methanol (2/1, v/v), and glacial acetic acid (3.5mg, 0.058mmol) was added dropwise. The mixture was stirred at room temperature under nitrogen protection for 1h. Then sodium cyanobohydride (2.9mg, 0.047mmol) was added. The mixture was further stirred for 1h. The solvent was removed under reduced pressure below 40°C, and the

crude product was purified by Flash chromatography to afford off-white solid compound **C235** (12mg, yield: 22.2%). LCMS: $[M + H]^+ = 930$.

**Preparation of 3-(5-(4-(4-(4-(1-(4-((5-chloro-4-((2-(dimethylphosphoryl)phenyl)amino)pyrimidin-2-yl)amino)-3-methoxyphenyl)piperidin-4-yl)piperazin-1-yl)butyl)phenyl)-1-oxoisoindolin-2-yl)piperidine-2,6-dione (C236)**

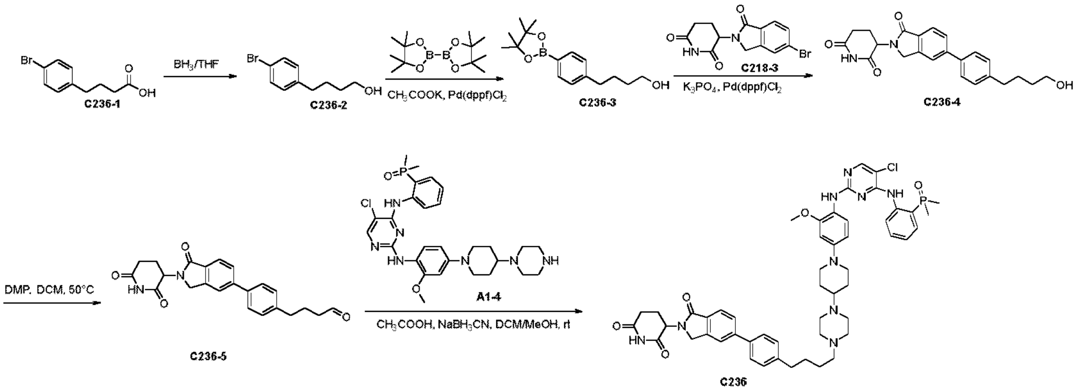


Step 1:

Compound **C236-1** (1g, 4.13mmol) was dissolved in 10 mL of dry THF. The mixture was cooled to -10°C, and the tetrahydrofuran complex of borane (1mol/L, 8.3 mL, 8.26mmol) was added dropwise under nitrogen protection. After the dropwise addition, the mixture was warmed to room temperature and stirred for 2h. The mixture was poured into 10 mL of cold water. The mixture was extracted with ethyl acetate, dried with anhydrous sodium sulfate, and filtered. The filtrate was concentrated. The crude product was purified by Flash chromatography to afford compound **C236-2** (600mg, yield: 63.7%).

Step 2:

Compound **C236-2** (635mg, 2.78mmol), bis(pinacolato)diboron (800mg, 3.15mmol), potassium acetate (820mg, 8.35mmol), and Pd(dppf)$Cl_2$ (61mg, 0.08mmol) were dissolved in 10 mL of 1,4-dioxane. The mixture was stirred at 100°C under nitrogen protection overnight. The solvent was removed under reduced pressure, and the crude product was purified by Flash chromatography to afford compound **C236-3** (500mg, yield: 65.1%).

Step 3:

**C218-3** (or **C1-1**) (100 mg, 0.311 mmol), compound **C236-3** (112mg, 0.404mmol), potassium phosphate (81 mg, 0.373mmol), and Pd(dppf)$Cl_2$ (23mg, 0.031mmol) were dissolved in 2 mL of DMF. The mixture was stirred at 90°C under nitrogen protection overnight. The solvent was removed under reduced pressure, and the crude product was purified by Flash chromatography to afford compound **C236-4** (60mg, yield: 49.6%). LCMS: $[M + H]^+ = 393$.

Step 4:

Compound **C236-4** (42mg, 0.107mmol) and Dess-Martin reagent (91mg, 0.214mmol) were dissolved in 10 mL of dichloromethane and stirred at 50°C for 2h. The mixture was cooled to room temperature, and saturated sodium bicarbonate aqueous solution and saturated sodium thiosulfate aqueous solution were added successively and stirred for 5 minutes respectively to quench the reaction. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C236-5** (39mg, yield: 93.3%). LCMS: $[M + H]^+ = 391$.

Step 5:

Compound **C236-5** (39mg, 0.1mmol) and compound **A1-4** (40mg, 0.07mmol) were dissolved in 2 mL of dichloromethane/methanol (2/1, v/v), and glacial acetic acid (6.0mg, 0.1mmol) was added dropwise. The mixture was stirred at room temperature under nitrogen protection for 1h. Then sodium cyanobohydride (4.4mg, 0.07mmol) was added. The mixture was further stirred for 1h. The solvent was removed under reduced pressure below 40°C, and the crude product was purified by Flash chromatography to afford off-white solid compound **C236** (2.6mg, yield: 2.8%). LCMS: $[M + H]^+$ = 944.

**Preparation of 3-(4-(6-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hexyl)-1-oxoisoindolin-2-yl)piperidine-2,6-dione (C238)**

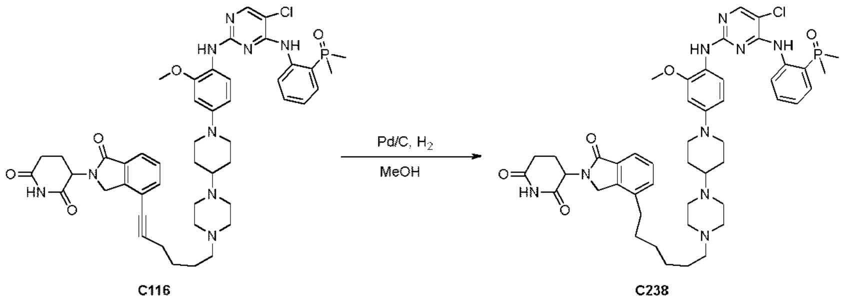


**C116** (100 mg, 0.112 mmol) was dissolved in methanol (10 mL), and Pd/C (30 mg, 10% Pd) was added. The atmosphere was replaced three times with nitrogen gas, and then replaced three times with hydrogen gas. The atmosphere was pressurized to 60 Psi with hydrogen gas. The mixture was reacted with stirring at room temperature for 1h. The reaction process was monitored by LCMS, and the raw materials were basically transformed into the title product. The atmosphere was replaced with nitrogen gas to remove hydrogen gas. The mixture was filtered to remove Pd/C. The filtrate was concentrated to afford a crude product, which was separated by Flash to afford white solid **C238** (49 mg, yield: 48.8%). LCMS: $[M + H]^+$ = 896.

**Preparation of 3-(4-(8-(4-(1-(4-((5-chloro-4-((2-(dimethylphosphoryl)phenyl)amino)pyrimidin-2-yl)amino)-3-methoxyphenyl)piperidin-4-yl)piperazin-1-yl)octyl)-1-oxoisoindolin-2-yl)piperidine-2,6-dione (C240)**

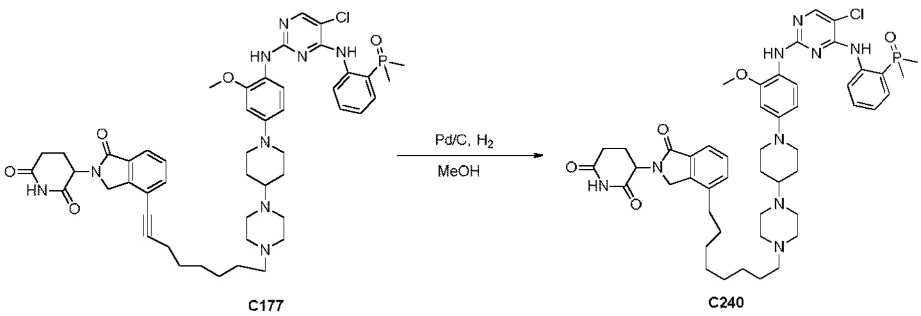


**C177** (50 mg, 0.051 mmol) was dissolved in methanol (10 mL), and Pd/C (20 mg, 10% Pd) was added. The atmosphere was replaced three times with nitrogen gas, and then replaced three times with hydrogen gas. The atmosphere was pressurized to 60 Psi with hydrogen gas. The mixture was reacted with stirring at room temperature for 1h. The reaction process was monitored by LCMS, and the raw materials were basically transformed into the title product. The atmosphere was replaced with nitrogen gas to remove hydrogen gas. The mixture was filtered to remove Pd/C. The filtrate was concentrated to afford a crude product, which was separated by Flash to afford white solid **C240**, 19 mg, yield: 40.4%. LCMS: $[M + H]^+$ = 924.

**Preparation of 3-(4-(7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hept-1-yn-1-yl)-7-fluoro-1-oxoisoindolin-2-yl)piperidine-2,6-dione (C243)**

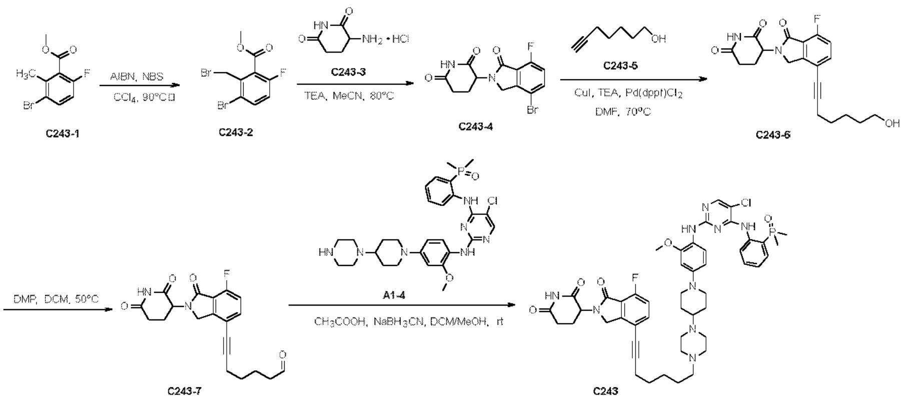


Step 1:

**C243-1** (1.0 g, 4.05 mmol) was dissolved in 20.0 mL of $CCl_4$. NBS (1.09 g, 6.08 mmol) and AIBN (265.7 mg, 1.62 mmol) were added under nitrogen protection. The mixture was heated to 90°C, and reacted under reflux for 20 h. TLC showed that one fifth of the raw materials were left. NBS (1.09 g, 6.08 mmol) and AIBN (265.7 mg, 1.62 mmol) were added to the mixture. After the addition was completed, the mixture was further reacted under reflux for 20 h. TLC showed that there was no raw material remaining. The mixture was cooled to room temperature, and then filtered with suction. The filtrate was concentrated under reduced pressure to afford a crude product, which was purified by Flash chromatography to afford light-yellow oily product **C243-2** (1.35 g, 100%).

Step 2:

**C243-2** (1.35 g, 4.18 mmol) and **C243-3** (891 mg, 5.43 mmol) were dispersed in 25.0 mL of anhydrous MeCN, and TEA (548.8 mg, 5.43 mmol) was added. The mixture was heated to 80°C, and reacted under reflux for 16h. LCMS showed that the reaction was completed. The mixture was cooled to room temperature, and then filtrated with suction. The filter cake was washed with MeCN for three times, and dried with baking to afford a title product **C243-4** (1.25 g, yield: 88.0%). LCMS: $[M + H]^+$ = 341, 343.

Step 3:

**C243-4** (200 mg, 0.588 mmol), CuI (22.3 mg, 0.118 mmol) and $Pd(dppf)Cl_2$ (86.2 mg, 0.118 mmol) were dispersed in 10.0 mL of anhydrous DMF. **C243-5** (131.8 mg, 1.176 mmol) and TEA (178.2 mg, 1.764 mmol) were added successively under $N_2$ protection. The mixture was heated to 70°C and reacted for 20 h. LCMS showed that the reaction was completed. The mixture was cooled to room temperature and directly purified by RP-Flash chromatography to afford light-yellow solid product **C243-6** (160 mg, 73.1%). LCMS: $[M + H]^+$ = 373.

Step 4:

**C243-6** (160 mg, 0.43 mmol) was dissolved in 80.0 mL of anhydrous DCM. Dess-Martin reagent (365 mg, 0.86 mmol) was added under $N_2$ protection. The mixture was heated to 50°C, and reacted under reflux for 2.0 h. TLC showed that the reaction was completed. The mixture was cooled to room temperature, and then 20 mL of saturated $NaHCO_3$ solution and 20 mL of saturated $Na_2S_2O_3$ solution were added. The mixture was stirred at room temperature for 5 min. The organic layer was separated, dried with anhydrous sodium sulfate, and filtered with suction. The filtrate was concentrated under reduced pressure to afford a crude product. The crude product was purified by Flash chromatography to afford light-yellow solid product **C243-7** (75 mg, 46.9%). LCMS: $[M + H]^+$ = 371.

Step 5:

**C243-7** (72 mg, 0.195 mmol) and **A1-4** (99.9 mg, 0.176 mmol) were dissolved in a mixture of 7.0 mL of anhydrous DCM and 0.7 mL of anhydrous MeOH. $CH_3COOH$ (17.6 mg, 0.293 mmol) was added under nitrogen protection. The mixture was stirred at room temperature for 0.5h. Then solid $NaBH_3CN$ (24.5 mg, 0.390 mmol) was added. The mixture was further reacted at room temperature for 2h. LCMS and TLC showed that the reaction was completed. The mixture was purified by Prep-HPLC to afford white solid pure product C243 (45 mg, yield: 25.0%). LCMS: $[M + H]^+ = 924$.

**Preparation of 3-(4-(6-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hex-1-yn-1-yl)-7-fluoro-1-oxoisoindolin-2-yl)piperidine-2,6-dione (C244)**

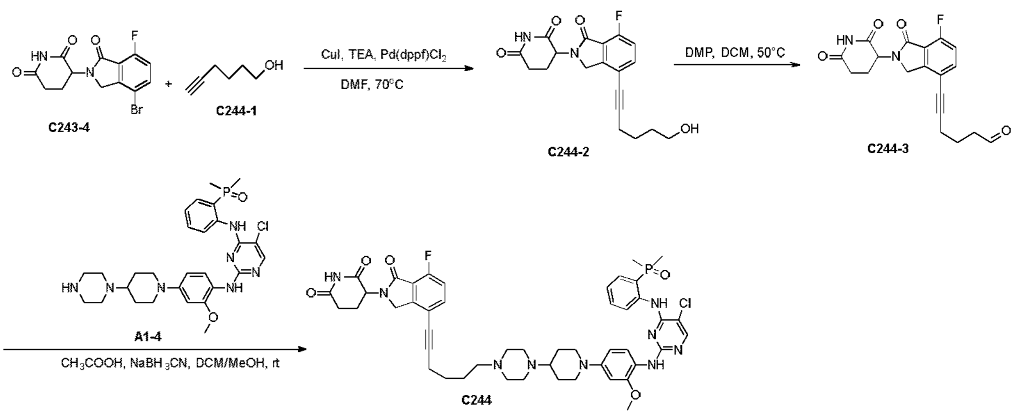


Step 1:

**C243-4** (170 mg, 0.5 mmol), CuI (19.0 mg, 0.1 mmol) and $Pd(dppf)Cl_2$ (73.1 mg, 0.1 mmol) were dispersed in 8.5 mL of anhydrous DMF. **C244-1** (122.5 mg, 1.25 mmol) and TEA (151.5 mg, 1.5 mmol) were added successively under $N_2$ protection. The mixture was heated to 70°C and reacted for 20 h. LCMS showed that the reaction was completed. The mixture was cooled to room temperature and directly purified by RP-Flash chromatography to afford light-yellow solid crude **C244-2** (210 mg, yield: 93.8%), and the crude product was directly used in the next step. LCMS: $[M + H]^+ = 359$.

Step 2:

**C244-2** (90 mg, 0.251 mmol) was dissolved in 45.0 mL of anhydrous DCM. Dess-Martin reagent (159.6 mg, 0.377 mmol) was added under $N_2$ protection. The mixture was heated to 50°C, and reacted under reflux for 2.0 h. TLC showed that the reaction was completed. The mixture was cooled to room temperature, and then 15 mL of saturated $NaHCO_3$ solution and 15 mL of saturated $Na_2S_2O_3$ solution were added. The mixture was stirred at room temperature for 5 min. The organic layer was separated, dried with anhydrous sodium sulfate, and filtered with suction. The filtrate was concentrated under reduced pressure to afford a crude product. The crude product was purified by Prep-TLC to afford light-yellow solid product **C244-3** (75 mg, 83.3%). LCMS: $[M + H]^+ = 357$.

Step 3:

**C244-3** (71.2 mg, 0.2 mmol) and **A1-4** (102.4 mg, 0.18 mmol) were dissolved in a mixture of 7.0 mL of anhydrous DCM and 0.7mL of anhydrous MeOH. $CH_3COOH$ (18.0 mg, 0.3 mmol) was added under nitrogen protection. The mixture was stirred at room temperature for 0.5h. Then solid $NaBH_3CN$ (25.1 mg, 0.4 mmol) was added. The mixture was further reacted at room temperature for 2h. LCMS and TLC showed that the reaction was completed. The mixture was purified by Prep-HPLC to afford white solid pure product **C244** (50 mg, yield: 27.5%). LCMS: $[M + H]^+ = 910$.

**Preparation of 3-(4-(7-(4-(1-(4-((4-((2-(dimethylphosphoryl)phenyl)amino)pyrimidin-2-yl)amino)-3-methoxyphenyl)piperidin-4-yl)piperazin-1-yl)heptyl)-1-oxoisoindolin-2-yl)piperidine-2,6-dione (C245)**

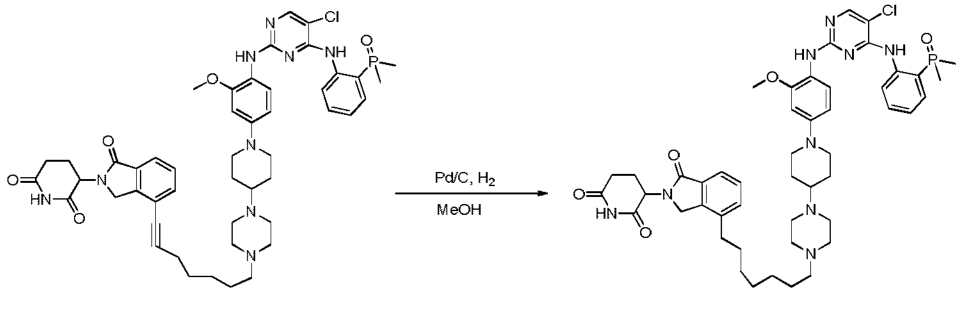


C1 C245

Compound **C1** (45 mg, 0.05 mmol) was dissolved in methanol (10 mL), and Pd/C (20 mg, 10% Pd) was added. The atmosphere was replaced three times with nitrogen gas, and then replaced three times with hydrogen gas. The atmosphere was pressurized to 60 Psi with hydrogen gas. The mixture was reacted with stirring at room temperature for 1h. The reaction process was monitored by LCMS until the raw materials were basically transformed into the title product. The atmosphere was replaced with nitrogen gas to remove hydrogen gas. The mixture was filtered to remove Pd/C. The filtrate was concentrated to afford a crude product, which was separated by Flash to afford 7 mg of white solid. Yield: 16%. LCMS: $[M + H]^+ = 876$.

**Preparation of 3-(4-((5-(4-(1-(4-((5-bromo-4-((5-(dimethylphosphoryl) quinoxalin-6-yl)amino)pyrimidin-2-yl)amino)-5-methoxy-2-methylphenyl)piperidin-4-yl)piperazin-1-yl)pentyl)oxy)-1-oxoisoindolin-2-yl)piperidine-2,6-dione (C246)**

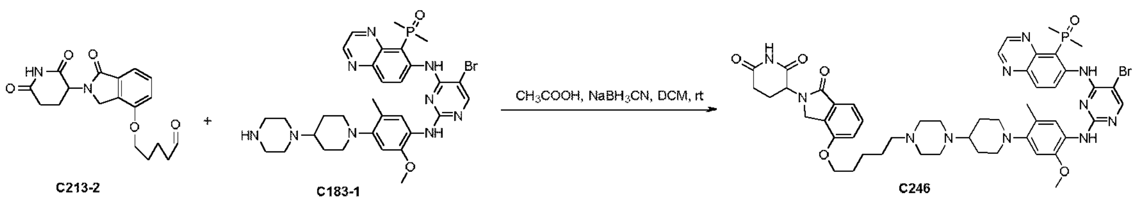


C213-2 C183-1 C246

**C213-2** (22 mg, 0.064 mmol) and **C183-1** (or **C158-1)** (30.4 mg, 0.045 mmol) were dissolved in 3.0 mL of anhydrous DCM. $CH_3COOH$ (5.8 mg, 0.096 mmol, dissolved in 0.5 mL of anhydrous DCM) was added dropwise under nitrogen protection. The mixture was stirred at room temperature for 30 min. Then $NaBH_3CN$ (8.03 mg, 0.128 mmol) was added. The mixture was reacted with stirring at room temperature for 2h. The mixture was purified by Prep-HPLC to afford yellow solid pure product **C246** (8.0 mg, yield: 12.5%). LCMS: $[M + H]^+ = 1008, 1010$.

**Preparation of 7-(5-(4-(1-(4-((5-chloro-4-((2-(dimethylphosphoryl)phenyl)amino)pyrimidin-2-yl)amino)-3-methoxyphenyl)piperidin-4-yl)piperazin-1-yl)pent-1-yn-1-yl)-2-(2,6-dioxopiperidin-3-yl)-3-oxoisoindoline-5-carbonitrile (C258)**

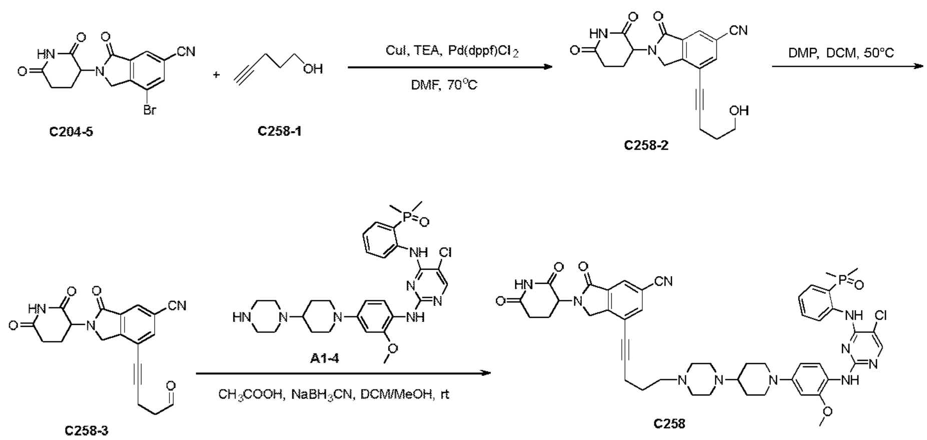


C204-5 C258-1 C258-2

C258-3 C258

Step 1:

**C204-5** (173.5 mg, 0.5 mmol), CuI (19.0 mg, 0.1 mmol) and Pd(dppf)$Cl_2$ (73.1 mg, 0.1 mmol) were dispersed in 10.0 mL of anhydrous DMF. **C258-1** (105 mg, 2.5 mmol) and TEA (151.5 mg, 1.5 mmol) were added successively under $N_2$ protection. The mixture was heated to 70°C and reacted for 20 h. LCMS showed that the reaction was completed. The mixture was cooled to room temperature and directly purified by RP-Flash chromatography to afford light-yellow solid product **C258-2** (180 mg, 100%). LCMS: $[M + H]^+ = 352$.

Step 2:

**C258-2** (180 mg, 0.513 mmol) was dissolved in 90.0 mL of anhydrous DCM. Dess-Martin reagent (435 mg, 1.026 mmol) was added under $N_2$ protection. The mixture was heated to 50°C, and reacted under reflux for 2.0 h. TLC showed that the reaction was completed. The mixture was cooled to room temperature, and then 20 mL of saturated $NaHCO_3$ solution and 20 mL of saturated $Na_2S_2O_3$ solution were added. The mixture was stirred at room temperature for 5 min. The organic layer was separated, dried with anhydrous sodium sulfate, and filtered with suction. The filtrate was concentrated under reduced pressure to afford a crude product. The crude product was purified by Prep-TLC to afford light-yellow solid product **C258-3** (105 mg, 58.3%). LCMS: $[M + H]^+ = 350$.

Step 3:

**C258-3** (93 mg, 0.266 mmol) and **A1-4** (129 mg, 0.226 mmol) were dissolved in a mixture of 7.0 mL of anhydrous DCM and 0.7 mL of anhydrous MeOH. $CH_3COOH$ (24 mg, 0.399 mmol) was added under nitrogen protection. The mixture was stirred at room temperature for 0.5h. Then solid $NaBH_3CN$ (33.5 mg, 0.532 mmol) was added. The mixture was further reacted at room temperature for 2h. LCMS and TLC showed that the reaction was completed. The mixture was purified by Prep-HPLC to afford white solid pure product **C258** (30 mg, yield: 12.5%). LCMS: $[M + H]^+ = 903$.

**Preparation of 7-(6-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hex-1-yn-1-yl)-2-(2,6-dioxopiperidin-3-yl)-3-oxoisoindoline-5-**carbonitrile **(C259)**

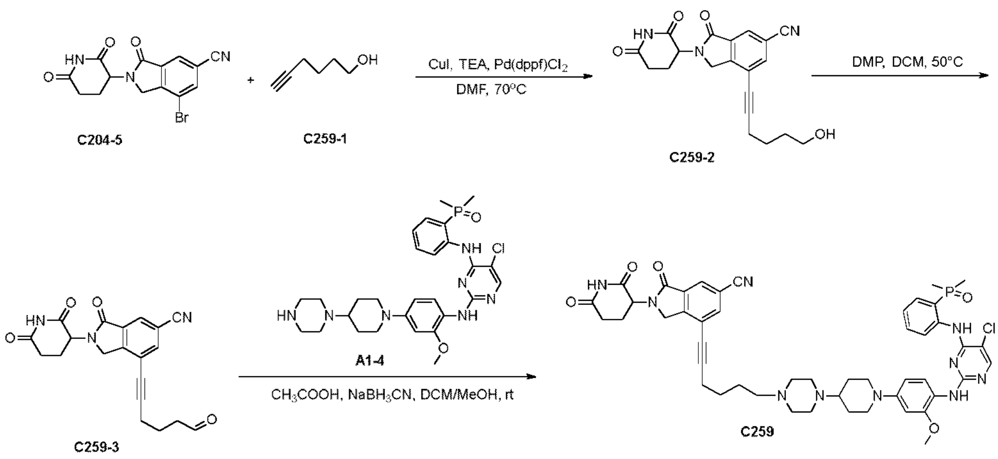


Step 1:

**C204-5** (173.5 mg, 0.5 mmol), CuI (19.0 mg, 0.1 mmol) and Pd(dppf)$Cl_2$ (73.1 mg, 0.1 mmol) were dispersed in 10.0 mL of anhydrous DMF. **C259-1** (122.5 mg, 2.5 mmol) and TEA (151.5 mg, 1.5 mmol) were added successively under $N_2$ protection. The mixture was heated to 70°C and reacted for 20 h. LCMS showed that the reaction was completed. The mixture was cooled to room temperature and directly purified by RP-Flash chromatography to afford light-yellow solid crude **C259-2** (210 mg, 100%). LCMS: $[M + H]^+ = 366$.

Step 2:

**C259-2** (210 mg, 0.575 mmol) was dissolved in 105 mL of anhydrous DCM. Dess-Martin reagent (439 mg, 1.035 mmol) was added under $N_2$ protection. The mixture was heated to 50°C, and reacted under reflux for 2.0 h. TLC showed that the reaction was completed. The mixture was cooled to room temperature, and then 20 mL of saturated $NaHCO_3$ solution and 20 mL of saturated $Na_2S_2O_3$ solution were added. The mixture was stirred at room temperature for 5 min. The organic layer was separated, dried with anhydrous sodium sulfate, and filtered with suction. The filtrate was concentrated under reduced pressure to afford a crude product. The crude product was purified by Prep-TLC to afford light-yellow solid product **C259-3** (130 mg, 62.5%). LCMS: $[M + H]^+ = 364$.

Step 3:

**C259-3** (120 mg, 0.33 mmol) and **A1-4** (160 mg, 0.28 mmol) were dissolved in a mixture of 8.0 mL of anhydrous DCM and 0.8 mL of anhydrous MeOH. $CH_3COOH$ (29.8 mg, 0.495 mmol) was added under nitrogen protection. The mixture was stirred at room temperature for 0.5h. Then solid $NaBH_3CN$ (41.5 mg, 0.66 mmol) was added. The mixture was further reacted at room temperature for 2h. LCMS and TLC showed that the reaction was completed. The mixture was purified by Prep-HPLC to afford white solid pure product **C259** (30 mg, yield: 9.93%). LCMS: $[M + H]^+ = 917$.

**Preparation of 3-(4-(7-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hept-1-yn-1-yl)-1-oxo-6-(trifluoromethyl)isoindolin-2-yl)piperidine-2,6-dione (C267)**

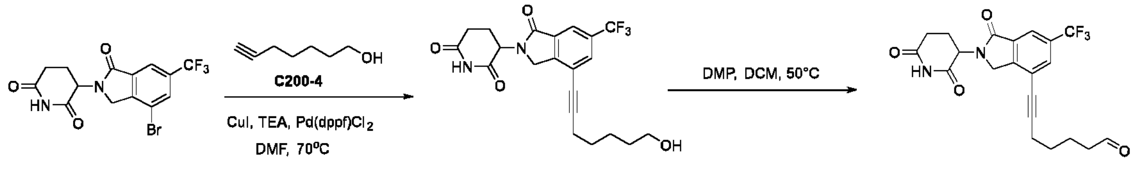


C267-1 C267-2 C267-3

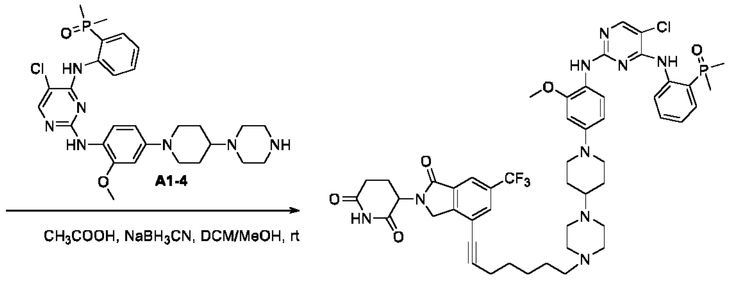


C267

Step 1:

Compound **C267-1** (or **C200-5**) (60mg, 0.154mmol), compound **C200-4** (43mg, 0.384mmol), cuprous iodide (2.9mg, 0.015mmol), $Pd(dppf)Cl_2$ (11.3mg, 0.015mmol) and triethylamine (46.6mg, 0.461mmol) were dissolved in 2 mL of anhydrous DMF. The mixture was stirred at 70°C under nitrogen protection overnight. The solvent was removed under reduced pressure. The crude product was purified by thin layer chromatography to afford compound **C267-2** (61mg, yield: 94.0%). LCMS: $[M + H]^+$ = 423.

Step 2:

Compound **C267-2** (81mg, 0.192mmol) and Dess-Martin reagent (163mg, 0.384mmol) were dissolved in 10 mL of dichloromethane and stirred at 50°C for 2h. The mixture was cooled to room temperature, and saturated sodium bicarbonate aqueous solution and saturated sodium thiosulfate aqueous solution were added successively and stirred for 5 minutes respectively to quench the reaction. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C267-3** (60mg, yield: 74.4%). LCMS: $[M + H]^+$ = 421.

Step 3:

Compound **267-3** (20mg, 0.048mmol) and compound **A1-4** (22mg, 0.038mmol) were dissolved in 2 mL of dichloromethane/methanol (2/1, v/v), and glacial acetic acid (2.9mg, 0.038mmol) was added dropwise. The mixture was stirred at room temperature under nitrogen protection for 1h. Then sodium cyanobohydride (2.4mg, 0.038mmol) was added. The mixture was further stirred for 1h. The solvent was removed under reduced pressure below 40°C, and the crude product was purified by Flash chromatography to afford off-white solid compound **C267** (12mg, yield: 46.3%). LCMS: $[M + H]^+$ = 974. $^1H$ NMR (400 MHz, DMSO-$d_6$) δ 11.17 (s, 1H), 11.04 (s, 1H), 8.48 (s, 1H), 8.06 (d,2H), 7.99-7.98 (m, 2H), 7.57-7.49 (m, 1H), 7.38 (d, 1H), 7.33 (d, 1H), 7.09 (t, 1H), 6.62 (d, 1H), 6.46 (dd, 1H), 5.32 (dd, 1H), 5.18 (dd,1H), 4.58 (d, 1H), 4.43 (d, 1H), 3.76 (s, 3H), 3.70 (d,2H), 2.93 (m, 1H), 2.69-2.60 (m, 4H), 2.52 (d,2H), 2.46 (m, 3H), 2.44-2.26 (m, 8H), 1.99 (m,4H), 1.82 (s, 1H), 1.78 (m, 3H), 1.75 (s, 3H), 1.60 (d, 2H), 1.46 (m, 3H).

**Preparation of 3-(4-(6-(4-(1-(4-((5-chloro-4-((2-(dimethylphosphoryl)phenyl)amino)pyrimidin-2-yl)amino)-3-methoxyphenyl)piperidin-4-yl)piperazin-1-yl)hex-1-yn-1-yl)-1-oxo-6-(trifluoromethyl)isoindolin-2-yl)piperidine-2,6-dione (C268)**

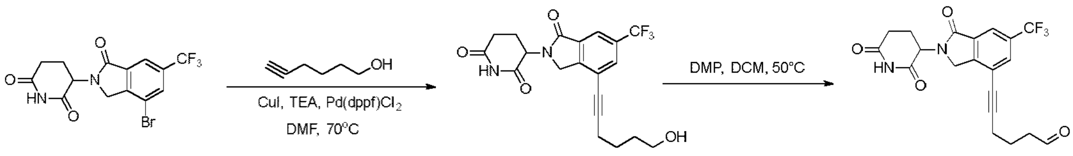


C268-1 C268-2 C268-3

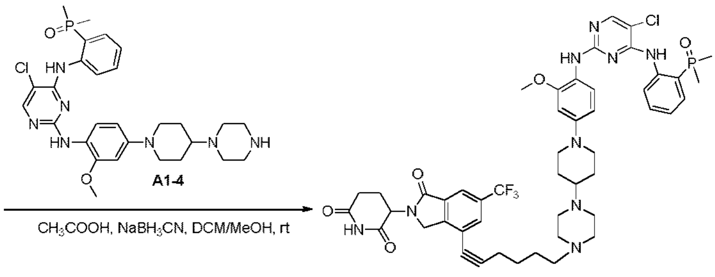


C268

Step 1:

Compound **C268-1** (or **C200-5**) (100mg, 0.256mmol), 5-alkynyl-1-hexanol (62.9mg, 0.641mmol), cuprous iodide (4.9mg, 0.026mmol), Pd(dppf)$Cl_2$ (18.8mg, 0.026mmol) and triethylamine (77.7mg, 0.769mmol) were dissolved in 3 mL of anhydrous DMF. The mixture was stirred at 70°C under nitrogen protection overnight. The solvent was removed under reduced pressure, and the crude product was purified by thin layer chromatography to afford compound **C268-2** (80mg, yield: 76.5%). LCMS: $[M + H]^+ = 409$.

Step 2:

Compound **C268-2** (126mg, 0.309mmol) and Dess-Martin reagent (262mg, 0.618mmol) were dissolved in 10 mL of dichloromethane and stirred at 50°C for 2h. The mixture was cooled to room temperature, and saturated sodium bicarbonate aqueous solution and saturated sodium thiosulfate aqueous solution were added successively and stirred for 5 minutes respectively to quench the reaction. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C268-3** (40mg, yield: 32.0%). LCMS: $[M + H]^+ = 407$.

Step 3:

Compound **C268-3** (39mg, 0.096mmol) and compound **A1-4** (38.3mg, 0.067mmol) were dissolved in 2 mL of dichloromethane/methanol (2/1, v/v), and glacial acetic acid (5.8mg, 0.096mmol) was added dropwise. The mixture was stirred at room temperature for 1h under nitrogen protection. Then sodium cyanobohydride (4.2mg, 0.067mmol) was added. The mixture was further stirred for 1h. The solvent was removed under reduced pressure below 40°C, and the crude product was purified by Flash chromatography to afford off-white solid compound **C268** (30mg, yield: 92.1%). LCMS: $[M + H]^+ = 960$. $^1$H NMR (400 MHz, DMSO-$d_6$) $\delta$ 11.17 (s, 1H), 11.04 (s, 1H), 8.48 (s, 1H), 8.06 (d,2H), 8.01-7.98 (m, 2H), 7.53 (m, 1H), 7.38 (d, 1H), 7.33 (d, 1H), 7.09 (t, 1H), 6.62 (d,1H), 6.47 (dd, 1H), 5.32(m, 1H), 5.18 (dd, 1H), 4.57 (d, 1H), 4.42 (d, 1H), 3.76 (s, 3H), 3.71 (d, 2H), 2.93 (m, 1H), 2.70-2.62 (m, 3H), 2.53 (d, 3H), 2.38 (m, 3H), 2.30 (s, 4H), 2.05-1.96 (m, 4H), 1.85 (d, 2H), 1.78 (s, 3H), 1.75 (s, 3H), 1.63 -1.58 (m, 4H), 1.53-1.46 (m, 2H).

**Preparation of 3-(4-(5-(4-(1-(4-((5-chloro-4-((2-(dimethylphosphoryl)phenyl)amino)pyrimidin-2-yl)amino)-3-methoxyphenyl)piperidin-4-yl)piperazin-1-yl)pent-1-yn-1-yl)-1-oxo-6-(trifluoromethyl)isoindolin-2-yl)piperidine-2,6-dione (C269)**

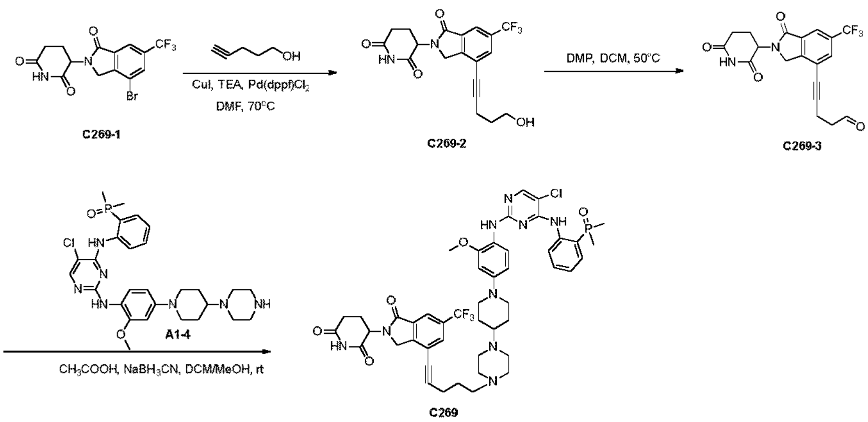


Step 1:

Compound **C269-1** (or **C200-5**) (60mg, 0.154mmol), 4-alkynyl-1-pentanol (32.3mg, 0.384mmol), cuprous iodide (2.9mg, 0.015mmol), Pd(dppf)$Cl_2$ (11.3mg, 0.015mmol) and triethylamine (46.6mg, 0.461mmol) were dissolved in 2 mL of anhydrous DMF. The mixture was stirred at 70°C under nitrogen protection overnight. The solvent was removed under reduced pressure, and the crude product was purified by thin layer chromatography to afford compound **C269-2** (58mg, yield: 95.8%). LCMS: $[M + H]^+$ = 395.

Step 2:

Compound **C269-2** (81mg, 0.206mmol) and Dess-Martin reagent (174mg, 0.410mmol) were dissolved in 10 mL of dichloromethane and stirred at 50°C for 2h. The mixture was cooled to room temperature, and saturated sodium bicarbonate aqueous solution and saturated sodium thiosulfate aqueous solution were added successively and stirred for 5 minutes respectively to quench the reaction. The organic phase was separated, and the aqueous phase was extracted with dichloromethane for three times. The organic phases were combined, dried with anhydrous sodium sulfate, and filtered. The filtrate was concentrated, and the crude product was purified by TLC to afford a white solid compound **C269-3** (60mg, yield: 74.4%). LCMS: $[M + H]^+$ = 393.

Step 3:

**C269-3** (31mg, 0.079mmol) and **A1-4** (36mg, 0.063mmol) were dissolved in 2 mL of dichloromethane/methanol (2/1, v/v), and glacial acetic acid (4.7mg, 0.079mmol) was added dropwise. The mixture was stirred at room temperature under nitrogen protection for 1h. Then sodium cyanobohydride (4.0mg, 0.063mmol) was added. The mixture was further stirred for 1h. The solvent was removed under reduced pressure below 40°C, and the crude product was purified by Flash chromatography to afford off-white solid compound **C269** (13mg, yield: 17.4%). LCMS: $[M + H]^+$ = 946. $^1$H NMR (400 MHz, DMSO-$d_6$) δ 11.17 (s, 1H), 11.04 (s, 1H), 8.48 (s, 1H), 8.06 (d, 2H), 8.01-7.98 (m, 2H), 7.53 (dd, 1H), 7.38 (d, 1H), 7.33 (d, 1H), 7.09 (t, 1H), 6.63 (d, 1H), 6.47 (dd, 1H), 5.32 (t, 1H), 5.19 (dd, 1H), 4.58 (d, 1H), 4.42 (d, 1H), 3.76 (s, 3H), 3.72 (d, 2H), 2.93 (m, 1H), 2.70-2.62 (m, 4H), 2.54 (m, 2H), 2.52 (d, 3H), 2.41 (m, 4H), 1.99 (dt, 5H), 1.86 (d, 2H), 1.78 (s, 3H), 1.74 (s, 3H), 1.55-1.45 (m, 3H).

**Preparation of 3-(4-(5-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)pentyl)-1-oxoisoindolin-2-yl)piperidine-2,6-dione (C285)**

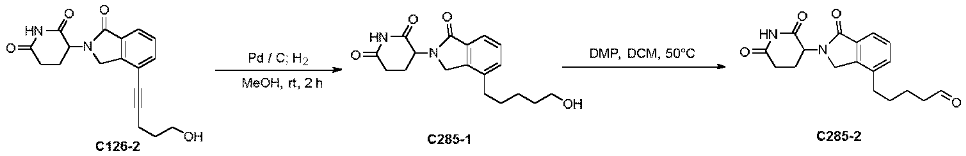


C126-2 C285-1 C285-2

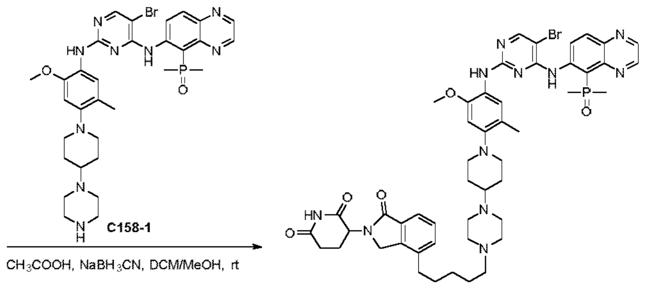


C158-1

C285

Step 1:

**C126-2** (250 mg, 0.767 mmol) was dissolved in 100.0 mL of anhydrous MeOH, and 10% Pd/C (125 mg) was added to the mixture. $H_2$ (0.4 atm) was bubbled into the mixture. The mixture was reacted at room temperature for 2h. LCMS showed that the reaction was completed. The mixture was filtered with suction to remove the solid catalyst. The solvent was removed from the filtrate under reduced pressure to afford white solid crude **C285-1** (230 mg, 92%). LCMS: $[M + H]^+ = 331$.

Step 2:

**C285-1** (230 mg, 0.697 mmol) was dissolved in 150 mL of anhydrous DCM. Dess-Martin reagent (591 mg, 1.394 mmol) was added under $N_2$ protection. The mixture was heated to 50°C, and reacted under reflux for 2.0 h. TLC showed that the reaction was completed. The mixture was cooled to room temperature, and then 30 mL of saturated $NaHCO_3$ solution and 30 mL of saturated $Na_2S_2O_3$ solution were added. The mixture was stirred at room temperature for 5 min. The organic layer was separated, dried with anhydrous sodium sulfate, and filtered with suction. The filtrate was concentrated under reduced pressure to afford a crude product. The crude product was purified by Flash chromatography to afford white solid product **C285-2** (210 mg, 91.3%). LCMS: $[M + H]^+ = 329$.

Step 3:

**C285-2** (49.2mg, 0.15 mmol) and **C158-1** (81.5 mg, 0.12 mmol) were dissolved in a mixture of 5.0 mL of anhydrous DCM and 0.5 mL of anhydrous MeOH. $CH_3COOH$ (13.5 mg, 0.225 mmol) was added under nitrogen protection. The mixture was stirred at room temperature for 0.5h. Then solid $NaBH_3CN$ (18.8 mg, 0.3 mmol) was added. The mixture was further reacted at room temperature for 2h. LCMS and TLC showed that the reaction was completed. The mixture was purified by Prep-HPLC to afford yellow solid pure product **C285** (38 mg, yield: 25.6%). LCMS: $[M + H]^+ = 992, 994$.

**Preparation of 3-(4-(6-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)hexyl)-1-oxoisoindolin-2-yl)piperidine-2,6-dione (C286)**

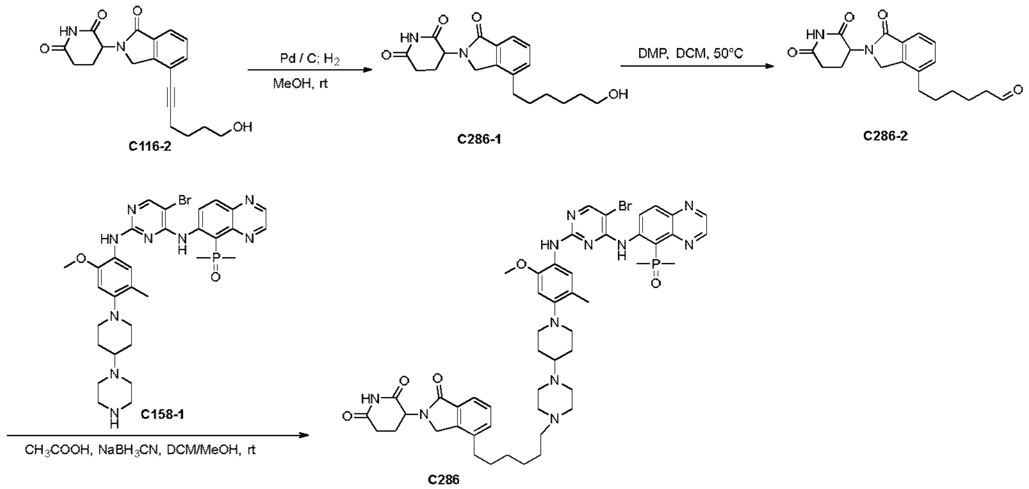


Step 1:

**C116-2** (150 mg, 0.441 mmol) was dissolved in 60.0 mL of anhydrous MeOH, and 10% Pd/C (150 mg) was added to the mixture. $H_2$ (0.4 atm) was bubbled into the mixture. The mixture was reacted at room temperature for 2h. LCMS showed that the reaction was completed. The mixture was filtered with suction to remove the solid catalyst. The solvent was removed from the filtrate under reduced pressure to afford white solid crude C286-1 (150 mg, 98.5%), which was directly used in the next step. LCMS: $[M + H]^+ = 345$.

Step 2:

**C286-1** (150 mg, 0.436 mmol) was dissolved in 100 mL of anhydrous DCM. Dess-Martin reagent (370 mg, 0.872 mmol) was added under $N_2$ protection. The mixture was heated to 50°C, and reacted under reflux for 2.0 h. TLC showed that the reaction was completed. The mixture was cooled to room temperature, and then 20 mL of saturated $NaHCO_3$ solution and 20 mL of saturated $Na_2S_2O_3$ solution were added. The mixture was stirred at room temperature for 5 min. The organic layer was separated, dried with anhydrous sodium sulfate, and filtered with suction. The filtrate was concentrated under reduced pressure to afford a crude product. The crude product was purified by Flash chromatography to afford white solid product **C286-2** (100 mg, 66.7%). LCMS: $[M + H]^+ = 343$.

Step 3:

**C286-2** (41 mg, 0.12 mmol) and **C158-1** (65.2 mg, 0.096 mmol) were dissolved in a mixture of 4.0 mL of anhydrous DCM and 0.4 mL of anhydrous MeOH. $CH_3COOH$ (10.8 mg, 0.18 mmol) was added under nitrogen protection. The mixture was stirred at room temperature for 0.5h. Then solid $NaBH_3CN$ (15.1 mg, 0.24 mmol) was added. The mixture was further reacted at room temperature for 2h. LCMS and TLC showed that the reaction was completed. The mixture was purified by Prep-HPLC to afford yellow solid pure product **C286** (20 mg, yield: 16.6%). LCMS: $[M + H]^+ =$ 1006, 1008.

**Preparation of 3-(4-(6-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)hexyl)-1-oxoisoindolin-2-yl)piperidine-2,6-dione (C299)**

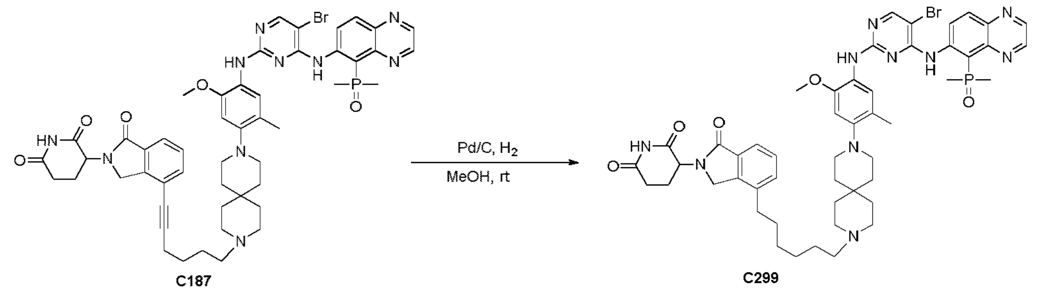


C187 C299

**C187** (20 mg, 0.02 mmol) was dissolved in 20.0 mL of MeOH, and 10% Pd/C (10 mg) was added. 0.4 atm of $H_2$ was bubbled into the system. The mixture was reacted at room temperature for 2h. LCMS showed that the reaction was completed. The mixture was filtered to remove the solid catalyst. The filtrate was concentrated to afford a crude product. The crude product was directly purified by RP-Flash chromatography to afford light-yellow solid crude product, which was further purified by Prep-HPLC to afford white solid product **C299** (2.5 mg, yield: 12.6%). LCMS: $[M + H]^+$ = 991, 993.

**Preparation of 3-(4-(4-(4-(1-(4-((5-bromo-4-((5-(dimethylphosphoryl)quinoxalin-6-yl)amino)pyrimidin-2-yl)amino)-5-methoxy-2-methylphenyl)piperidin-4-yl)piperazin-1-yl)butyl)-1-oxoisoindolin-2-yl)piperidine-2,6-dione (C301)**

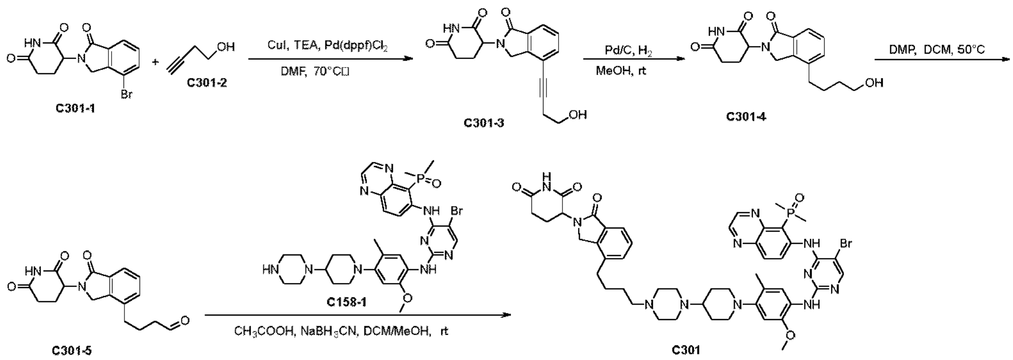


C301-1 C301-2 C301-3 C301-4 C301-5 C158-1 C301

Step 1:

**C301-1** (644 mg, 2.0 mmol), CuI (38 mg, 0.2 mmol) and $Pd(dppf)Cl_2$ (146.2 mg, 0.2 mmol) were dispersed in 20.0 mL of anhydrous DMF. **C301-2** (280.4 mg, 4.0 mmol) and TEA (606 mg, 6.0 mmol) were added successively under $N_2$ protection. The mixture was heated to 70°C and reacted for 20 h. LCMS showed that the reaction was completed. The mixture was cooled to room temperature and purified by RP-Flash chromatography to afford light-yellow solid crude **C301-3** (450 mg, 72.1%). LCMS: $[M + H]^+$ = 313.

Step 2:

**C301-3** (450 mg, 1.44 mmol) was dissolved in 90.0 mL of anhydrous MeOH, and 10% Pd/C (225 mg) was added to the mixture. $H_2$ (0.4 atm) was bubbled into the mixture. The mixture was reacted at room temperature for 2h. LCMS showed that the reaction was completed. The mixture was filtered with suction to remove the solid catalyst. The solvent was removed from the filtrate under reduced pressure to afford white solid crude **C301-4** (450 mg, 98.9%), and the crude product was directly used in the next step. LCMS: $[M + H]^+$ = 317.

Step 3:

**C301-4** (420 mg, 1.33 mmol) was dissolved in 150 mL of anhydrous DCM. Dess-Martin reagent (1010 mg, 2.39 mmol) was added under $N_2$ protection. The mixture was heated to 50°C, and reacted under reflux for 2.0 h. TLC showed that the reaction was completed. The mixture was cooled to room temperature, and then 30 mL of saturated

$NaHCO_3$ solution and 30 mL of saturated $Na_2S_2O_3$ solution were added. The mixture was stirred at room temperature for 5 min. The organic layer was separated, dried with anhydrous sodium sulfate, and filtered with suction. The filtrate was concentrated under reduced pressure to afford a crude product. The crude product was purified by Flash chromatography to afford white solid product **C301-5** (350 mg, 83.3%). LCMS: $[M + H]^+$ = 315.

Step 4:

**C301-5** (47.1 mg, 0.15 mmol) and **C158-1** (81.5 mg, 0.12 mmol) were dissolved in a mixture of 5.0 mL of anhydrous DCM and 0.5 mL of anhydrous MeOH. $CH_3COOH$ (13.5 mg, 0.225 mmol) was added under nitrogen protection. The mixture was stirred at room temperature for 0.5h. Then solid $NaBH_3CN$ (18.8 mg, 0.3 mmol) was added. The mixture was further reacted at room temperature for 2h. LCMS and TLC showed that the reaction was completed. The mixture was purified by RP-Flash to afford yellow solid pure product **C301** (35 mg, yield: 23.8%). LCMS: $[M + H]^+$ = 978, 980.

**Preparation of 3-(4-(4-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)butyl)-1-oxoisoindolin-2-yl)piperidine-2,6-dione (C302)**

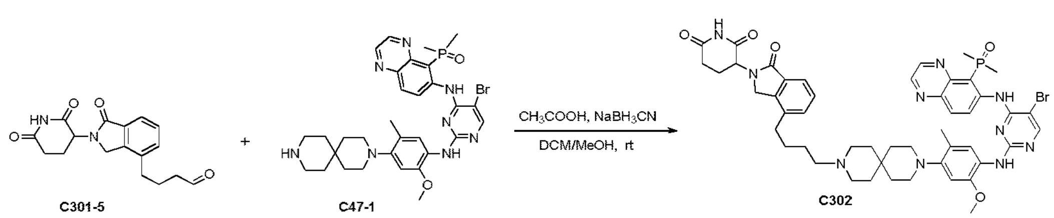


C301-5 C47-1 C302

**C301-5** (47.1 mg, 0.15 mmol) and **C47-1** (79.7 mg, 0.12 mmol) were dissolved in a mixture of 5.0 mL of anhydrous DCM and 0.5 mL of anhydrous MeOH. $CH_3COOH$ (13.5 mg, 0.225 mmol) was added under nitrogen protection. The mixture was stirred at room temperature for 0.5h. Then solid $NaBH_3CN$ (18.8 mg, 0.3 mmol) was added. The mixture was further reacted at room temperature for 2h. LCMS and TLC showed that the reaction was completed. The mixture was directly purified by Prep-TLC to afford a crude product, which was further purified by RP-Flash to afford yellow solid pure product **C302** (35 mg, yield: 24.3%). LCMS: $[M + H]^+$ = 963, 965.

**Preparation of 3-(4-(3-(2-(9-(4-((5-bromo-4-((5-(dimethylphosphoryl)quinoxalin-6-yl)amino)pyrimidin-2-yl)amino)-5-methoxy-2-methylphenyl)-3,9-diazaspiro[5.5]undecan-3-yl)ethoxy)prop-1-yn-1-yl)-6-fluoro-1-oxoisoindolin-2-yl)piperidine-2,6-dione (C310)**

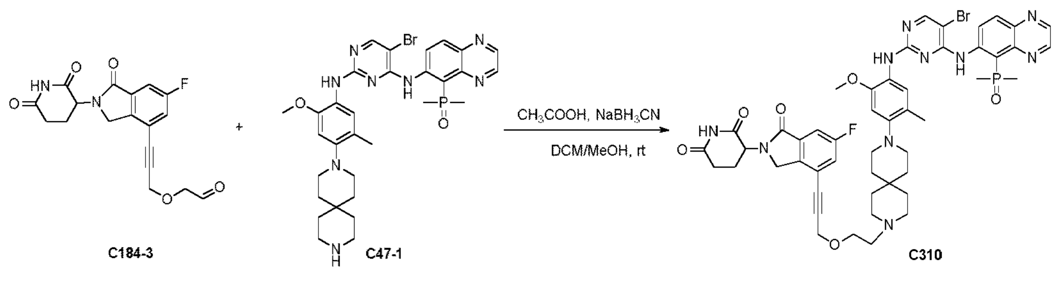


C184-3 C47-1 C310

**C184-3** (40 mg, 0.112 mmol) and **C47-1** (55.6 mg, 0.084 mmol) were dissolved in a mixture of 5.0 mL of anhydrous DCM and 0.5 mL of anhydrous methanol. $CH_3COOH$ (10.08 mg, 0.168 mmol) was added under nitrogen protection. The mixture was stirred at room temperature for 0.5h. Then solid $NaBH_3CN$ (14.1 mg, 0.224 mmol) was added. The mixture was further reacted at room temperature for 2h. LCMS and TLC showed that the reaction was completed. The mixture was purified by Prep-HPLC to afford yellow solid pure product **C310** (21 mg, yield: 18.8%). LCMS: $[M + H]^+$ = 1007, 1009. $^1$H-NMR: (400 MHz, DMSO-$d_6$) δ 11.026 (s, 1H), 8.865 (dd, *J* = 2.0 Hz, 9.6 Hz, 3H), 8.265 (s, 2H), 7.938 (d, *J* = 9.6 Hz, 1H), 7.676-7.611 (m, 3H), 7.324 (s, 1H), 6.810 (s, 1H), 5.187 (dd, *J* = 5.2Hz, 13.2 Hz, 1H), 4.470 (s, 3H), 4.360 (d, *J* = 17.6 Hz, 1H), 3.782 (s, 3H), 3.677 (t, *J* = 5.6 Hz, 2H), 2.991-2.913 (m, 1H),

2.8891-2.784 (m, 4H), 2.681-2.503 (m, 4H), 2.460-2.426 (m, 5H), 2.076 (s, 3H), 2.040 (s, 3H), 2.004 (s, 3H), 1.561-1.521 (m, 8H).

**EGFR inhibitory activity assay**

1. Assay method

(1) A compound stock solution was prepared and diluted 3x to obtain a compound dilution; 10 nL of the compound dilution was transferred to a 384-well plate (784075, Greiner) by Echo 550;
(2) The plate was sealed, and centrifuged at 1,000 g for 1 min;
(3) 2x $EGFR^{Del19/T790M/C797S}$ and $EGFR^{L858R/T790M/C797S}$ protein working solutions were prepared with 1x kinase buffer, respectively;
(4) 5 μl of the 2x EGFR protein working solution was added to the 384-well plate from step (2), centrifuged at 1,000 g for 30 s, and allowed to stand at room temperature (mixed thoroughly) for 10 min;
(5) A mixture of 2x TK-substrate-biotin (2 μM) and ATP was prepared with 1x Kinase buffer;
(6) 5μL of the TK-substrate-biotin and ATP (the mixture prepared in step (5)) was added to the 384-well plate from step (4) to initiate the reaction;
(7) The mixture was centrifuged at 1,000 g for 30 s; the plate was sealed, and allowed to stand (and reacted) at room temperature for 40 min;
(8) 4X Sa-XL 665 and TK-antibody-Cryptate were prepared with detection buffer;
(9) 5μL of the Sa-XL 665 and 5μL of the TK-antibody-Cryptate were added successively to the 384-well plate from step (7);
(10) The mixture was centrifuged at 1,000 g for 30 s, and allowed to stand (reacted) at room temperature for 1h;
(11) The fluorescence values were read at 615 nm and 665 nm by enzyme labeling instrument (PerkinElmer, 74785).

2. Data analysis

The ratio (665/615) of each well was calculated.
Formula of inhibition rate (%):

$$\text{Inhibition}(\%) = \left[1 - \frac{\text{Ratio}_{cmpd} - \overline{\text{Ratio}_{positive}}}{\overline{\text{Ratio}_{vehicle}} - \overline{\text{Ratio}_{positive}}}\right] \times 100$$

$\text{Ratio}_{cmpd}$: Ratio (665/615) value of assay compound.
$\overline{\text{Ratio}_{positive}}$ : Average ratio (665/615) value of positive control.
$\overline{\text{Ratio}_{vehicle}}$ : Average ratio (665/615) value of negative control.
The nonlinear regression curve (dose response-variable slope) between the value of inhibition rate (%) and the logarithm of compound concentration was fitted with graphpad prism 8.0, the effect dose curve of compound was drawn, and the $IC_{50}$ value was calculated.

3. Assay result

The $IC_{50}$ values of the inhibitory activity of the compound of the present disclosure on mutant $EGFR^{Del19/T790M/C797S}$ and $EGFR^{L8S8R/T790M/C797S}$ are shown in the table below.

| Compound No. | $IC_{50}^{\#}$ (nM) | |
|---|---|---|
| | $EGFR^{Del19/T790M/C797S}$ | $EGFR^{L858R/T790M/C797S}$ |
| A1 | B | A |
| A2 | B | A |
| A3 | A | A |
| A6 | | A |

(continued)

| Compound No. | $IC_{50}$# (nM) | |
|---|---|---|
| | $EGFR^{Del19/T790M/C797S}$ | $EGFR^{L858R/T790M/C797S}$ |
| A8 | A | A |
| A10 | A | A |
| A11 | A | A |
| A12 | | A |
| A13 | | B |
| A17 | A | A |
| A18 | A | A |
| C1 | | A |
| C4 | | B |
| C6 | | B |
| C25 | | B |
| C78 | | A |
| C88 | | A |
| C93 | | A |
| C94 | | A |
| C99 | | A |
| C101 | | A |
| C103 | | C |
| C104 | | A |
| C106 | | A |
| C107 | | A |
| C109 | | A |
| C110 | | A |
| C112 | | A |
| C116 | A | A |
| C148 | | A |
| C153 | A | A |
| C164 | | A |
| C167 | A | A |
| C176 | | A |
| C177 | A | A |
| C178 | A | A |
| C187 | A | A |
| C192 | A | A |
| Osimertinib | C | D |
| #Note: A represents $IC_{50}$ of 1-100 nM, B represents $IC_{50}$ of 101-200 nM, C represents $IC_{50}$ of 201-300 nM, and D represents $IC_{50}$ of >300 nM. | | |

Conclusion: the compounds of the present disclosure have strong inhibitory activity on mutant $EGFR^{Del19/T790M/C797S}$ and $EGFR^{L8S8R/T790M/C797S}$.

**Assay of activity on Ba/F3($EGFR^{Del19/T790M/C797S}$) and Ba/F3($EGFR^{L858R/T790M/C797S}$) cells**

1. Assay method

(1) Ba/F3($EGFR^{Del19/T790M/C797S}$) and Ba/F3($EGFR^{L8S8R/T790M/C797S}$) cells were respectively cultured according to the requirements of ATCC, incubated in an incubator at 37°C and 5% $CO_2$, and analyzed by index; cells with viability of > 90% can be used in the assay, and cells were seeded in a 384-well plate (PerkinElmer, 6007680) with 700 cells/well, 30 μL/well.
(2) A compound stock solution was prepared, and then diluted 3x to obtain a compound dilution. 30 nL of the compound dilution was added to a 384-well plate by Echo (Labcyte, Echo550). The cells were incubated in an incubator at 37°C and 5% $CO_2$ for 72h.
(3) 30 μL of CTG was added to each well, and the 384-well plate was shaken on a Plate shaker (QILINBEIER, QB-9002). The 384-well plate was incubated at 37°C and 5% $CO_2$ in the dark for 30 min, and the chemiluminescence value was read by Envision (PerkinElmer, EnVision 2104).

2. Data analysis

The percent inhibition rate (% inhibition) was calculated by the following formula

$$\text{Inhibition(\%)} = \left[1 - \frac{LUM_{cmpd} - \overline{LUM}_{positive}}{\overline{LUM}_{vehicle} - \overline{LUM}_{positive}}\right] \times 100$$

$LUM_{cmpd}$: Luminescence value of assay compound.
$\overline{LUM}_{positive}$: Average LUM value of positive drug at a concentration of 10 μM.
$\overline{LUM}_{vehicle}$: Average LUM value of negative control group without drug treatment.
The nonlinear regression curve (dose response-variable slope) between the value of inhibition rate (%) and the logarithm of compound concentration was fitted with graphpad prism 8.0, the effect dose curve of compound was drawn, and the $IC_{50}$ value was calculated.

$$Y = Bottom + (Top\text{-}Bottom)/(1+10^{\wedge}((LogIC_{50}\text{-}X)^{*}HillSlope))$$

X-axis: logarithm of compound concentration; Y axis: inhibition rate (% inhibition).

3. Assay result

The IC50 values of the inhibitory activity of the compound of the present disclosure on Ba/F3($EGFR^{Del19/T790M/C797S}$) and Ba/F3($EGFR^{L858R/T790M/C797S}$) cells are shown in the table below.

| Compound No. | $IC_{50}^{\#}$ (nM) | |
|---|---|---|
| | Ba/F3($EGFR^{Del19/T790M/C797S}$) | Ba/F3($EGFR^{L858R/T790M/C797S}$) |
| A1 | | C |
| A2 | | C |
| A10 | A | A |
| A11 | A | B |
| A12 | | A |
| A13 | | B |

(continued)

| Compound No. | $IC_{50}$# (nM) | |
|---|---|---|
| | Ba/F3(EGFR$^{Del19/T790M/C797S}$) | Ba/F3(EGFR $^{L858R/T790M/C797S}$) |
| C1 | | A |
| C4 | | B |
| C6 | | C |
| C25 | | C |
| C47 | B | B |
| C78 | | D |
| C88 | | D |
| C93 | | D |
| C94 | | D |
| C99 | | D |
| C101 | | D |
| C103 | | |
| C104 | | D |
| C106 | | D |
| C107 | | B |
| C109 | | D |
| C110 | | D |
| C116 | A | |
| C126 | A | |
| C148 | | D |
| C150 | | D |
| C153 | A | A |
| C158 | A | B |
| C162 | D | D |
| C163 | A | |
| C164 | A | |
| C166 | A | B |
| C167 | | A |
| C169 | A | A |
| C171 | A | |
| C172 | A | |
| C173 | A | |
| C174 | A | A |
| C176 | | A |
| C177 | A | |
| C178 | A | |
| C179 | A | |

(continued)

| Compound No. | $IC_{50}$# (nM) | |
|---|---|---|
| | Ba/F3(EGFR$^{Del19/T790M/C797S}$) | Ba/F3(EGFR $^{L858R/T790M/C797S}$) |
| C181 | A | |
| C182 | A | |
| C183 | A | A |
| C184 | A | |
| C185 | A | |
| C186 | A | |
| C187 | A | |
| C188 | A | |
| C189 | A | |
| C190 | A | |
| C191 | A | |
| C192 | A | |
| C193 | A | |
| C194 | A | |
| C195 | A | |
| C196 | A | |
| C200 | B | |
| C202 | A | |
| C204 | A | |
| C211 | A | |
| C212 | A | |
| C213 | A | A |
| C214 | A | A |
| C215 | A | |
| C218 | A | |
| C227 | A | |
| C228 | A | |
| C229 | A | |
| C235 | A | A |
| C236 | A | |
| C238 | A | |
| C240 | A | |
| C243 | A | |
| C244 | A | A |
| C245 | A | |
| C246 | A | A |
| C258 | A | |

(continued)

| Compound No. | $IC_{50}$# (nM) | |
|---|---|---|
| | Ba/F3(EGFR$^{Del19/T790M/C797S}$) | Ba/F3(EGFR $^{L858R/T790M/C797S}$) |
| C259 | A | |
| C267 | A | |
| C268 | A | |
| C269 | A | |
| C285 | A | A |
| C286 | A | |
| C299 | A | A |
| C301 | A | |
| C302 | A | |
| C310 | A | |
| A1-4 | D | D |
| C47-1 | | B |
| C158-1 | A | |
| Osimertinib | D | D |
| #Note: A represents $IC_{50}$ of 1-100 nM, B represents $IC_{50}$ of 101-200 nM, C represents $IC_{50}$ of 201-300 nM, and D represents $IC_{50}$ of >300 nM. | | |

Conclusion: The compounds of the present disclosure have strong inhibitory activity on mutant Ba/F3(EGFR$^{Del19/T790M/C797S}$) and Ba/F3(EGFR$^{L858R/T790M/C797S}$) cells.

**Assay of activity of compounds to induce EGFR$^{L858R/T790M/C797S}$ and EGFR$^{Del19/T790M/C797S}$ protein degradation**

In order to further explain the reason why the compounds of the present disclosure have inhibitory activity on Ba/F3(EGFR$^{L858R/T790M/C797S}$) and Ba/F3(EGFR$^{Del19/T790M/C797S}$) cells, representative compounds C176 and C213 were selected to study the mechanism of action of the compounds, and observe their effects on EGFR$^{L858R/T790M/C797S}$ and EGFR$^{Del19/T790M/C797S}$ protein levels.

(1) Cell culture:

Ba/F3(EGFR$^{L858R/T790M/C797S}$) and Ba/F3(EGFR$^{Del19/T790M/C797S}$) cells were cultured according to the culture conditions recommended by ATCC, and analyzed by index.
Complete medium: 1640 medium, 10% FBS, 1x glutamine, 1x penicillin-streptomycin.
Culture conditions: incubated at 37°C, 95% air, 5% $CO_2$ incubator.

(2) Compound stock solution: 10 mM stock solution in DMSO, stored at -20°C.
(3) Preparation of cell suspension:
The cells in the cell culture bottle were collected, and the cells with viability of > 90% can be used in the assay. Cells were seeded in a 96-well plate with 40 μL cells, 1*$10^5$ cells/well.
(4) Compound treatment:
Compound was diluted 3x with DMSO, starting at 1.0 mM (for EGFR$^{L858R/T790M/C797S}$ assay) and 5 mM (for EGFR$^{Del19/T790M/C797S}$ assay), respectively, into 10 concentrations to prepare working solutions.
(5) Compound was pipetted into the 96-well plate to treat cells in a 37°C, 95% air, 5% $CO_2$ incubator for 24h.
(6) Detection:

1) 1 μg/mL of EGF activated cells were treated for 10 min;
2) After the compound treatment, lysis buffer was added to lyse cells; 10 μL of cell lysate was transferred to a

384-well plate; in addition, control lysate and negative control plus 5 μL of acceptor mix were added to the 384-well plate, and shaken on a shaker for 1-2 min;
3) 5 μL of donor mix was added to each well; the 384-well plate was sealed, shaken on a shaker for 1-2 min, left in the dark at room temperature overnight, and read with an enzyme labeling instrument.

(7) Data analysis:

Alpha counts were fitted by logarithmic treatment of compound concentration with Graphpad Prism 8.0.

$$Y=Bottom + (Top-Bottom)/(1+10^((LogIC_{50}-X)*HillSlope))$$

X: logarithm of compound concentration; Y: Alpha Counts.

(8) Assay result:

The effects of the compounds of the present disclosure on mutant $EGFR^{L858R/T790M/C797S}$ and $EGFR^{Del19/T790M/C797S}$ protein levels are shown in Figures 1 and 2.
As shown in FIG. 1, the assay results show that in the range of 0.01 nM to 1000 nM, $EGFR^{L858R/T790M/C797S}$ protein level decreases with the increase of the concentration of compound C176, and compound C176 significantly reduces $EGFR^{L858R/T790M/C797S}$ protein level with a $DC_{50}$ of 31.37 nM, which proves that the compounds of the present disclosure have significant degradation effect on $EGFR^{L858R/T790M/C797S}$ protein in a dose-dependent manner.

As shown in FIG. 2, the assay results show that in the range of 0.1 nM to 10000 nM, $EGFR^{Del19/T790M/C797S}$ protein level decreases with the increase of the concentration of compound C213, and compound C213 significantly reduces $EGFR^{Del19/T790M/C797S}$ protein level with a $DC_{50}$ of 11.28 nM, which proves that the compounds of the present disclosure have significant degradation effect on $EGFR^{Del19/T790M/C797S}$ protein in a dose-dependent manner.
Conclusion: the compounds of the present disclosure can significantly induce the degradation of $EGFR^{Del19/T790M/C797S}$ and $EGFR^{L858R/T790M/C797S}$ proteins in cells in a dose-dependent manner.

**Assay of activity on ALK kinase**

1. Assay method

(1) A compound stock solution was prepared;
(2) The compound stock solution was diluted 3x to obtain a compound dilution;
(3) 100 nL of the compound dilution was transferred to ProxiPlate-384 Plus (Perkin Elmer, Perkin Elmer), in duplicates, by Echo 550;
(4) 2x ALK and $ALK^{G1202R}$ kinase working solutions were prepared with 1x kinase buffer;
(5) 5 μl of the 2x kinase working solution was added to the reaction plate, centrifuged at 1,000 rpm for 1 min, and incubated at 25°C for 15 min;
(6) A mixture of 2x TK-substrate-biotin (2μM) and ATP was prepared with 1x Kinase buffer;
(7) 5μl of the STK-substrate-biotin and ATP (the mixture prepared in step (6)) was added to initiate the reaction;
(8) The mixture was centrifuged at 1,000g for 30s; the plate was sealed, and incubated at 25°C for 60 min;
(9) 4X Sa-XL 665 and TK-antibody-Cryptate were prepared with detection buffer;
(10) 5μL of the Sa-XL 665 and 5μL of the TK-antibody-Cryptate were added successively;
(11) The mixture was centrifuged at 1,000g for 1 min, and incubated at 25°C for 60 min;
(12) The fluorescence values were read at 615 nm and 665 nm by enzyme labeling instrument (PerkinElmer, 74785).

2. Data analysis

The ratio (665/615) value of each well was calculated.
Formula of inhibition rate (%):

$$\text{Inhibition}(\%) = \left[1-\frac{\text{Ratio}_{cmpd} - \overline{\text{Ratio}_{positive}}}{\overline{\text{Ratio}_{vehicle}} - \overline{\text{Ratio}_{positive}}}\right] \times 100$$

$\text{Ratio}_{cmpd}$: Ratio (665/615) value of assay compound.

$\overline{\text{Ratio}_{positive}}$ : Average ratio (665/615) value of positive control.

$\overline{\text{Ratio}_{vehicle}}$ : Average ratio (665/615) value of negative control.

The nonlinear regression curve (dose response-variable slope) between the value of inhibition rate (%) and the logarithm of compound concentration was fitted with graphpad prism 8.0, the effect dose curve of compound was drawn, and the $IC_{50}$ value was calculated.

$$Y = \text{Bottom} + (\text{Top-Bottom})/(1+10\text{^}((\text{LogIC}_{50}\text{-X})*\text{HillSlope}))$$

X-axis: logarithm of compound concentration; Y axis: inhibition rate (% inhibition)

3. Assay result

The $IC_{50}$ values of the inhibitory activity of the compounds of the present disclosure on ALK and $ALK^{G1202R}$ are shown in the table below.

| Compound No. | $IC_{50}$# (nM) | |
|---|---|---|
| | ALK | $ALK^{G1202R}$ |
| A1 | B | |
| A2 | A | |
| A10 | A | B |
| A11 | A | B |
| A12 | A | B |
| A13 | A | D |
| C1 | A | |
| C4 | A | B |
| C6 | A | A |
| C93 | A | A |
| C94 | A | A |
| C103 | A | D |
| C106 | A | C |
| C107 | A | B |
| C109 | A | A |
| C116 | A | C |
| C153 | A | C |
| C167 | A | C |
| C177 | A | C |
| C178 | A | C |
| C190 | A | C |
| C192 | A | C |

(continued)

| Compound No. | $IC_{50}$# (nM) | |
|---|---|---|
| | ALK | $ALK^{G1202R}$ |
| C193 | A | C |
| C194 | A | D |
| Crizotinib | A | D |
| Repotrectinib | A | B |
| #Note: A represents $IC_{50}$ of 1-100 nM, B represents $IC_{50}$ of 101-200 nM, C represents $IC_{50}$ of 201-300 nM, and D represents $IC_{50}$ of >300 nM. | | |

Conclusion: the compounds of the present disclosure have strong inhibitory activity on ALK and $ALK^{G1202R}$.

**Assay of activity on Ba/F3(EML4-ALK) cells**

1. Assay method

(1) Ba/F3(EML4-ALK) cells were cultured according to the requirements of ATCC, incubated in an incubator at 37°C and 5% $CO_2$, and analyzed by index; cells with viability of > 90% can be used in the assay, and cells were seeded in a 384-well plate (PerkinElmer, 6007680) with 700 cells/well, 30μl/well.
(2) A compound stock solution was prepared, and diluted 3x to obtain a compound dilution; 30 nL of the serial dilution was added to a 384-well plate (784075, Greiner) by Echo 550, and cells were incubated in an incubator at 37°C and 5% $CO_2$ for 72h.
(3) 30 μL of CTG was added to each well, and the 384-well plate was shaken on a Plate shaker (QILINBEIER, QB-9002); the 384-well plate was incubated in the dark at 37°C and 5% $CO_2$ for 30 min, and the chemiluminescence value was read by Envision (PerkinElmer, EnVision 2104).

2. Data analysis

The percent inhibition rate (% inhibition) was calculated by the formula below

$$\text{Inhibition}(\%) = \left[1 - \frac{LUM_{cmpd} - \overline{LUM}_{positive}}{\overline{LUM}_{vehicle} - \overline{LUM}_{positive}}\right] \times 100$$

$LUM_{cmpd}$: Luminescence value of assay compound.
$\overline{LUM}_{positive}$: Average LUM value of positive drug at a concentration of 10 μM.
$\overline{LUM}$vehicle. Average LUM value of negative control group without drug treatment.
The nonlinear regression curve (dose response-variable slope) between the value of inhibition rate (%) and the logarithm of compound concentration was fitted with graphpad prism 8.0, the effect dose curve of compound was drawn, and the $IC_{50}$ value was calculated.

$$Y = Bottom + (Top-Bottom)/(1+10^((LogIC_{50}-X)*HillSlope))$$

X-axis: logarithm of compound concentration; Y axis: inhibition rate (% inhibition).

3. Assay result

The $IC_{50}$ values of the inhibitory activity of the compounds of the present disclosure on Ba/F3(EML4-ALK) cells are shown in the table below.

| Compound No. | $IC_{50}$# (nM) |
|---|---|
| | Ba/F3(EML4-ALK) |
| A10 | A |
| A11 | A |
| A12 | A |
| A13 | A |
| C1 | A |
| C47 | B |
| C116 | A |
| C148 | A |
| C153 | A |
| C158 | C |
| C163 | A |
| C164 | A |
| C167 | A |
| C171 | B |
| C172 | A |
| C173 | A |
| C176 | A |
| C177 | A |
| C178 | A |
| C181 | A |
| C182 | A |
| C183 | A |
| C184 | A |
| C186 | A |
| C187 | A |
| C188 | D |
| C189 | B |
| C190 | A |
| C191 | B |
| C192 | A |
| C193 | A |
| C194 | A |
| C195 | A |
| C196 | A |
| C211 | A |
| C214 | A |
| C310 | A |

(continued)

| Compound No. | $IC_{50}$# (nM) |
|---|---|
| | Ba/F3(EML4-ALK) |
| Crizotinib | A |
| [4]Note: A represents $IC_{50}$ of 1-100 nM, B represents $IC_{50}$ of 101-200 nM, C represents $IC_{50}$ of 201-300 nM, and D represents $IC_{50}$ of >300 nM. | |

Conclusion: the compounds of the present disclosure have strong inhibitory activity on Ba/F3(EML4-ALK) cells.

The above is a further detailed description of the present disclosure in connection with the specific alternative embodiments, and the specific embodiments of the present disclosure are not limited to the description. It will be apparent to those skilled in the art that the present disclosure may be practiced by making various simple deduction and replacement, without departing from the spirit and scope of the invention.

## Claims

1. A compound of general formula (X), or a pharmaceutically acceptable salt, enantiomer, diastereomer, racemate, solvate, hydrate, polymorph, prodrug, or isotopic variant thereof, or mixture thereof:

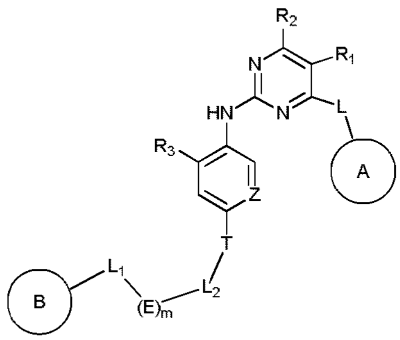

(X)

wherein

ring A is selected from the following optionally substituted groups: $C_{3-7}$ cycloalkyl, 4- to 8-membered heterocyclyl, $C_{6-10}$ aryl or 5- to 10-membered heteroaryl, wherein the substituent is selected from halogen, -CN, $C_{1-6}$ alkyl, $C_{1-6}$ haloalkyl, $C_{2-6}$ alkenyl, $C_{3-7}$ cycloalkyl, 4- to 8-membered heterocyclyl, -P(O)($C_{1-6}$ alkyl)$_2$, -P(O)($C_{2-6}$ alkenyl)$_2$, -O-$C_{1-6}$ alkyl, -O-$C_{1-6}$ haloalkyl, -O-$C_{2-6}$ alkenyl, -O-$C_{3-7}$ cycloalkyl, -O-4- to 8-membered heterocyclyl, -NH-$C_{1-6}$ alkyl, -NH-$C_{2-6}$ alkenyl, -NH-$C_{3-7}$ cycloalkyl, -NH-4- to 8-membered heterocyclyl, -C(O)-$C_{1-6}$ alkyl, -C(O)-$C_{2-6}$ alkenyl, -C(O)-$C_{3-7}$ cycloalkyl, -C(O)-4- to 8-membered heterocyclyl, - S(O)$_2$-$C_{1-6}$ alkyl, -S(O)$_2$-$C_{2-6}$ alkenyl, -S(O)$_2$-$C_{3-7}$ cycloalkyl, -S(O)$_2$-4- to 8-membered heterocyclyl, -NHC(O)-$C_{1-6}$ alkyl, -NHC(O)-$C_{2-6}$ alkenyl, -NHC(O)-$C_{3-7}$ cycloalkyl, - NHC(O)-4- to 8-membered heterocyclyl, -NHS(O)$_2$-$C_{1-6}$ alkyl, -NHS(O)$_2$-$C_{2-6}$ alkenyl, - NHS(O)z-$C_{3-7}$ cycloalkyl or -NHS(O)$_2$-4- to 8-membered heterocyclyl;

ring B is selected from the following group:

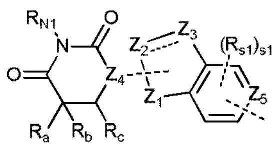

;

 represents single bond or double bond;

 represents that the point of attachment to the rest of the molecule can be located at the available point of

the ring;

$Z_1$ is O, S, N or C atom, which is optionally substituted with one or two $R_{Z1}$; or $Z_1$ is absent, and thus $Z_4$ is connected to $Z_2$, $Z_3$ or the C atom connected to $Z_1$ on the aromatic ring, and the $Z_2$ and the C atom on the aromatic ring that are connected to $Z_1$ are connected to Rw respectively; or $Z_1$, $Z_2$ and $Z_3$ are all absent, and thus $Z_4$ is connected to one of the C atoms connected to $Z_1$ or $Z_3$ on the aromatic ring, and the other C atom on the aromatic ring is connected to Rw;

$Z_2$ is O, S, N or C atom, which is optionally substituted with one or two $R_{Z2}$;

$Z_3$ is O, S, N or C atom, which is optionally substituted with one or two $R_{Z3}$; with the proviso that when - - - - represents double bond, $Z_2$ is N or C atom, and $Z_3$ is N or C atom;

$Z_4$ is N or $CR_{Z4}$;

$Z_5$ is N or $CR_{Z5}$;

$R_a$, $R_b$ and $R_c$ are independently H, halogen, $-(CH_2)_{0-5}$-OR", $-(CH_2)_{0-5}$-NR"R‴, $C_{1-6}$ alkyl or $C_{1-6}$ haloalkyl; or $R_a$ and $R_b$ are taken together with the carbon atom to which they are attached to form C=O, $C_{3-7}$ cycloalkyl or 4- to 8-membered heterocyclyl; or $R_a$ and $R_c$ are taken together with the carbon atoms to which they are attached to form $C_{3-7}$ cycloalkyl or 4-to 8-membered heterocyclyl; or $R_a$ and $R_c$ are taken together to form bond;

$R_{N1}$ is H, $C_{1-6}$ alkyl or $C_{1-6}$ haloalkyl, alternatively H;

Rzi is absent, H, CN, halogen, $-(CH_2)_{0-5}$-OR", $-(CH_2)_{0-5}$-NR"R‴, $C_{1-6}$ alkyl, $C_{1-6}$ haloalkyl, $-(CH_2)_{0-5}$-$C_{3-7}$ cycloalkyl or $-(CH_2)_{0-5}$-4- to 8-membered heterocyclyl; or two $R_{Z1}$ are taken together with $Z_1$ to form C=O, $C_{3-7}$ cycloalkyl or 4- to 8-membered heterocyclyl;

$R_{Z2}$ is absent, H, CN, halogen, $-(CH_2)_{0-5}$-OR", $-(CH_2)_{0-5}$-NR"R‴, $C_{1-6}$ alkyl, $C_{1-6}$ haloalkyl, $-(CH_2)_{0-5}$-$C_{3-7}$ cycloalkyl or $-(CH_2)_{0-5}$-4- to 8-membered heterocyclyl; or two $R_{Z2}$ are taken together with $Z_2$ to form C=O, $C_{3-7}$ cycloalkyl or 4- to 8-membered heterocyclyl;

$R_{Z3}$ is absent, H, CN, halogen, $-(CH_2)_{0-5}$-OR", $-(CH_2)_{0-5}$-NR"R‴, $C_{1-6}$ alkyl, $C_{1-6}$ haloalkyl, $-(CH_2)_{0-5}$-$C_{3-7}$ cycloalkyl or $-(CH_2)_{0-5}$-4- to 8-membered heterocyclyl; or two $R_{Z3}$ are taken together with $Z_3$ to form C=O, $C_{3-7}$ cycloalkyl or 4- to 8-membered heterocyclyl;

$R_{Z4}$ is H, CN, halogen, $-(CH_2)_{0-5}$-OR", $-(CH_2)_{0-5}$-NR"R‴, $C_{1-6}$ alkyl or $C_{1-6}$ haloalkyl;

$R_{Z5}$ is H, CN, halogen, $-(CH_2)_{0-5}$-OR", $-(CH_2)_{0-5}$-NR"R‴, $C_{1-6}$ alkyl, $C_{1-6}$ haloalkyl, - $(CH_2)_{0-5}$-$C_{3-7}$ cycloalkyl or $-(CH_2)_{0-5}$-4- to 8-membered heterocyclyl;

or the ring in which $Z_4$ is located is absent;

wherein $R_w$ is H, CN, halogen, $-(CH_2)_{0-5}$-OR", $-(CH_2)_{0-5}$-NR"R‴, -$C_{1-6}$ alkyl, $C_{1-6}$ haloalkyl, $-(CH_2)_{0-5}$-$C_{3-7}$ cycloalkyl, $-(CH_2)_{0-5}$-4- to 8-membered heterocyclyl, $C_{2-6}$ alkenyl, $C_{2-6}$ alkynyl, $-(CH_2)_{0-5}$-$C_{3-10}$ halocycloalkyl, $-(CH_2)_{0-5}$-$C_{6-10}$ aryl or $-(CH_2)_{0-5}$-5- to 14-membered heteroaryl;

R" is H, $C_{1-6}$ alkyl, $C_{1-6}$ haloalkyl or $-(CH_2)_{0-5}$-$C_{3-7}$ cycloalkyl;

R‴ is H, $C_{1-6}$ alkyl or $C_{1-6}$ haloalkyl;

$L_1$ is selected from bond, -O-, $-S(O)_p-$, -S(O)(=NR*)-, $-NR^{\#}-$, $-CR^{\#}R^{\#'}-$, $-C_aR^{\#}R^{\#'}-C_bR^{\#}R^{\#'}-$, -N=S(O)(R*)- or -S(O)(R*)=N-;

$L_2$ is selected from bond, -O-, $-S(O)_p-$, -S(O)(=NR*)-, $-NR^{\#}-$, $-CR^{\#}R^{\#'}-$, $-C_aR^{\#}R^{\#'}-C_bR^{\#}R^{\#'}-$, -N=S(O)(R*)- or -S(O)(R*)=N-;

wherein one of $C_aR^{\#}R^{\#'}$ or $C_bR^{\#}R^{\#'}$ can be replaced by O, $S(O)_p$, S(O)(=NR*) or $NR^{\#}$, and when one of $C_aR^{\#}R^{\#'}$ or $C_bR^{\#}R^{\#'}$ is replaced by O, S or $NR^{\#}$, the other of $C_aR^{\#}R^{\#'}$ or $C_bR^{\#}R^{\#'}$ can also be replaced by $S(O)_q$;

E is independently selected from: bond, $-C_cR^{\#}R^{\#'}-C_dR^{\#}R^{\#'}-C_eR^{\#}R^{\#'}$,

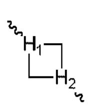,
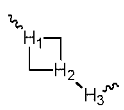,

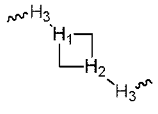,
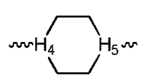
or
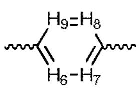;

wherein one of $C_cR^{\#}R^{\#'}$, $C_dR^{\#}R^{\#'}$ or $C_eR^{\#}R^{\#'}$, or both of $C_cR^{\#}R^{\#'}$ and $C_eR\#R\#'$ can be replaced by O, $S(O)_p$, S(O)(=NR*) or $NR^{\#}$, and when one of $C_cR^{\#}R^{\#'}$, $C_dR^{\#}R^{\#'}$ or $C_eR\#R\#'$ is replaced by O, S or $NR^{\#}$, the other adjacent one or two of $C_cR^{\#}R^{\#'}$, $C_dR^{\#}R^{\#'}$ or $C_eR\#R\#'$ can also be replaced by $S(O)_q$;

or two E moieties can be taken together to form $-CH_2CH_2OCH_2CH_2-$, - $OCH_2CH_2CH_2CH_2-$, $-CH_2CH_2CH_2CH_2O-$,

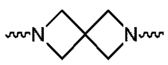, 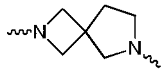, 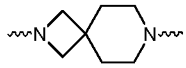,

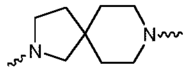, 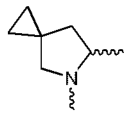 or 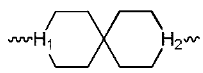;

wherein ~~~ represents the point of attachment to $L_1$ or $L_2$;

$H_1$ and $H_2$ are N or C atom, $H_3$ is O, S, N or C atom, and $H_1$ and $H_3$, and $H_2$ and $H_3$ are not heteroatoms at the same time;

$H_4$ and $H_5$ are N or C atom;

$H_6$, $H_7$, $H_8$ and $H_9$ are C or N atom;

p is 0, 1 or 2;

q is 1 or 2;

R* is H, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl or 5- to 14-membered heteroaryl;

$R^{\#}$ is H, halogen, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl or 5- to 14-membered heteroaryl;

$R^{\#'}$ is H, halogen, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl or 5- to 14-membered heteroaryl;

or, $R^{\#}$ and $R^{\#}$ on adjacent atoms can be taken together to form bond, and $R^{\#'}$ and $R^{\#'}$ on adjacent atoms can be taken together to form bond;

or, $R^{\#}$ and $R^{\#'}$ on the same or different atoms can be taken together to form =O, or $C_{3-7}$ cycloalkyl, 4- to 8-membered heterocyclyl, $C_{6-10}$ aryl or 5- to 6-membered heteroaryl, wherein the $C_{3-7}$ cycloalkyl, 4- to 8-membered heterocyclyl, $C_{6-10}$ aryl or 5- to 6-membered heteroaryl is optionally substituted with $R_x$, wherein the $R_x$ is H, CN, halogen, $C_{1-6}$ alkyl or $C_{1-6}$ haloalkyl;

m is 0, 1, 2, 3, 4, 5, 6, 7, 8, 9 or 10;

$R_{s1}$ is selected from H, CN, halogen, $-(CH_2)_{0-5}$-OR", $-(CH_2)_{0-5}$-NR"R‴, $C_{1-6}$ alkyl, $C_{1-6}$ haloalkyl, $-(CH_2)_{0-5}$-4- to 8-membered heterocyclyl, $C_{2-6}$ alkenyl, $C_{2-6}$ alkynyl, $-(CH_2)_{0-5}-C_{3-7}$ cycloalkyl, $-(CH_2)_{0-5}-C_{3-10}$ halocycloalkyl, $-(CH_2)_{0-5}-C_{6-10}$ aryl, $-(CH_2)_{0-5}$-5- to 14-membered heteroaryl, $-C(O)R_W$, $-S(O)R_W$ or $-S(O)_2R_W$;

s1 is 0, 1, 2 or 3;

R' is selected from H, $-C(O)-C_{1-6}$ alkyl, $-C(O)-C_{1-6}$ haloalkyl, $-C(O)-C_{2-6}$ alkenyl or - $C(O)-C_{6-10}$ aryl;

L is bond, -O- or -NR-;

wherein R is H or $C_{1-6}$ alkyl;

Z is -N= or $-C(R_4)=$;

T is selected from bond, $C_{2-6}$ heteroalkylene, 4- to 12-membered heterocyclylene, or 5-to 6-membered heterocyclylene, wherein the 5- to 6-membered heterocyclylene is substituted with 5- to 6-membered heterocyclylene;

$R_1$ is selected from H, halogen, cyano, $C_{1-6}$ alkyl, $C_{3-7}$ cycloalkyl, 4- to 8-membered heterocyclyl, $C_{1-6}$ haloalkyl, $C_{2-6}$ alkenyl or $C_{2-6}$ alkynyl;

or, when L is -NR-, $R_1$ and R are taken together with the atoms to which they are attached to form optionally substituted 5- to 6-membered heterocyclyl, wherein the substituent is selected from halogen, oxo, $C_{1-6}$ alkyl, $C_{1-6}$ haloalkyl, $C_{6-10}$ aryl, wherein the $C_{6-10}$ aryl is mono- or poly-substituted with halogen;

$R_2$ is H, halogen, hydroxyl, amino, $C_{1-6}$ alkyl or $C_{1-6}$ haloalkyl;

or $R_1$ and $R_2$ are taken together with the atoms to which they are attached to form 5- to 6-membered heterocyclyl or 5- to 6-membered heteroaryl;

$R_3$ is selected from H, $-O-C_{1-6}$ alkyl or $-O-C_{1-6}$ haloalkyl;

$R_4$ is selected from H, halogen, $C_{1-6}$ alkyl, $C_{1-6}$ haloalkyl, $-NHC(O)-C_{1-6}$ alkyl or - $NHC(O)-C_{2-6}$ alkenyl;

if the above groups are H or H-containing groups, the one or more H atom(s) may be substituted with D atom(s);

the groups containing OH, NH, $NH_2$, CH, $CH_2$, or $CH_3$ in Li, E, $L_2$, and T, or the above alkyl, alkylene, haloalkyl, alkenyl, alkynyl, cycloalkyl, halocycloalkyl, heterocyclyl, aryl, and heteroaryl are each optionally substituted with 1, 2, 3 or more $R^s$ or isotopic variants thereof at each occurrence, wherein the $R^s$ is independently selected from the following groups at each occurrence: halogen, hydroxyl, amino, cyano, nitro, $C_{1-6}$ alkyl, $C_{1-6}$ haloalkyl, $C_{2-6}$ alkenyl, $C_{2-6}$ alkynyl, $C_{3-10}$ cycloalkyl, $C_{3-10}$ halocycloalkyl, 3- to 10-membered heterocyclyl, $C_{6-10}$ aryl, 5- to 14-membered heteroaryl, $C_{6-12}$ aralkyl, $-OR^{a'}$, $-OC(O)R^{a'}$, $-C(O)R^{a'}$, - $C(O)OR^{a'}$, $-C(O)NR^{a'}R^{b'}$, $-S(O)_nR^{a'}$,

$-S(O)_nOR^{a'}$, $-S(O)_nNR^{a'}R^{b'}$, $-NR^{a'}R^{b'}$, $-NR^{a'}C(O)R^{b'}$, $-NR^{a'}-C(O)OR^{b'}$, $-NR^{a'}-S(O)_n-R^{b'}$, $-NR^{a'}C(O)NR^{a'}R^{b'}$, $-C_{1-6}$ alkylene-$R^{a'}$, $-C_{1-6}$ alkylene-$OR^{a'}$, $-C_{1-6}$ alkylene-$OC(O)R^{a'}$, $-C_{1-6}$ alkylene-$C(O)OR^{a'}$, $-C_{1-6}$ alkylene-$S(O)_nR^{a'}$, $-C_{1-6}$ alkylene$S(O)_nOR^{a'}$, $-C_{1-6}$ alkylene-$OC(O)NR^{a'}R^{b'}$, $-C_{1-6}$ alkylene-$C(O)NR^{a'}R^{b'}$, $-C_{1-6}$ alkylene-$NR^{a'}-C(O)NR^{a'}R^{b'}$, $-C_{1-6}$ alkylene-$OS(O)_nR^{a'}$, $-C_{1-6}$ alkylene-$S(O)_nNR^{a'}R^{b'}$, $-C_{1-6}$ alkylene-$NR^{a'}-S(O)_nNR^{a'}R^{b'}$, $-C_{1-6}$ alkylene-$NR^{a'}R^{b'}$ and $-O-C_{1-6}$ alkylene-$NR^{a'}R^{b'}$, and wherein the hydroxyl, amino, alkyl, alkylene, cycloalkyl, heterocyclyl, aryl, heteroaryl and aralkyl described with respect to the substituent $R^s$ are further optionally substituted with 1, 2, 3 or more substituents independently selected from the following groups or an isotopic variant thereof: halogen, OH, amino, cyano, nitro, $C_{1-6}$ alkyl, $C_{1-6}$ haloalkyl, $C_{1-6}$ alkyl hydroxyl, $C_{3-6}$ cycloalkyl, 3- to 10-membered heterocyclyl, $C_{6-10}$ aryl, 5- to 14-membered heteroaryl and $C_{6-12}$ aralkyl;
n is independently 1 or 2 at each occurrence;
each of $R^{a'}$ and $R^{b'}$ is independently selected from H, $C_{1-6}$ alkyl, $C_{2-6}$ alkenyl, $C_{2-6}$ alkynyl, $C_{1-6}$ alkyl-O-, $C_{1-6}$ alkyl-S-, $C_{3-10}$ cycloalkyl, 3- to 10-membered heterocyclyl, $C_{6-10}$ aryl, 5- to 14-membered heteroaryl and $C_{6-12}$ aralkyl at each occurrence.

**2.** The compound of general formula (X), or the pharmaceutically acceptable salt, enantiomer, diastereomer, racemate, solvate, hydrate, polymorph, prodrug, or isotopic variant thereof, or mixture thereof according to claim 1, which has the structure of general formula (I):

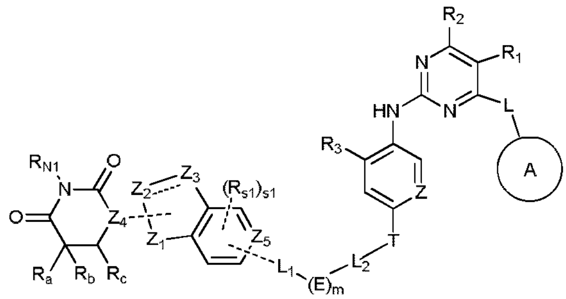
(I)

wherein each group is as defined in claim 1.

**3.** The compound of general formula (I), or the pharmaceutically acceptable salt, enantiomer, diastereomer, racemate, solvate, hydrate, polymorph, prodrug, or isotopic variant thereof, or mixture thereof according to claim 2, wherein ring A is the following groups:

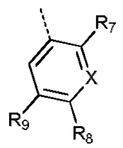
,
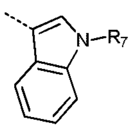
, or
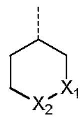
;

wherein

$R_7$ is selected from $C_{1-6}$ alkyl, $C_{1-6}$ haloalkyl, $C_{2-6}$ alkenyl, $C_{3-7}$ cycloalkyl, $-P(O)(C_{1-6}\ alkyl)_2$ or $-P(O)(C_{2-6}\ alkenyl)_2$;
$R_8$ is selected from H, $-NH-C_{1-6}$ alkyl, $-NH-C_{2-6}$ alkenyl, $-NH-C_{3-7}$ cycloalkyl, -NH-4- to 8-membered heterocyclyl, $-NHC(O)-C_{1-6}$ alkyl, $-NHC(O)-C_{2-6}$ alkenyl, $-NHC(O)-C_{3-7}$ cycloalkyl, -NHC(O)-4- to 8-membered heterocyclyl, $-NHS(O)_2-C_{1-6}$ alkyl or $-NHS(O)_2-C_{3-7}$ cycloalkyl;
$R_9$ is selected from H, halogen, $C_{1-6}$ alkyl, -CN, $C_{1-6}$ haloalkyl, $-O-C_{1-6}$ alkyl, $-O-C_{1-6}$ haloalkyl, $C_{3-7}$ cycloalkyl, $-O-C_{3-7}$ cycloalkyl, $-NH-C_{1-6}$ alkyl, $-NH-C_{2-6}$ alkenyl, $-NH-C_{3-7}$ cycloalkyl, -NH-4- to 8-membered heterocyclyl, $-NHC(O)-C_{1-6}$ alkyl, $-NHC(O)-C_{2-6}$ alkenyl, $-NHC(O)-C_{3-7}$ cycloalkyl, -NHC(O)-4- to 8-membered heterocyclyl, $-NHS(O)_2-C_{1-6}$ alkyl or $-NHS(O)_2-C_{3-7}$ cycloalkyl;
X is $-C(R_x)=$ or -N=;
wherein $R_x$ is H, or $R_x$ and $R_8$ are taken together with the C atoms to which they are attached to form 5- to 6-membered heterocyclyl or 5- to 6-membered heteroaryl; alternatively, $R_x$ and $R_8$ are taken together with the C atoms to which they are attached to form 5- to 6-membered heteroaryl, alternatively pyrazinyl;
$X_1$ is $-CH(R_{X1})-$ or $-N(R_{X1})-$;
$X_2$ is $-CH(R_{X2})-$ or $-N(R_{X2})-$;

wherein Rxi is selected from H, $C_{1-6}$ alkyl, $C_{3-7}$ cycloalkyl, -O-$C_{1-6}$ alkyl, -O-$C_{3-7}$ cycloalkyl, -NH-$C_{1-6}$ alkyl, -NH-$C_{3-7}$ cycloalkyl, -$S(O)_2$-$C_{1-6}$ alkyl, -$S(O)_2$-$C_{3-7}$ cycloalkyl, - NHS$(O)_2$-$C_{1-6}$ alkyl, -NHS$(O)_2$-$C_{3-7}$ cycloalkyl, -C(O)-$C_{1-6}$ alkyl, -C(O)-$C_{2-6}$ alkenyl, -C(O)-$C_{3-7}$ cycloalkyl, -NHC(O)-$C_{1-6}$ alkyl, -NHC(O)-$C_{3-7}$ cycloalkyl or -C(O)-4- to 8-membered heterocyclyl; $R_{X2}$ is selected from H, $C_{1-6}$ alkyl, $C_{3-7}$ cycloalkyl, -O-$C_{1-6}$ alkyl, -O-$C_{3-7}$ cycloalkyl, -NH-$C_{1-6}$ alkyl, -NH-$C_{3-7}$ cycloalkyl, -C(O)-$C_{1-6}$ alkyl, -C(O)-$C_{3-7}$ cycloalkyl, - C(O)-4- to 8-membered heterocyclyl, -$S(O)_2$-$C_{1-6}$ alkyl, -$S(O)_2$-$C_{3-7}$ cycloalkyl, -NHS$(O)_2$-$C_{1-6}$ alkyl, -NHS$(O)_2$-$C_{3-7}$ cycloalkyl, -NHC(O)-$C_{1-6}$ alkyl, -NHC(O)-$C_{2-6}$ alkenyl, -NHC(O)-$C_{3-7}$ cycloalkyl or -NHC(O)-4- to 8-membered heterocyclyl;
----- represents the point of attachment to L.

**4.** The compound of general formula (I), or the pharmaceutically acceptable salt, enantiomer, diastereomer, racemate, solvate, hydrate, polymorph, prodrug, or isotopic variant thereof, or mixture thereof according to claim 2 or 3, wherein T is
bond,

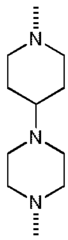 , 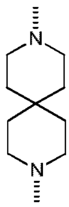 , 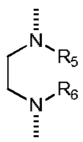 , 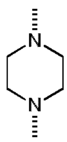 , 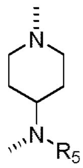 or 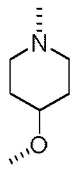 ;

wherein

$R_5$ is H or $C_{1-6}$ alkyl;
$R_6$ is H or $C_{1-6}$ alkyl;
or $R_5$ and $R_6$ are connected to form $C_{1-6}$ alkylene;
'''''' represents the point of attachment to parent core or $L_2$.

**5.** The compound of general formula (I), or the pharmaceutically acceptable salt, enantiomer, diastereomer, racemate, solvate, hydrate, polymorph, prodrug, or isotopic variant thereof, or mixture thereof according to any one of claims 2-4, wherein
when L is -NR-, $R_1$ and R are taken together to form the following groups: - C(O)N($R_N$)C(O)- or -C($C_{1-6}$ alkyl)=C($R_N$)C(O)-;
wherein

$R_N$ is selected from $C_{1-6}$ alkyl or

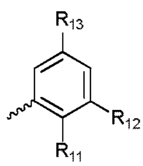 ;

$R_{11}$ is H or halogen;
$R_{12}$ is H or halogen;
$R_{13}$ is H or halogen;

~~~~ represents the point of attachment.

**6.** The compound of general formula (I), or the pharmaceutically acceptable salt, enantiomer, diastereomer, racemate, solvate, hydrate, polymorph, prodrug, or isotopic variant thereof, or mixture thereof according to any one of claims 2-5, wherein $R_1$ and $R_2$ are taken together with the atoms to which they are attached to form 5- to 6-membered heteroaryl; alternatively form pyrrolyl.

**7.** The compound of general formula (I), or the pharmaceutically acceptable salt, enantiomer, diastereomer, racemate,
~~~~

solvate, hydrate, polymorph, prodrug, or isotopic variant thereof, or mixture thereof according to claim 2, wherein ring A is selected from the following optionally substituted groups: $C_{3-7}$ cycloalkyl, 4- to 8-membered heterocyclyl, $C_{6-10}$ aryl or 5- to 10-membered heteroaryl, wherein the substituent is selected from -F, -Cl, -Br, -Me, -OMe, $-CF_3$, $-OCF_3$, -CN, -NHMe, cyclopropyl, $-P(O)Me_2$, $-NHC(O)CH_2CH_3$, $-C(O)CH=CH_2$, $-NHS(O)_2CH_2CH_3$, -NH-cyclopropyl, $-NHC(O)CH=CH_2$ or $-C(O)CH_2CH_3$; ring A is alternatively the following groups:

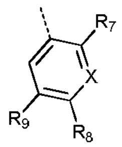 , 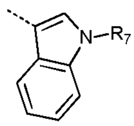 or 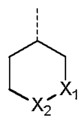 ;

wherein

$R_7$ is selected from -Me, cyclopropyl or $-P(O)Me_2$;
$R_8$ is selected from H, -NHMe, $-NHC(O)CH_2CH_3$ or -NH-cyclopropyl;
$R_9$ is selected from H, -F, -Cl, -Br, -Me, $-CF_3$, -OMe, $-OCF_3$, -CN, $-NHC(O)CH_2CH_3$, - $NHS(O)_2CH_2CH_3$, $-NHC(O)CH=CH_2$ or -NH-cyclopropyl;
X is $-C(R_x)=$ or -N=;
wherein $R_x$ is H, or $R_x$ and $R_8$ are taken together with the C atoms to which they are attached to form 5- to 6-membered heterocyclyl or 5- to 6-membered heteroaryl; alternatively 5- to 6-membered heteroaryl (alternatively pyrazinyl);
$X_1$ is $-CH_2-$ or $-N(R_{x1})-$;
$X_2$ is $-CH(R_{X2})-$ or $-N(R_{X2})-$;
wherein $R_{X1}$ is $-C(O)CH_2CH_3$ or $-C(O)CH=CH_2$; $R_{X2}$ is H, -Me, -OMe, -NHMe, - $C(O)CH_2CH_3$, $-NHS(O)_2CH_2CH_3$ or $-NHC(O)CH_2CH_3$;
----- represents the point of attachment to L;
T is selected from bond, $C_{2-6}$ heteroalkylene, 4- to 12-membered heterocyclylene, or 5-to 6-membered heterocyclylene, wherein the 5- to 6-membered heterocyclylene is substituted with 5- to 6-membered heterocyclylene; or is alternatively the following groups:

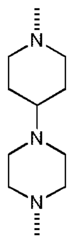 , 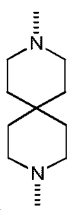 , 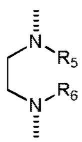 , 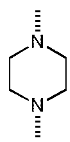 , 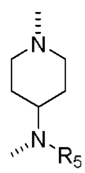 or 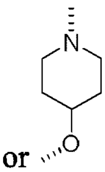 ;

wherein

$R_5$ is -Me;
$R_6$ is -Me;
or $R_5$ and $R_6$ are connected to form $-CH_2CH_2-$;

'''''' represents the point of attachment to parent core or $L_2$;
$R_1$ is selected from H, -Cl, -Br, $-CH_3$, $CF_3$, cyclopropyl or $-CH=CH_2$;
or, when L is -NR-, $R_1$ and R are taken together with the atoms to which they are attached to form optionally substituted 5- to 6-membered heterocyclyl, wherein the substituent is selected from halogen, oxo, -iPr, -Et, or phenyl, wherein the phenyl is mono- or poly-substituted with halogen; $R_1$ and R are alternatively taken together to form the following groups: $-C(O)N(R_N)C(O)-$ or $-C(CH_3)=C(R_N)C(O)-$;
wherein

$R_N$ is selected from -iPr, -Et or

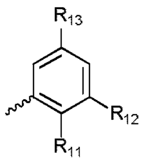 ;

$R_{11}$ is -Cl or -Br;
$R_{12}$ is H or -F;
$R_{13}$ is H or -F;

~~~ represents the point of attachment;
$R_2$ is H;
or $R_1$ and $R_2$ are taken together with the atoms to which they are attached to form 5- to 6-membered heterocyclyl or 5- to 6-membered heteroaryl; alternatively form pyrrolyl;
$R_3$ is selected from H or -OMe;
$R_4$ is selected from H, -F, -Me, $-CF_3$ or $-NHC(O)CH=CH_2$;
other groups are as defined in claim 2.

**8.** The compound of general formula (I), or the pharmaceutically acceptable salt, enantiomer, diastereomer, racemate, solvate, hydrate, polymorph, prodrug, or isotopic variant thereof, or mixture thereof according to any one of claims 2-7, wherein the compound of general formula (I) has the structure of the following general formulas:

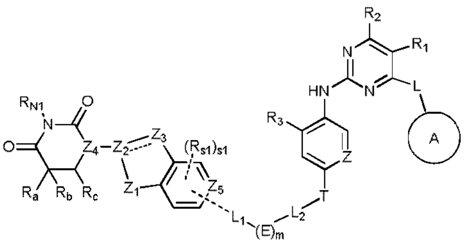 (I-A), 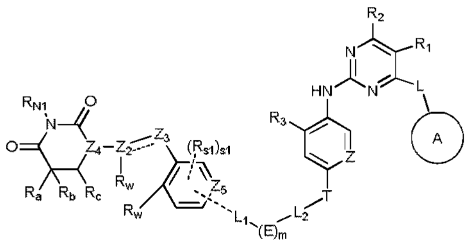 (I-B),

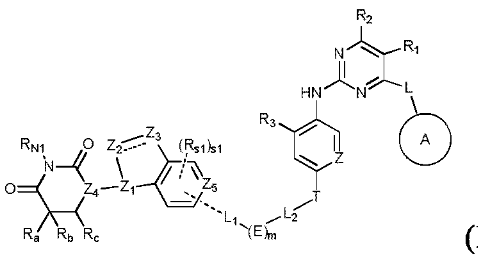 (I-C), 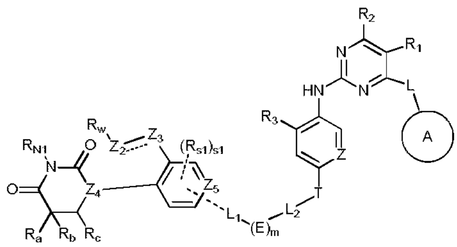 (I-D),

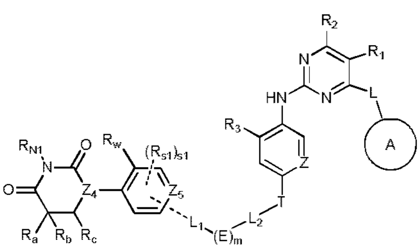 (I-E), 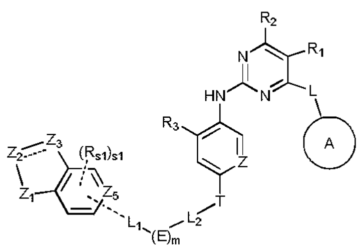 (I-F),
~~~

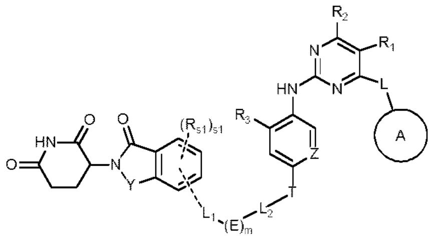

(I-G),

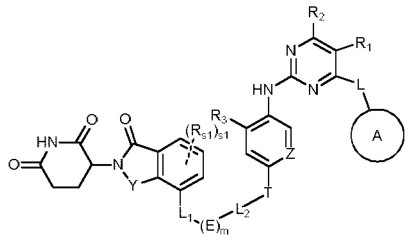

(I-H),

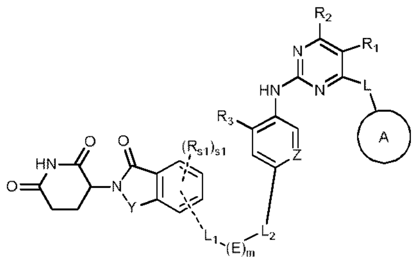

(I-I),

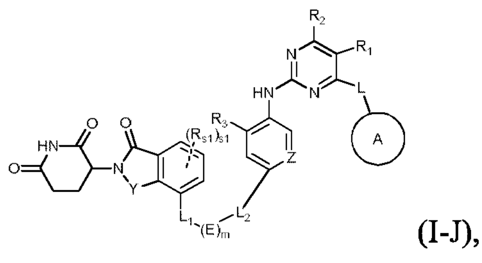

(I-J),

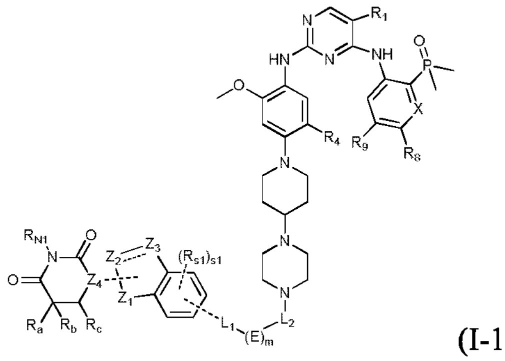

(I-1),

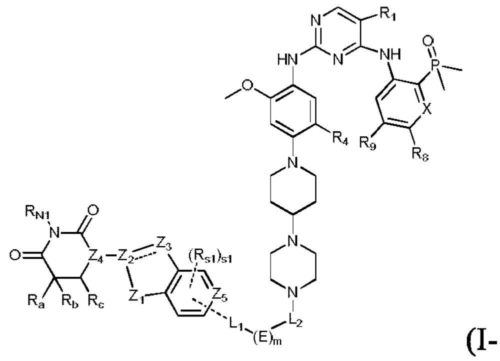

(I-1-A),

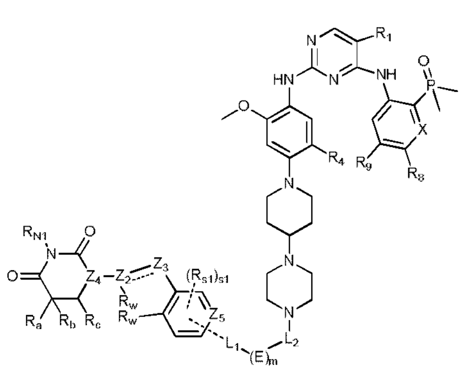

(I-1-B),

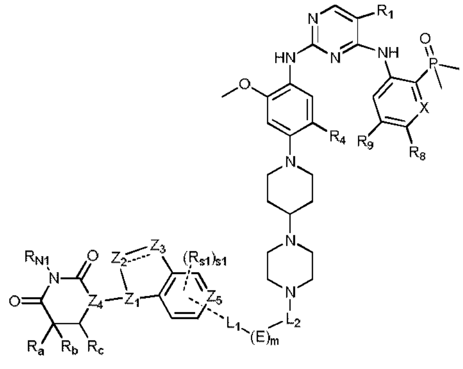

(I-1-C),

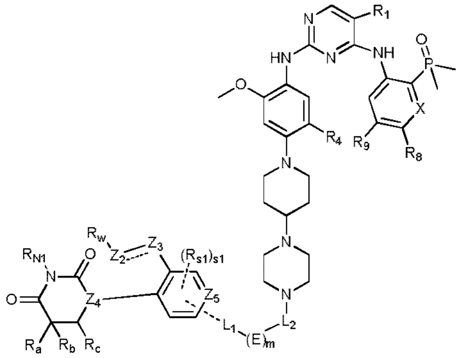

(I-1-D),

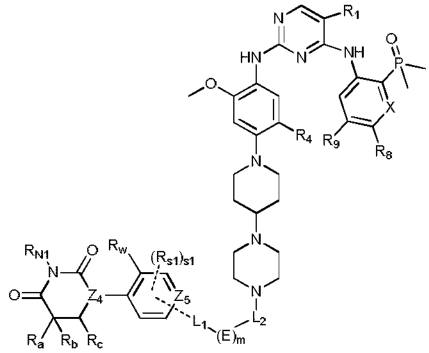

(I-1-E),

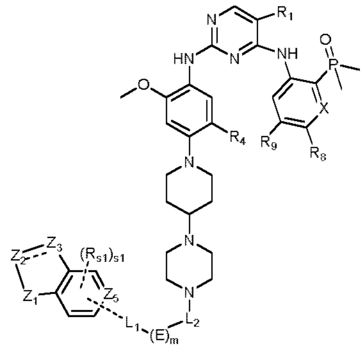
(I-1-F),

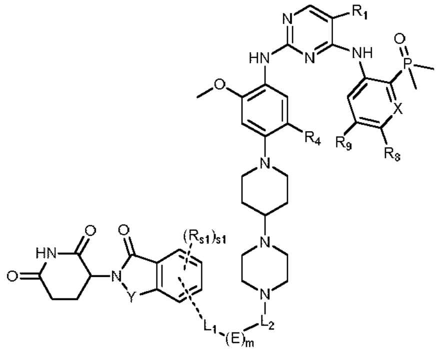
(I-1-G),

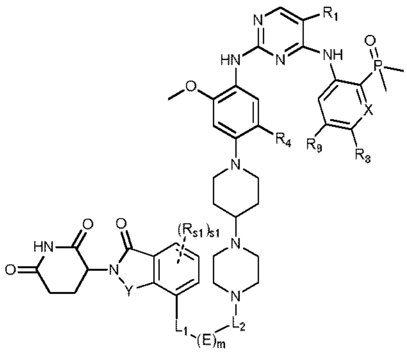
(I-1-H),

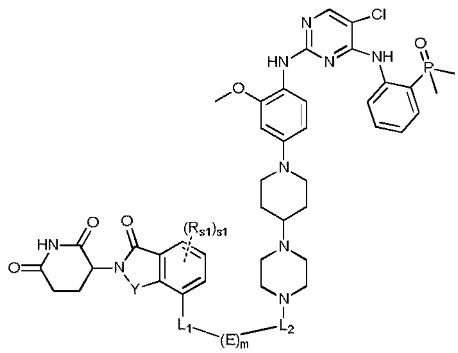
(I-1-H'),

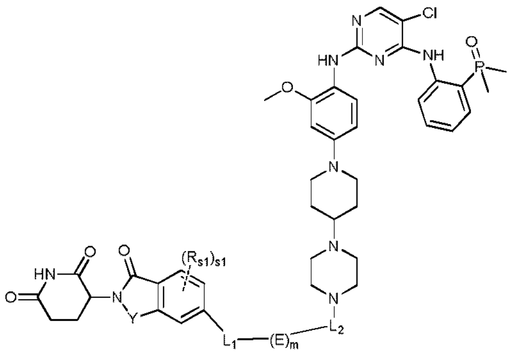
(I-1-H''),

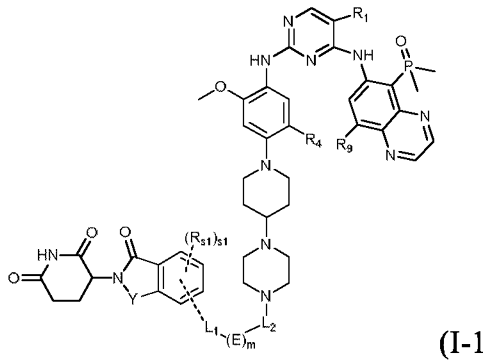
(I-1-I),

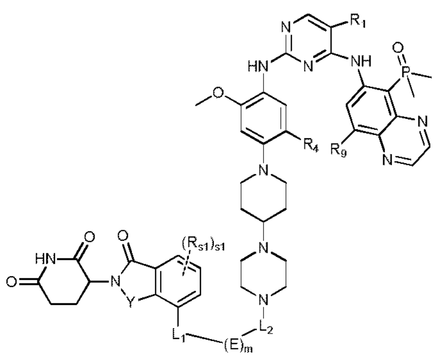
(I-1-I'),

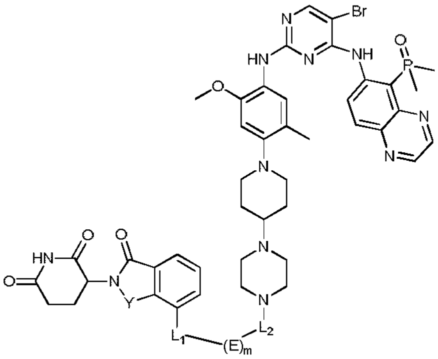
(I-1-I''),

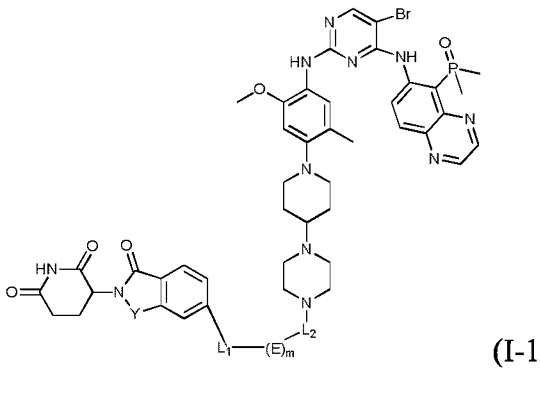

(I-1-I'''),

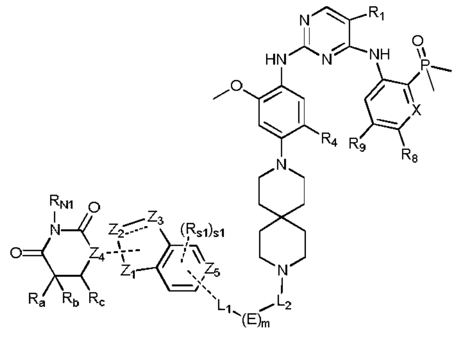

(I-2),

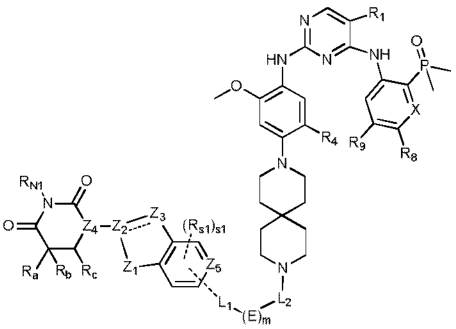

(I-2-A),

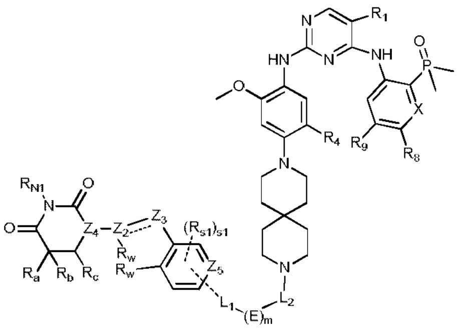

(I-2-B),

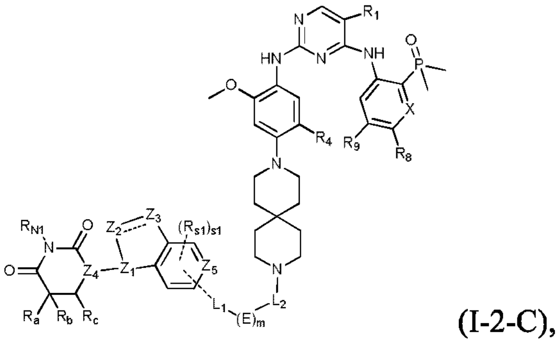

(I-2-C),

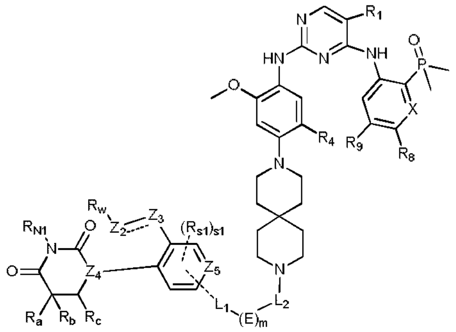

(I-2-D),

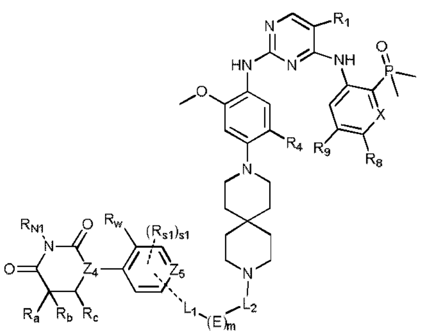

(I-2-E),

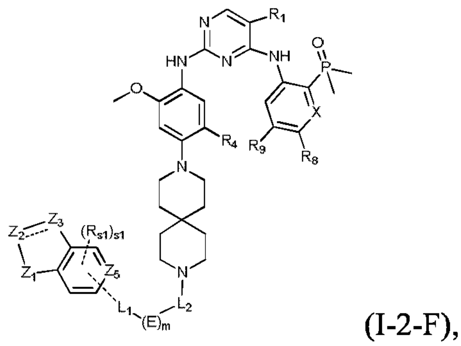

(I-2-F),

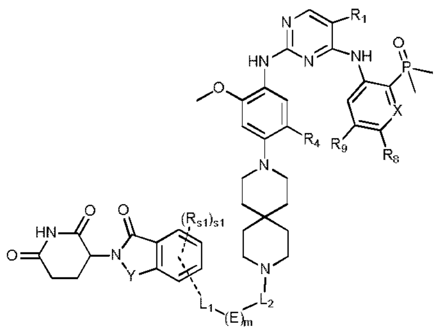
(I-2-G),

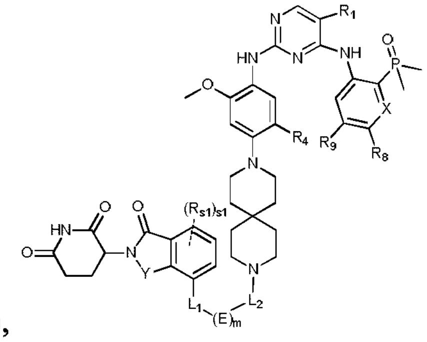
(I-2-H),

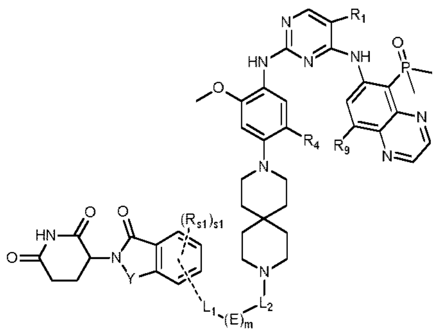
(I-2-I),

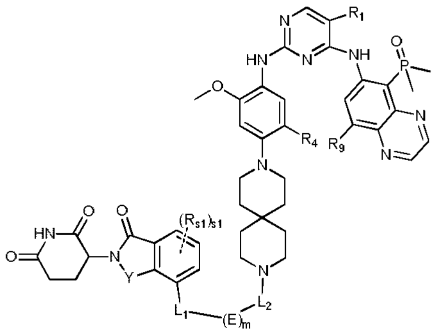
(I-2-I'),

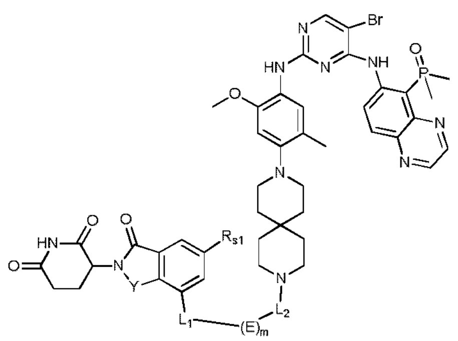
(I-2-I''),

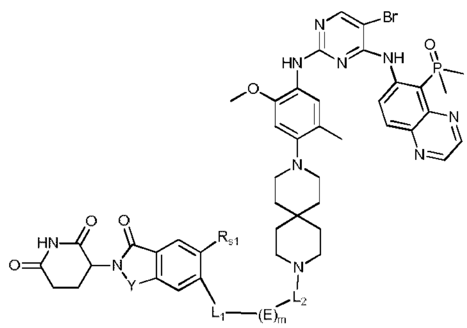
(I-2-I'''),

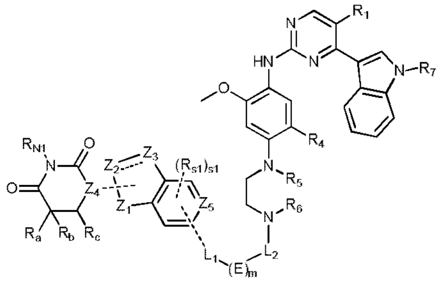
(I-3),

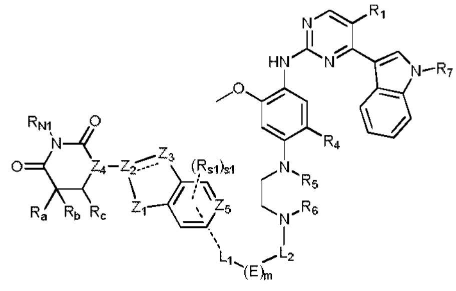
(I-3-A),

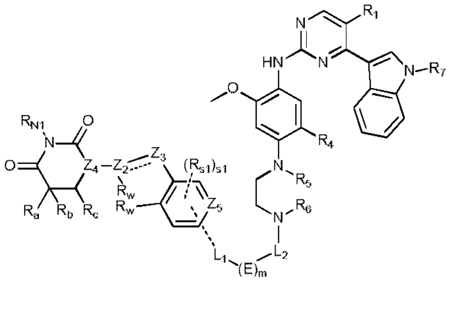
(I-3-B),

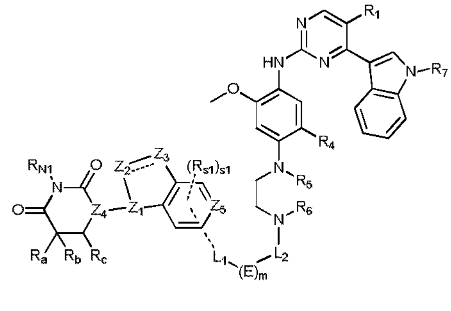
(I-3-C),

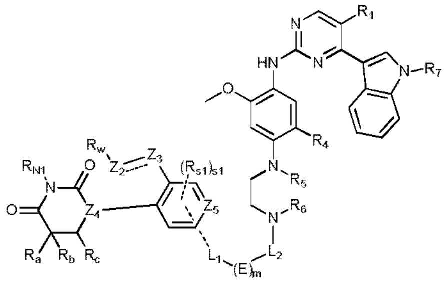 (I-3-D),

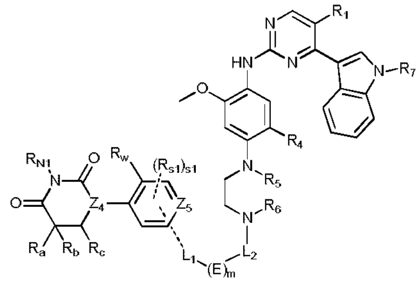 (I-3-E),

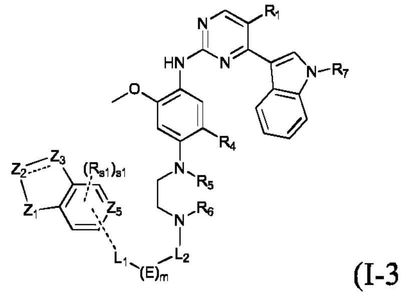 (I-3-F),

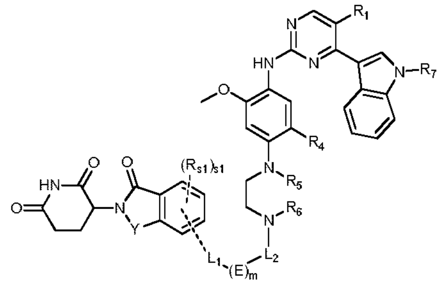 (I-3-G),

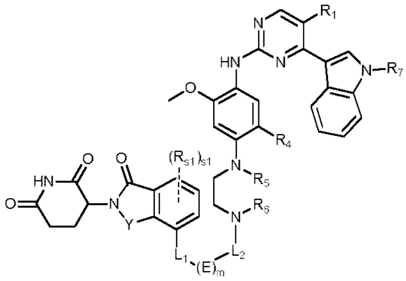 (I-3-H),

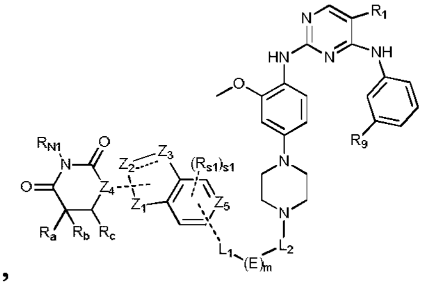 (I-4),

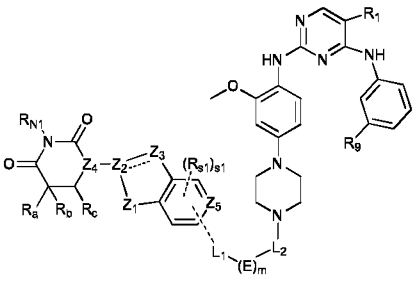 (I-4-A),

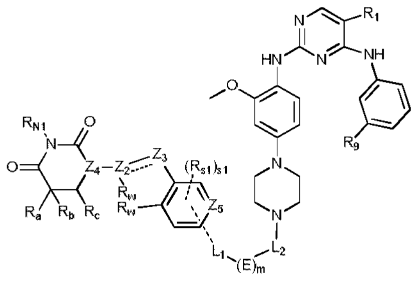 (I-4-B),

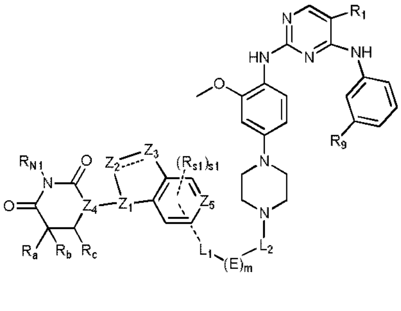 (I-4-C),

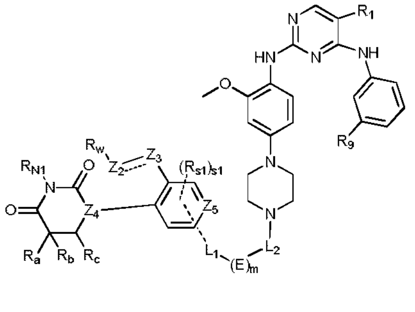 (I-4-D),

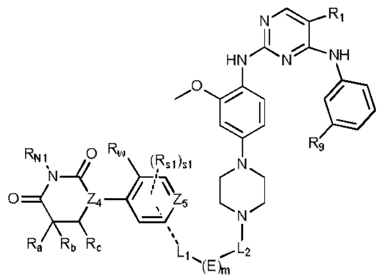 (I-4-E), 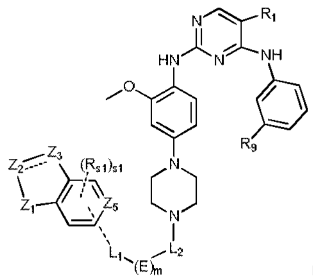 (I-4-F),

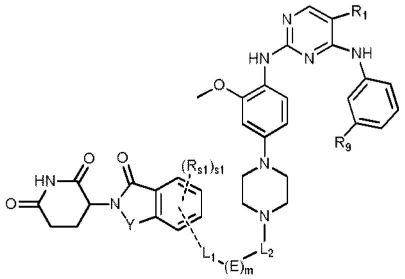 (I-4-G), 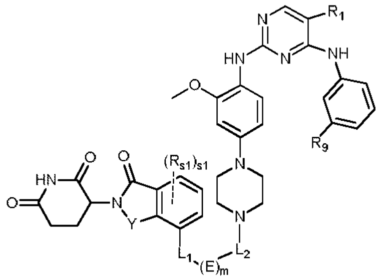 (I-4-H),

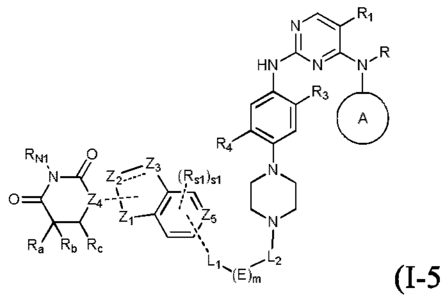 (I-5), 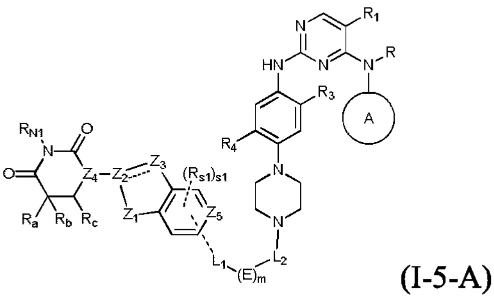 (I-5-A),

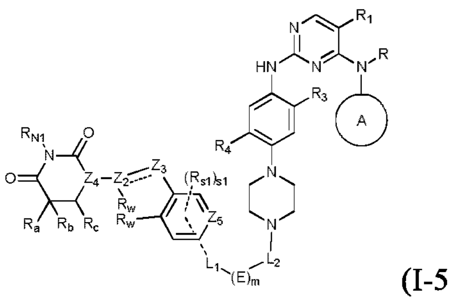 (I-5-B), 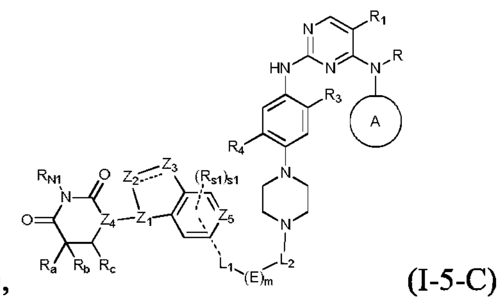 (I-5-C),

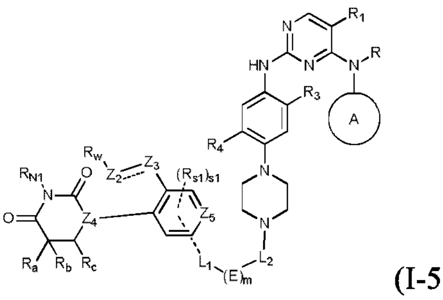 (I-5-D), 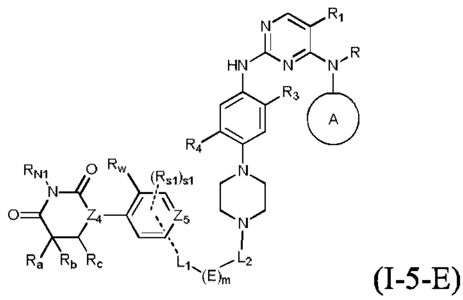 (I-5-E),

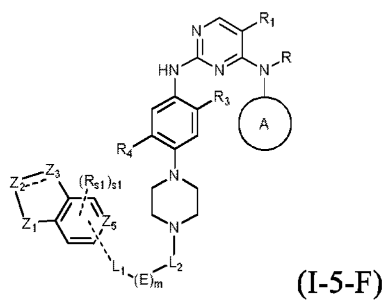 (I-5-F), 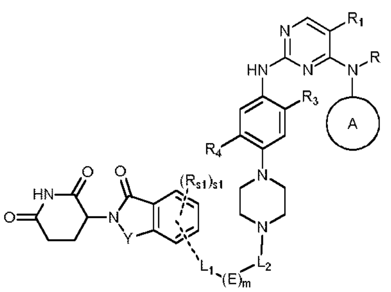 (I-5-G),

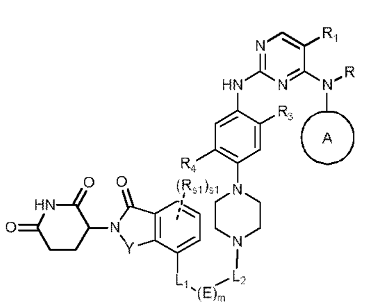 (I-5-H), 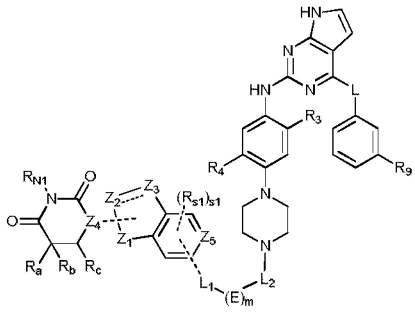 (I-6),

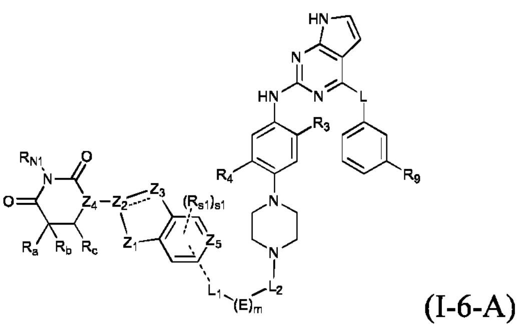 (I-6-A), 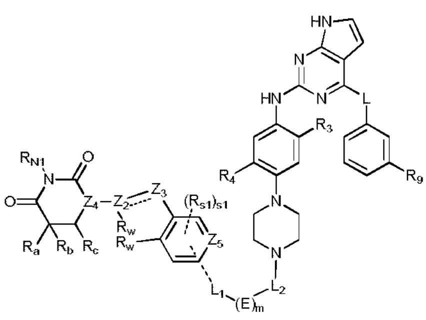 (I-6-B),

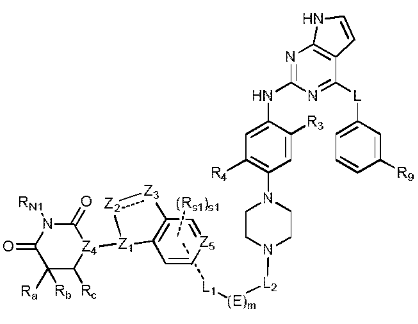 (I-6-C), 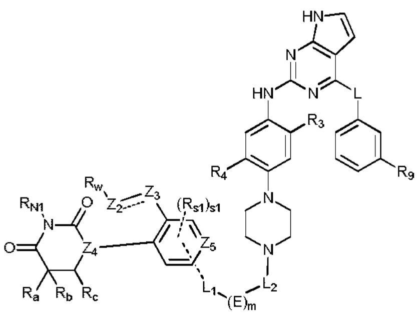 (I-6-D),

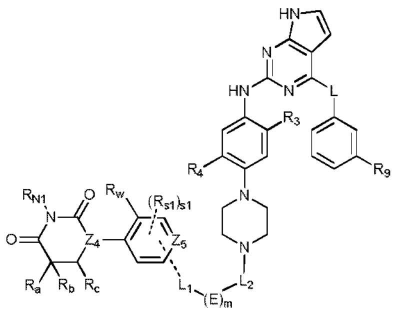 (I-6-E), 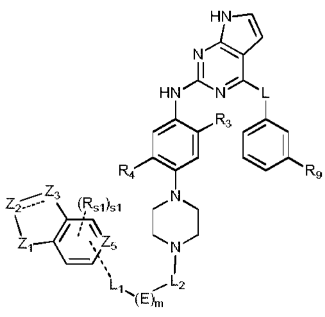 (I-6-F),

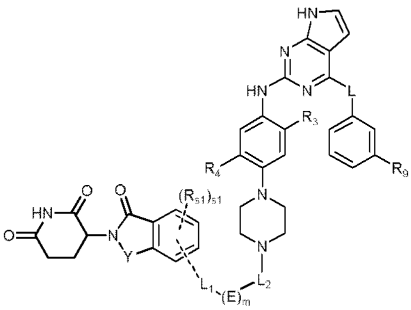 (I-6-G), 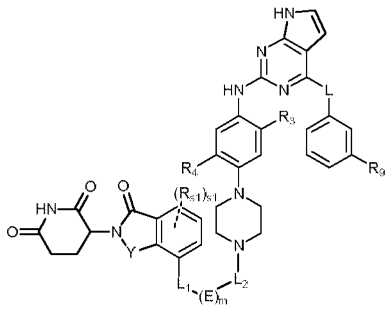 (I-6-H),

wherein the definition of Y is the same as that of $Z_1$, and other groups are as defined in claims 2-7.

**9.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (I-1), (I-1-A), (I-1-B), (I-1-C), (I-1-D), (I-1-E), (I-1-F), (I-1-G), (I-1-H) or (I-1-I):
wherein

$R_1$ is selected from -Cl, -Br, $-CF_3$ or $-CH=CH_2$; alternatively, $R_1$ is selected from -Cl or - Br;
$R_4$ is H or -Me;
$R_8$ is H, -NHMe, $-NHC(O)CH_2CH_3$ or -NH-cyclopropyl;
$R_9$ is H, -F, -Cl, -Br, -Me, $-CF_3$, -OMe, $-OCF_3$, -CN, $-NHC(O)CH_2CH_3$, - $NHS(O)_2CH_2CH_3$ or $-NHC(O)CH=CH_2$;
X is $-C(R_x)=$ or -N=;
$R_x$ is H, or $R_x$ and $R_8$ are taken together with the C atoms to which they are attached to form pyrazinyl;
and other groups are as defined in claims 2-7.

**10.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (I-G):

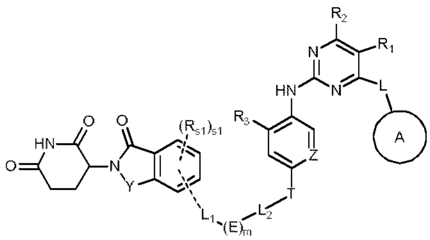

(I-G),

wherein
ring A is the following group:

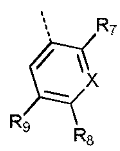 ;

wherein

$R_7$ is $-P(O)(C_{1-6}\text{ alkyl})_2$;
$R_8$ is H;
$R_9$ is selected from H, halogen, $C_{1-6}$ alkyl, -CN or $C_{1-6}$ haloalkyl;
X is $-C(R_x)=$;
wherein $R_x$ is H, or $R_x$ and $R_8$ are taken together with the C atoms to which they are attached to form 5- to 6-membered heteroaryl; alternatively form pyrazinyl;

Y is C atom, which is optionally substituted with one or two Rzi, wherein Rzi is H, CN or halogen; or two Rzi are taken together with Y to form C=O; alternatively, Y is $CH_2$ or C=O; alternatively, Y is $CH_2$;
$L_1$ is $-C_aR^{\#}R^{\#'}-C_bR^{\#}R^{\#'}-$;
$L_2$ is selected from bond, $-CR^{\#}R^{\#'}-$ or $-C_aR^{\#}R^{\#'}-C_bR^{\#}R^{\#'}-$;
wherein one of $C_aR^{\#}R^{\#'}$ or $C_bR^{\#}R^{\#'}$ can be replaced by O, $S(O)_p$ or $NR^{\#}$;
E is independently selected from: bond or $-C_cR^{\#}R^{\#'}-C_dR^{\#}R^{\#'}-C_eR^{\#}R^{\#'}$;
wherein one of $C_cR^{\#}R^{\#'}$, $C_dR^{\#}R^{\#'}$ or $C_eR^{\#}R^{\#'}$, or both of $C_cR^{\#}R^{\#'}$ and $C_e$R#R#' can be replaced by O, $S(O)_p$ or $NR^{\#}$;
p is 0, 1 or 2;
$R^{\#}$ is H, halogen, $C_{1-6}$ alkyl, $C_{1-6}$ haloalkyl, $C_{2-6}$ alkenyl or $C_{2-6}$ alkynyl;
$R^{\#'}$ is H, halogen, $C_{1-6}$ alkyl, $C_{1-6}$ haloalkyl, $C_{2-6}$ alkenyl or $C_{2-6}$ alkynyl;
or, $R^{\#}$ and $R^{\#}$ on adjacent atoms can be taken together to form bond, and $R^{\#'}$ and $R^{\#'}$ on adjacent atoms can be taken together to form bond;
or, $R^{\#}$ and $R^{\#'}$ on the same or different atoms can be taken together to form =O;
m is 0, 1, 2, 3, 4 or 5;
$R_{s1}$ is selected from H, CN, halogen, OH, $NH_2$, $C_{2-6}$ alkenyl, $C_{2-6}$ alkynyl, -O-$C_{1-6}$ alkyl, -O-$C_{1-6}$ haloalkyl, -NH-$C_{1-6}$ alkyl, $C_{1-6}$ alkyl or $C_{1-6}$ haloalkyl;
s1 is 0, 1, 2 or 3;
L is -NR-; wherein R is H or $C_{1-6}$ alkyl;
Z is $-C(R_4)=$;
T is

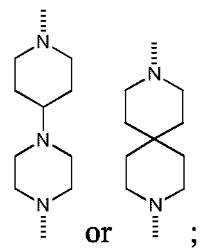

$R_1$ is selected from H, halogen, cyano, $C_{1-6}$ alkyl, $C_{1-6}$ haloalkyl, $C_{2-6}$ alkenyl or $C_{2-6}$ alkynyl;
$R_2$ is H, halogen, hydroxyl, amino, $C_{1-6}$ alkyl or $C_{1-6}$ haloalkyl;
$R_3$ is selected from H, -O-$C_{1-6}$ alkyl or -O-$C_{1-6}$ haloalkyl;
$R_4$ is selected from H, halogen, $C_{1-6}$ alkyl or $C_{1-6}$ haloalkyl;
if the above groups are H or H-containing groups, the one or more H atom(s) may be substituted with D atom(s).

**11.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (I-1-G), (I-1-H), (I-1-H'), or (I-1-H"):

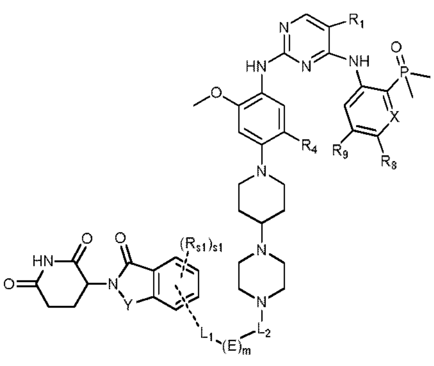

(I-1-G),

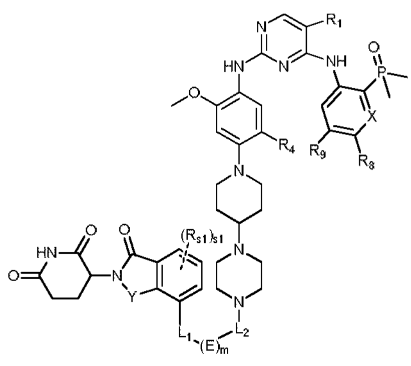

(I-1-H),

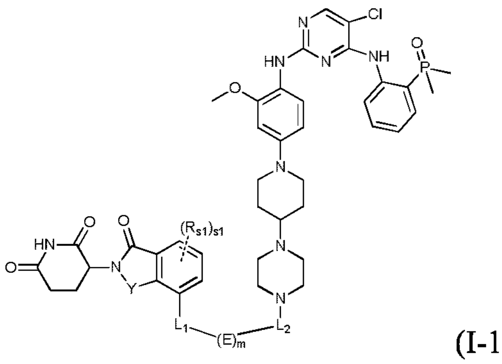

(I-1-H'),

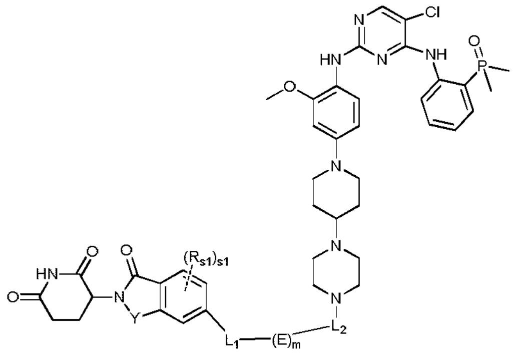

(I-1-H''),

wherein

X is -CH=;

Y is C atom, which is optionally substituted with one or two Rzi, wherein Rzi is H, CN or halogen; or two Rzi are taken together with Y to form C=O; alternatively, Y is $CH_2$ or C=O;

$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is H or halogen;

$R_4$ is selected from H, halogen, $C_{1-6}$ alkyl or $C_{1-6}$ haloalkyl; alternatively, $R_4$ is H;

$R_5$ is H;

$R_9$ is selected from H, halogen, $C_{1-6}$ alkyl, -CN or $C_{1-6}$ haloalkyl; alternatively, $R_9$ is H;

$L_1$ is selected from $-CH_2CH_2-$, -CH=CH-, -C≡C-, $-OCH_2-$, $-SCH_2-$, $-S(O)CH_2-$, - $S(O)_2CH_2-$, $-NHCH_2-$, $-N(Me)CH_2-$, $-C(O)CH_2-$, $-CH_2C(O)-$, -OC(O)-, -SC(O)-, -NHC(O)- or -N(Me)C(O)-, and one or more H atom(s) in the above groups can be substituted with D atom(s); alternatively, $L_1$ is selected from $-CH_2CH_2-$, -CH=CH-, -C=C-, $-OCH_2-$ or $-NHCH_2-$; alternatively, $L_1$ is selected from $-CH_2CH_2-$, -C=C- or $-OCH_2-$;

$L_2$ is selected from bond, $-CH_2-$ or $-CH_2CH_2-$, and one or more H atom(s) in the above groups can be substituted with D atom(s);

E is $-CH_2CH_2CH_2-$, $-CH_2CH_2O-$, $-CH_2OCH_2-$, $-OCH_2CH_2-$, $-CH_2CH_2S-$, $-CH_2SCH_2-$ or $-SCH_2CH_2-$, and one or more H atom(s) in the above groups can be substituted with D atom(s); alternatively, E is $-CH_2CH_2CH_2-$;

m is 0, 1, 2 or 3; alternatively, the chain length of $-L_1-(E)_m-L_2-$ is alternatively 4 to 12 bond lengths, still alternatively 5, 6, 7, 8, 9 or 10 bond lengths;

$R_{s1}$ is selected from H, CN, halogen, OH, $NH_2$, $C_{2-6}$ alkenyl, $C_{2-6}$ alkynyl, $-O-C_{1-6}$ alkyl, $-O-C_{1-6}$ haloalkyl, $-NH-C_{1-6}$ alkyl, $C_{1-6}$ alkyl or $C_{1-6}$ haloalkyl; alternatively, $R_{s1}$ is H, CN or halogen;

s1 is 0, 1 or 2.

**12.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (I-1-G), (I-1-H), (I-1-H'), or (I-1-H''):

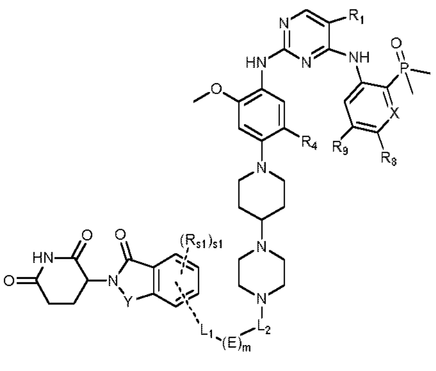

(I-1-G),

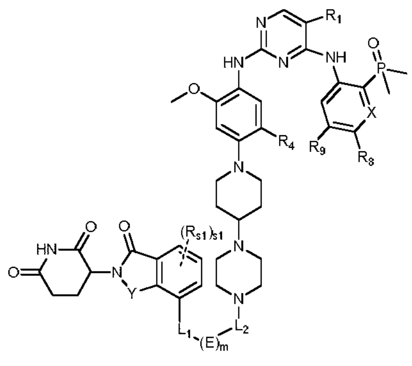

(I-1-H),

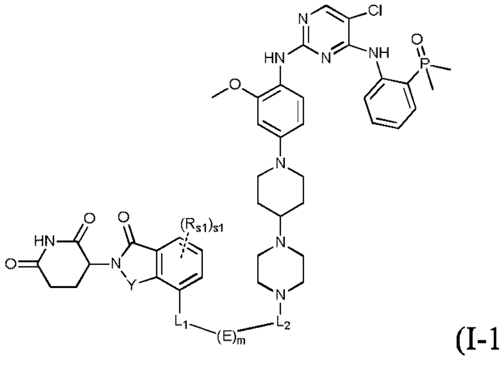

(I-1-H'),

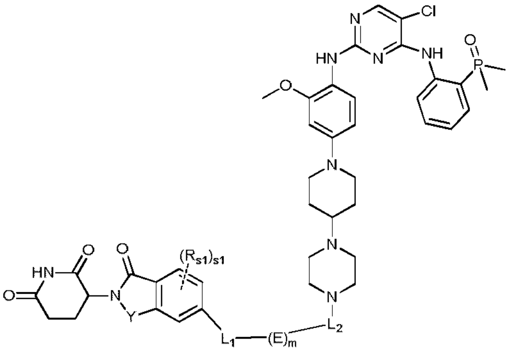

(I-1-H''),

wherein

X is -CH=;
Y is C atom, which is optionally substituted with one or two Rzi, wherein Rzi is H, CN or halogen; or two Rzi are taken together with Y to form C=O; alternatively, Y is $CH_2$;
$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is halogen;
$R_4$ is selected from H, halogen, $C_{1-6}$ alkyl or $C_{1-6}$ haloalkyl; alternatively, $R_4$ is H;
$R_8$ is H;
$R_9$ is selected from H, halogen, $C_{1-6}$ alkyl, -CN or $C_{1-6}$ haloalkyl; alternatively, $R_9$ is H;
$L_1$ is selected from $-CH_2CH_2-$, -CH=CH-, -C=C-, $-OCH_2-$, $-SCH_2-$, $-S(O)CH_2-$, - $S(O)_2CH_2-$, $-NHCH_2-$, $-N(Me)CH_2-$, $-C(O)CH_2-$, $-CH_2C(O)-$, -OC(O)-, -SC(O)-, -NHC(O)- or -N(Me)C(O)-, and one or more H atom(s) in the above groups can be substituted with D atom(s); alternatively, $L_1$ is selected from $-CH_2CH_2-$, -CH=CH-, -C=C-, $-OCH_2-$ or $-NHCH_2-$; alternatively, $L_1$ is selected from $-CH_2CH_2-$, -C=C-, $-OCH_2-$ or $-NHCH_2-$;
$L_2$ is selected from bond, $-CH_2-$ or $-CH_2CH_2-$, and one or more H atom(s) in the above groups can be substituted with D atom(s);
E is $-CH_2CH_2CH_2-$, $-CH_2CH_2O-$, $-CH_2OCH_2-$, $-OCH_2CH_2-$, $-CH_2CH_2S-$, $-CH_2SCH_2-$ or $-SCH_2CH_2-$, and one or more H atom(s) in the above groups can be substituted with D atom(s); alternatively, E is $-CH_2CH_2CH_2-$;
m is 0, 1, 2 or 3; alternatively, the chain length of $-L_1-(E)_m-L_2-$ is alternatively 4 to 14 bond lengths, still alternatively 5, 6, 7, 8, 9 or 10 bond lengths;
$R_{s1}$ is selected from H, CN, halogen, OH, $NH_2$, $C_{2-6}$ alkenyl, $C_{2-6}$ alkynyl, $-O-C_{1-6}$ alkyl, $-O-C_{1-6}$ haloalkyl, $-NH-C_{1-6}$ alkyl, $C_{1-6}$ alkyl or $C_{1-6}$ haloalkyl; alternatively, $R_{s1}$ is H or halogen;
s1 is 0, 1 or 2.

**13.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (I-1-G), (I-1-H), (I-1-H'), or (I-1-H"):

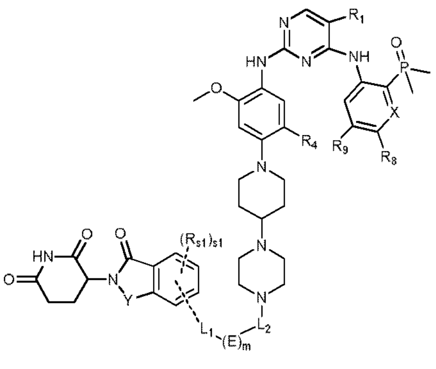

(I-1-G),

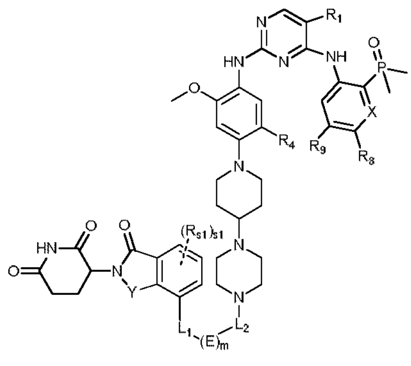

(I-1-H),

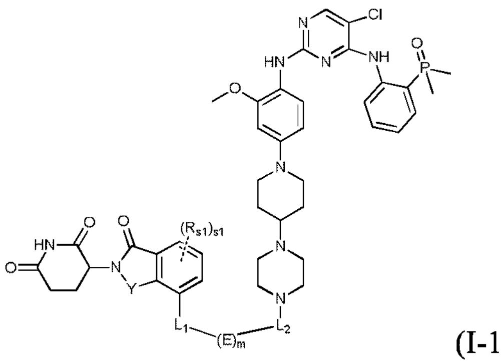

(I-1-H'),

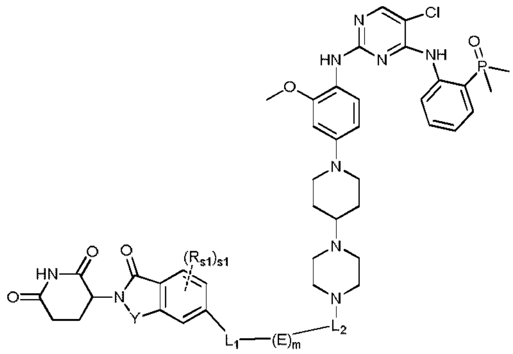

(I-1-H''),

wherein

X is -CH=;
Y is C atom, which is optionally substituted with one or two Rzi, wherein Rzi is H, CN or halogen; or two Rzi are taken together with Y to form C=O; alternatively, Y is $CH_2$ or C=O;
$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is halogen;
$R_4$ is selected from H, halogen, $C_{1-6}$ alkyl or $C_{1-6}$ haloalkyl; alternatively, $R_4$ is H;
$R_8$ is H;
$R_9$ is selected from H, halogen, $C_{1-6}$ alkyl, -CN or $C_{1-6}$ haloalkyl; alternatively, $R_9$ is H;
$L_1$ is selected from $-CH_2CH_2-$, -CH=CH-, -C=C-, $-OCH_2-$, $-SCH_2-$, $-S(O)CH_2-$, - $S(O)_2CH_2-$, $-NHCH_2-$, $-N(Me)CH_2-$, $-C(O)CH_2-$, $-CH_2C(O)-$, -OC(O)-, -SC(O)-, -NHC(O)- or -N(Me)C(O)-, and one or more H atom(s) in the above groups can be substituted with D atom(s); alternatively, $L_1$ is selected from $-CH_2CH_2-$, -CH=CH-, -C=C-, $-OCH_2-$ or $-NHCH_2-$; alternatively, $L_1$ is selected from -C=C-, $-OCH_2-$ or $-NHCH_2-$;
$L_2$ is selected from bond, $-CH_2-$ or $-CH_2CH_2-$, and one or more H atom(s) in the above groups can be substituted with D atom(s);
E is $-CH_2CH_2CH_2-$, $-CH_2CH_2O-$, $-CH_2OCH_2-$, $-OCH_2CH_2-$, $-CH_2CH_2S-$, $-CH_2SCH_2-$ or $-SCH_2CH_2-$, and one or more H atom(s) in the above groups can be substituted with D atom(s); alternatively, E is $-CH_2CH_2CH_2-$;
m is 1, 2 or 3; alternatively, the chain length of $-L_1-(E)_m-L_2-$ is less than 14 bond lengths; alternatively, the chain length is 5-10 bond lengths;
$R_{s1}$ is selected from H, CN, halogen, OH, $NH_2$, $C_{2-6}$ alkenyl, $C_{2-6}$ alkynyl, $-O-C_{1-6}$ alkyl, $-O-C_{1-6}$ haloalkyl, $-NH-C_{1-6}$ alkyl, $C_{1-6}$ alkyl or $C_{1-6}$ haloalkyl; alternatively, $R_{s1}$ is H or halogen;
s1 is 0, 1 or 2.

**14.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (I-1-I), (I-1-I'), (I-1-I"), or (I-1-I'''):

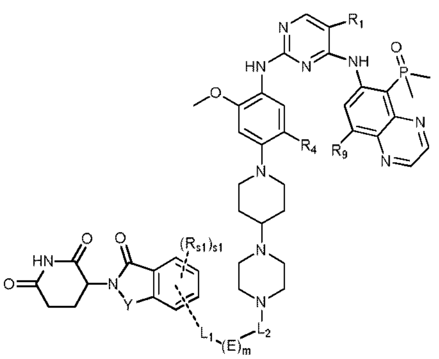

(I-1-I),

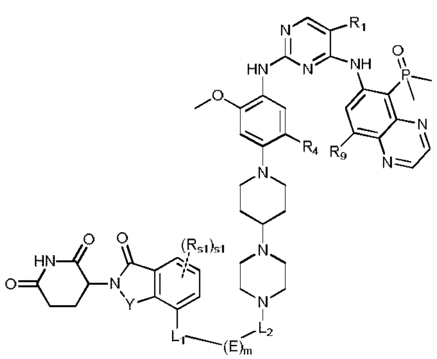

(I-1-I'),

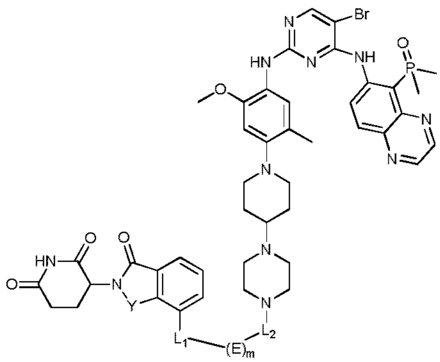

(I-1-I"),

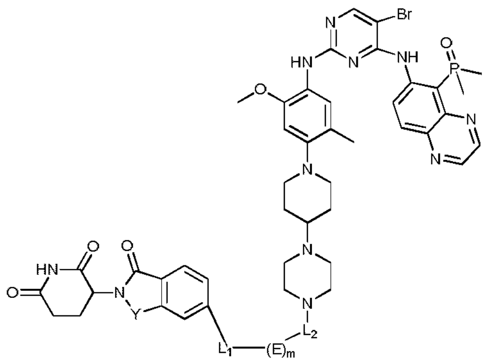

(I-1-I'''),

wherein

Y is C atom, which is optionally substituted with one or two Rzi, wherein Rzi is H, CN or halogen; or two Rzi are taken together with Y to form C=O; alternatively, Y is $CH_2$ or C=O;
$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is halogen;
$R_4$ is selected from H, halogen, $C_{1-6}$ alkyl or $C_{1-6}$ haloalkyl; alternatively, $R_4$ is selected from $C_{1-6}$ alkyl or $C_{1-6}$ haloalkyl;
$R_9$ is selected from H, halogen, $C_{1-6}$ alkyl, -CN or $C_{1-6}$ haloalkyl; alternatively, $R_9$ is H;
$L_1$ is selected from $-CH_2CH_2-$, -CH=CH-, -C=C-, $-OCH_2-$, $-SCH_2-$, $-S(O)CH_2-$, - $S(O)_2CH_2-$, $-NHCH_2-$, $-N(Me)CH_2-$, $-C(O)CH_2-$, $-CH_2C(O)-$, -OC(O)-, -SC(O)-, -NHC(O)- or -N(Me)C(O)-; alternatively, $L_1$ is selected from $-CH_2CH_2-$, -CH=CH-, -C=C- or $-OCH_2-$; alternatively, $L_1$ is selected from $-CH_2CH_2-$, -C=C- or $-OCH_2-$;
$L_2$ is selected from bond, $-CH_2-$ or $-CH_2CH_2-$;
E is $-CH_2CH_2CH_2-$, $-CH_2CH_2O-$, $-CH_2OCH_2-$, $-OCH_2CH_2-$, $-CH_2CH_2S-$, $-CH_2SCH_2-$ or $-SCH_2CH_2-$; alternatively, E is $-CH_2CH_2CH_2-$;
m is 1 or 2; alternatively, the chain length of $-L_1-(E)_m-L_2-$ is 4 to 14 bond lengths, still alternatively 5, 6, 7, 8, 9 or 10 bond lengths.
$R_{s1}$ is selected from H, CN, halogen, OH, $NH_2$, $C_{2-6}$ alkenyl, $C_{2-6}$ alkynyl, $-O-C_{1-6}$ alkyl, $-O-C_{1-6}$ haloalkyl, $-NH-C_{1-6}$ alkyl, $C_{1-6}$ alkyl or $C_{1-6}$ haloalkyl; alternatively, $R_{s1}$ is H, halogen or CN;
s1 is 0, 1 or 2.

**15.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (I-1-I), (I-1-I'), (I-1-I"), or (I-1-I'''):

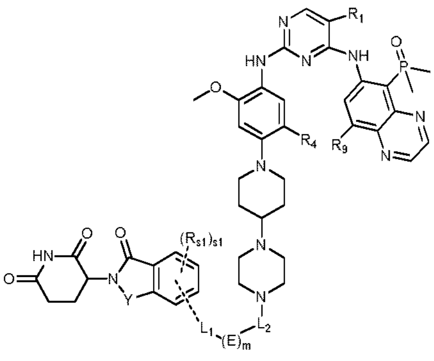

(I-1-I),

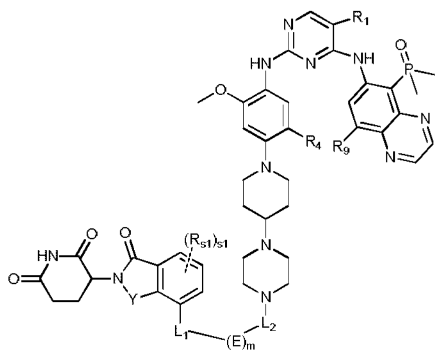

(I-1-I'),

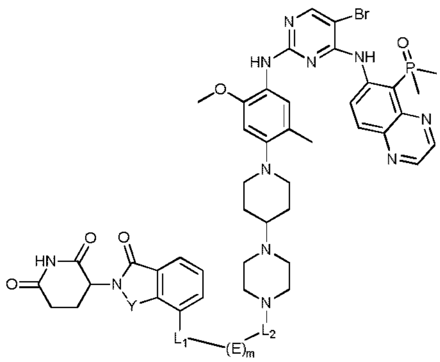

(I-1-I''),

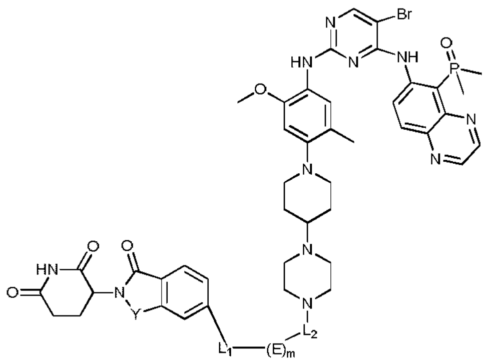

(I-1-I'''),

wherein

Y is C atom, which is optionally substituted with one or two Rzi, wherein Rzi is H, CN or halogen; or two Rzi are taken together with Y to form C=O; alternatively, Y is $CH_2$;
$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is halogen;
$R_4$ is selected from H, halogen, $C_{1-6}$ alkyl or $C_{1-6}$ haloalkyl; alternatively, $R_4$ is selected from $C_{1-6}$ alkyl or $C_{1-6}$ haloalkyl;
$R_9$ is selected from H, halogen, $C_{1-6}$ alkyl, -CN or $C_{1-6}$ haloalkyl; alternatively, $R_9$ is H;
$L_1$ is selected from $-CH_2CH_2-$, -CH=CH-, -C=C-, $-OCH_2-$, $-SCH_2-$, $-S(O)CH_2-$, - $S(O)_2CH_2-$, $-NHCH_2-$, $-N(Me)CH_2-$, $-C(O)CH_2-$, $-CH_2C(O)-$, -OC(O)-, -SC(O)-, -NHC(O)- or -N(Me)C(O)-; alternatively, $L_1$ is selected from $-CH_2CH_2-$, -CH=CH-, -C=C- or $-OCH_2-$; alternatively, $L_1$ is selected from -C=C- or $-OCH_2-$;
$L_2$ is selected from bond, $-CH_2-$ or $-CH_2CH_2-$;
E is $-CH_2CH_2CH_2-$;
m is 1 or 2; alternatively, the chain length of $-L_1-(E)_m-L_2-$ is less than 10 bond lengths; alternatively, the chain length is 6 to 9 bond lengths, still alternatively 6, 7, 8 or 9 bond lengths;
$R_{s1}$ is selected from H, CN, halogen, OH, $NH_2$, $C_{2-6}$ alkenyl, $C_{2-6}$ alkynyl, $-O-C_{1-6}$ alkyl, $-O-C_{1-6}$ haloalkyl, $-NH-C_{1-6}$ alkyl, $C_{1-6}$ alkyl or $C_{1-6}$ haloalkyl; alternatively, $R_{s1}$ is H or halogen;
s1 is 0, 1 or 2.

**16.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (I-2-I), (I-2-I'), (I-2-I''), or (I-2-I'''):

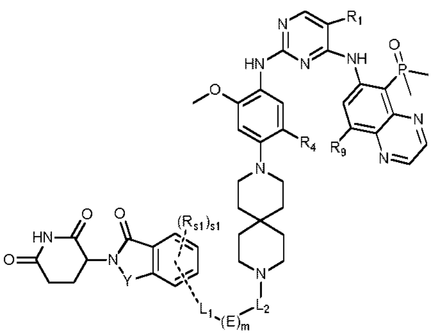

(I-2-I),

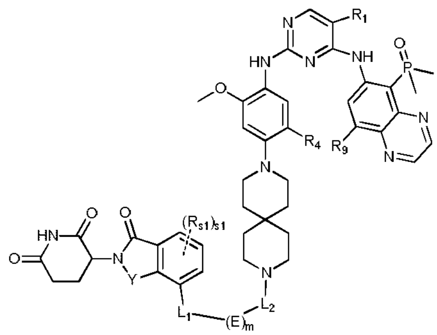

(I-2-I'),

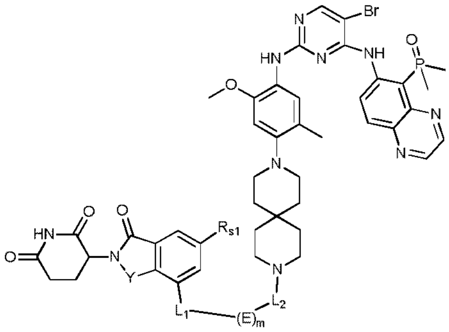

(I-2-I''),

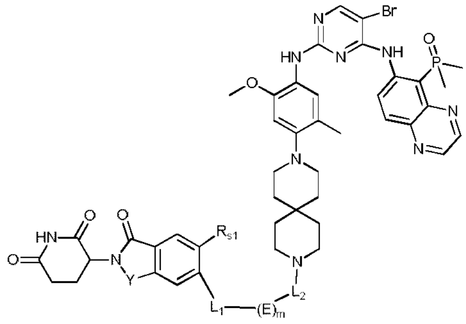

(I-2-I'''),

wherein

Y is C atom, which is optionally substituted with one or two Rzi, wherein Rzi is H, CN or halogen; or two Rzi are taken together with Y to form C=O; alternatively, Y is $CH_2$;
$R_1$ is selected from H, halogen, cyano, $C_{1-6}$ alkyl, $C_{1-6}$ haloalkyl, $C_{2-6}$ alkenyl or $C_{2-6}$ alkynyl; alternatively, $R_1$ is halogen;
$R_4$ is selected from H, halogen, $C_{1-6}$ alkyl or $C_{1-6}$ haloalkyl; alternatively, $R_4$ is selected from $C_{1-6}$ alkyl or $C_{1-6}$ haloalkyl;
$R_9$ is selected from H, halogen, $C_{1-6}$ alkyl, -CN or $C_{1-6}$ haloalkyl; alternatively, $R_9$ is H;
$L_1$ is selected from $-CH_2CH_2-$, -CH=CH-, -C=C-, $-OCH_2-$, $-SCH_2-$, $-S(O)CH_2-$,$-S(O)_2CH_2-$, $-NHCH_2-$, $-N(Me)CH_2-$, $-C(O)CH_2-$, $-CH_2C(O)-$, -OC(O)-, -SC(O)-, -NHC(O)- or -N(Me)C(O)-; alternatively, $L_1$ is selected from $-CH_2CH_2-$, -CH=CH-, -C=C- or $-OCH_2-$; alternatively, $L_1$ is selected from -C=C- or $-OCH_2-$;
$L_2$ is selected from bond, $-CH_2-$ or $-CH_2CH_2-$;
E is $-CH_2CH_2CH_2-$, $-CH_2CH_2O-$, $-CH_2OCH_2-$, $-OCH_2CH_2-$, $-CH_2CH_2S-$, $-CH_2SCH_2-$ or $-SCH_2CH_2-$; alternatively, E is $-CH_2CH_2CH_2-$;
m is 1 or 2; alternatively, the chain length of $-L_1-(E)_m-L_2-$ is less than 10 bond lengths; alternatively, the chain length is 6 to 9 bond lengths, still alternatively 6, 7, 8 or 9 bond lengths;
$R_{s1}$ is selected from H, CN, halogen, OH, $NH_2$, $C_{2-6}$ alkenyl, $C_{2-6}$ alkynyl, -O-$C_{1-6}$ alkyl, -O-$C_{1-6}$ haloalkyl, -NH-$C_{1-6}$ alkyl, $C_{1-6}$ alkyl or $C_{1-6}$ haloalkyl; alternatively, $R_{s1}$ is H or halogen;
s1 is 0, 1 or 2.

**17.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (1-2), (I-2-A), (I-2-B), (I-2-C), (I-2-D), (I-2-E), (I-2-F), (I-2-G), (I-2-H) or (I-2-I):
wherein

$R_1$ is -Cl, -Br or $-CH=CH_2$;
$R_4$ is H or -Me;
$R_8$ is H, -NHMe, $-NHC(O)CH_2CH_3$ or -NH-cyclopropyl;
$R_9$ is H, -F, -Cl, -Br, -Me, $-CF_3$, -OMe, $-OCF_3$, -CN, $-NHC(O)CH_2CH_3$, - $NHS(O)_2CH_2CH_3$ or $-NHC(O)CH=CH_2$;
X is $-C(R_x)=$;
$R_x$ is H, or $R_x$ and $R_8$ are taken together with the C atoms to which they are attached to form pyrazinyl;
and other groups are as defined in claims 2-7.

**18.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (1-3), (I-3-A), (I-3-B), (I-3-C), (I-3-D), (I-3-E), (I-3-F), (I-3-G) or (I-3-H):
wherein

$R_1$ is H, -Cl or $-CH=CH_2$;
$R_4$ is $-NHC(O)CH=CH_2$;
$R_5$ is -Me;
$R_6$ is -Me;
or $R_5$ and $R_6$ are connected to form $-CH_2CH_2-$;

$R_7$ is -Me or cyclopropyl;
and other groups are as defined in claims 2-7.

**19.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (1-4), (I-4-A), (I-4-B), (I-4-C), (I-4-D), (I-4-E), (I-4-F), (I-4-G) or (I-4-H):
wherein

$R_1$ is $-CF_3$;
$R_9$ is H, -F, -Cl, -Br, -Me, $-CF_3$, -OMe, $-OCF_3$, -CN, $-NHC(O)CH=CH_2$ or -NH-cyclopropyl;
and other groups are as defined in claims 2-7.

**20.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (1-5), (I-5-A), (I-5-B), (I-5-C), (I-5-D), (I-5-E), (I-5-F), (I-5-G) or (I-5-H):
wherein
ring A is the following optionally substituted groups: $C_{3-7}$ cycloalkyl or $C_{6-10}$ aryl, wherein the substituent is selected from -F, -Cl, -Br, -Me, -OMe, $-CF_3$, $-OCF_3$, -CN, -NHMe, $-P(O)Me_2$, $-NHC(O)CH_2CH_3$, $-C(O)CH=CH_2$, $-NHS(O)_2CH_2CH_3$, -NH-cyclopropyl, - $NHC(O)CH=CH_2$ or $-C(O)CH_2CH_3$; ring A is alternatively the following groups:

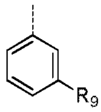 or 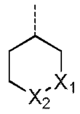 ;

wherein

$R_9$ is H, -F, -Cl, -Br, -Me, $-CF_3$, -OMe, $-OCF_3$, -CN, $-NHC(O)CH_2CH_3$, - $NHS(O)_2CH_2CH_3$ or $-NHC(O)CH=CH_2$;
$X_1$ is $-CH_2-$ or $-N(R_{X1})-$;
$X_2$ is $-CH(R_{X2})-$ or $-N(R_{X2})-$;
wherein
$R_{X1}$ is $-C(O)CH_2CH_3$ or $-C(O)CH=CH_2$;
$R_{X2}$ is H, -Me, -OMe, -NHMe, $-NHS(O)_2CH_2CH_3$, $-C(O)CH_2CH_3$ or $-NHC(O)CH_2CH_3$;
----- represents the point of attachment;
$R_1$ and R are taken together with the atoms to which they are attached to form optionally substituted 5- to 6-membered heterocyclyl, wherein the substituent is selected from halogen, oxo, -iPr, -Et, or phenyl, wherein the phenyl is mono- or poly-substituted with halogen; $R_1$ and R are alternatively taken together to form: $-C(O)N(R_N)C(O)-$ or $-C(CH_3)=C(R_N)C(O)-$;
wherein

$R_N$ is selected from -iPr, -Et or

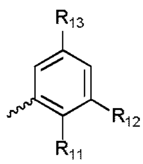 ;

$R_{11}$ is -Cl, -Br;
$R_{12}$ is H or -F;
$R_{13}$ is H or -F;

~~~ represents the point of attachment;
R3 is H or -OMe;
$R_4$ is H or -Me;
~~~

and other groups are as defined in claims 2-7.

**21.** The compound of general formula (I), or the pharmaceutically acceptable salt, enantiomer, diastereomer, racemate, solvate, hydrate, polymorph, prodrug, or isotopic variant thereof, or mixture thereof according to claim 20, wherein the compound of general formula (I) is a compound of general formula (1-5-1) or (I'-5-1):

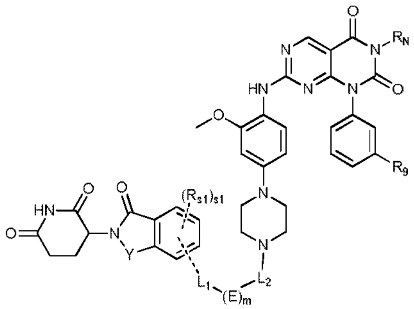

(I-5-1) or

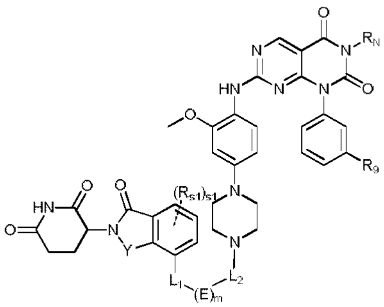

(I'-5-1)

wherein

$R_9$ is $-NHC(O)CH_2CH_3$ or $-NHC(O)CH=CH_2$;
$R_N$ is -iPr or -Et;
and other groups are as defined in claims 2-7.

**22.** The compound of general formula (I), or the pharmaceutically acceptable salt, enantiomer, diastereomer, racemate, solvate, hydrate, polymorph, prodrug, or isotopic variant thereof, or mixture thereof according to claim 20, wherein the compound of general formula (I) is a compound of general formula (1-5-2) or (I'-5-2):

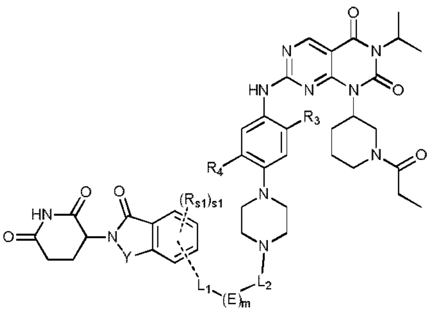

(I-5-2) or

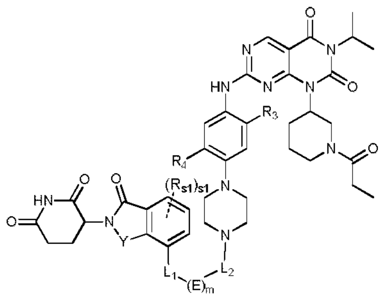

(I'-5-2)

wherein

$R_3$ is H or -OMe;
$R_4$ is H or -Me;
and other groups are as defined in claims 2-7.

**23.** The compound of general formula (I), or the pharmaceutically acceptable salt, enantiomer, diastereomer, racemate, solvate, hydrate, polymorph, prodrug, or isotopic variant thereof, or mixture thereof according to claim 20, wherein the compound of general formula (I) is a compound of general formula (1-5-3) or (I'-5-3):

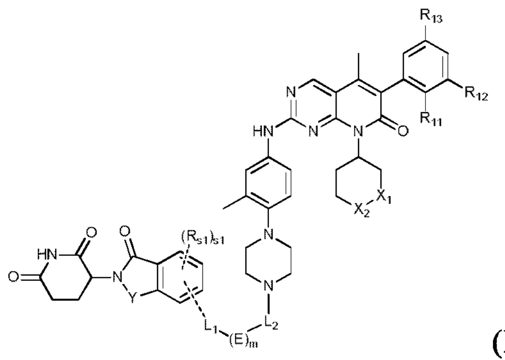

(I-5-3) or

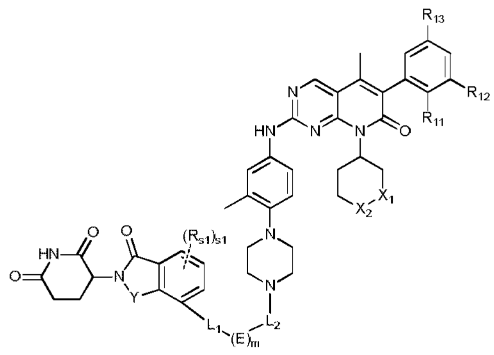

(I'-5-3)

wherein

$X_1$ is $-CH_2-$ or -N(Rxi)-;
$X_2$ is $-CH(R_{X2})-$ or $-N(R_{X2})-$;
wherein
$R_{X1}$ is $-C(O)CH_2CH_3$;
$R_{X2}$ is H, -Me, -OMe, -NHMe, $-C(O)CH_2CH_3$, $-NHS(O)_2CH_2CH_3$ or $-NHC(O)CH_2CH_3$;
$R_{11}$ is -Cl or -Br;
$R_{12}$ is H or -F;
$R_{13}$ is H or -F;
and other groups are as defined in claims 2-7.

**24.** The compound of general formula (I), or the pharmaceutically acceptable salt, enantiomer, diastereomer, racemate, solvate, hydrate, polymorph, prodrug, or isotopic variant thereof, or mixture thereof according to claim 8, wherein the compound of general formula (I) is a compound of general formula (1-6), (I-6-A), (I-6-B), (I-6-C), (I-6-D), (I-6-E), (I-6-F), (I-6-G) or (I-6-H):
wherein

L is -O- or -NH-;
$R_3$ is H or -OMe;
$R_4$ is H or -F;
$R_9$ is $-CF_3$, -OMe, $-OCF_3$, -CN, $-NHC(O)CH_2CH_3$ or $-NHC(O)CH=CH_2$;
and other groups are as defined in claims 2-7.

**25.** The compound of general formula (X), or the pharmaceutically acceptable salt, enantiomer, diastereomer, racemate, solvate, hydrate, polymorph, prodrug, or isotopic variant thereof, or mixture thereof according to any one of claims 1-7, wherein

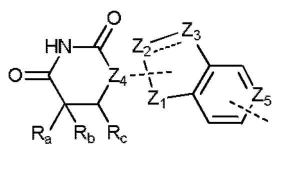

is selected from the following groups:

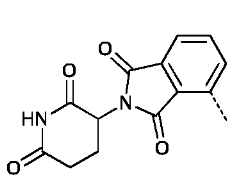 , 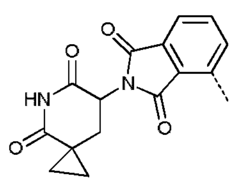 , 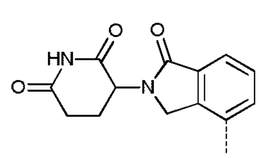 , 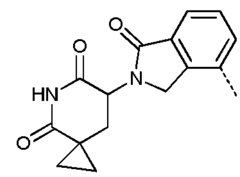 ,

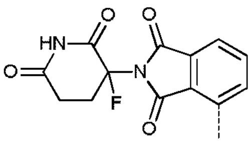 , 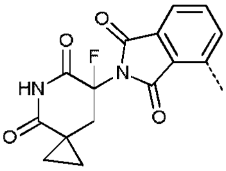 , 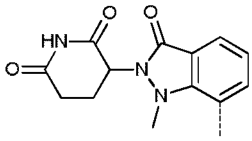 , 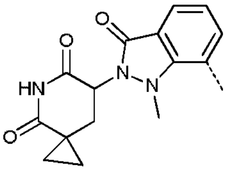 ,

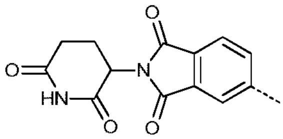 , 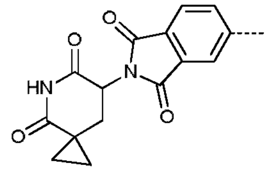 , 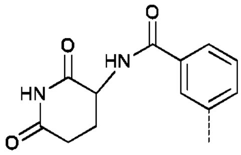 , 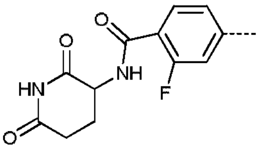 ,

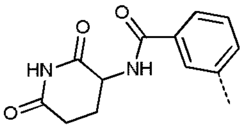 , 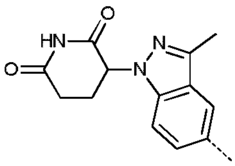 , 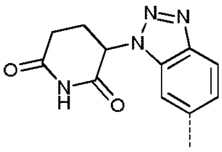 , 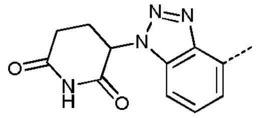 ,

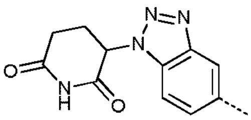 , 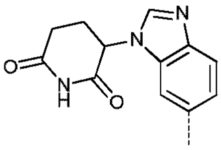 , 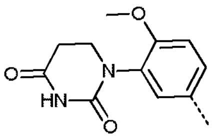 , 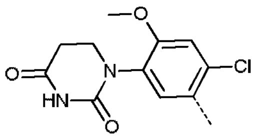 ,

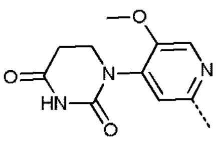 , 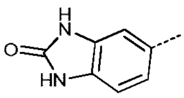 , 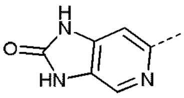 , 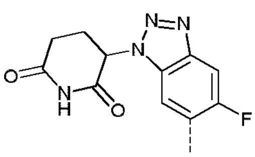 , 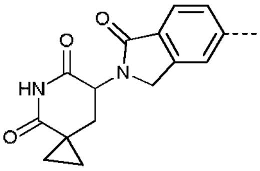 ,

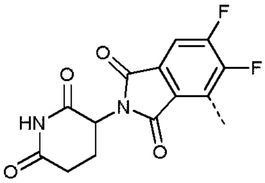 , 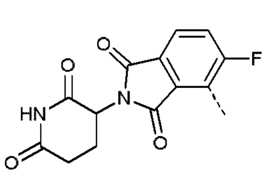 , 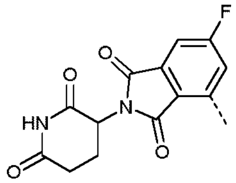 , 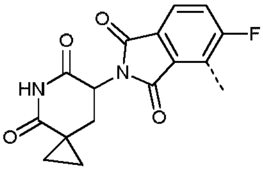 ,

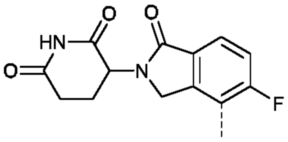 , 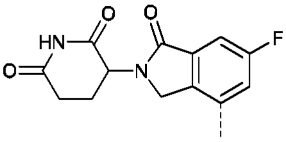 , 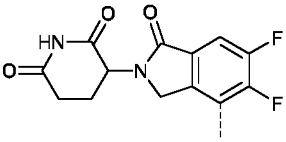 ,

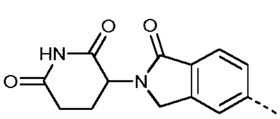 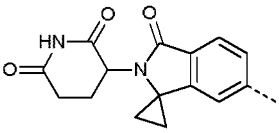 , 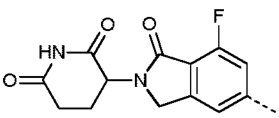 , 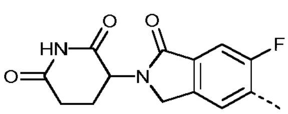 ,

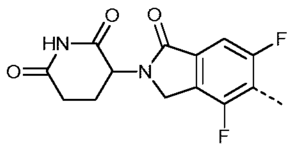 , 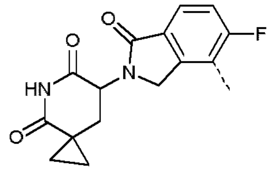 , 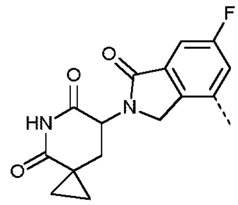 , 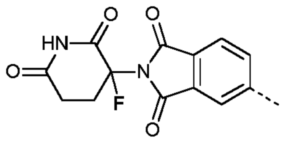 ,

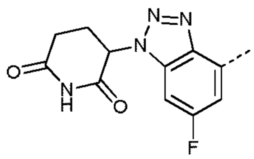 , 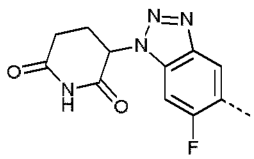 , 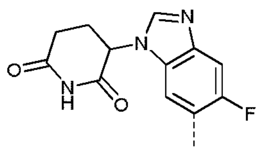 , 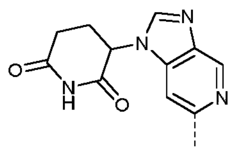 ,

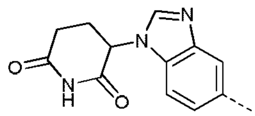 , 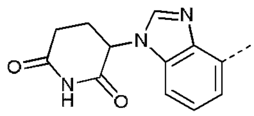 , 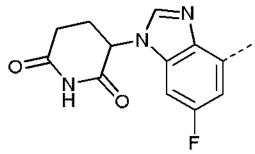 , 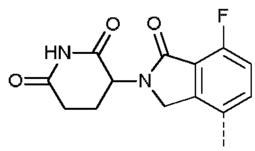 ,

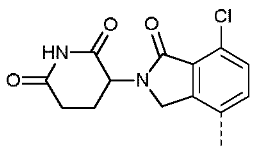 , 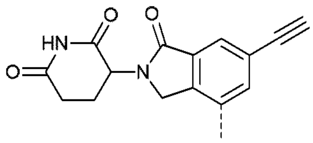 , 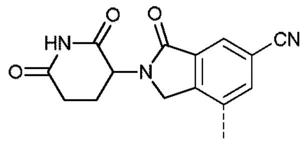 , 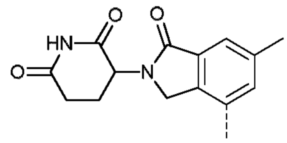 ,

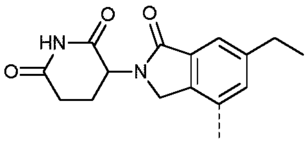 , 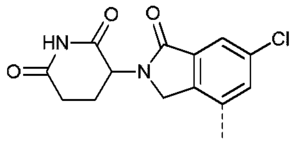 or 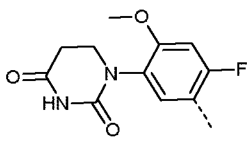 ;

and one or more H atom(s) in the above groups can be substituted with D atom(s).

**26.** The compound of general formula (X), or the pharmaceutically acceptable salt, enantiomer, diastereomer, racemate, solvate, hydrate, polymorph, prodrug, or isotopic variant thereof, or mixture thereof according to any one of claims 1-7, wherein $L_1$ and $L_2$ are independently selected from bond, -O-, -S-, -S(O)-, $-S(O)_2-$, - S(O)(=NH)-, -S(O)(=NMe)-,

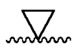 , 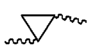 ,

-NH-, -N(Me)-,

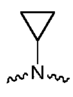 ,

$-N(CF_3)-$, $-CH_2-$, - CH(OMe)-, -CH(Cl)-, -CH(F)-, $-CF_2-$, $-CH(CF_3)-$, -C(O)-, $-CH_2CH_2-$, -CH=CH-, -C=C-, - $OCH_2-$, $-CH_2O-$, $-SCH_2-$, $-CH_2S-$, $-S(O)CH_2-$, $-CH_2S(O)-$, $-S(O)_2CH_2-$, $-CH_2S(O)_2-$, - $NHCH_2-$, $-N(Me)CH_2-$, $-CH_2NH-$, $-CH_2N(Me)-$, $-C(O)CH_2-$, $-CH_2C(O)-$, $-C(O)CMe_2-$, - $CMe_2C(O)-$, -OC(O)-, -C(O)O-, -SC(O)-, -C(O)S-, -NHC(O)-, -N(Me)C(O)-, -C(O)NH-, - C(O)N(Me)-, -S(O)=NH-, -NH=S(O)-, -N=S(O)Me-, -S(O)Me=N-,

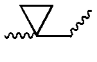 or 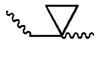 ;

and one or more H atom(s) in the above groups can be substituted with D atom(s).

**27.** The compound of general formula (I), or the pharmaceutically acceptable salt, enantiomer, diastereomer, racemate, solvate, hydrate, polymorph, prodrug, or isotopic variant thereof, or mixture thereof according to any one of claims 1-7, wherein E is selected from bond, $-CH_2CH_2CH_2-$, $-CH_2CH=CH-$, $-CH=CHCH_2-$, - $CH_2C\equiv C-$, $-C\equiv CCH_2-$, $-CH_2CH_2C(O)-$, $-CH_2C(O)CH_2-$, $-C(O)CH_2CH_2-$, $-CH_2CH_2S(O)_2-$, - $CH_2S()_2CH_2-$, $-S(O)_2CH_2CH_2-$, $-C(O)CH=CH-$, $-C(O)C\equiv C-$, $-CH_2CH_2O-$, $-CH_2OCH_2-$, - $OCH_2CH_2-$, $-CH_2CH_2S-$, $-CH_2SCH_2-$, $-SCH_2CH_2-$, $-C(O)CH_2O-$, $-OCH_2C(O)-$, $-CH_2C(O)O-$, $-C(O)CH_2S-$, $-SCH_2C(O)-$, $-CH_2C(O)S-$, $-OC(O)CH_2-$, $-C(O)OCH_2-$, $-CH_2OC(O)-$, - $SC(O)CH_2-$, $-C(O)SCH_2-$, $-CH_2SC(O)-$, $-CH_2CH_2NH-$, $-CH_2NHCH_2-$, $-NHCH_2CH_2-$, - $CH_2CH_2NMe-$, $-CH_2NMeCH_2-$, $-NMeCH_2CH_2-$, $-C(O)CH_2NH-$, $-NHCH_2C(O)-$, - $CH_2C(O)NH-$, $-NHC(O)CH_2-$, $-C(O)NHCH_2-$, $-CH_2NHC(O)-$,

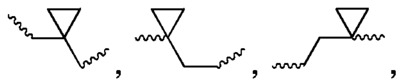,

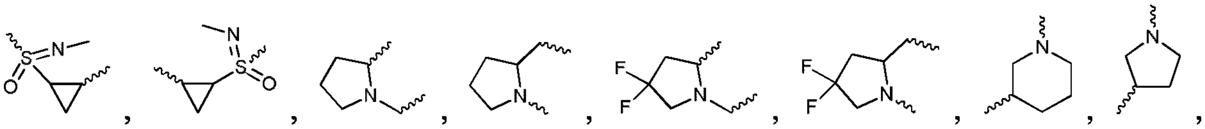,

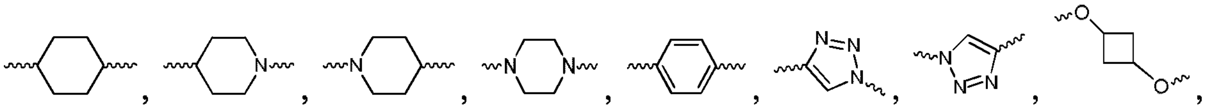,

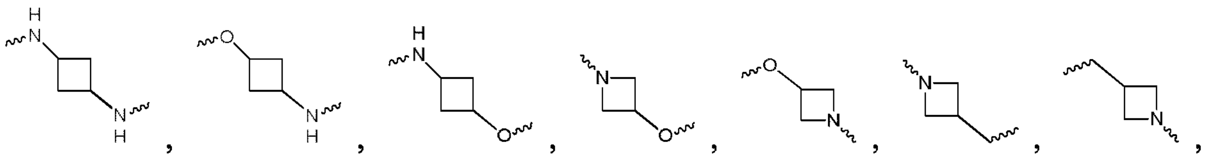,

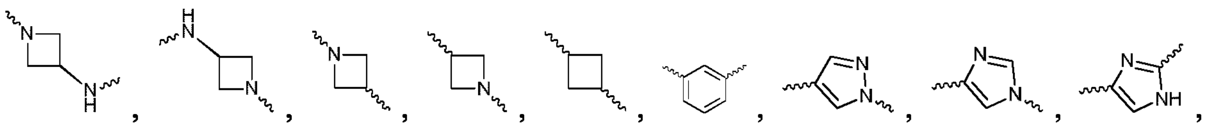,

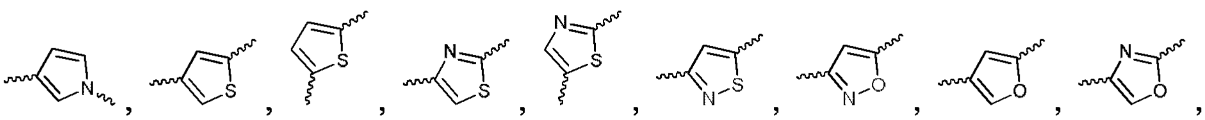,

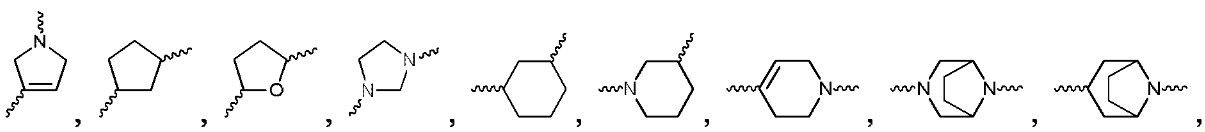,

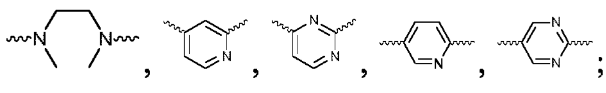;

or two E moieties or two E' moieties can be taken together to form $-CH_2CH_2OCH_2CH_2-$, $-OCH_2CH_2CH_2CH_2-$, $-CH_2CH_2CH_2CH_2O-$,

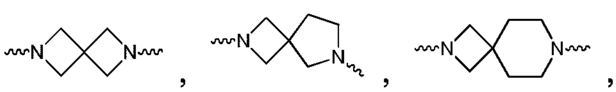,

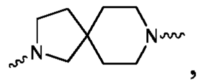, 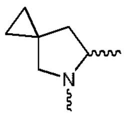 or 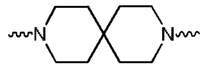;

and one or more H atom(s) in the above groups can be substituted with D atom(s).

**28.** The compound of general formula (I), or the pharmaceutically acceptable salt, enantiomer, diastereomer, racemate, solvate, hydrate, polymorph, prodrug, or isotopic variant thereof, or mixture thereof according to claim 1, the compound is selected from:

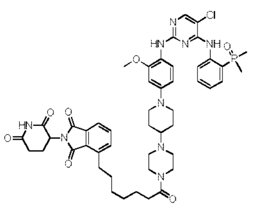, 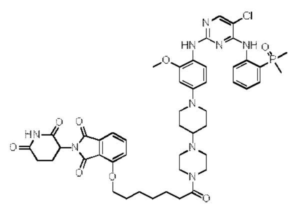, 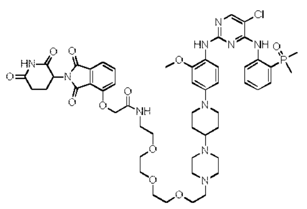, 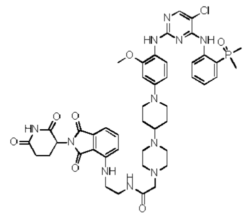,

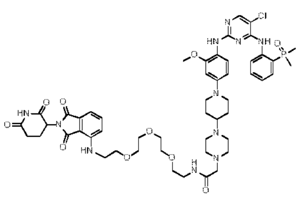, 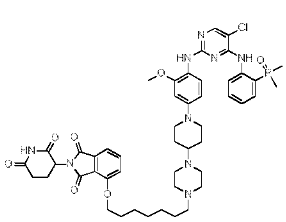, 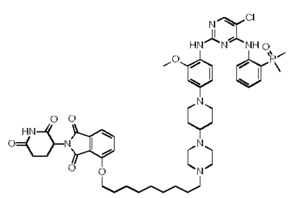, 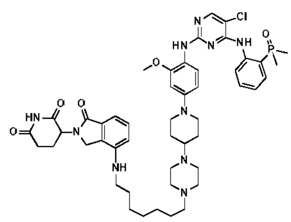,

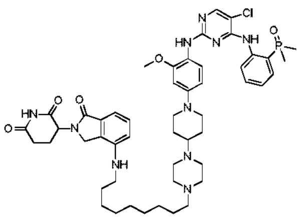, 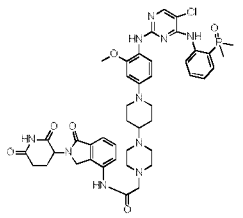, 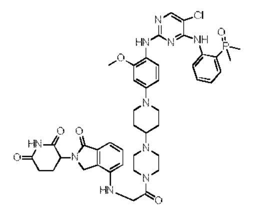, 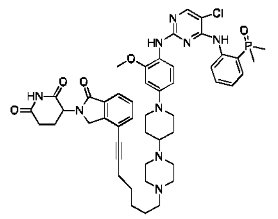,

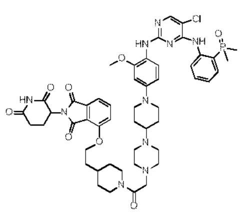, 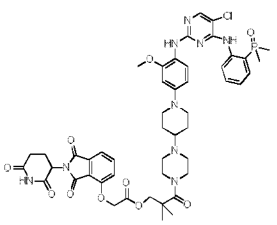, 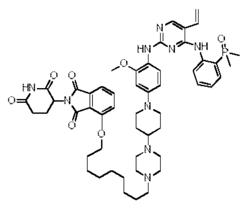, 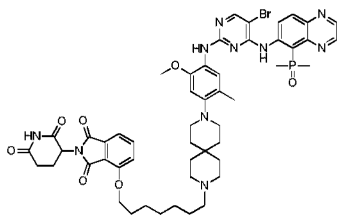,

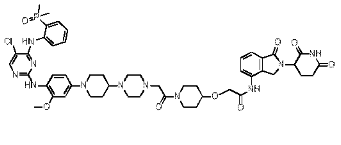, 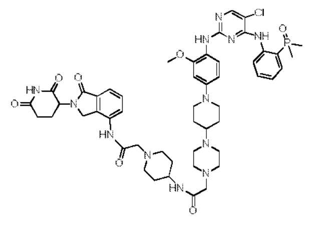, 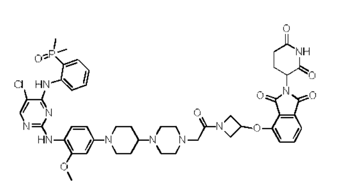,

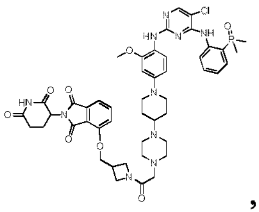, 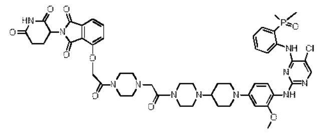, 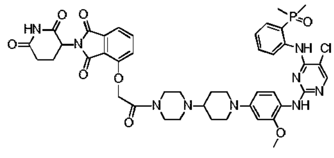, 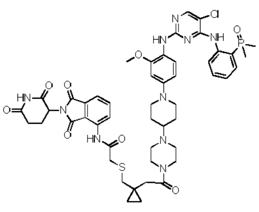,

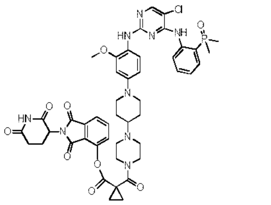, 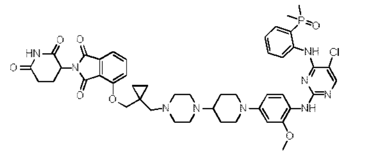, 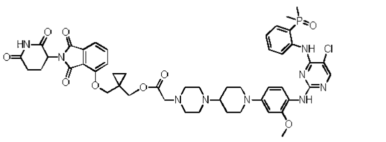,

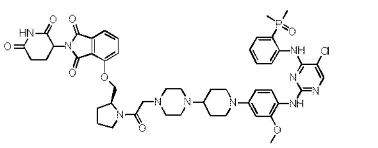, 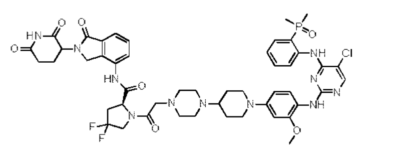, 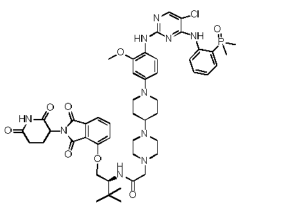,

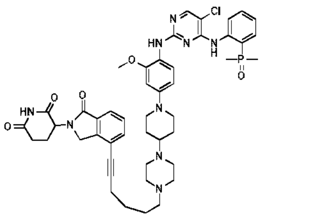, 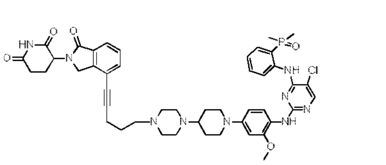, 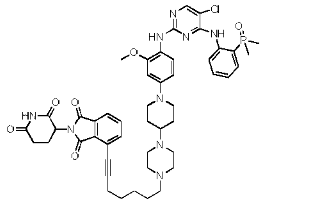,

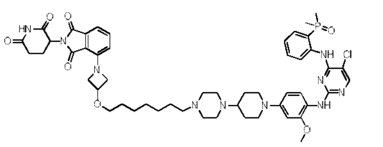, 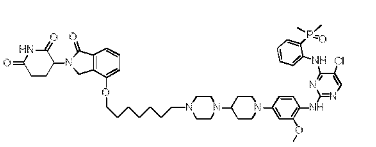, 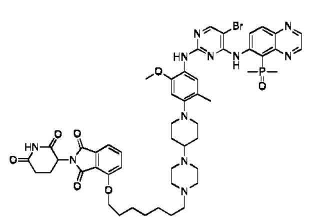,

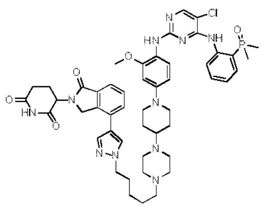, 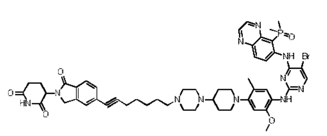, 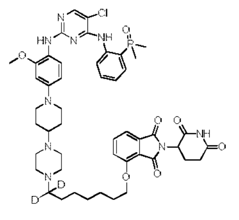, 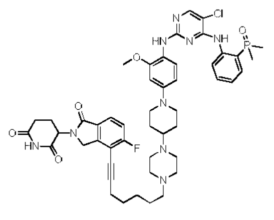,

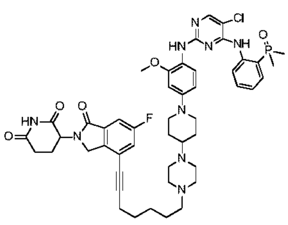, 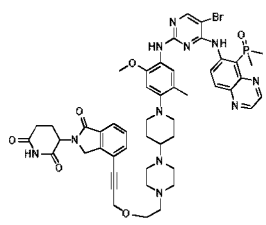, 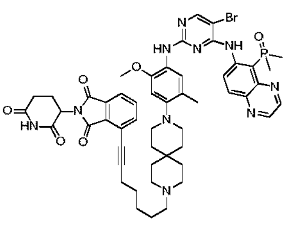, 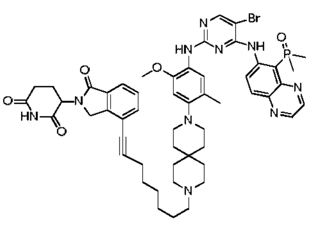,

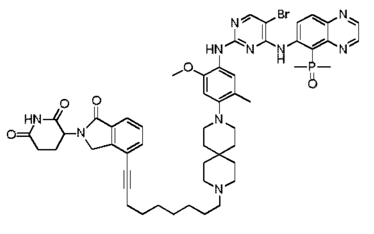,
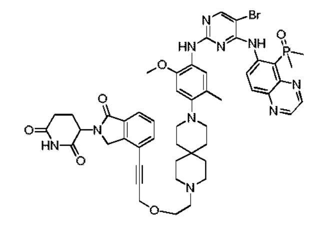,
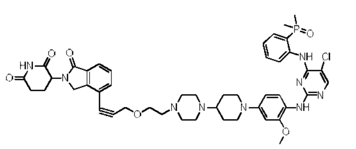,

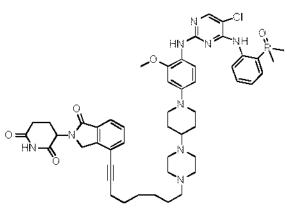,
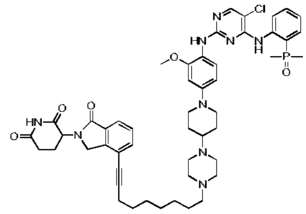,
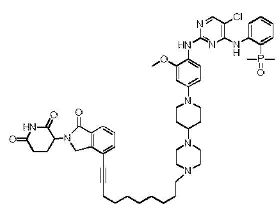,
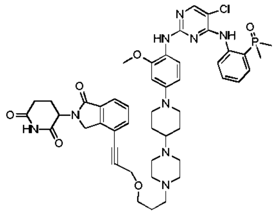,

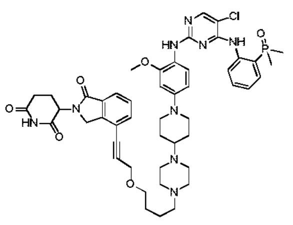,
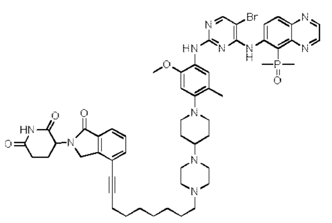,
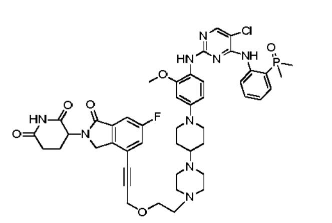,

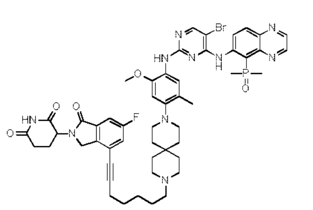,
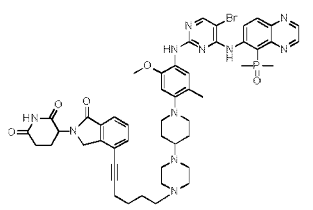,
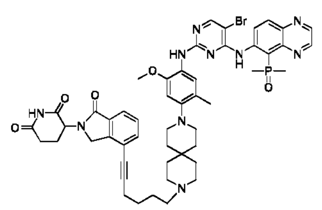,

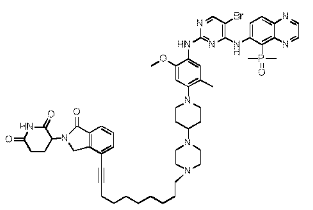,
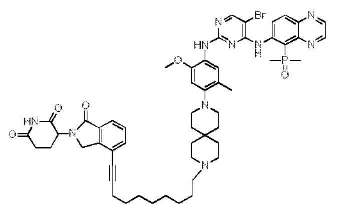,
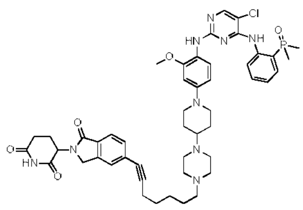,

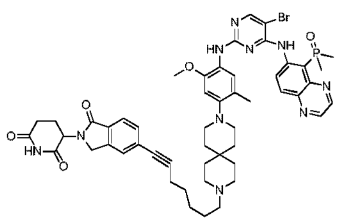,
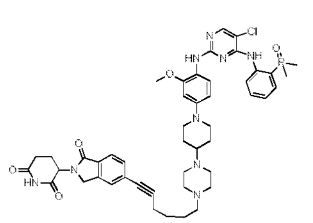,
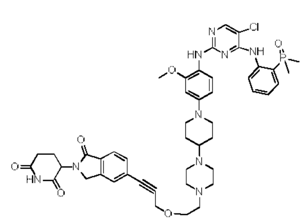,

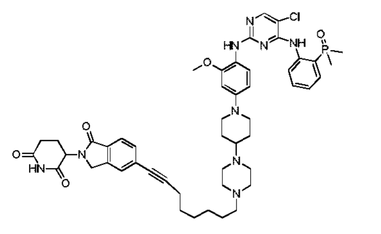 , 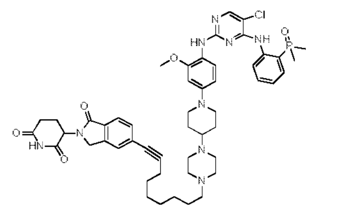 , 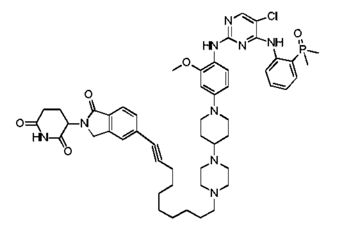 ,

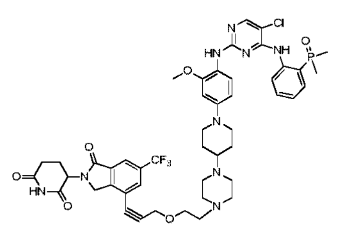 , 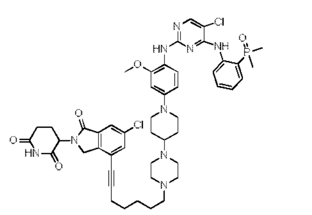 , 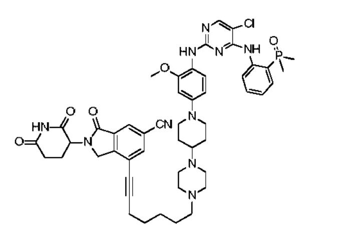 ,

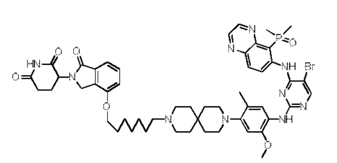 , 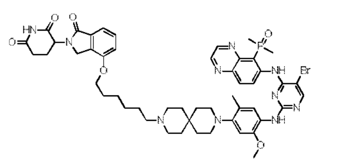 , 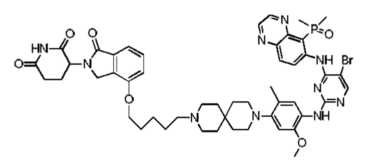 ,

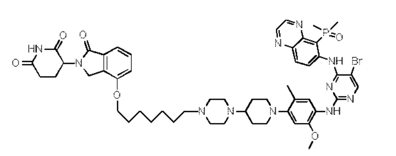 , 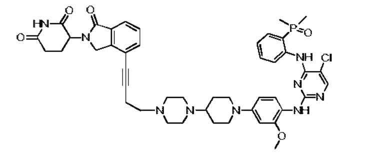 , 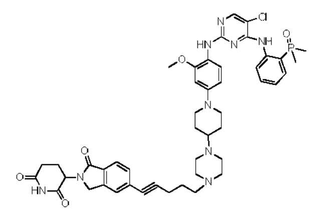 ,

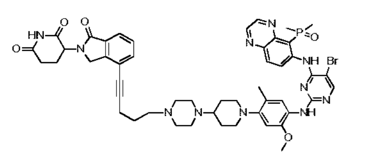 , 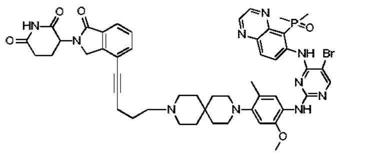 , 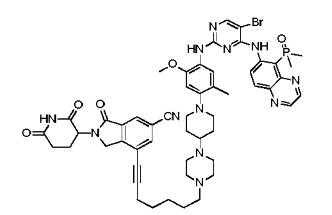 ,

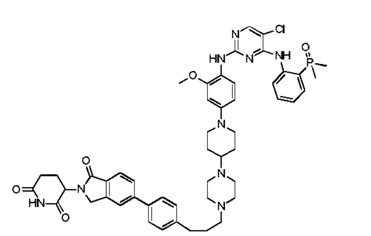 , 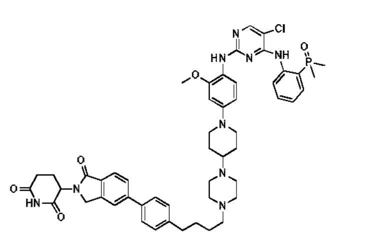 , 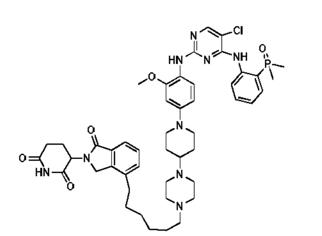 ,

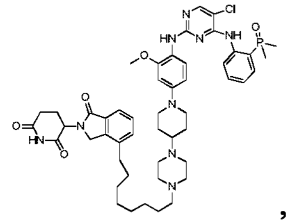 , 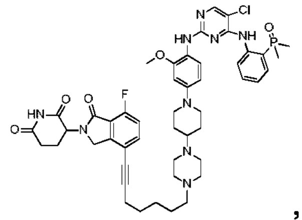 , 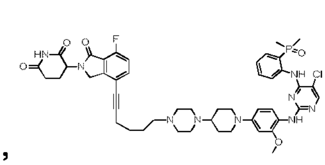 , 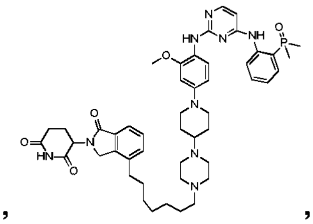 ,

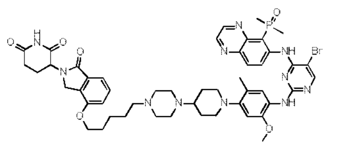, 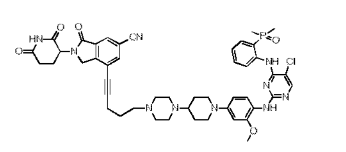, 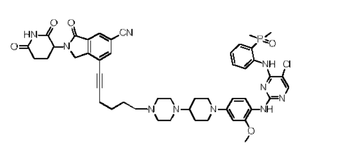,

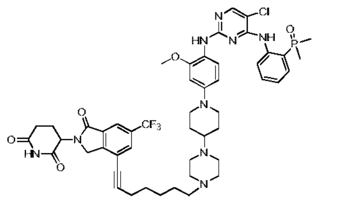, 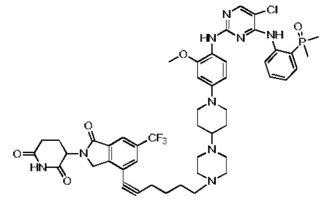, 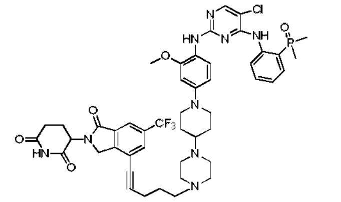,

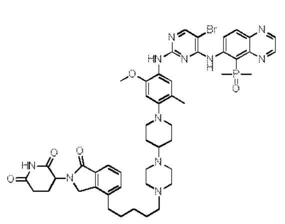, 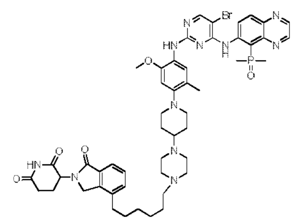, 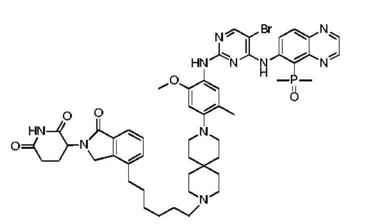,

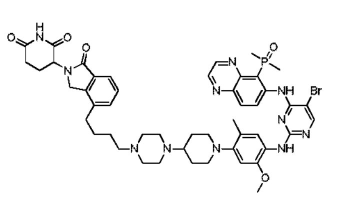, 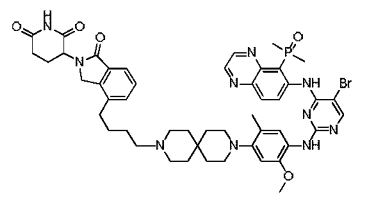, 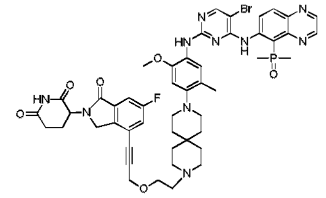,

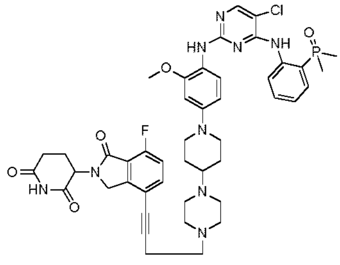, 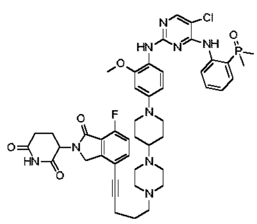, 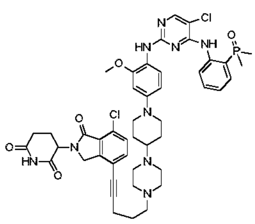, 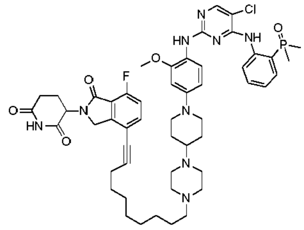,

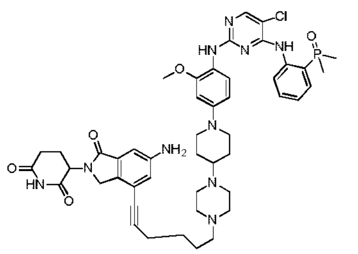, 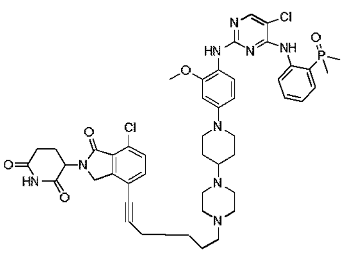, 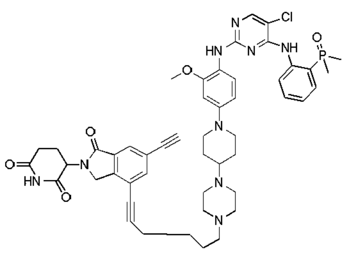,

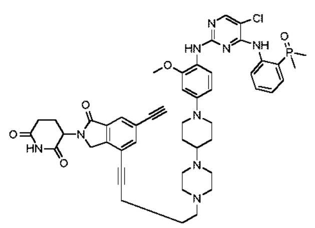,
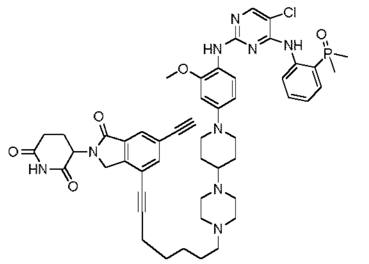,
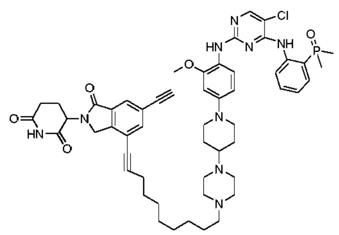,
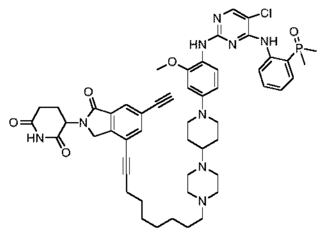,
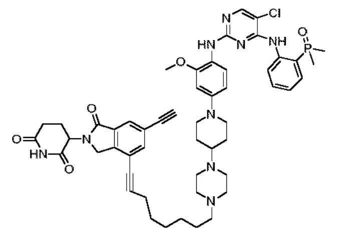,
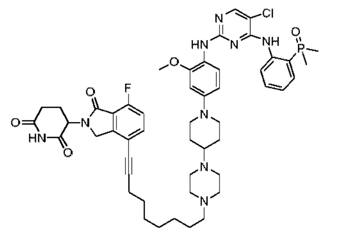,
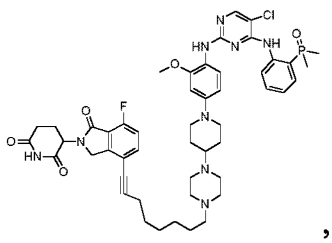,
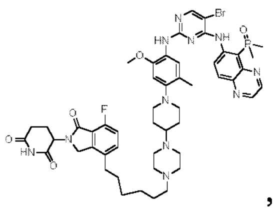,
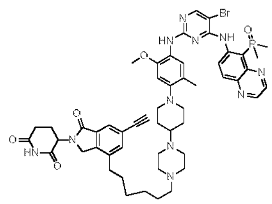,
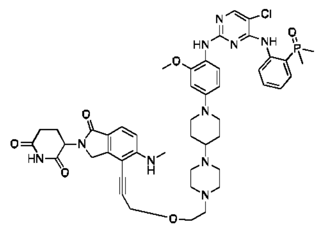,
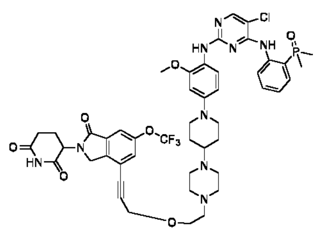,
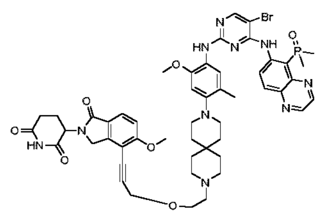,
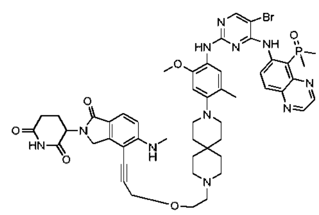,
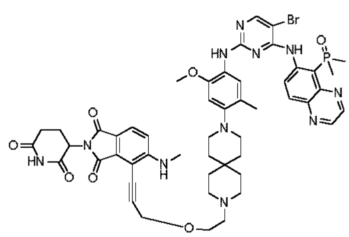,
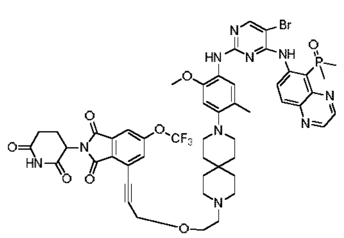 or
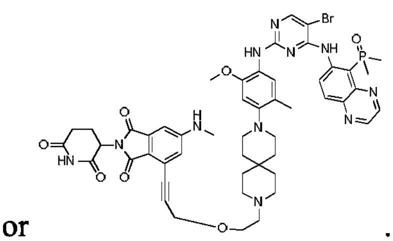.

**29.** A pharmaceutical composition, comprising:

the compound, or the pharmaceutically acceptable salt, enantiomer, diastereomer, racemate, solvate, hydrate, polymorph, prodrug or isotopic variant thereof according to any one of claims 1-28; and
pharmaceutically acceptable excipient (s);
alternatively, the pharmaceutical composition further comprises other therapeutic agent (s).

**30.** Use of the compound, or the pharmaceutically acceptable salt, enantiomer, diastereomer, racemate, solvate, hydrate, polymorph, prodrug or isotopic variant thereof according to any one of claims 1-28 in the manufacture of a medicament for treating and/or preventing diseases mediated by EGFR kinase and/or ALK kinase.

**31.** A method of treating and/or preventing diseases mediated by EGFR kinase and/or ALK kinase in a subject, which

comprises administering to the subject the compound, or the pharmaceutically acceptable salt, enantiomer, diastereomer, racemate, solvate, hydrate, polymorph, prodrug or isotopic variant thereof according to any one of claims 1-28, or the pharmaceutical composition according to claim 29.

**32.** The compound, or the pharmaceutically acceptable salt, enantiomer, diastereomer, racemate, solvate, hydrate, polymorph, prodrug or isotopic variant thereof according to any one of claims 1-28, or the pharmaceutical composition according to claim 29, for use in treating and/or preventing diseases mediated by EGFR kinase and/or ALK kinase.

**33.** The use according to claim 30, or the method according to claim 31, or the compound or composition for use according to claim 32, wherein the diseases mediated by EGFR kinase and/or ALK kinase include cancer, such as ovarian cancer, cervical cancer, colorectal cancer, breast cancer, pancreatic cancer, glioma, glioblastoma, melanoma, prostate cancer, leukemia, lymphoma, non-Hodgkin's lymphoma, gastric cancer, lung cancer, hepatocellular cancer, stomach cancer, gastrointestinal stromal tumor (GIST), thyroid cancer, cancer of bile duct, endometrial cancer, kidney cancer, anaplastic large cell lymphoma, acute myeloid leukemia (AML), multiple myeloma, melanoma, mesothelioma.

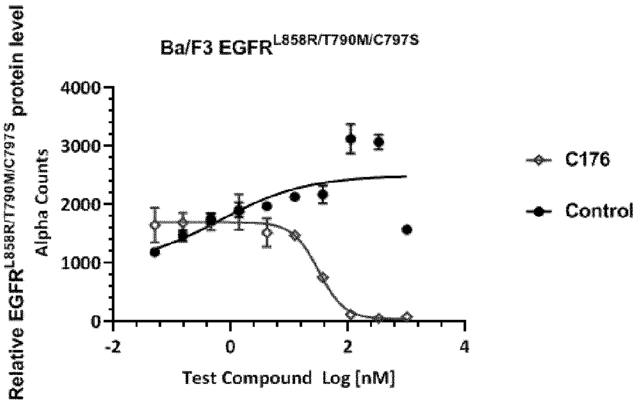
Ba/F3 EGFR$^{L858R/T790M/C797S}$
Relative EGFR$^{L858R/T790M/C797S}$ protein level
Alpha Counts
4000
3000
2000
1000
0
-2
0
2
4
Test Compound Log [nM]
C176
Control

FIG.1

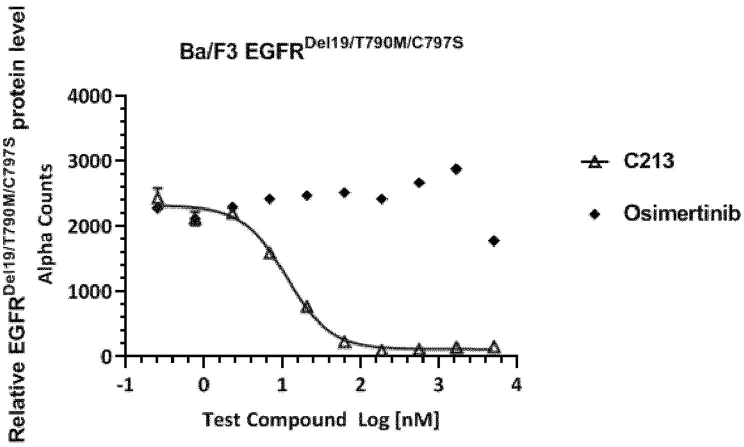
Ba/F3 EGFR$^{Del19/T790M/C797S}$
Relative EGFR$^{Del19/T790M/C797S}$ protein level
Alpha Counts
4000
3000
2000
1000
0
-1
0
1
2
3
4
Test Compound Log [nM]
C213
Osimertinib

FIG.2

## INTERNATIONAL SEARCH REPORT

International application No.
**PCT/CN2020/110442**

**A. CLASSIFICATION OF SUBJECT MATTER**

A61K 31/506(2006.01)i; C07D 401/12(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; C07D; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOXTX, EPTXT, USTXT, CNKI, 读秀, 中国药物专利数据库, Pubmed, ISI_Web of Science, Science Direct, STNext: structure search, 嘧啶, structure, pyrimidine

**C. DOCUMENTS CONSIDERED TO BE RELEVANT**

| Category* | Citation of document, with indication, where appropriate, of the relevant passages | Relevant to claim No. |
|---|---|---|
| PX | CN 110684015 A (SICHUAN UNIVERSITY) 14 January 2020 (2020-01-14) claims 1-12 | 1-33 |
| PX | WO 2019196812 A1 (SHANGHAI TECH UNIVERSITY) 17 October 2019 (2019-10-17) claims 1-91 | 1-33 |
| PX | CN 111285849 A (SHANGHAI QINGDONG BIOTECHNOLOGY CO., LTD.) 16 June 2020 (2020-06-16) claims 1-22 | 1-33 |
| X | WO 2019113071 A1 (ICAHN SCHOOL MED. MOUNT SINAI, et al.) 13 June 2019 (2019-06-13) table 1, description page 39 lines 8-27, page 40 lines 10-34, pages 425-10, page 47 lines 1-24 | 1-33 |
| X | CN 109422733 A (SHANGHAI MEIZHI PHARMACEUTICAL TECHNOLOGY CO., LTD.) 05 March 2019 (2019-03-05) claims 1-10, description paragraphs 0006-0072 | 1-33 |
| X | CN 109912655 A (SHANGHAI TECH UNIVERSITY) 21 June 2019 (2019-06-21) claims 1-44 | 1-33 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 November 2020** | **25 November 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/110442**

**C. DOCUMENTS CONSIDERED TO BE RELEVANT**

| Category* | Citation of document, with indication, where appropriate, of the relevant passages | Relevant to claim No. |
|---|---|---|
| X | CN 109928956 A (HANGZHOU OULIAN PHARMACEUTICAL TECHNOLOGY CO., LTD.) 25 June 2019 (2019-06-25)<br>claims 1-10 | 1-33 |
| X | WO 2018119441 A1 (ARVINAS INC. et al.) 28 June 2018 (2018-06-28)<br>figure 2, claims 1-47 | 1-33 |
| X | WO 2018033556 A1 (GLAXOSMITHKLINE IP. DEV. LTD.) 22 February 2018 (2018-02-22)<br>claims 1-21 | 1-33 |
| X | WO 2016105518 A1 (DANA-FARBER CANCER INST., INC.) 30 June 2016 (2016-06-30)<br>claims 1-24 | 1-33 |
| X | POWELL, Chelsea E. et al.,. "Chemically Induced Degradation of Anaplastic Lymphoma Kinase(ALK),"<br>*J. Med. Chem.*, Vol. 61, 16 April 2018 (2018-04-16),<br>pp. 4249-4255 | 1-33 |

Form PCT/ISA/210 (second sheet) (January 2015)

**INTERNATIONAL SEARCH REPORT**

International application No.
**PCT/CN2020/110442**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **31**，**33**（部分）
because they relate to subject matter not required to be searched by this Authority, namely:

[1] Claim 31 sets forth treating and/or preventing EGFR and/or ALK kinase-mediated diseases in a subject, and claim 33 (in part) sets forth a use of the method of claim 31 or the compound or composition of claim 31, thus including a method for the treatment of the human or animal body, and not complying with PCT Rule 39.1(iv). The search for claims 31 and 33 (in part) is provided on the basis of the following amendment: a use of the compound or pharmaceutically acceptable salt in any one of claims 1-28, or the pharmaceutical composition of claim 29 in preparing a drug to treat and/or prevent EGFR and/or ALK kinase-mediated diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2020/110442**

| Patent document cited in search report | Publication date (day/month/year) | Patent family member(s) | Publication date (day/month/year) |
|---|---|---|---|
| CN 110684015 A | 14 January 2020 | None | |
| WO 2019196812 A1 | 17 October 2019 | CN 110357889 A | 22 October 2019 |
| CN 111285849 A | 16 June 2020 | None | |
| WO 2019113071 A1 | 13 June 2019 | None | |
| CN 109422733 A | 05 March 2019 | WO 2019042444 A1 | 07 March 2019 |
| | | US 2020199106 A1 | 25 June 2020 |
| | | CN 109963844 A | 02 July 2019 |
| CN 109912655 A | 21 June 2019 | CA 3085645 A1 | 20 June 2019 |
| | | AU 2018382122 A1 | 16 July 2020 |
| | | WO 2019114770 A1 | 20 June 2019 |
| CN 109928956 A | 25 June 2019 | None | |
| WO 2018119441 A1 | 28 June 2018 | WO 2018119441 A8 | 23 May 2019 |
| | | WO 2018119441 A9 | 10 October 2019 |
| | | KR 20190101406 A | 30 August 2019 |
| | | JP 2020505327 A | 20 February 2020 |
| | | AU 2017382406 A1 | 18 April 2019 |
| | | CA 3047586 A1 | 28 June 2018 |
| | | EP 3559002 A1 | 30 October 2019 |
| | | MX 2019007646 A | 06 September 2019 |
| | | CO 2019007892 A2 | 09 October 2019 |
| | | BR 112019012682 A2 | 17 December 2019 |
| | | IL 267398 D0 | 29 August 2019 |
| | | CN 110753693 A | 04 February 2020 |
| | | US 2018193470 A1 | 12 July 2018 |
| WO 2018033556 A1 | 22 February 2018 | CN 109563076 A | 02 April 2019 |
| | | JP 2019524832 A | 05 September 2019 |
| | | KR 20190039567 A | 12 April 2019 |
| | | AU 2017313307 A1 | 21 February 2019 |
| | | BR 112019002096 A2 | 14 May 2019 |
| | | CA 3033001 A1 | 22 February 2018 |
| | | US 2019210996 A1 | 11 July 2019 |
| | | EP 3500563 A1 | 26 June 2019 |
| | | GB 201614134 D0 | 05 October 2016 |
| WO 2016105518 A1 | 30 June 2016 | US 2018009779 A1 | 11 January 2018 |
| | | JP 2018502097 A | 25 January 2018 |
| | | US 10125114 B2 | 13 November 2018 |
| | | US 10669253 B2 | 02 June 2020 |
| | | EP 3256470 A1 | 20 December 2017 |
| | | CN 107257800 B | 30 June 2020 |
| | | CN 107257800 A | 17 October 2017 |
| | | US 2019071415 A1 | 07 March 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5376645 A


### Non-patent literature cited in the description


- *Nature Medicine,* 2015, vol. 21 (6), 560-562
- *JAMA Oncol.,* 2018, vol. 4 (11), 1527-1534
- **BERGE S. M. et al.** Pharmaceutical Salts. *J. Pharm. Sci.,* 1977, vol. 66, 1-19
- **T. HIGUCHI ; V. STELLA.** Prodrugs as Novel Delivery Systems. *A.C.S. Symposium Series,* vol. 14
- Bioreversible Carriers in Drug Design. American Pharmaceutical Association and Pergamon Press, 1987
- **D. FLEISHER ; S. RAMON ; H. BARBRA.** Improved oral drug delivery: solubility limitations overcome by the use of prodrugs. *Advanced Drug Delivery Reviews,* 1996, vol. 19 (2), 115-130
- Remington 's Pharmaceutical Sciences. Mack Publishing Company, 1985